# EUROPEAN PATENT APPLICATION

(11) **EP 4 659 812 A1**
(43) Date of publication of application: **10.12.2025**
(21) Application number: 24218177.4
(22) Date of filing: 06.12.2024
(51) Int. Cl.: A61P 35/00, A61K 31/366, A61K 39/395, C07K 16/32

(54) **DETECTION METHODS AND COMBINATION THERAPIES OF CELLS WITH EXTRACELLULAR RAS EXPRESSION**

(30) Priority: 07.06.2024 WO PCT/EP2024/065774
(71) Applicant: Bumm, Thomas, 91443 Scheinfeld (DE); Bumm UG, 40789 Monheim (DE)
(72) Inventor: Bumm, Thomas, Scheinfeld, 91443 (DE); Bumm, Martin Malte Johannes, Rommerskirchen 41569 (DE)
(74) Representative: Hoffmann Eitle

(57) **Abstract**

The present invention relates to a combination of an antigen binding protein (ABP) or an isolated nucleic acid comprising a sequence encoding the ABP and a modulator compound for use as medicament. The ABP comprises at least a first antigen binding domain capable of binding to an extracellular Ras antigen. The invention also relates to a pharmaceutical composition comprising the combination and a composition or kit comprising an antigen binding protein (ABP) or an isolated nucleic acid comprising a sequence encoding the ABP and a modulator compound. The invention also relates to an *in vitro* 3D cell culture model and an *in vitro* method for detecting the expression of predominantly extracellular Ras proteins on the surface of predominantly living cells.

## Description

### FIELD OF THE INVENTION

The present invention pertains to targeting extracellular Ras antigen by a combination of an antigen-binding protein and a modulator compound for use as medicament, in particular for use in a method of diagnosis, prevention and/or treatment of a proliferative disorder, such as cancer, and/or for use in adoptive, target-cell specific immunotherapy and/or for use in drug development and/or for use in a method of diagnosis, prevention and/or treatment of a non-malignant disease. The invention also relates to a pharmaceutical composition, a composition and a kit. Further, an *in vitro* 3D cell culture model is provided and an *in vitro* method for detecting the expression of predominantly extracellular Ras proteins on the surface of predominantly living cells.

### BACKGROUND OF THE INVENTION

The superfamily of Ras ("Rat sarcoma virus") proteins belong to a class of cytosolic proteins called small GTPase. The Ras superfamily comprise over 150 members and is divided into five major families: Ras, Rho, Arf/Sar, Ran, and Rab. Ras proteins are binary molecular switches that cycle between active guanosine triphosphate (GTP)-bound and inactive guanosine diphosphate (GDP)-bound states, transmitting signals within cells (cellular signal transduction), and are important for regulating cell growth, cell proliferation, and cell differentiation.

The RAS family is comprised of 36 genes, with more than 40 different proteins reported. There are three RAS genes in the human genome, often mutated in human cancer, namely Kirsten rat sarcoma viral oncogene homolog (KRAS), neuroblastoma RAS viral (v-RAS) oncogene homolog (NRAS), and Harvey rat sarcoma viral oncogene homolog (HRAS). For KRAS, two isoforms arise from alternative RNA splicing, namely KRAS4A and KRAS4B. Thus, due to alternative splicing of the mRNA and post-translational modifications of the proteins, there are different post-translational modified Ras Proteins expressed in the cytosol of cells.

The amino-terminal catalytic domains of H-Ras, N-Ras and K-Ras are highly conserved (90-100% identical), but the carboxy terminal sequences diverge significantly, carrying the hypervariable domain (HVR). The HVR comprises the anchor sequences, important for attachment to the cytoplasmic part of the membrane and to sort, or target, to different membrane microdomains. The interaction of Ras with the plasma membrane is highly dynamic and Ras is present on endosomes and other intracellular membranes, such as the endoplasmic reticulum (ER) and Golgi [1].

There are many different proteins in the cell with high binding affinity and/or specificity for Ras, important for regulating the Ras signaling cascade. The Ras GTPases are continuously cycling between inactive (RAS•GDP) and active (RAS•GTP) conformations in a process modulated by negative (GTPase Activating Proteins, GAPs) and positive (Guanine nucleotide Exchange Factors, GEFs) regulators.

Owing to the Ras family proteins' essential role in modulating a wide range of cellular processes, several human diseases are caused by the dysregulation or dysfunction of Ras related signaling pathways. These include cancer, developmental-, neurocognitive- and neurodegenerative disorders, as well as metabolic and cardiovascular diseases [2].

Mutations in Ras genes can lead to the production of permanently activated Ras proteins, which can cause overactive signaling inside the cell, even in the absence of incoming signals. Accumulation of constantly active Ras proteins permanently stimulating cell growth plays an important part in cancer cell proliferation and survival. Thus, Ras genes are protooncogenic, i.e. mutations in Ras genes can render them oncogenic, and this is causally linked to the development of certain types of cancer. Importantly, the three Ras genes in humans (HRAS, KRAS, and NRAS) are the most common oncogenes in human cancer, and oncogenic mutations that permanently activate Ras (gain-of-function) are found in 20 to 27% of all human tumors (range 0.8%-90%) (e.g., pancreatic cancer, but also lung or colorectal carcinoma are often characterized by a high percentage of oncogenic Ras mutations).

Prior art reports small Ras peptides to be expressed on the cell surface via the human major histocompatibility complex (MHC) known as the human leukocyte antigen (HLA). Therefore, all current cancer immunotherapies against extracellular Ras use T-cell receptors (TCR) or antibody single-chain variable fragments (scFv) targeting Ras mutant peptides expressed via HLA class I or II [3-7].

It was recently found (PCT/EP2024/065774) that extracellular Ras protein is also accessible on cancer cells outside the MHC system. This Ras expression on the cell surface can be used for immunotherapy with different formats of antibodies, chimeric antigen receptor T cells (CART) or small chemical binders. In contrast to prior art, Antigen Binding Proteins (ABPs) against extracellular Ras independent of the MHC system are not restricted to a specific HLA genotype. Twelve different bi-specific T cell recruiting Diabodies had been designed targeting extracellular Ras and targeting the human CD3 receptor complex on T cells being effective in killing tumor cells. The binding sites for these Ras ABPs are in the normally intracellular Ras domains, adapted to the different harsh and densely packed conditions of the cytosol.

Prior art also reports on combination therapies in tumor therapy, for example the combination of antiangiogenic agents like Bevacizumab^{®} with immune checkpoint inhibitors, for increasing treatment efficiency. However, 3D tumor models, where the tumor microenvironment (TME) is supporting the tumor cells and possibly impede the immune cells, had not been used. In addition, the receptors targeted in prior art immunotherapies are native receptors having transmembrane domains reaching into the cytosol. It is a particular challenge to target extracellular Ras because it only reaches a short distance into the lipid layer and may be redistributed to the cytosol or intracellular membrane.

Hence, it was an object of the present invention to provide a proper model for testing combination therapies comprising an ABP targeting extracellular Ras antigen not in the context of HLA/MHC and to identify suitable combinations for use as medicament, in particular for use in a method of diagnosis, prevention and/or treatment of a proliferative disorder, such as cancer. In addition, it was aimed at providing an ABP targeting extracellular Ras antigen for use in a method of diagnosis, prevention and/or treatment of a non-malignant disease.

There is also a need for new methods to predominantly detect extracellular Ras expression on viable cells. Therefore, it is an object of the present invention to provide such methods involving an ABP targeting extracellular Ras antigen. In addition, was aimed to provide such methods for use in drug development. Those methods and compositions preferably have a good signal to noise ratio to avoid false positive and false negative results.

### SUMMARY OF THE INVENTION

It has presently found by using a 3D tumor cell model that combinations of an ABP of the present invention with at least one other compound surprisingly have a very good anti-tumor effect which is better than an ABP alone. In addition, it has been shown that the ABP of the present invention bind to extracellular Ras antigen not in the context of or not restricted to, a human leukocyte antigen (HLA)-peptide complex, or specific HLA alleles, presented on the cell surface by the major histocompatibility complex (MHC). Further, a stimulating effect on tumor cells using ABPs alone has been demonstrated.

Further, different methods have been identified for detecting predominantly extracellular Ras protein on the surface of predominantly living cells.

In a first aspect of the invention, a combination of an antigen binding protein (ABP) or an isolated nucleic acid comprising a sequence encoding the ABP and a modulator compound for use as medicament is provided, wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

In a preferred embodiment, said extracellular Ras antigen is not part of, or is not restricted to, a human leukocyte antigen (HLA)-peptide complex, or specific HLA alleles, presented on the cell surface by the major histocompatibility complex (MHC).

In another preferred embodiment, said ABP comprises one or more additional antigen binding domain(s) that is capable of binding to antigen(s) present on a mammalian T-cell, preferably a human cluster of differentiation 3 (CD3) antigen or a human T cell receptor (TCR).

In a further preferred embodiment, the first antigen binding domain comprises an amino acid sequence having a sequence identity of at least 70%, more preferably at least 80%, even more preferably at least 90%, and most preferably at least 95% sequence identity, to an amino acid sequence selected from SEQ ID NO: 20 to 31, 66 to 71, 134 to 178 and 290 to 396, or, in each case independently, optionally with no more than ten, nine, eight, seven, six, five, four, preferably no more than three, two or one, amino acid substitution(s), insertion(s) or deletion(s) compared to these sequences.

The isolated nucleic acid may be comprised in an expression construct, preferably further comprising promoter and/or terminator sequences. The isolated nucleic acid and/or the expression construct may be comprised in a recombinant host cell.

In a preferred embodiment, the ABP is PCC04D diabody (SEQ ID NOs 23 and 231).

In another preferred embodiment, the ABP enhances a cell-mediated immune response, such as the immune response mediated by an activated cytotoxic T-cell (CTL) to a mammalian cell expressing said extracellular Ras antigen.

Further, the ABP may be selected from the group consisting of an immunoglobulin molecule, such as an IgG, IgE, IgD, IgA, or IgM immunoglobulin, preferably an IgG immunoglobulin, a monoclonal antibody, a chimeric antibody, a bispecifc ABP, such as a bispecific antibody, a CDR-grafted antibody, a humanized antibody, a single domain antibody, such as a VHH single domain antibody, a hemibody antibody, a single-chain KeyLock-antibody, a diabody, a single chain diabody, a variable domain of the antibody heavy chain or antibody light chain, a multispecific antibody, a Chimeric Antigen Receptor (CAR), alternative protein binders including monobodies (derived from fibronectin type III), anticalins (derived from lipocalins), affibodies (derived from immunoglobulin-binding protein A), DARPins (Designed Ankyrin Repeat Proteins), proteins with repeating motifs like leucine-rich repeats (LRRs), ankyrin repeats (ARs), Armadillo repeats (Arms), tetratricopeptide repeats (TPRs), and/or a fragment of an antibody, such as a fragment of a monoclonal antibody, for example a single chain Fv (scFv), (scFv)2, a Fv, a disulfide linked Fv, Fab, Fab', F(ab')2 or a scFv-Fc, preferably wherein said ABP is a bispecific antibody or a diabody.

In another embodiment, the ABP comprises at least one antigen binding domain capable of binding with one, two, three, four, or preferable more than five amino acids to a Ras antigen, wherein the at least one antigen binding domain is alone or in a bipartite complex with another Ras binding protein, or in a tripartite complex with another Ras binding protein and the membrane, or in a multipartite complex with one or more Ras binding proteins and/or the membrane, and wherein said amino acids in the ABP optionally comprise:
(i) a domain comprising the RBD-CRD region (amino acids 52 to 188) of the human RAF1 protein as set forth in SEQ ID NO: 23,
(ii) optionally wherein one or multiple amino acids as depicted in Tables A and B are in contact with KRAS residues, or wherein one or multiple amino acids as depicted in Table C are in contact with the membrane, or wherein one or multiple amino acids as depicted in Table D are in contact with KRAS residues in a tripartite complex comprised of RBD-CRD, KRAS and the membrane, or
(iii) a domain comprising the CDC25H region (amino acids 780 to 1019) of the human SOS1 protein as set forth in SEQ ID NO: 24, optionally wherein one or multiple amino acids as depicted in Table E are in contact with KRAS residues, or
(iv) a domain comprising the CDC25 region (amino acids 1038 to 1270) of the human RASGRF1 protein as set forth in SEQ ID NO: 29, optionally wherein one or multiple amino acids as depicted in Table F are in contact with Ras residues in a tripartite complex with Ras, Sos1 and RasGRF1, or
(v) a domain comprising the RAS binding region (amino acids 274 to 364) of the human RASSF5 protein (UniProt Q8WWW0-1) as set forth in SEQ ID NO: 377, optionally wherein one or multiple amino acids are in contact with RAS residues, or
(vi) a domain comprising the RAS binding region (amino acids 201 to 363) of a splice variant of the human RASSF5 protein (UniProt Q8WWW0-2) as set forth in SEQ ID NO: 378, optionally wherein one or multiple amino acids are in contact with RAS residues, or
(vii)a domain comprising the RAS binding region (amino acids 201 to 363) of the human RASSF1 protein (UniProt Q9NS23-1) as set forth in SEQ ID NO: 379, optionally wherein one or multiple amino acids are in contact with RAS residues, or
(viii) a domain comprising the RAS binding region (amino acids 176 to 264) of the human RASSF2 protein (UniProt P50749-1) as set forth in SEQ ID NO: 380, optionally wherein one or multiple amino acids are in contact with RAS residues, or
(ix) a domain comprising the RAS binding region (amino acids 6 to 89) of the human RASSF7 protein (UniProt Q02833-1) as set forth in SEQ ID NO: 381, optionally wherein one or multiple amino acids are in contact with RAS residues, or
(x) a domain comprising the RAS binding region (amino acids 19 to 91) of the human ARAF protein (UniProt P10398-1) as set forth in SEQ ID NO: 382, optionally wherein one or multiple amino acids are in contact with RAS residues, or
(xi) a domain comprising the RAS binding region (amino acids 19 to 148) of the human ARAF protein (UniProt P10398-1) as set forth in SEQ ID NO: 383, optionally wherein one or multiple amino acids are in contact with RAS residues, or
(xii)a domain comprising the RAS binding region (amino acids 151 to 232) of the human BRAF protein (UniProt P15056) as set forth in SEQ ID NO: 384, optionally wherein one or multiple amino acids are in contact with RAS residues, or
(xiii) a domain comprising the RAS binding region (amino acids 151 to 320) of the human BRAF protein (UniProt P15056) as set forth in SEQ ID NO: 385, optionally wherein one or multiple amino acids are in contact with RAS residues, or
(xiv) a domain comprising the RAS binding region (amino acids 56 to 131) of the human RAF1 protein (UniProt P04049-1) as set forth in SEQ ID NO: 386, optionally wherein one or multiple amino acids are in contact with RAS residues, or
(xv) a domain comprising the RAS binding region (amino acids 1235 to 1451) of the human NF1 protein (UniProt P21359-1) as set forth in SEQ ID NO: 387, optionally wherein one or multiple amino acids are in contact with RAS residues, or
(xvi) a domain comprising the RAS binding region (amino acids 748 to 942) of the human RASA1 protein (UniProt P20936-1) as set forth in SEQ ID NO: 388, optionally wherein one or multiple amino acids are in contact with RAS residues, or
(xvii) a domain comprising the RAS binding region (amino acids 302 to 512) of the human RASA4 protein (UniProt O43374-1) as set forth in SEQ ID NO: 389, optionally wherein one or multiple amino acids are in contact with RAS residues, or
(xviii) a domain comprising the RAS binding region (amino acids 938 to 1130) of the human RGS12 protein (UniProt O14924-1) as set forth in SEQ ID NO: 390, optionally wherein one or multiple amino acids are in contact with RAS residues.

In another preferred embodiment, the ABP comprises at least one antigen binding domain capable of binding to an extracellular Ras antigen, preferably provided that said extracellular Ras antigen is not part of, or is not restricted to, a HLA-peptide complex, or specific HLA alleles presented on the cell surface, and wherein said Ras binding ABP comprises a diabody format as set forth in SEQ ID NO: 228 to 239, wherein in each case independently comprise a sequence having no more than three or two, preferably no more than one amino acid substitution(s), deletion(s) or insertion(s) compared to SEQ ID NO: 224, and 228 to 239, respectively; or comprising a sequence having at least 75% sequence identity, preferably at least 80% sequence identity, more preferably at least 90% sequence identity, and most preferably at last 95% sequence identity with SEQ ID NO: 224, and 228 to 239.

In a preferred embodiment, the combination is for use in a method of diagnosis, prevention and/or treatment of a proliferative disorder, such as cancer, and/or for use in adoptive, target-cell specific immunotherapy and/or for use in drug development and/or for use in a method of diagnosis, prevention and/or treatment of a non-malignant disease.

In a second aspect of the invention, a pharmaceutical composition for use as medicament is provided comprising the combination as disclosed *supra* and a pharmaceutically acceptable carrier, stabilizer and/or excipient.

In a third aspect of the invention, a composition comprising an antigen binding protein (ABP) or an isolated nucleic acid comprising a sequence encoding the ABP and a modulator compound is provided, wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

In a preferred embodiment, the composition is a pharmaceutical composition, preferably additionally comprising a pharmaceutically acceptable carrier, stabilizer and/or excipient.

In a fourth aspect of the invention. a kit comprising an antigen binding protein (ABP) or an isolated nucleic acid comprising a sequence encoding the ABP and a modulator compound is provided, wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

In a fifth aspect of the invention, an *in vitro* 3D cell culture model is provided comprising tumor cells and at least one non-malignant cell selected from the group comprising or consisting of a fibroblast, an endothelial cell of an arterial blood vessel, an endothelial cell of a venous blood vessel, an endothelial cell of a lymphatic vessel, a tissue macrophage, a fat cell, an osteoblast, a chondrocyte, a smooth muscle cell, a preadipocyte, a pericyte, a mesenchymal stem cell, a melanocyte, a keratinocyte, hematopoietic progenitors, a dendritic cell, a skeletal muscle cell, a T cell and a B cell, preferably wherein the at least non-malignant cell is a T cell, a fibroblast and an endothelial cell.

In a preferred embodiment, the *in vitro* 3D cell culture model further comprises the combination or the ABP as disclosed *supra* for testing the influence of the combination on the viability and extracellular Ras expression of the at least one tumor cell.

In a sixth aspect of the invention, an *in vitro* method for detecting the expression of predominantly extracellular Ras proteins on the surface of predominantly living cells is provided selected from the group consisting of
a) a method using a Ras protein labeling compound with substantially no penetration inside the cell and/or predominantly binding to the extracellular Ras protein
b) a method using an agent blocking binding to intracellular Ras proteins and/or using a washing step to substantially remove binding to intracellular Ras proteins
c) a method using a Ras protein labeling compound as of a) and one or more labeling compounds binding to intracellular proteins not belonging to the Ras protein family
d) a method using a dual-antigen protein-protein interaction (PPI) reporter
e) a method using cells expressing an altered Ras protein, preferably a Ras-tag fusion protein, wherein the tag can be detected on the extracellular side of the cell, optionally wherein the tag requires a second tag to be detectable (split-tag PPI)
f) a method using an ABP as defined *supra*
g) a method using 2D, 3D cell culture and/or spheroid or organoid cultures and/or stem cell-based embryo-like structures
h) a method combining two or more of a) to g).

In a seventh aspect of the invention an *in vitro* method for detecting modulation of cell activity induced by binding of ABPs or compounds to extracellular Ras on the surface of predominantly living cells is provided selected from the group consisting of
a) a method using an agent to detect a cytokine level
b) a method using at least one electrode to detect a change in cells' electrical potential
c) a method using genetically modified cells with a reporter plasmid to detect and/or measure activation of a signaling cascade
d) a method combining two or more of a) to c).

In an eighth aspect of the invention, an *in silico* method for detecting modulation of extracellular Ras on the surface of artificial cells is provided selected from the group consisting of
a) a method using artificial cell membranes
b) a method using artificial intelligence for calculating the extracellular Ras interface with small molecule compounds, with other proteins, with the lipid cell membrane and/or with the glycocalyx.

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the spirit and scope of the disclosed invention will become readily apparent to those skilled in the art from reading the following.

### DETAILED DESCRIPTION OF THE INVENTION

The present invention relates to novel combinations which comprise an ABP and a modulator compound for use as medicament. Both components (ABP and modulator compound) will now be described in detail.

### Combinations of ABPs and modulator compounds

In the present invention, a 3D co-culture model was used to test combinations of ABPs according to the present invention with modulator compounds.

According to a first aspect, a combination is provided, wherein the combination comprises
- an antigen binding protein (ABP) or an isolated nucleic acid comprising a sequence encoding the ABP and
- a modulator compound
for use as medicament, wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

"Combination" according to the present invention means that the at least two components of the combination, an ABP and a modulator compound, are administered in a timely manner but not necessarily together. It is possible that first an ABP is administered and then a modulator compound. It is also possible that first a modulator compound is administered and then an ABP. Both components can also be administered essentially together.

According to the present invention, the term "capable of" binding to shall refer to a binding domain that is able to bind to a certain antigen, or is specific for a particular antigen, i.e. is specifically binding to said antigen. Moreover, the term binding domain "capable of binding" shall further refer to a binding domain that is specific for a particular antigen.

### ABPs

The ABPs are preferably part of a combination or composition or kit. However, it is also intended that the ABP are used without a modulator compound. The ABP comprises at least a first antigen binding domain capable of binding to an extracellular Ras antigen. In one preferred embodiment, said extracellular Ras antigen is not part of, or is not restricted to, a human leukocyte antigen (HLA)-peptide complex, or specific HLA alleles, presented on the cell surface by the major histocompatibility complex (MHC). In another embodiment, said ABP comprises one or more additional antigen binding domain(s) that is capable of binding to antigen(s) present on a mammalian T-cell, preferably a human cluster of differentiation 3 (CD3) antigen or a human T cell receptor (TCR). In a further preferred embodiment, the first antigen binding domain comprises an amino acid sequence having a sequence identity of at least 70%, more preferably at least 80%, even more preferably at least 90%, and most preferably at least 95% sequence identity, to an amino acid sequence selected from SEQ ID NO: 20 to 31, 66 to 71, 134 to 178 and 290 to 396, or, in each case independently, optionally with no more than ten, nine, eight, seven, six, five, four, preferably no more than three, two or one, amino acid substitution(s), insertion(s) or deletion(s) compared to these sequences.

Thus, the present invention relates to an ABP having a binding domain for Ras, which mediates tumor specificity. Moreover, the ABP of the present invention preferably also has a binding domain for CD3, which enables the binding to T cells. Most preferably, the present invention relates to an ABP having a binding domain for Ras and a binding domain for CD3. In a particularly preferred example, the present invention relates to a bispecific antibody having a binding domain for Ras and a binding domain for CD3.

Ras antigen is often used herein synonymously with Ras protein. When expressed on a cell and being present on a cell surface, the Ras antigen is a Ras protein.

The inventors recently found extracellular accessible Ras protein on cancer cells. The inventor then developed bi-specific ABPs that are able to bind to Ras proteins on the cell surface of cancer cells and CD3 positive T cells, and, thereby, eliminate cancerous cells via a T cell-induced immune response. Importantly, the binding of the anti Ras directed bi-specific ABPs to the tumor cells is preferably not restricted to specific HLA alleles.

The ABP according to the present invention binds to an epitope displayed by one or more extracellular accessible domain(s) of a Ras protein. Importantly, extracellular accessible Ras protein provides a multitude of epitopes including an epitope covering a part of the Ras wildtype sequence or part of Ras mutated sequence. Thus, the ABPs of the present invention can target various cells that aberrantly express mutant Ras or wildtype Ras proteins. Therefore, the ABPs of the present invention are highly beneficial over ABPs of the prior art, which target patient-specific RAS neoantigens in complex with MHC. Such RAS neoantigens are the result of mutations during oncogenesis, and allow individualized therapies, *i.e.* therapies for a small HLA matched patient group because such neoantigens are expressed specific in a certain set of HLA alleles.

Importantly, the ABPs of the present invention target extracellular accessible Ras protein that preferably differs largely in its tertiary structure from Ras epitopes presented by short peptide fragments in complex with HLAs. Therefore, the ABPs of the present invention preferably differ from ABPs targeting Ras neoantigenes in complex with HLAs. Importantly, the ABPs of the present invention allow targeting extracellular accessible Ras protein in aggressive tumors on Ras mutated cancer cells from the cell outside.

In one preferred embodiment, the ABP of the present invention can recruit immune cells like natural killer (NK) cells or antigen presenting cells (like dendritic cells).

In a preferred embodiment, the extracellular Ras antigen is selected from a Kirsten rat sarcoma viral oncogene homolog (KRAS), such as KRAS4A or KRAS4B, a neuroblastoma RAS viral (v-RAS) oncogene homolog (NRAS), and a Harvey rat sarcoma viral oncogene homolog (HRAS). Of note, the two KRAS isoforms KRAS4A and KRAS4B arise from alternative RNA splicing. For HRAS, two isoforms arise from an alternate exon resulting in a frameshift and an early stop codon compared to wild type isoform 1. The encoded HRAS isoform 2 has a shorter and distinct C-terminus compared to wild type isoform 1. Thus, there is a total of five different Ras Proteins expressed in human cells, and the extracellular Ras antigen according to the present invention can be any of these.

The present invention further relates to an ABP, wherein the ABP comprises at least a first antigen binding domain capable of binding to an extracellular Ras antigen, a paralogue, an orthologue or other variant thereof.

In the present invention, the Ras antigen is of murine or human origin, preferably is a human Ras antigen or protein.

In another preferred embodiment, which can be combined with any and all other specifically preferred embodiments and aspects of the present invention, the first antigen binding domain of the ABP according to the present invention is capable of binding to an extracellular human Ras antigen. Preferably the binding is not restricted to specific HLA alleles.

Particularly preferred is that the extracellular Ras antigen comprises, preferably consists of, the amino acid sequence according to any one of SEQ ID NOs: 1, 3, 5, 7, 9 or 179 to 182, or comprises, preferably consists of, a sequence having at least 75% sequence identity, preferably at least 80%, more preferably at least 90%, and most preferably at least 95% sequence identity with any one of SEQ ID NOs: 1, 3, 5, 7, 9, or 179 to 182.

Even more preferred is that the extracellular Ras antigen comprises, preferably consists of, the amino acid sequence according to any one of SEQ ID NOs: 179 to 182, or comprises, preferably consists of, a sequence having at least 75% sequence identity, preferably at least 80%, more preferably at least 90%, and most preferably at least 95% sequence identity with any one of SEQ ID NOs: 179 to 182. SEQ ID NO: 179 corresponds to the amino acid sequence of KRAS4A, wherein the protease amino acids have been removed. SEQ ID NO: 180 corresponds to the amino acid sequence of KRAS4B, wherein the protease amino acids have been removed. SEQ ID NO: 181 corresponds to the amino acid sequence NRAS, wherein the protease amino acids have been removed. SEQ ID NO: 182 corresponds to the amino acid sequence HRAS, wherein the protease amino acids have been removed. Thus, the sequences according to any one of SEQ ID NOs: 179 to 182 corresponds to the respective wild type Ras proteins, after they have been post-translationally modified. The removal of the protease amino acids in any of the respective wild type Ras proteins is important for enabling the binding of the proteins to the cell membrane.

According to one preferred embodiment, the present invention thus pertains to an ABP, wherein the ABP comprises at least a first antigen binding domain specifically binding to an extracellular Ras antigen according to any one of SEQ ID NOs: 1, 3, 5, 7, 9, or 179 to 182.

Alternative target antigens with possible extracellular expression on the cell surface for ABP are also possible and shown in Table 1.

Table 1: Alternative target antigens with possible extracellular expression on the cell surface for ABP of this invention. Genes belonging to the RAS superfamily divided into the five major families Ras, Rho, Arf/Sara, Ran and Rab. Also genes belonging to the Gα subfamily and genes belonging to the Src family kinase containing a membrane-targeting region at their N-terminus, which is myristoylated and sometimes palmitoylated, similar to the HVR regions of KRAS, NRAS and HRAS. All sequences were retrieved from the online databases on December 02, 2024.

| **Gene name** | **NCBI Gene ID** | **UniProt Number** |
|---|---|---|
| KRAS | 3845 | P01116 |
| | | P01116-1 |
| | | P01116-2 |
| | | G3V5T7 |
| | | G3V4K2 |
| | | A0A8I5KQ21 |
| | | A0A8I5KR86 |
| | | A0A8I5KQU3 |
| | | A0A8I5KUB5 |
| | | A0A8I5KYH6 |
| | | A0A8I5KXN3 |
| HRAS | 3265 | P01112 |
| | | P01112-1 |
| | | P01112-2 |
| | | A0A804HJ06 |
| | | A0A0J9YXG8 |
| | | A0A804HKM6 |
| | | A0A8C8MQR2 |
| ERAS | 3266 | Q7Z444 |
| RALA | 5898 | P11233 |
| | | H7C3P7 |
| | | C9JPE8 |
| RALB | 5899 | P11234 |
| | | P11234-1 |
| | | P11234-2 |
| | | P11234-3 |
| | | C9J6B1 |
| | | C9JQB3 |
| RRAS | 6237 | P10301 |
| RRAS2 | 22800 | P62070 |
| | | P62070-1 |
| | | P62070-2 |
| | | P62070-3 |
| | | P62070-4 |
| | | E9PK85 |
| MRAS | 22808 | 014807 |
| | | 014807-1 |
| | | 014807-2 |
| | | C9J8Q6 |
| RIT1 | 6016 | Q92963 |
| | | Q92963-1 |
| | | Q92963-2 |
| | | Q92963-3 |
| | | A0A494C0S1 |
| | | V9GYC3 |
| RIT2 | 6014 | Q99578 |
| | | Q99578-1 |
| | | Q99578-2 |
| | | A0A3B3ITB4 |
| | | K7EMR8 |
| RAP1A | 5906 | P62834 |
| RAP1B | 5908 | P61224 |
| | | P61224-1 |
| | | P61224-2 |
| | | P61224-3 |
| | | P61224-4 |
| | | E7ESV4 |
| | | B7ZB78 |
| | | F5GZG1 |
| | | F5GX62 |
| | | F5GYB5 |
| | | F5H7Y6 |
| | | F5H004 |
| RAP2A | 5911 | P10114 |
| RAP2C | 57826 | Q9Y3L5 |
| | | A0A087X2C3 |
| RAP2B | 5912 | P61225 |
| DIRAS1 | 148252 | O95057 |
| | | K7EN06 |
| DIRAS2 | 54769 | Q96H U8 |
| | | A0A1B0GVC3 |
| DIRAS3 | 9077 | 095661 |
| RASD1 | 51655 | Q9Y272 |
| | | Q9Y272-1 |
| | | Q9Y272-2 |
| RASD2 | 23551 | Q96D21 |
| RASL10B | 91608 | Q96S79 |
| RASL10A | 10633 | Q92737 |
| | | Q92737-1 |
| | | Q92737-2 |
| NKIRAS1 | 28512 | Q9NYS0 |
| | | G5E9P3 |
| NKIRAS2 | 28511 | Q9NYR9 |
| | | Q9NYR9-1 |
| | | Q9NYR9-2 |
| | | Q9NYR9-3 |
| | | Q9NYR9-4 |
| | | H7BXP1 |
| | | K7ERG2 |
| RERG | 85004 | Q96A58 |
| | | Q96A58-1 |
| | | Q96A58-2 |
| | | F5H252 |
| RASL11B | 65997 | Q9BPW5 |
| RASL11A | 387496 | Q6T310 |
| RASL12 | 51285 | Q9NYN1 |
| | | Q9NYN1-1 |
| | | Q9NYN1-2 |
| | | Q9NYN1-3 |
| GEM | 2669 | P55040 |
| RRAD | 6236 | P55042 |
| | | J3KRG9 |
| | | J3KSM6 |
| REM1 | 28954 | O75628 |
| REM2 | 161253 | Q8IYK8 |
| | | Q8IYK8-1 |
| | | Q8IYK8-2 |
| RHEB | 6009 | Q15382 |
| RHEBL1 | 121268 | Q8TAI7 |
| | | Q8TAI7-1 |
| | | Q8TAI7-2 |
| | | F8W1T5 |
| RAC1 | 5879 | P63000 |
| | | P63000-1 |
| | | P63000-2 |
| | | A0A994J6T1 |
| RAC2 | 5880 | P15153 |
| | | B1AH80 |
| | | B1AH77 |
| | | B1AH78 |
| RAC3 | 5881 | P60763 |
| | | J3KSC4 |
| | | J3QLK0 |
| RHOG | 391 | P84095 |
| RHOJ | 57381 | Q9H4E5 |
| | | Q9H4E5-1 |
| | | Q9H4E5-2 |
| | | G3V476 |
| | | G3V4H1 |
| RHOQ | 23433 | P17081 |
| CDC42 | 998 | P60953 |
| | | P60953-1 |
| | | P60953-2 |
| | | Q5JYX0 |
| | | A0A494BZX6 |
| | | A0A590UJK8 |
| | | A0A494C1M1 |
| | | A0A8Q3WLC5 |
| | | A0A8Q3SI43 |
| RHOU | 58480 | Q7L0Q8 |
| | | Q7L0Q8-1 |
| | | Q7L0Q8-2 |
| RHOV | 171177 | Q96L33 |
| RHOH | 399 | Q 15669 |
| | | D6RG23 |
| | | D6RA52 |
| RHOA | 387 | P61586 |
| | | C9JX21 |
| | | C9JNR4 |
| | | A0A7I2YQV1 |
| | | A0A7I2V3G1 |
| RHOC | 389 | P08134 |
| | | Q5JR05 |
| | | Q5JR07 |
| | | Q5JR08 |
| | | E9PQH6 |
| | | E9PN11 |
| RHOB | 388 | P62745 |
| RHOD | 29984 | 000212 |
| | | E9PIG5 |
| RHOF | 54509 | Q9HBH0 |
| | | Q9HBH0-1 |
| | | Q9HBH0-2 |
| | | F5GXB1 |
| | | V9GY67 |
| RND3 | 390 | P61587 |
| | | Q53RZ3 |
| | | E9PFH1 |
| RND2 | 8153 | P52198 |
| RND1 | 27289 | Q92730 |
| | | H0YHG7 |
| RHOBTB1 | 9886 | O94844 |
| RHOBTB2 | 23221 | Q9BYZ6 |
| | | Q9BYZ6-1 |
| | | Q9BYZ6-2 |
| | | Q9BYZ6-3 |
| | | A0A8I5KV41 |
| RHOT1 | 55288 | Q8IXI2 |
| | | Q8IXI2-1 |
| | | Q8IXI2-2 |
| | | Q8IXI2-3 |
| | | Q8IXI2-4 |
| | | Q8IXI2-5 |
| | | Q8IXI2-6 |
| | | Q8IXI2-7 |
| | | H7BXZ6 |
| RHOT2 | 89941 | Q8IXI1 |
| | | Q8IXI1-1 |
| | | Q8IXI1-2 |
| | | A0A8V8TM48 |
| BLK | 640 | P51451 |
| FGR | 2268 | P09769 |
| | | Q5TGY6 |
| FRK | 2444 | P42685 |
| | | P42685-1 |
| | | P42685-2 |
| FYN | 2534 | P06241 |
| | | P06241-1 |
| | | P06241-2 |
| | | P06241-3 |
| HCK | 3055 | P08631 |
| | | P08631-1 |
| | | P08631-2 |
| | | P08631-3 |
| | | P08631-4 |
| | | J3KPD6 |
| | | H0Y3C5 |
| LCK | 3932 | P06239 |
| | | P06239-1 |
| | | P06239-2 |
| | | P06239-3 |
| | | E9PAP0 |
| | | E9PJ92 |
| | | E9PKQ8 |
| LYN | 4067 | P07948 |
| | | P07948-1 |
| | | P07948-2 |
| | | E5RJ37 |
| SRC | 6714 | P12931 |
| | | P12931-1 |
| | | P12931-2 |
| | | P12931-3 |
| | | A0A8I5KYU4 |
| YES1 | 7525 | P07947 |
| | | J3QRU1 |
| RAN | 5901 | P62826 |
| | | J3KQE5 |
| | | B5M DF5 |
| | | F5H018 |
| RABL2A | 11159 | Q9UBK7 |
| | | Q9UBK7-1 |
| | | Q9UBK7-2 |
| | | Q9UBK7-3 |
| | | B7ZBD5 |
| | | B7ZBD4 |
| RABL2B | 11158 | Q9UNT1 |
| | | Q9UNT1-1 |
| | | Q9UNT1-2 |
| | | Q9UNT1-3 |
| | | A8MXF6 |
| RABL3 | 285282 | Q5HYI8 |
| | | F8WDC7 |
| | | C9JXM3 |
| IFT22 | 64792 | Q9H7X7 |
| | | Q9H7X7-1 |
| | | Q9H7X7-2 |
| | | Q9H7X7-3 |
| RAB1A | 5861 | P62820 |
| | | P62820-1 |
| | | P62820-2 |
| | | P62820-3 |
| | | E7END7 |
| RAB1B | 81876 | Q9H0U4 |
| | | E9PLD0 |
| RAB35 | 11021 | Q15286 |
| | | Q15286-1 |
| | | Q15286-2 |
| | | F5H7F8 |
| | | F5H157 |
| RAB13 | 5872 | P51153 |
| | | A0A087WWB9 |
| RAB8A | 4218 | P61006 |
| | | P61006-1 |
| | | P61006-2 |
| RAB8B | 51762 | Q92930 |
| | | H0YNE9 |
| RAB10 | 10890 | P61026 |
| RAB12 | 201475 | Q6IQ22 |
| RAB3A | 5864 | P20336 |
| | | M0R257 |
| | | S4R3Q3 |
| RAB3C | 115827 | Q96E17 |
| RAB3B | 5865 | P20337 |
| RAB3D | 9545 | 095716 |
| RAB40A | 142684 | Q8WXH6 |
| RAB40B | 10966 | Q12829 |
| | | H0YFJ5 |
| RAB40C | 57799 | Q96S21 |
| | | Q96S21-1 |
| | | Q96S21-2 |
| | | H3BTC6 |
| | | H3BPA5 |
| | | H3BNV8 |
| | | H3BME4 |
| RAB15 | 376267 | P59190 |
| | | P59190-1 |
| | | P59190-2 |
| | | A0A2R8Y7G7 |
| | | A0A2R8YFB8 |
| | | G3V562 |
| | | G3V196 |
| RAB44 | 401258 | Q7Z6P3 |
| RAB27A | 5873 | P51159 |
| | | P51159-1 |
| | | P51159-2 |
| | | H3BVH7 |
| | | H3BS49 |
| | | H3BN55 |
| RAB27B | 5874 | 000194 |
| | | K7EJ38 |
| RASEF | 158158 | Q8IZ41 |
| | | Q8IZ41-1 |
| | | Q8IZ41-2 |
| RAB26 | 25837 | Q9ULW5 |
| | | Q9ULW5-1 |
| | | Q9ULW5-2 |
| | | H3BQ97 |
| RAB37 | 326624 | Q96AX2 |
| | | Q96AX2-1 |
| | | Q96AX2-2 |
| | | Q96AX2-3 |
| | | Q96AX2-4 |
| | | A8MSP2 |
| | | A8MTC6 |
| | | B7Z3L0 |
| | | A0A9H3ZVF6 |
| RAB2A | 5862 | P61019 |
| | | P61019-1 |
| | | P61019-2 |
| | | E9PKL7 |
| RAB2B | 84932 | Q8WUD1 |
| | | Q8WUD1-1 |
| | | Q8WUD1-2 |
| | | A0A3B3ITL1 |
| RAB4A | 5867 | P20338 |
| | | A0A087WYT5 |
| RAB4B | 53916 | P61018 |
| | | P61018-1 |
| | | P61018-2 |
| | | M0R0X1 |
| RAB14 | 51552 | P61106 |
| | | X6RFL8 |
| | | A0A994J4B9 |
| | | A0A994J451 |
| RAB11A | 8766 | P62491 |
| | | P62491-1 |
| | | P62491-2 |
| | | H3BSC1 |
| | | B4DQU5 |
| | | H3BMH2 |
| RAB11B | 9230 | Q15907 |
| | | Q15907-1 |
| | | Q15907-2 |
| RAB25 | 57111 | P57735 |
| RAB39A | 54734 | Q14964 |
| RAB39B | 116442 | Q96DA2 |
| RAB42 | 115273 | Q8N4Z0 |
| | | Q8N4Z0-1 |
| | | Q8N4Z0-2 |
| RAB19 | 401409 | A4D1S5 |
| | | A4D1S5-1 |
| | | A4D1S5-2 |
| RAB43 | 339122 | Q86YS6 |
| | | Q86YS6-1 |
| | | Q86YS6-2 |
| RAB30 | 27314 | Q15771 |
| | | Q15771-1 |
| | | Q15771-2 |
| | | H0YDK7 |
| | | E9PS06 |
| | | E9PNB9 |
| | | E9PMJ1 |
| RAB33A | 9363 | Q14088 |
| RAB33B | 83452 | Q9H082 |
| | | A0A494C0Z5 |
| RAB18 | 22931 | Q9NP72 |
| | | Q9NP72-1 |
| | | Q9NP72-2 |
| | | Q9NP72-3 |
| | | A0A8C8NLQ3 |
| | | B7Z4P9 |
| | | Q5W0J0 |
| RAB17 | 64284 | Q9H0T7 |
| | | Q9H0T7-1 |
| | | Q9H0T7-2 |
| | | C9J0T6 |
| | | H7C1P7 |
| RAB5A | 5868 | P20339 |
| | | P20339-1 |
| | | P20339-2 |
| RAB5C | 5878 | P51148 |
| | | P51148-1 |
| | | P51148-2 |
| | | F8VVK3 |
| | | K7ENY4 |
| RAB5B | 5869 | P61020 |
| | | P61020-1 |
| | | P61020-2 |
| | | F8VUA5 |
| RAB22A | 57403 | Q9U L26 |
| RAB31 | 11031 | Q13636 |
| RAB21 | 23011 | Q9U L25 |
| RAB20 | 55647 | Q9NX57 |
| RAB24 | 53917 | Q969Q5 F8W8H5 |
| RAB6A | 5870 | P20340 |
| | | P20340-1 |
| | | P20340-2 |
| | | P20340-3 |
| | | P20340-4 |
| RAB6C | 84084 | Q9H0N0 |
| RAB6B | 51560 | Q9NRW1 |
| | | Q9NRW1-1 |
| | | Q9NRW1-2 |
| | | J3KR73 |
| RAB41 | 347517 | Q5JT25 |
| | | Q5JT25-1 |
| | | Q5JT25-2 |
| RAB34 | 83871 | Q9BZG1 |
| | | Q9BZG1-1 |
| | | Q9BZG1-2 |
| | | Q9BZG1-4 |
| | | E7ES60 |
| | | K7EIF2 |
| | | C9JY26 |
| | | P0DI83 |
| | | A0A1B0GTQ2 |
| | | A0A1B0GWB1 |
| | | C9JBG0 |
| | | Q96PJ7 |
| | | A0A1C7CYW6 |
| RAB36 | 9609 | O95755 |
| | | 095755-1 |
| | | O95755-2 |
| RAB29 | 8934 | 014966 |
| | | 014966-1 |
| | | 014966-2 |
| | | 014966-3 |
| RAB32 | 10981 | Q13637 |
| RAB38 | 23682 | P57729 |
| | | H0YDB7 |
| RAB23 | 51715 | Q9ULC3 |
| RAB28 | 9364 | P51157 |
| | | P51157-1 |
| | | P51157-2 |
| | | P51157-3 |
| | | H0Y9S6 |
| IFT27 | 11020 | Q9BW83 |
| | | Q9BW83-1 |
| | | Q9BW83-2 |
| | | B1AH58 |
| | | H0Y6C7 |
| | | F5GZ09 |
| RAB7A | 7879 | P51149 |
| | | A0A6Q8PH84 |
| | | A0A6Q8PGE6 |
| | | A0A6Q8PG52 |
| | | C9J8S3 |
| | | C9IZZ0 |
| RAB7B | 338382 | Q96AH8 |
| | | A0A096LP44 |
| RAB9B | 51209 | Q9NP90 |
| RAB9A | 9367 | P51151 |
| ARF1 | 375 | P84077 |
| | | A0A8V8TQC0 |
| | | A0A8V8TQP8 |
| | | A0A8V8TNZ0 |
| | | A0A8V8TNZ5 |
| ARF3 | 377 | P61204 |
| | | P61204-1 |
| | | P61204-2 |
| | | F5H0C7 |
| ARF4 | 378 | P18085 |
| | | C9JPM4 |
| | | C9JAK5 |
| ARF5 | 381 | P84085 |
| | | C9J1Z8 |
| ARF6 | 382 | P62330 |
| ARFRP1 | 10139 | Q13795 |
| | | Q13795-1 |
| | | Q13795-2 |
| | | Q13795-3 |
| | | Q13795-4 |
| SARA1 | 56681 | Q9NR31 |
| | | Q9NR31-1 |
| | | Q9NR31-2 |
| | | Q5SQT8 |
| | | H0Y5E8 |
| SAR1B | 51128 | Q9Y6B6 |
| | | D6RDB2 |
| | | D6RD69 |
| GNAL | 2774 | P38405 |
| | | P38405-1 |
| | | P38405-2 |
| | | P38405-3 |
| | | K7EQ80 K7EPE2 |
| GNAS | 2778 | O95467 |
| | | 095467-1 |
| | | Q5JWF2-1 |
| | | Q5JWF2-2 |
| | | Q5JWF2-3 |
| | | P63092-1 |
| | | P63092-2 |
| | | P63092-3 |
| | | P63092-4 |
| | | Q5JWF2 |
| | | P63092 |
| | | Q5JWE9 |
| | | H0Y7E8 |
| | | A0A804HIH4 |
| | | A0A590UJY2 |
| | | A0A590UJS2 |
| | | A0A590UJQ9 |
| | | A0A590UJX6 |
| | | A0A590UK28 |
| | | A0A590UK00 |
| | | A0A590UJF0 |
| | | A0A7I2V5R6 |
| | | P84996 |
| GNAI1 | 2770 | P63096 |
| | | P63096-1 |
| | | P63096-2 |
| | | A0A3B3ITM0 |
| | | A0A3B3IUA8 |
| GNAI3 | 2773 | P08754 |
| GNAI2 | 2771 | P04899 |
| | | P04899-1 |
| | | P04899-2 |
| | | P04899-3 |
| | | P04899-4 |
| | | P04899-5 |
| | | P04899-6 |
| GNAO1 | 2775 | P09471 |
| | | P09471-1 |
| | | P09471-2 |
| | | H3BTM2 |
| | | H3BNR5 |
| | | A0A1W2PP38 |
| | | A0A1W2PPG6 |
| GNAT1 | 2779 | P11488 |
| | | C9JCV8 |
| GNAT2 | 2780 | P19087 |
| GNAT3 | 346562 | A8MTJ3 |
| GNAZ | 2781 | P19086 |
| GNA11 | 2767 | P29992 |
| | | K7EL62 |
| GNAQ | 2776 | P50148 |
| GNA14 | 9630 | O95837 |
| GNA15 | 2769 | P30679 |
| GNA12 | 2768 | Q03113 |
| | | Q03113-1 |
| | | Q03113-2 |
| | | Q03113-3 |
| | | E9PC54 |
| GNA13 | 10672 | Q14344 |
| | | Q14344-1 |
| | | Q14344-2 |

In one embodiment, the extracellular Ras antigen is a mutant Ras antigen according to any one of SEQ ID NOs: 11 to 19, 77 to 119, or 183 to 223. In one preferred embodiment, the targeted protein is a mutant Ras protein expressed on the extracellular site of a cancer cell. Thus, an ABP of the present invention can target Ras mutated oncogenic cells.

In a further embodiment, the extracellular antigen is a mutant version of any antigen shown in Table 1.

In another preferred embodiment, the cells targeted by the ABP of the present invention are malignant cancer cells and/or Ras mutated cells.

In another preferred embodiment, the malignant cells targeted by the ABP of the present invention have no mutation in Ras, but show Ras cellular signaling dependency. Thus, Ras is important for the tumor cell survival and proliferation.

In a further embodiment, the ABP according to the present invention binds to an extracellular accessible Ras antigen with an EC₅₀ higher than 0.01 nM, preferably higher than 0.1 nM, even more preferably higher than 1 nM, even more preferably higher than 10 nM, even more preferably higher than 20 nM, and most preferably higher than 30 nM.

In a further preferred embodiment, the ABP enhances a cell-mediated immune response, such as the immune response mediated by an activated cytotoxic T-cell (CTL) to a mammalian cell expressing said extracellular accessible Ras antigen.

In a specific embodiment, the preferred one or more additional antigen binding domain(s) of the ABP enhances a cell-mediated immune response, such as that mediated by an activated cytotoxic T-cell (CTL), to a mammalian cell expressing said extracellular Ras antigen.

Thus, the preferable one or more additional antigen binding domain(s) of the ABP that is capable of binding to antigen(s) present on a mammalian T-cell, preferably the human cluster of differentiation 3 (CD3) antigen or a human T cell receptor (TCR), preferably increases the activity of immune cells, such as T-cells.

In one specific embodiment, the specific T cell response is induced by a specific anti-CD3 binding of the at least one second antigen binding domain of the ABP according to the present invention to antigens present on a human cluster of differentiation 3 (CD3) antigen.

In a preferred embodiment, the antigen binding domain(s) that is capable of binding to antigen(s) present on a mammalian T-cell, preferably a human cluster of differentiation 3 (CD3) antigen, comprises an amino acid sequence according to SEQ ID No: 48 or 49.

In another specific embodiment, the one or more additional antigen binding domain(s) of the ABP enhances a cell-mediated immune response, such as that mediated by an activated natural killer (NK) cell, to a mammalian cell expressing said extracellular Ras antigen.

In another specific embodiment, the one or more additional antigen binding domain(s) of the ABP enhances a cell-mediated immune response, such as that mediated by an activated natural killer T cell (NKT cell), to a mammalian cell expressing said extracellular Ras antigen.

In another specific embodiment, the one or more additional antigen binding domain(s) of the ABP enhances a cell-mediated immune response, such as that mediated by an activated regulatory T cell (TReg), to a mammalian cell expressing said extracellular Ras antigen.

In another specific embodiment, the one or more additional antigen binding domain(s) of the ABP enhances a cell-mediated immune response, such as that mediated by an activated antigen-presenting cell (APC) or accessory cell, to a mammalian cell expressing said extracellular Ras antigen.

In a further preferred embodiment, the antigen binding domain capable of binding to an extracellular Ras antigen, preferably provided that said extracellular Ras antigen is not part of a, or is not restricted to, human leukocyte antigen (HLA)-peptide complex, or specific HLA alleles, presented on the cell surface by the major histocompatibility complex (MHC), comprises an amino sequence according to any one of SEQ ID Nos: 20 to 31, or 66 to 71, or 134 to 178.

In one embodiment, the anti CD3 binder of the present invention was derived from the diL2K clone. In another embodiment, the anti CD3 binder of the present invention was derived from the UCHT1 clone. The anti-CD3 antibodies according to the present invention are herein sometimes referred to as diL2K and/or UCHT1. In one embodiment, the anti-CD3 antibodies diL2K and/or UCHT1 bind to the epsilon chain of the CD3 receptor complex.

In a further embodiment, the ABP of the present invention increases type-I cytokine secretion by said immune cells, for example T-cells or CTLs, such as one or more cytokines independently selected from the list consisting of: IFN-gamma, IL-2 and TNF-alpha.

Another embodiment relates to the ABP according to the present invention, wherein said ABP is selected from the group consisting of: an immunoglobulin molecule, such as an IgG, IgE, IgD, IgA, or IgM immunoglobulin, preferably an IgG immunoglobulin, a monoclonal antibody, a chimeric antibody, a bispecifc ABP, such as a bispecific antibody, a CDR-grafted antibody, a humanized antibody, a single domain antibody, a hemibody antibody, a single-chain KeyLock-antibody, a diabody, a single chain diabody, a variable domain of the antibody heavy chain or antibody light chain, a multispecific antibody, a chimeric antigen receptor (CAR), and a fragment of an antibody, such as a fragment of a monoclonal antibody, for example a single chain variable fragment (scFv), (scFv)2, a Fv, a disulfide linked Fv, Fab, Fab', F(ab')2 or a scFv-Fc, preferably wherein said ABP is a bispecific antibody or a CAR. The ABP can also be selected from the group of alternative protein binders including monobodies (derived from fibronectin type III), anticalins (derived from lipocalins), affibodies (derived from immunoglobulin-binding protein A) or DARPins (Designed Ankyrin Repeat Proteins). These alternative binders usually have a "constant" scaffold and a "variable" site for target antigen binding. Many types of alternative scaffolds are based on proteins with repeating motifs like leucine-rich repeats (LRRs), ankyrin repeats (ARs), Armadillo repeats (Arms), and tetratricopeptide repeats (TPRs).

The invention also includes novel dual antigen restricted ABP constructs, such as antibodies termed hemibodies, or antibodies using prodrug-activating chain exchange for a targeted immunotherapy of patients with cancer.

In one embodiment, the ABP comprises a heavy chain immunoglobulin constant domain selected from the group consisting of:
a human IgM constant domain,
a human IgG1 constant domain,
a human IgG2 constant domain,
a human IgG3 constant domain,
a human IgG4 constant domain,
a human IgE constant domain,
a human IgA constant domain,
and human IgD constant domain,
an IgG constant domain variant with one or more mutations altering binding strength to Fc neonatal receptor, Fc gamma receptors, or C1q.

A further embodiment pertains to the ABP according to the present invention, wherein one, preferably two, heavy chain variable domain(s) and one, preferably two, light chain variable domain(s), each comprise an antibody framework having at least a portion of a human antibody consensus framework sequence.

In another embodiment, the ABP comprises two heavy chain immunoglobulin constant domains, wherein both constant domains have specific mutations to reduce or abrogate the binding to the Fc gamma receptors (such as effector silent mutations), comprises the mutations L234A, L235A and P329A (numbering using Eu numbering), and a mutation to abrogate glycosylation of the constant domain (such as aglycosylation mutations), comprises the mutation N297A (numbering using Eu numbering) according to any one of SEQ ID NOs: 42, 43, and 120 to 133. The corresponding mutations in SEQ ID NO: 42 are 22A, 23A, 85A and 117A, and in SEQ ID NO: 43 are 14A, 15A, 77A and 109A. In a particularly preferred embodiment, the ABP is a bispecific antibody, and comprises at least one additional antigen binding domain that binds to a human cluster of differentiation 3 (CD3) antigen. Accordingly, the present invention provides T cell-engaging antibodies with a Ras binding domain. Importantly, the antibodies of the present invention are thereby able to redirect cytotoxic T cells against tumor cells.

In another particularly preferred embodiment, the ABP is an antibody with a toxic payload directly labeled to it. These Antibody-Drug Conjugates (ADCs) can carry a toxic payload like ozogamicin/calicheamicin, Vedotin (Monomethylauristatin E), Maytansinoide, camptothecin, auristatin or Tesirin (SG-3199). The toxic payload can also be a radioactive label like Yttrium-90, lodine-131, Samarium-153, Lutetium-177, Astatine-211, Lead-212/bismuth-212, Radium-223, Actinium-225 or Thorium-227.

In a further embodiment, the ABP is used to carry a nucleic acid, such as an RNA molecule, like a siRNA molecule, into RAS expressing cells.

In another particularly preferred embodiment, the ABP is part of an oncolytic virus, for a virus-mediated immune response, such as that mediated by DNA or RNA viruses, to a mammalian cell expressing said extracellular Ras antigen.

Another preferred embodiment relates to a fragment of an ABP according to the present invention, such as a fragment of a bispecific antibody that comprises at least a first antigen binding domain capable of binding to an extracellular Ras antigen, preferably provided that said extracellular Ras antigen is not part of a, or is not restricted to, HLA-peptide complex, or specific HLA alleles, presented on the cell surface by MHC, and preferably comprises at least one additional antigen binding domain that binds to a human cluster of differentiation 3 (CD3) antigen.

In a further embodiment, said ABP according to the present invention is isolated and/or substantially pure.

Yet another embodiment relates to the ABP according to the present invention, wherein said ABP is not cell membrane permeable.

A further embodiment relates to the ABP according to the present invention, wherein said ABP comprises an effector group.

In one embodiment, the ABP according to the present invention is labelled.

Another embodiment pertains to the ABP according to the present invention, wherein said ABP is Fc receptor binding attenuated.

A further embodiment pertains to the ABP according to the present invention, wherein the ABP comprises at least a first antigen binding domain capable of binding to an extracellular Ras antigen, preferably provided that said extracellular Ras antigen is not part of a, or is not restricted to, HLA-peptide complex, or specific HLA alleles, presented on the cell surface by MHC, and wherein said ABP optionally comprises one or more additional antigen binding domain(s) that is capable of binding to antigen(s) present on a mammalian T-cell, preferably a human cluster of differentiation 3 (CD3) antigen or a human T cell receptor (TCR), comprising:
(i) a heavy chain variable domain comprising the CDRH1 region set forth in SEQ ID NO: 50 or 53, the CDRH2 region set forth in SEQ ID NO: 51 or 54, and the CDRH3 region set forth in SEQ ID NO: 52 or 55, or wherein in each case independently the CDRH1, CDRH2 and/or CDRH3 comprise a sequence having no more than three or two, preferably no more than one amino acid substitution(s), deletion(s) or insertion(s) compared to SEQ ID NO: 50 or 53, SEQ ID NO: 51 or 54, or SEQ ID NO: 52 or 55, respectively; or comprising a CDRH1, CDRH2 or CDRH3 sequence having at least 75% sequence identity, preferably at least 80% sequence identity, more preferably at least 90% sequence identity, and most preferably at last 95% sequence identity with SEQ ID NO: 50 or 53, SEQ ID NO: 51 or 54, or SEQ ID NO: 52 or 55; and
(ii) a light chain variable domain comprising the CDRL1 region set forth in SEQ ID NO:56 or 59, the CDRL2 region set forth in SEQ ID NO: 57 or 60, and the CDRL3 region set forth in SEQ ID NO: 58 or 61 or wherein in each case independently CDRL1, CDRL2 and/or CDRL3 comprise a sequence having no more than three or two, preferably no more than one amino acid substitution(s), deletion(s) or insertion(s) compared to SEQ ID NO: 56 or 59, SEQ ID NO: 57 or 60, or SEQ ID NO: 58 or 61, respectively; or comprising a CDRL1, CDRL2 or CDRL3 sequence having at least 75% sequence identity, preferably at least 80% sequence identity, more preferably at least 90% sequence identity, and most preferably at last 95% sequence identity with SEQ ID NO: 56 or 59, SEQ ID NO: 57 or 60, or SEQ ID NO: 58 or 61.

A further embodiment pertains to the ABP according to the present invention, wherein the heavy chain variable region comprises the amino acid sequence having a sequence identity of at least 70%, preferably at least 80%, more preferably at least 90%, and most preferably at last 95% sequence identity, to an amino acid sequence selected from SEQ ID NO: 62 and/or 64, or, in each case independently, optionally with no more than ten, nine, eight, seven, six, five, four, preferably no more than three, two or one, amino acid substitution(s), insertion(s) or deletion(s) compared to these sequences; and/or wherein the light chain variable region comprises the amino acid sequence having a sequence identity of at least 70%, preferably at least 80%, more preferably at least 90%, and most preferably at last 95% sequence identity, to the amino acid sequence selected from SEQ ID NO: 63 and/or 65, or, in each case independently, optionally with no more than ten, nine, eight, seven, six, five, four, preferably no more than three, two or one, amino acid substitution(s), insertion(s) or deletion(s) compared to these sequences. In this embodiment, the heavy chain variable region and the light chain variable region is referring to the anti-CD3 binding domain.

A further embodiment pertains to the ABP according to the present invention, wherein the ABP comprises at least an antigen binding domain from an antibody variable heavy chain and variable light chain capable of binding to an extracellular Ras antigen, preferably provided that said extracellular Ras antigen is not part of, or is not restricted to, a HLA-peptide complex, or specific HLA alleles presented on the cell surface and wherein said Ras binding ABP optionally comprises:
(i) a heavy chain variable domain comprising the CDRH1 region set forth in any one of SEQ ID NOs: 135, 149, 162, 292, 300, or 338, the CDRH2 region set forth in SEQ ID NOs: 137, 151, 164, 293, 301, or 339, and the CDRH3 region set forth in any one of SEQ ID NOs: 139, 153, 166, 294, 302, or 340, or wherein in each case independently the CDRH1, CDRH2, and/or CDRH3 comprise a sequence having no more than three or two, preferably no more than one amino acid substitution(s), deletion(s) or insertion(s) compared to any one of SEQ ID NOs: 135, 149, 162, 292, 300, or 338, SEQ ID NOs: 137, 151, 164, 293, 301, or 339, or SEQ ID NOs: 139, 153, 166, 294, 302, or 340, respectively; or comprising a CDRH1, CDRH2 or CDRH3 sequence having at least 75% sequence identity, preferably at least 80% sequence identity, more preferably at least 90% sequence identity, and most preferably at last 95% sequence identity with any one of SEQ ID NOs: 135, 149, 162, 292, 300, or 338, SEQ ID NOs: 137, 151, 164, 293, 301, or 339, or SEQ ID NO: 139, 153, 166, 294, 302, or 340, respectively; and
(ii) a light chain variable domain comprising the CDRL1 region set forth in any one of SEQ ID NOs: 142, 156, 169, 295, 303, or 341, the CDRL2 region set forth in any one of SEQ ID NOs: 144, 158, 171, 296, 304, or 342 and the CDRL3 region set forth in any one of SEQ ID NOs: 146, 160, 173, 297, 305, or 343 or wherein in each case independently CDRL1, CDRL2, and/or CDRL3 comprise a sequence having no more than three or two, preferably no more than one amino acid substitution(s), deletion(s) or insertion(s) compared to any one of SEQ ID NOs: 142, 156, 169, 144, 158, 171, 146, 160, 173, 295, 303, 341, 296, 304, 342, 297, 305, or 343, respectively; or comprising a CDRL1, CDRL2, and/or CDRL3 sequence having at least 75% sequence identity, preferably at least 80% sequence identity, more preferably at least 90% sequence identity, and most preferably at last 95% sequence identity with any one of SEQ ID NOs: 142, 156, 169, 144, 158, 171, 146, 160, 173, 295, 303, 341, 296, 304, 342, 297, 305, or 343 respectively.

The amino acid residues, CDRs and FRs were determined according to the IMGT numbering system and Kabat by IgBLAST set forth in any one of SEQ ID NOs: 134 to 174, 292 to 297, 300 to 305, and 338 to 343.

Yet another embodiment pertains to the ABP according to the present invention, for an individual antibody the amino acids in the antibody CDR regions, which are in direct contact with the Ras antigen, preferably provided that said Ras antigen is not part of a, or is not restricted to, a HLA-peptide complex, or specific HLA alleles presented on the cell surface, can change significantly, depending on the type of Ras antigen bound, mutated or not, as seen in the Tables below.

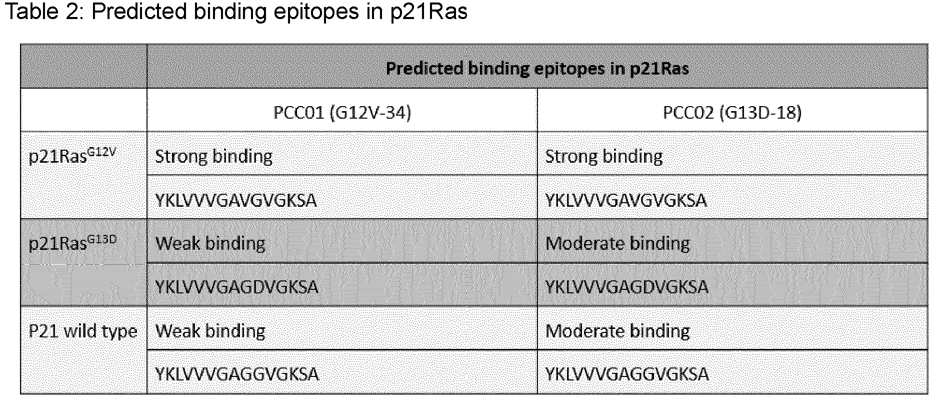

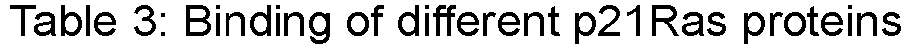

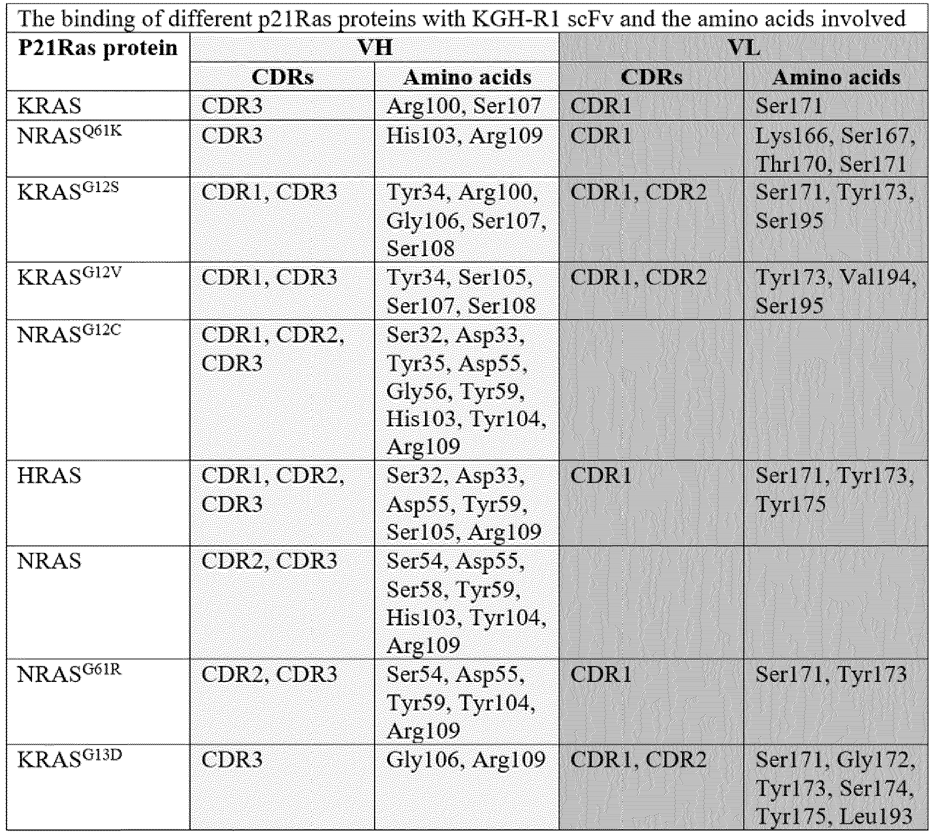

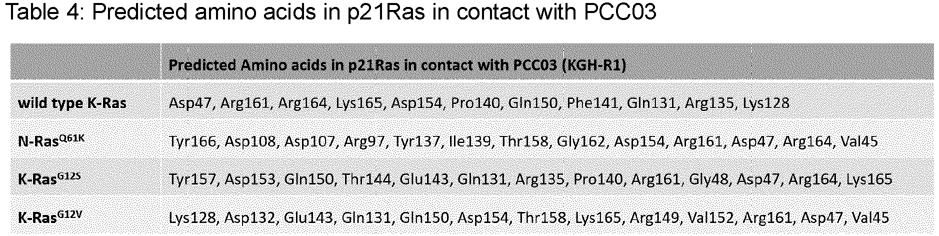

Yet another embodiment pertains to the ABP according to the present invention, comprising at least one antibody heavy chain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NOs: 66, 68, 70, 391, 393, or 395; and/or comprising at least one antibody light chain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s), or no amino acid change, compared to a sequence selected from selected from SEQ ID NOs: 67, 69, 71, 392, 394, or 396. In this embodiment, the heavy chain variable region and the light chain variable region is referring to the anti-Ras binding domain.

A further embodiment pertains to the ABP according to the present invention, wherein the ABP comprises at least a fibronectin type III domain (FN3) binder, also known as a monobody, capable of binding to an extracellular Ras antigen, preferably provided that said extracellular Ras antigen is not part of, or is not restricted to, a HLA-peptide complex, or specific HLA alleles presented on the cell surface and wherein said Ras binding ABP optionally comprises:
(i) With four different antigen binding loops (BC, DE, FG and CD) similar to antibody CDR regions comprising the BC loop region set forth in SEQ ID NOs: 175, 307, 312, 349, 354, or 359 the DE loop region set forth in SEQ ID NOs: 176, 309, 314, 351, 356, or 361, the FG loop region set forth in SEQ ID NOs: 177, 310, 315, 352, 357, or 362, and the CD loop region set forth in SEQ ID NOs: 178, 308, 313, 350, 355, or 360, or wherein in each case independently BC, DE, FG and CD comprise a sequence having no more than three or two, preferably no more than one amino acid substitution(s), deletion(s) or insertion(s) compared to any one of SEQ ID NOs: 175, 176, 177, 178, 307 to 310, 312 to 315, 349 to 352, 354 to 357, or 359 to 362, respectively; or comprising a BC, DE, FG and CD sequence having at least 75% sequence identity, preferably at least 80% sequence identity, more preferably at least 90% sequence identity, and most preferably at last 95% sequence identity with SEQ ID NOs: 175, 176, 177, 178, 307 to 310, 312 to 315, 349 to 352, 354 to 357, or 359 to 362.
(ii) Yet another embodiment pertains to the ABP according to the present invention, comprising at least one monobody having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NOs: 25, 26, 306, 311, 348, 353, or 358.

A further embodiment pertains to the ABP according to the present invention, wherein the ABP comprises at least a single variable domain of a heavy chain (VHH) antibody binder, also known as nanobody, capable of binding to an extracellular Ras antigen, preferably provided that said extracellular Ras antigen is not part of, or is not restricted to, a HLA-peptide complex, or specific HLA alleles presented on the cell surface and wherein said Ras binding ABP optionally comprises:
(i) a single heavy chain variable domain comprising the CDR1 region set forth in any one of SEQ ID NOs: 317, 321, 325, 329, 333, or 345, the CDR2 region set forth in SEQ ID NOs: 318, 322, 326, 330, 334, or 346, and the CDR3 region set forth in any one of SEQ ID NOs: 319, 323, 327, 331, 335, or 347, or wherein in each case independently the CDR1, CDR2, and/or CDR3 comprise a sequence having no more than three or two, preferably no more than one amino acid substitution(s), deletion(s) or insertion(s) compared to any one of SEQ ID NOs: 317, 321, 325, 329, 333, 345, 318, 322, 326, 330, 334, 346, 319, 323, 327, 331, 335, or 347 respectively; or comprising a CDR1, CDR2 or CDR3 sequence having at least 75% sequence identity, preferably at least 80% sequence identity, more preferably at least 90% sequence identity, and most preferably at last 95% sequence identity with any one of SEQ ID NOs: 317, 321, 325, 329, 333, 345, 318, 322, 326, 330, 334, 346, 319, 323, 327, 331, 335, or 347, respectively; and
(ii) Yet another embodiment pertains to the ABP according to the present invention, comprising at least one nanobody having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NOs: 316, 320, 324, 328, 332, or 344.

A further embodiment pertains to the ABP according to the present invention, wherein the ABP comprises at least an affinity binder based on a structurally robust protease inhibitor scaffold, also known as affimer, capable of binding to an extracellular Ras antigen, preferably provided that said extracellular Ras antigen is not part of, or is not restricted to, a HLA-peptide complex, or specific HLA alleles presented on the cell surface and wherein said Ras binding ABP optionally comprises:
(i) a single chain variable domain comprising the VR1 region set forth in any one of SEQ ID NOs: 364, 367, or 370, and the VR2 region set forth in SEQ ID NOs: 365, 368, or 371, or wherein in each case independently the VR1, and/or VR2 comprise a sequence having no more than three or two, preferably no more than one amino acid substitution(s), deletion(s) or insertion(s) compared to any one of SEQ ID NOs: 364, 367, 370, 365, 368, or 371, respectively; or comprising a VR1, or VR2 sequence having at least 75% sequence identity, preferably at least 80% sequence identity, more preferably at least 90% sequence identity, and most preferably at last 95% sequence identity with any one of SEQ ID NOs: 364, 367, 370, 365, 368, or 371, respectively; and
(ii) having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NOs: 363, 366 or 369.

A further embodiment pertains to the ABP according to the present invention, wherein the ABP comprises at least a designed ankyrin repeat protein, also known as DARPin, capable of binding to an extracellular Ras antigen, preferably provided that said extracellular Ras antigen is not part of, or is not restricted to, a HLA-peptide complex, or specific HLA alleles presented on the cell surface and wherein said Ras binding ABP optionally comprises:
(i) a single chain variable domain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NOs: 372 or 373.

A further embodiment pertains to the ABP according to the present invention, wherein the ABP comprises at least a charge-neutralized variants of the Sso7d protein (UniProt P39476) from the hyperthermophilic archaeon Sulfolobus solfataricus, capable of binding to an extracellular Ras antigen, preferably provided that said extracellular Ras antigen is not part of, or is not restricted to, a HLA-peptide complex, or specific HLA alleles presented on the cell surface and wherein said Ras binding ABP optionally comprises:
(i) a single chain variable domain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NOs: 374, 375 or 376.

For one embodiment, the sequences of two FN3 based ABP, with the individual Ras binding loops and the amino acid differences are seen in the Table 5 below:

**Table 5. Amino acid sequences for PCC06 and PCC07**

| **Amino acid sequences for PCC06 and PCC07 FN3 binding loops (underlined) and the amino acids in direct contact with RAS (bold)** | | | |
|---|---|---|---|
| PCC06 = NS1_Crystal | GSVSSVPTKLEVVAATPTSLLISWDAPAVTVDYYVITYGETGGNSPVQKF EVPGSKSTATISGLKPGVDYTITVYAWGWHGQVYYYMGSPISINYRT | | |
| PCC07 = NS1_Patent | SSVPTKLEVVAATSLLISWDAPAVTVDYYVITYGETGGNSPVQKFEVPGS KSTATISGLKPGVDYTITVYAWGWHGQVYYYMGSPISINYRT | | |

| **Individual antigen binding loops of PCC06 and PCC07** | | | |
|---|---|---|---|
| BC | **DY**Y**V** | | |
| CD | GNSPV | | |
| DE | KF**E** | | |
| FG | **W**G**W**HG**QVYYY**MG | | |

| **Difference between PCC06 and PCC07 Amino acid inserts (grey box)** | | | |
|---|---|---|---|
| PCC06 = NS1_Crystal 97 amino acids | GSVSSVPTKLEVVAATPTSLLISWDAPAVTVDYYVITYGETGGNSPVQKFE VPGSKSTATISGLKPGVDYTITVYAWGWHGQVYYYMGSPISINYRT | | |
| PCC07 = NS1_Patent 92 amino acids | SSVPTKLEVVAATSLLISWDAPAVTVDYYVITYGETGGNSPVQKFEVPGS KSTATISGLKPGVDYTITVYAWGWHGQVYYYMGSPISINYRT | | |

| **Predicted binding sites in p21Ras for PCC06 and PCC07** | | | |
|---|---|---|---|
| | The α4-β6-α5 interface (amino acids 126 -167) | | |
| Kras4A | DTKQAQDLARSYGIPFIETSAKTRQRVEDAFYTLVREIRQYR | | |
| Kras4b | DTKQAQDLARSYGIPFIETSAKTRQGVDDAFYTLVREIRKHK | | |
| H-Ras | ESRQAQDLARSYGIPYIETSAKTRQGVEDAFYTLVREIRQHK | | |

Yet another embodiment pertains to the ABP according to the present invention, comprising a human protein or a part of a human protein with a known binding site to a Ras antigen, wherein the human protein is binding to Ras in its active and/or inactive state, preferably wherein the binding site is a direct binding site.

In one embodiment, the part of a human protein binding to an extracellular Ras antigen, preferably provided that said extracellular Ras antigen is not part of, or is not restricted to, a HLA-peptide complex, or specific HLA alleles presented on the cell surface, has an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from any one of SEQ ID NOs: 23, 24, 27, 28, 29, 30, 31, and 377 to 390. In one embodiment, these proteins are referred to as "alternative Ras binders".

In one embodiment, the ABP according to the present invention comprises an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from any one of SEQ ID NOs: 23, 24, 27, 28, 29, 30, 31 and 377 to 390.

A further embodiment pertains to the "alternative Ras binders" for ABP according to the present invention, wherein the ABP comprises at least one antigen binding domain capable of binding with one, two, three, four or preferable more than five amino acids to Ras antigen, wherein the at least one antigen binding domain alone or in a bipartite complex with another Ras binding protein, or in a tripartite complex with another Ras binding protein and the membrane, or in a multipartite complex with one or more Ras binding proteins and/or the membrane, and wherein said amino acids in the ABP optionally comprises:
(i) a domain comprising the RBD-CRD region (amino acids 52 to 188) of the human RAF1 protein (UniProt P04049) as set forth in SEQ ID NO: 23, optionally wherein one or multiple amino acids as depicted in Table A and B are in contact with KRAS residues, or wherein one or multiple amino acids as depicted in Table C are in contact with the membrane, or wherein one or multiple amino acids as depicted in Table D are in contact with KRAS residues in a tripartite complex comprised of RBD-CRD, KRAS and the membrane, or
(ii) a domain comprising the CDC25H region (amino acids 780 to 1019) of the human SOS1 protein (UniProt Q07889) as set forth in SEQ ID NO: 24, optionally wherein one or multiple amino acids as depicted in Table E are in contact with KRAS residues, or
(iii) a domain comprising the CDC25 region (amino acids 1038 to 1270) of the human RASGRF1 protein (UniProt Q13972) as set forth in SEQ ID NO: 29, optionally wherein one or multiple amino acids as depicted in Table F are in contact with Ras residues in a tripartite complex with Ras, Sos1 and RasGRF1, or
(iv) a domain comprising the RAS binding region (amino acids 274 to 364) of the human RASSF5 protein (UniProt Q8WWW0-1) as set forth in SEQ ID NO: 377, optionally wherein one or multiple amino acids are in contact with RAS residues, or
(v) a domain comprising the RAS binding region (amino acids 201 to 363) of a splice variant of the human RASSF5 protein (UniProt Q8WWW0-2) as set forth in SEQ ID NO: 378, optionally wherein one or multiple amino acids are in contact with RAS residues, or
(vi) a domain comprising the RAS binding region (amino acids 201 to 363) of the human RASSF1 protein (UniProt Q9NS23-1) as set forth in SEQ ID NO: 379, optionally wherein one or multiple amino acids are in contact with RAS residues, or
(vii)a domain comprising the RAS binding region (amino acids 176 to 264) of the human RASSF2 protein (UniProt P50749-1) as set forth in SEQ ID NO: 380, optionally wherein one or multiple amino acids are in contact with RAS residues, or
(viii) a domain comprising the RAS binding region (amino acids 6 to 89) of the human RASSF7 protein (UniProt Q02833-1) as set forth in SEQ ID NO: 381, optionally wherein one or multiple amino acids are in contact with RAS residues, or
(ix) a domain comprising the RAS binding region (amino acids 19 to 91) of the human ARAF protein (UniProt P10398-1) as set forth in SEQ ID NO: 382, optionally wherein one or multiple amino acids are in contact with RAS residues, or
(x) a domain comprising the RAS binding region (amino acids 19 to 148) of the human ARAF protein (UniProt P10398-1) as set forth in SEQ ID NO: 383, optionally wherein one or multiple amino acids are in contact with RAS residues, or
(xi) a domain comprising the RAS binding region (amino acids 151 to 232) of the human BRAF protein (UniProt P15056) as set forth in SEQ ID NO: 384, optionally wherein one or multiple amino acids are in contact with RAS residues, or
(xii)a domain comprising the RAS binding region (amino acids 151 to 320) of the human BRAF protein (UniProt P15056) as set forth in SEQ ID NO: 385, optionally wherein one or multiple amino acids are in contact with RAS residues, or
(xiii) a domain comprising the RAS binding region (amino acids 56 to 131) of the human RAF1 protein (UniProt P04049-1) as set forth in SEQ ID NO: 386, optionally wherein one or multiple amino acids are in contact with RAS residues, or
(xiv) a domain comprising the RAS binding region (amino acids 1235 to 1451) of the human NF1 protein (UniProt P21359-1) as set forth in SEQ ID NO: 387, optionally wherein one or multiple amino acids are in contact with RAS residues, or
(xv) a domain comprising the RAS binding region (amino acids 748 to 942) of the human RASA1 protein (UniProt P20936-1) as set forth in SEQ ID NO: 388, optionally wherein one or multiple amino acids are in contact with RAS residues, or
(xvi) a domain comprising the RAS binding region (amino acids 302 to 512) of the human RASA4 protein (UniProt 043374-1) as set forth in SEQ ID NO: 389, optionally wherein one or multiple amino acids are in contact with RAS residues, or
(xvii) a domain comprising the RAS binding region (amino acids 938 to 1130) of the human RGS12 protein (UniProt 014924-1) as set forth in SEQ ID NO: 390, optionally wherein one or multiple amino acids are in contact with RAS residues.

Tables A to F (from PCT/EP2024/065774, the disclosure of which is hereby incorporated by reference in its entirety.) show amino acids of an ABP according to the present invention, which can bind Ras (obtained from crystal structures).

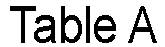

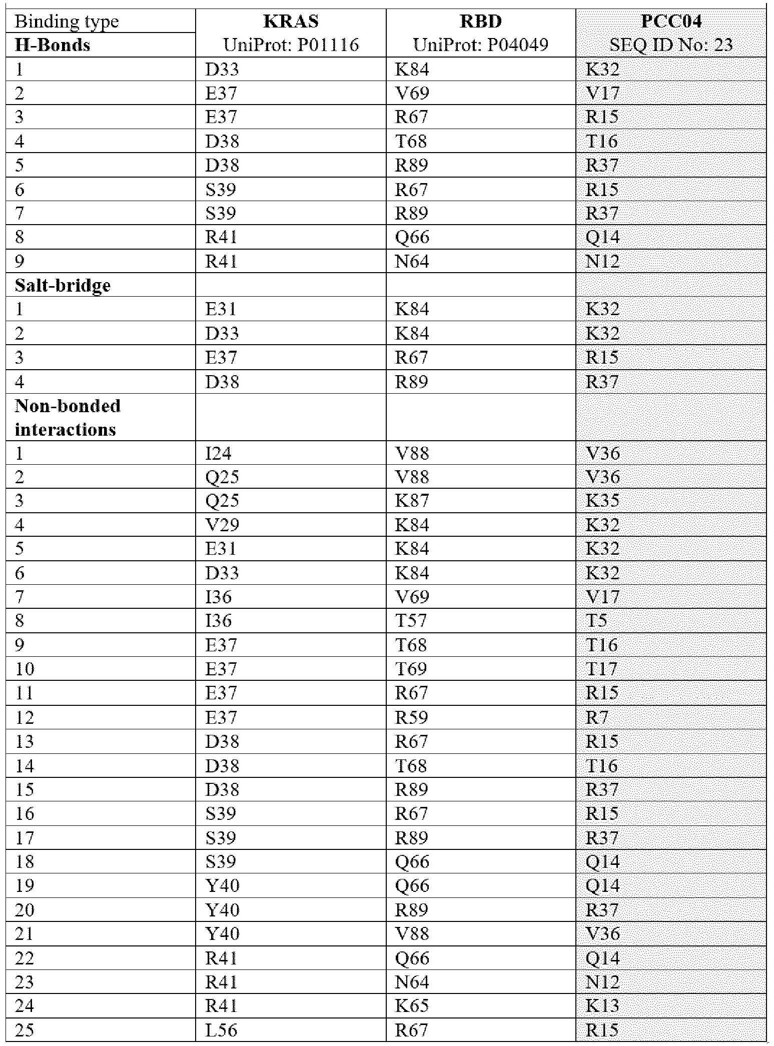

Table A shows the amino acids interactions present at the bipartite complex KRAS-RAF1(RBDCRD) for the interface KRAS - RBD from a crystal structure, the corresponding amino acids in PCC04 (SEQ ID NO: 23) and the type of binding between the amino acids. The amino acids in KRAS involved are conserved across all four RAS isoforms (KRas4b, KRas4a, NRas, HRas).

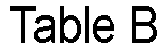

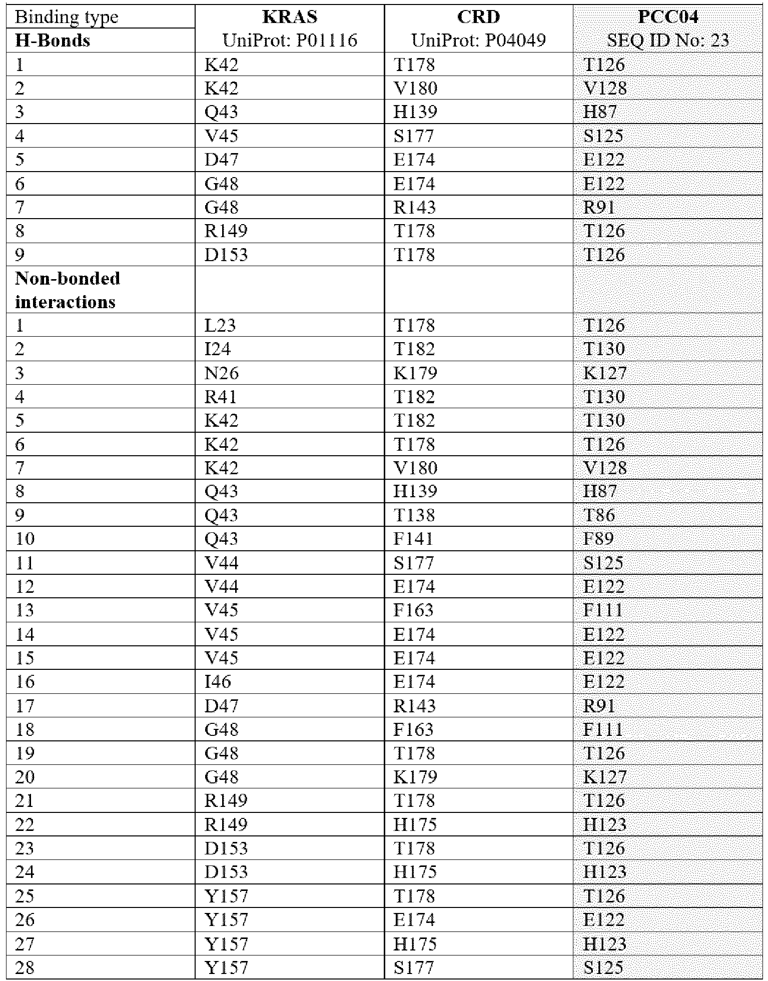

Table B shows the amino acids interactions present at the bipartite complex KRAS-RAF1(RBDCRD) for the interface KRAS - CRD from a crystal structure, the corresponding amino acids in PCC04 (SEQ ID NO: 23) and the type of binding between the amino acids. The amino acids in KRAS involved are conserved across all four RAS isoforms (KRas4b, KRas4a, NRas, HRas).

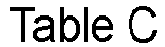

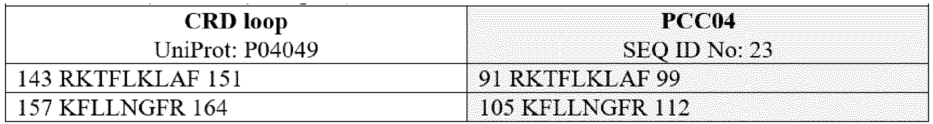

Table C shows the amino acids present at the membrane interaction of CRD loop residues in the structure of KRAS-RAF1(RBDCRD) complex, and the corresponding amino acids in the PCC04 sequence (SEQ ID NO: 23).

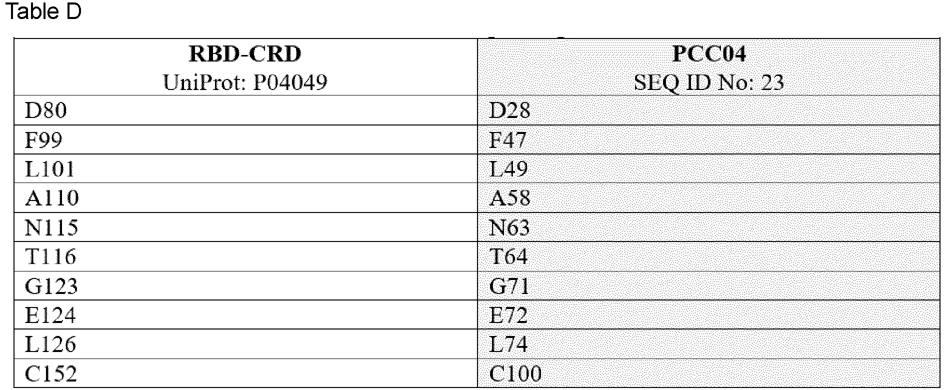

Table D shows the amino acids interactions present at the tripartite complex comprised of RBD-CRD, KRAS and the membrane (Nanodisc), and the corresponding amino acids in the PCC04 sequence (SEQ ID NO: 23).

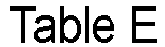

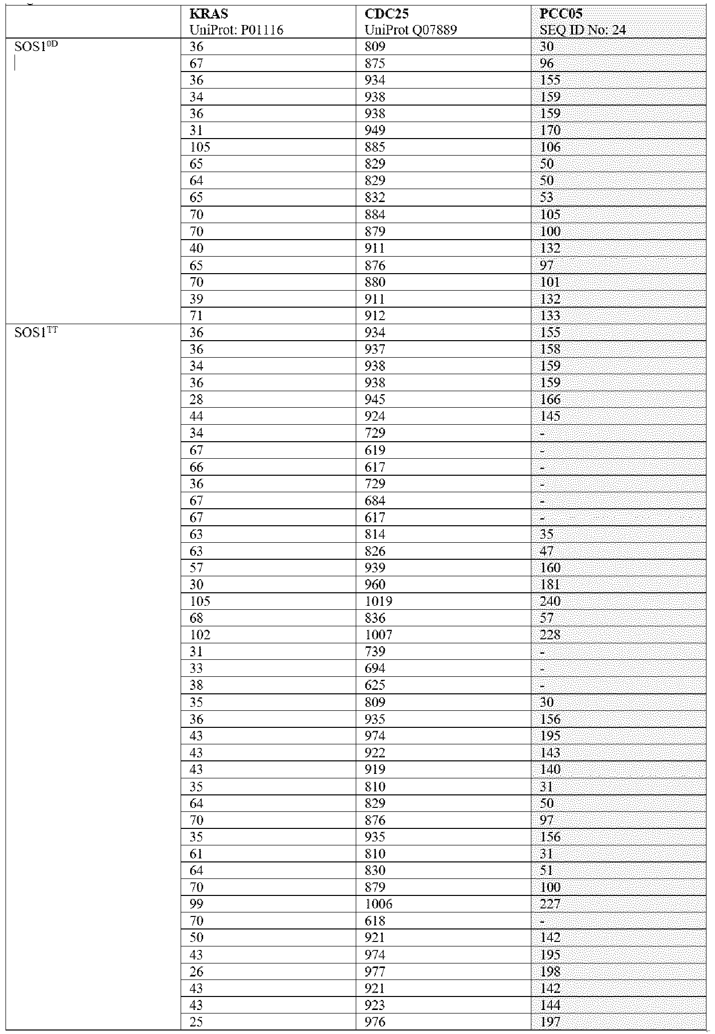

Table E shows the intermolecular interacting residue pairs between SOS1 and Kras4B, for the CDC25 domain and RAS with GTP- and/or GDP bound to from a crystal structure. Here, "T" and "D" denote GTP and GDP. SOS1^{TD} denotes GTP- and GDP-bound KRas4B interacting with SOS1 at the REM allosteric and CDC25 catalytic sites, respectively. The corresponding amino acids in the PCC05 binder (SEQ ID NOs: 24) are included, the amino acids not part of the PCC05 sequence (-).

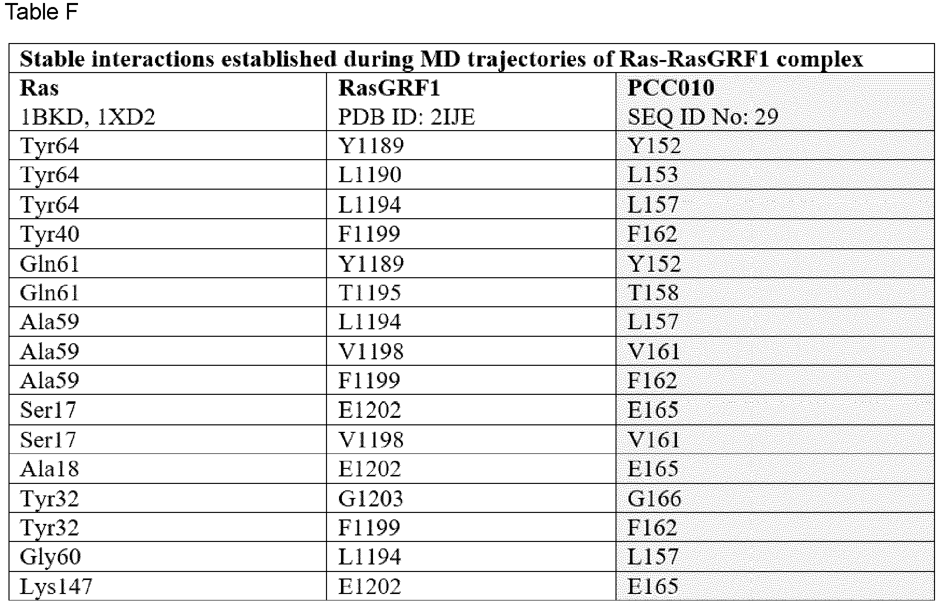

Table F shows the hot-spot amino acids in the Ras-RasGRF1 complex important for a stable interaction using a Molecular Dynamics (MD) simulation. Of note, the numbering for the RasGRF1 residues in the original report is 11 numbers shorter as the corresponding numbers in UniProt (Q13972), but the amino acid sequence is the same [8]. The RasGRF1 numbers in this Figure are corrected for the UniProt numbers. The corresponding amino acids in the PCC010 binder (SEQ ID NOs: 29) are included.

A further particularly preferred embodiment pertains to the ABP according to the present invention, wherein the ABP comprises at least an antigen binding domain capable of binding to an extracellular Ras antigen, preferably provided that said extracellular Ras antigen is not part of, or is not restricted to, a HLA-peptide complex, or specific HLA alleles presented on the cell surface, and wherein said Ras binding ABP optionally comprises a diabody format set forth in SEQ ID NO: 228 to 239, wherein in each case independently comprise a sequence having no more than three or two, preferably no more than one amino acid substitution(s), deletion(s) or insertion(s) compared to SEQ ID NO: 228 to 239, respectively; or comprising a sequence having at least 75% sequence identity, preferably at least 80% sequence identity, more preferably at least 90% sequence identity, and most preferably at last 95% sequence identity with SEQ ID NO: 228 to 239.

In another embodiment, the ABP is a Chimeric Antigen Receptor (CAR) comprising from N-terminus to C-terminus (i) an extracellular domain comprising the at least first antigen binding domain, (ii) an extracellular hinge domain, (iii) a transmembrane domain, and (iv) a cytoplasmic domain, optionally wherein the ABP is a CAR which is expressed by a T cell (CAR T cell).

Optionally, the extracellular hinge domain, the transmembrane domain and the cytoplasmic domain comprise the amino acid sequence having a sequence identity of at least 70%, preferably at least 80%, more preferably at least 90%, and most preferably at last 95% sequence identity, to an amino acid sequence selected from SEQ ID NO: 72 or, in each case independently, optionally with no more than ten, nine, eight, seven, six, five, four, preferably no more than three, two or one, amino acid substitution(s), insertion(s) or deletion(s) compared to this sequence.

In a preferred embodiment, the CAR according to the present invention specifically binds to an extracellular Ras antigen according to the present invention.

In another embodiment, the CAR molecule according to the present invention is a CAR molecule of the first, second, third, fourth, fifth or next generation, optionally comprising one additional antigen binding domain, such as an scFv antigen binding domain, VHH, DARPIN, or variable NAR-domain.

In one embodiment, the CAR according to the present invention is a dual-antigen CAR.

In one embodiment, when the CAR molecule is expressed in a T cell, such as a CD3+ T cell or a CD4+ T cell or CD8+ T cell, the T cell demonstrates activity against at least 2 different cancer types, at least 3 different cancer types, at least 4 different cancer types, at least 5 different cancer types, at least 6 different cancer types, at least 7 different cancer types, at least 8 different cancer types, at least 9 different cancer types, or at least 10 different cancer types.

Importantly, the novel anti-Ras directed immunotherapy of the present invention can be used for cell-based therapies like chimeric antigen receptors (CARs) transfected T cells for treating different types of Ras-dependent cancers. In one embodiment, CAR-T cells have activity against Ras tumor antigens.

In the present invention it is also intended that the combination comprises an isolated nucleic acid comprising a sequence encoding for an ABP according to the present invention. The ABP may also be a CAR.

According to a further embodiment, said isolated nucleic acid comprises a sequence encoding for an antigen binding fragment or a monomer, such as a heavy or light chain, of an ABP according to the present invention.

In one embodiment, the isolated amino acid relates to the amino acid sequence of SEQ ID NO. 72 or their complementary sequences or sequences that have at least 95 % sequence identity with said sequence.

The isolated nucleic acid may be comprised in an expression construct, preferably further comprising promoter and/or terminator sequences for expressing the ABP according to the present invention. The isolated nucleic acid and/or the expression construct may be comprised in a recombinant (host) cell.

According to a further embodiment, said expression construct is for expressing an antigen binding fragment or a monomer, such as a heavy or light chain, of an ABP according to the present invention, or for a CAR according to the present invention.

In one embodiment, the ABPs according to the present invention have protein linkers connecting the different Ras binding domains, connecting the Ras binding domains with the antibody constant domains, connecting the Ras binding domain with the CAR hinge or transmembrane domain, or connecting the detection and/or purification tags with the ABP.

In yet another embodiment, the protein linker comprises an amino acid sequence having a sequence identity of at least 70%, preferably at least 80%, more preferably at least 90%, and most preferably at last 95% sequence identity, to an amino acid sequence selected from SEQ ID NO: 34, 35, 36, 37, 38, 39, 40, or 41, or, in each case independently, optionally with no more than ten, nine, eight, seven, six, five, four, preferably no more than three, two or one, amino acid substitution(s), insertion(s) or deletion(s) compared to these sequences.

Four exemplary protein linkers between the Ig constant domain and Ras binders have an amino acid sequence selected from:
SEQ ID NO 36: GA
SEQ ID NO 37: AS
SEQ ID NO 38: GS
SEQ ID NO 39: GG

Of note, a protein linker comprising the amino acid sequence of SEQ ID NO 38 (GS) can also be a protein linker between a His tag and Ras binders.

In one embodiment, the ABP according to the present invention, has one, two, or more protein tags for detection and/or purification.

In a further embodiment, the protein tag is a His tag and/or a CL7 tag.

In yet another embodiment, the protein tag comprises an amino acid sequence having a sequence identity of at least 70%, preferably at least 80%, more preferably at least 90%, and most preferably at last 95% sequence identity, to an amino acid sequence selected from SEQ ID NO: 32, 33, 45, 46 or, in each case independently, optionally with no more than ten, nine, eight, seven, six, five, four, preferably no more than three, two or one, amino acid substitution(s), insertion(s) or deletion(s) compared to these sequences.

In one embodiment, the protein tag is a protein tag for detection and/or purification, wherein the protein tag has a specific proteolytic cleavage site to be cut off in the production and purification process from the final ABP.

In another embodiment, the proteolytic cleavage site comprises an amino acid sequence having a sequence identity of at least 70%, preferably at least 80%, more preferably at least 90%, and most preferably at last 95% sequence identity, to an amino acid sequence selected from SEQ ID NO: 47 or, in each case independently, optionally with no more than ten, nine, eight, seven, six, five, four, preferably no more than three, two or one, amino acid substitution(s), insertion(s) or deletion(s) compared to these sequences.

In one embodiment, the ABP according to the present invention, has two tags.

In another embodiment, the ABP according to the present invention, has a His tag and a CL7 tag.

In yet another embodiment, the expression construct encoding for the ABP according to the present invention, has a His tag and a CL7 tag.

In one embodiment, the CL7 tag is cleaved during the final purification of the ABP according to the present invention, and one, two, three, four, five, six, seven, eight, nine, or ten amino acids remain on the N-terminus on the ABP according to the present invention. In a preferred embodiment, the CL7 tag is cleaved during the final purification of the ABP according to the present invention, and two amino acids remain on the N-terminus on the ABP according to the present invention.

In yet another embodiment, the His tag of the expression construct is also present in the ABP according to the present invention, preferably the antibody according to the present invention. The His tag can be used for the detection of the ABP according to the present invention in serum or tissue.

In one embodiment, the ABP according to the present invention, or the CAR according to the present invention, have a protein secretion leader to express the CAR on the cell surface and/or for extracellular secretion.

In another embodiment, the protein secretion leader has an amino acid sequence having a sequence identity of at least 70%, preferably at least 80%, more preferably at least 90%, and most preferably at last 95% sequence identity, to an amino acid sequence selected from SEQ ID NO: 44 or, in each case independently, optionally with no more than ten, nine, eight, seven, six, five, four, preferably no more than three, two or one, amino acid substitution(s), insertion(s) or deletion(s) compared to these sequences.

The present invention further pertains to a nucleic acid construct (NAC) comprising an isolated nucleic acid according to the present invention and one or more additional sequence features permitting the expression of the encoded ABP, or a component of said ABP (such as an antibody heavy chain or light chain) in a (host) cell.

The invention also relates to a recombinant host cell expressing an ABP according to the present invention, or comprising an isolated nucleic acid according to the present invention, or an expression construct according to the present invention, which is preferably selected from effector cells of the immune system, such as lymphocytes, for example CD8 positive cytotoxic lymphocytes, CD4 positive T cells, T helper cells, or Th17 T cells, natural killer (NK) cells, natural killer T (NKT) cells, dendritic cells, killer dendritic cells, B cells, γδ T cells, and a lymphocyte preparation containing NK cells and NKT mast cells. Thus, the present invention further pertains to a host cell transformed with an isolated nucleic acid according to the present invention, or an expression construct according to the present invention.

In a preferred embodiment, the recombinant host cell expresses an ABP capable of binding a Ras antigen, wherein the Ras antigen comprises, preferably consists of, the amino acid sequence according to any one of SEQ ID NOs: 1, 3, 5, 7, 9, 11 to 19, 77 to 119, or 179 to 223, or comprises, preferably consists of, a sequence having at least 75% sequence identity, preferably at least 80%, more preferably at least 90%, and most preferably at least 95% sequence identity with any one of SEQ ID NOs: 1, 3, 5, 7, 9, 11 to 19, 77 to 119, or 179 to 223.

In one example, said recombinant host cell is a Chinese Hamster Overay (CHO) cell, a Human Embryonic Kidney (HEK) cell, a Pichia pastoris cell, a Saccharomyces cerevisiae cell, a Hansenula polymorpha cell, a Schizosaccharomyces pombe cell, a Leishmania tarentolae cell, a Spodoptera frugiperda cell, or a Trichopulsia ni High Five cell.

In one example, said recombinant host is a prokaryote cell, is an Escherichia coli cell, a Caulobacter crescentus cell, a Lactobacillus zeae cell, a Lactococcus lactis cell, a Bacillus brevis cell, a Bacillus subtilis cell, a Bacillus megaterium cell, a Caulobacter crescentus cell, or a Corynebacterium species cell like a Corynebacterium glutamicum cell.

A further embodiment pertains to a therapeutically effective amount of the above recombinant host cell for use in the treatment of a proliferative disorder, such as cancer, and/or for use in adoptive, target-cell specific immunotherapy, in a subject in need thereof. Preferably, the cancer is selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

Yet another embodiment of the present invention pertains to an engineered immune cell (genetically modified cell) expressing the ABP, wherein preferably the engineered immune cell is selected from the group consisting of a cytotoxic T cell, a helper T cell, a natural killer T cell, a γδT cell, and a NK cell. In a preferred embodiment, the cytotoxic T cell is a CD4+ T cell, a CD8+ T cell, a gamma delta T cell, or a natural killer cell.

The present invention also relates to a pharmaceutical composition comprising: (i) an ABP according to the present invention, or (ii) an isolated nucleic acid according to the present invention, or (iii) an expression construct according to the present invention, or (iv) a recombinant host cell according to the present invention, and a pharmaceutically acceptable carrier, stabilizer and/or excipient.

The present invention also relates to the inventive combination for use in a method of diagnosis, prevention and/or treatment of a proliferative disorder, such as cancer, and/or for use in adoptive, target-cell specific immunotherapy and/or for use in a method of diagnosis, prevention and/or treatment of a non-malignant disease. Preferably, the cancer is selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

In a preferred embodiment, the ABPs of the present invention used in the combination or alone are bispecific antibodies for use in the treatment of different types of cancer, such as cancer characterized by (a) mutated Ras gene(s), or cancers with no mutation in Ras gene(s) but with increased activation of the Ras signaling cascade(s).

In a preferred embodiment, the therapeutic compounds of the present invention are tri-specific or multi-specific antibodies for use in the treatment of different types of cancer, such as cancer characterized by (a) mutated Ras gene(s), or cancers with no mutation in Ras gene(s) but with increased activation of the Ras signaling cascade(s).

Importantly, target-cell specific immunotherapy is a very promising treatment strategy for, e.g., the treatment of cancer. When using target-cell specific immunotherapy, endogenous cells of the patient are used to destroy cancer cells via induction of an immune response, whereas treatment strategies for cancer not based on immunotherapy often use external compounds to destroy cancer cells. It is often difficult to control targeting using such external compounds, and such treatment strategies, therefore, risk harming healthy cells. Therefore, target-cell specific immunotherapeutic treatment strategies are highly advantageous over the use of external compounds.

In a preferred embodiment, the cancer is metastatic, stage III cancer, or stage IV cancer, optionally wherein the cancer is a Ras-dependent cancer.

In another embodiment, which can be combined with any aspect and any other embodiment of the present invention, the compounds or combinations of the present invention are for use in the diagnosis, prevention and/or treatment of RASopathies. RASopathies are a heterogenic group of developmental conditions, in which germline mutations in the Ras gene induce an overactivation of the RAS-MAPK signaling cascade. The result of an overactivation of the RAS-MAPK signaling cascade can be a malformation of the heart or other organs, an impairment of growth or developmental defects, and an enhanced risk for cancer.

In a preferred embodiment, which can be combined with any aspect and any other embodiment of the present invention, a disorder to be diagnosed, prevented and/or treated by any of the compounds of the present invention is a RAS driven disorder. Thus, the present invention provides strategies to target various RAS driven disorders with ABPs from the extra cellular site.
[2] In one embodiment, the combination according to the present invention or the ABP of the present invention is for use in modulating a cell-mediated immune response in a subject, optionally wherein the modulating the immune response is an inhibition of a cell-mediated immune response.

The inhibition of a cell-mediated immune response may be an inhibition of proliferation of an immune cell, such as a lymphocyte, and/or is an inhibition of cytokine expression in an immune cell, such as a lymphocyte.

The inhibition of a cell mediated immune response may be a reduction of proliferation/activity of effector memory T cells and/or increase of proliferation/activity of regulatory T cells (TREGs).

Another aspect relates to a diagnostic use of the combination or ABPs of the present invention to detect cells aberrantly expressing Ras protein on the surface of cells *in vitro* (for example by ELISA) or *in vivo* (for example by PET-CT with a Ras ligand) or *in vitro* by novel methods as disclosed *infra.*

A further aspect of the present invention relates to a method for the diagnosis, prevention and/or treatment of a proliferative disorder, such as cancer, in a subject, comprising the administration of a therapeutically effective amount of a combination according to the present invention, an ABP according to the present invention, an isolated nucleic acid according to the present invention, an expression construct according to the present invention, a recombinant host cell according to the present invention, or a pharmaceutical composition according to the present invention, to the subject, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3).

One particularly preferred example of a cancer to be treated is NEC-G3 of the gastrointestinal tract or small cell lung cancer (NEC-G3 lung carcinoma).

A further aspect of the present invention relates to the use of a combination according to the present invention or an ABP according to the present invention, an isolated nucleic acid according to the present invention, an expression construct according to the present invention, a recombinant host cell according to the present invention, or a pharmaceutical composition according to the present invention for the manufacture of a medicament for the treatment of a proliferative disorder, such as cancer, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

Yet another aspect of the present invention pertains to the use of a combination according to the present invention, an ABP according to the present invention, an isolated nucleic acid according to the present invention, an expression construct according to the present invention, a recombinant host cell according to the present invention, or a pharmaceutical composition for generating target-specific effector cells, in particular for generating cytotoxic T cells, or for killing tumor cells

Yet another aspect of the present invention relates to a method of modulating a cell-mediated immune response in a human cell that expresses an extracellular Ras antigen, comprising contacting said cell with a combination according to the present invention or an ABP according to the present invention, an isolated nucleic acid according to the present invention, an expression construct according to the present invention, a recombinant host cell according to the present invention, or a pharmaceutical composition according to the present invention, in the presence of an immune cell, such as a T-cell, thereby modulating, preferably enhancing, the cell-mediated immune response.

ABPs, preferably diabodies, can also have an effect on healthy cells. Thus, an ABP is provided for use in a method of diagnosis, prevention and/or treatment of a non-malignant disease. For example, healthy cells can proliferate upon treatment with ABCs of the present invention. This effect can be used in the field of regenerative medicine, for example. It can also be used in a method for enhancing wound healing in the treatment of aging or in lifestyle medicine.

The expression of functional extracellular Ras protein on non-malign cells opens new fields for use in humans or animals like for example: (i) Treating other disease than cancer like metabolic disease, hypercholesterinemia, hypertriglyceridemia, adrenoleukodystrophy, Diabetes type 1 or 2, Gaucher disease, Hereditary hemochromatosis, Lesch-Nyhan syndrome, obesity, acid-base imbalance, metabolic brain diseases, disorders of sodium, calcium or potassium metabolism, kidney disease, DNA repair-deficiency disorders, glucose metabolism disorders other than diabetes, hyperlactatemia, iron metabolism disorders, lipid metabolism disorders, malabsorption syndromes, metabolic syndrome X, inborn error of metabolism, mitochondrial diseases, phosphorus metabolism disorders, porphyrias, proteostasis deficiencies, metabolic skin diseases, wasting syndrome, chronic fatigue syndrome, water-electrolyte imbalance, wound healing or aging. (ii) Treat auto-immune disease of the integumentary system, of the digestive system, of the heart and vascular system, of the urinary system, of the nervous system, of the endocrine system, of the respiratory system, of the blood, of the reproductive system, of the eye, of the musculoskeletal system or to treat a combination of two or more of these systems. (iii) Treating neurological disorders like dementia of Alzheimer form, vascular dementia, Lewy body dementias, younger onset dementia, frontotemporal dementia, alcohol-related brain injury, virus infection associated dementia, chronic traumatic encephalopathy dementia. (iv) Treating forms of depression like major depression, persistent depressive disorders (formerly called dysthymia), bipolar disorder, seasonal affective disorder, perinatal depression or premenstrual dysphoric disorder. (v) Treating disease of the heart or blood vessels like systolic heart failure, diastolic heart failure, right-sided heart failure, congestive heart failure, mixed heart failure, heart failure with reduced ejection fraction, heart failure with preserved ejection fraction, coronary heart disease, myocardial infarction (MI), myocardial remodeling after MI, genetic cardiomyopathy, heart valve disease, arterial hypertension, pulmonary hypertension or myocardial arrhythmias. (vi) To be used for chronic liver disease like alcoholic liver disease, non-alcoholic fatty liver disease, chronic viral hepatitis, alpha-1 antitrypsin deficiency, liver cirrhosis, primary biliary cirrhosis, primary sclerosing cholangitis or autoimmune hepatitis. (vii) To be used for chronic kidney disease like atypical hemolytic uremic syndrome, Alport syndrome, amyloidosis, APOL1-mediated kidney disease, cardiovascular-kidney-metabolic syndrome, complement 3 glomerulopathy (C3G), congenital abnormalities of the kidneys and urinary tract, kidney cystinosis, Fabry disease, focal segmental glomerulosclerosis, glomerulonephritis, goodpasture syndrome, granulomatosis with polyangiitis, hemolytic uremic syndrome, IgA nephropathy, interstitial nephritis, lupus nephritis, minimal change kidney disease, polycystic kidney disease, primary hyperoxaluria and kidney vasculitis. (vii) To be used in hematologic disorders like sickle cell anemia, thalassemia intermedia or major, immunthrombocytopenia, paroxysmal nocturnal hemoglobinuria or hemolytic anemias. (viii) To be used for fertility treatment. (ix) To be used in diagnostics of non-cancer related disease. (x) To be used *in vitro* to stimulate or protect eukaryote cells in cell culture.

ABPs can also activate or stimulate cells to proliferate. Using cells with a reporter plasmid and/or vector may then be used to detect and measure the activation of specific signaling cascades, for example to measure cell viability or activation, the latter for example with a reporter plasmid and/or vector resulting in light emission after activation.

### Diabodies:

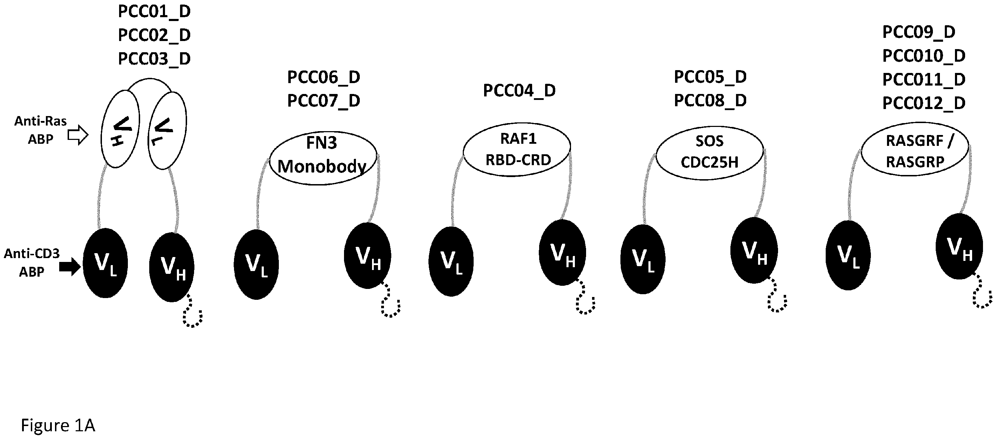

In one preferred embodiment, the ABP is a diabody. Twelve different bi-specific T cell recruiting Diabodies had been designed previously (Figure 1A; PCC01_D - PCC012_D; SEQ ID NOs: 228 to 239). Diabodies are small single-chain bi-specific antibodies. All 12 diabodies have shown high *in vitro* killing activity against tumor cells.

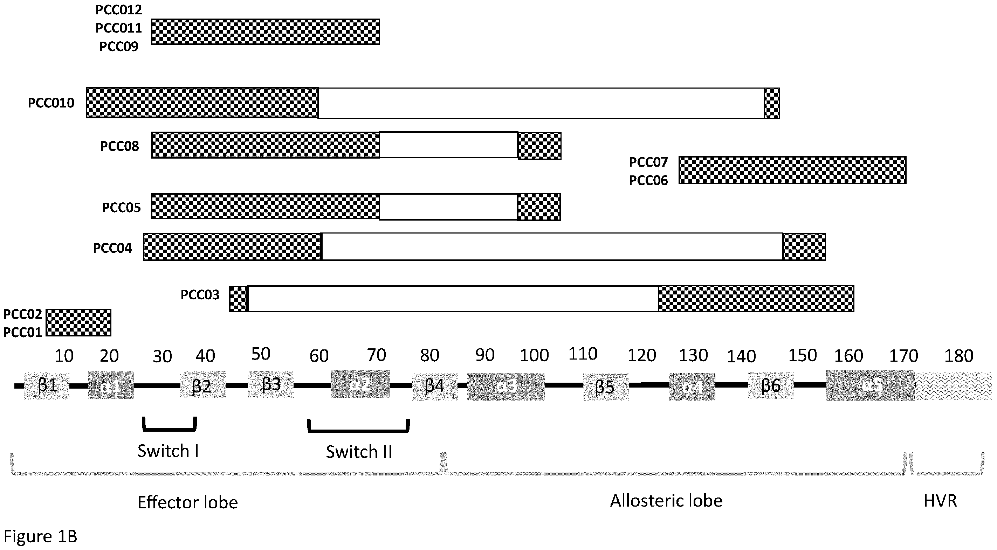

The predicted binding sites are shown in Figure 1B. The original scFv sequences for PCC01 (G12V-34, SEQ ID NOs: 20, 66, 67, 122, 134 to 147, 228) and PCC02 (G13D-18, SEQ ID NOs: 21, 68, 69, 123, 148 to 160, 229) are from a murine scFv combinatorial phage display library, extracted from mice immunized with Ras mutant peptides [9]. The ScFv sequence (KGH-R1) used for PCC03 (SEQ ID NoNO: 22, 70, 71, 124, 161 to 174, 230) is from a murine scFv combinatorial phage display library, extracted from mice immunized with human wild-type K-Ras, N-Ras and H-Ras proteins [10]. The ScFv from clone KGH-R1 showed strong binding to human primary solid tumor tissues and its binding site with K-Ras, H-Ras and N-Ras was analyzed by molecular docking method [11]. The results showed that KGH-R1 ScFv binds to Ras proteins mainly through hydrogen bonding and salt bridges. The sequences for PCC06 (SEQ ID NOs: 25, 127, 175 to 178, 233) and PCC07 (SEQ ID NOs: 26, 128, 175 to 178, 234) are derivative from the same original monobody FN3 clone (NS1) [12,13]. The NS1 selectively binds both H-Ras and K-Ras and potently inhibits H-Ras and K-Ras mediated cell signaling and transformation. The NS1 was isolated from the "side-and-loop" combinatorial library constructed in the phage display format against the H-Ras, H-Ras/GDP and H-Ras/GTP proteins. The NS1 monobody is a fibronectin type III domain (FN3) binder. A 1.4 A-resolution crystal structure of NS1 in complex with GDP-loaded H-Ras revealed interaction of NS1 with the α4-β6-α5 region within the so-called allosteric lobe, which lie on the surface of Ras protein.

The sequence for PCC04 (SEQ ID NOs: 23 and 231) is the known Ras binding domain (RBD-CRD) from the human protein RAF1 (P04049). There are several crystal structure data for the complex of KRAS with RAF1 (RBD-CRD) [14,15]. The amino acids in KRAS involved in the binding are conserved across all four RAS isoforms (KRas4b, KRas4a, NRas, HRas). Of interest, the reported amino acids interactions present at the tripartite complex comprised of RBD-CRD, KRAS and the lipid membrane (artificial Nanodisc), are to some part different from the amino acids reported for the interactions between bipartite complex RBD-CRD and KRAS. This high flexibility of the RBD-CRD domain against different conformations of RAS is important. It is known for RAS to change its position and conformation in contact with the lipid membrane and bound signaling partners. Therefore, an ABP with the ability to bind different conformations of RAS at the cell membrane, will have an advantage over ABP targeting only a specific Ras conformation. This has been recently demonstrated for the present Ras targeting ABPs in the Diabody format, with the highest killing function for the PCC04 construct over the antibody and monobody based constructs (PCC01, PCC02, PCC03, PCC06 and PCC07) (PCT/EP2024/065774).

### 2D cell culture

2D cell culture models can for example be used to test the influence of compounds, such as a combination of an ABP and a modulator compound, on the viability of tumor cells. Thus, for example T cells and tumor cells are cultured together. Preferred cell ratios are: Effector to tumor (E:T) ratio 1:1, 1:3, 1:5, 1:10; 1:20 or 2:1, 3:1, 5:1, 10:1, 20:1, 50:1, 100:1

### 3D model

Different cell culture models can be used for testing anti-tumor activity of an ABP or a combination of the present invention. In one embodiment, a mix of human and animal cells is used. In another embodiment, only tumor cells are used. In another embodiment, tumor and effector cells, preferably T cells, NK cells or NKT cells, are used.

In order to better resemble the natural *in vivo* conditions, a new *in vitro* cell culture model was developed. In regular *in vitro* cell culture, tumor cells grow adherent, semi adherent or in suspension on the plastic surface in 2 dimensions (2D) (used in prior art PCT/EP2024/065774). In contrast, *in vivo* tumor cells grow in a complex tumor microenvironment (TME) in 3 dimensions. The importance of the TME is well known for tumor progression and has been shown to influence response to immune checkpoint blockade (ICB).

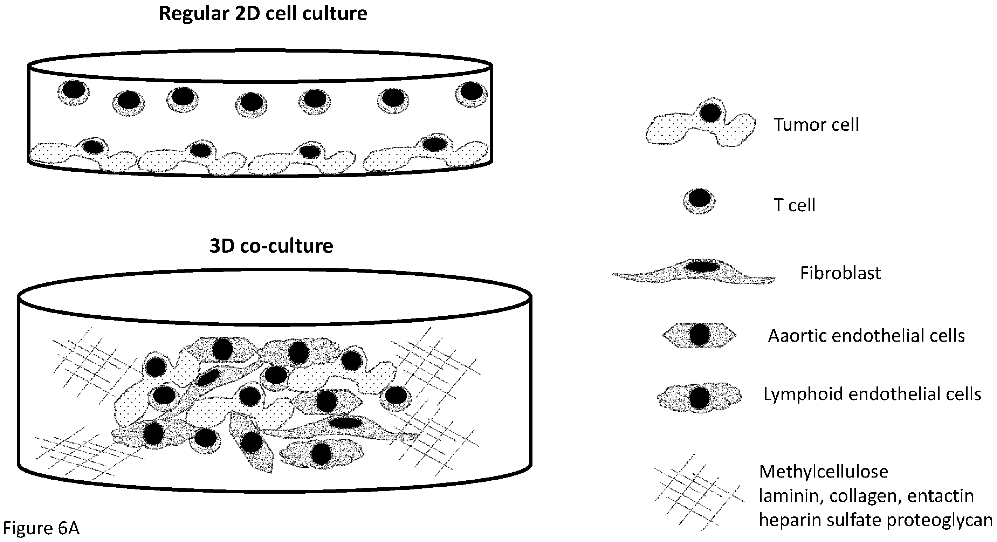

Thus, to study the effect of the TME on extracellular Ras targeting ABPs of the present invention, a new full-humanized *in vitro* co-culture assay resembling the complex human TME was developed (Figure 6A).

This co-culture model comprises tumor cells and at least one non-malignant cell selected from the group comprising or consisting of a fibroblast, an endothelial cell of a blood vessel, an endothelial cell of a lymphatic vessel, a tissue macrophage, a fat cell, an osteoblast, a chondrocyte, a smooth muscle cell, a preadipocyte, a pericyte, a mesenchymal stem cell, a melanocyte, a keratinocyte, hematopoietic progenitors, a dendritic cell, a skeletal muscle cell, a T cell and a B cell.

Preferred cell ratios are: Healthy to tumor (H:T) ratio 1:1, 1:3, 1:5, 1:10; 1:20, 1:50; 1:100 or 2:1, 3:1, 5:1, 7:1, 10:1, 15:1, 20:1, 30:1, 50:1, 100:1; 250:1, 500:1, 1000:1, 5000:1.

According to the present invention, an *in vitro* 3D cell culture model is provided comprising tumor cells and at least one non-malignant cell selected from the group comprising or consisting of a fibroblast, an endothelial cell of an arterial blood vessel, an endothelial cell of a venous blood vessel, an endothelial cell of a lymphatic vessel, a tissue macrophage, a fat cell, an osteoblast, a chondrocyte, a smooth muscle cell, a preadipocyte, a pericyte, a mesenchymal stem cell, a melanocyte, a keratinocyte, hematopoietic progenitors, a dendritic cell, a skeletal muscle cell, a T cell and a B cell, preferably wherein the at least non-malignant cell is a T cell, a fibroblast and an endothelial cell.

The *in vitro* 3D cell culture model preferably further comprises the combination according to the present invention or the ABP according to the present invention for testing the influence of the combination on the viability and extracellular Ras expression of the at least one tumor cell.

The *in vitro* 3D cell culture model may further comprise a cellulose, preferably methylcellulose, and/or Matrigel^{®}.

In one preferred embodiment, the *in vitro* 3D cell culture model comprises at least one tumor cell, at least one T cell, at least one fibroblast, at least one aortic endothelial cell, at least one lymphoid endothelial cell and a cellulose, preferably methylcellulose, and further comprises the combination according to the present invention for testing the influence of the combination on the viability and extracellular Ras expression of the at least one tumor cell.

Preferably, the cells are human cells.

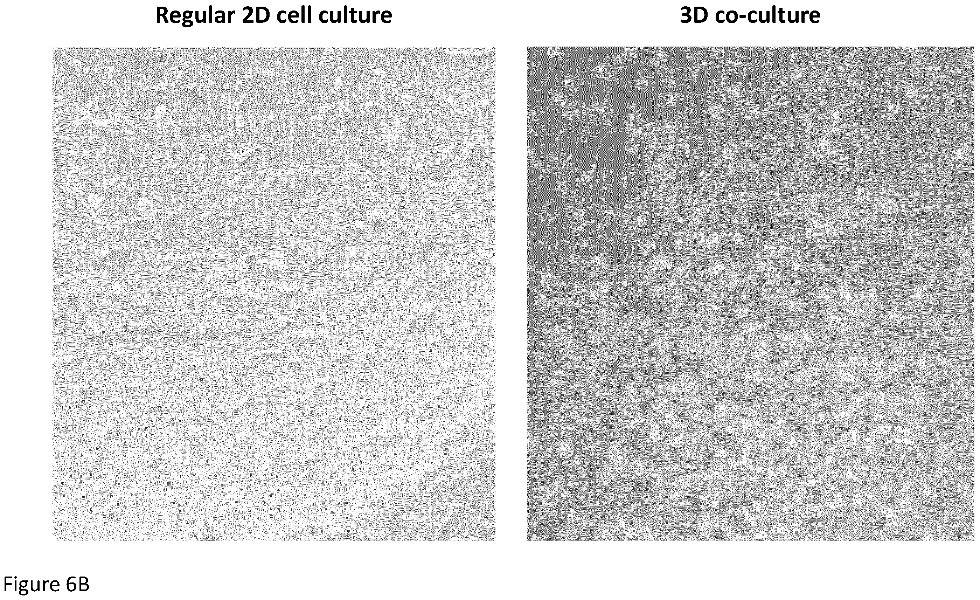

In contrast to regular cell culture, tumor cells preferably grow in the co-culture assay 3 dimensionally (3D), forming cluster of cells and spheroids (Figure 6B). This is advantageous because spheroids recapitulate important tumor features including cellular heterogeneity, cell signaling pathways, cell-cell and cell- extracellular matrix (ECM) interactions, gene expression patterns similar to those of *in vivo* conditions and a tumor morphology composed of different cell layers.

There is another major advantage of this co-culture assay. This is preferably a full-humanized assay with different types of preferably healthy human cells, resembling to some part the complex human TME. This is advantageous because some cytokines, ligands, receptors or hormones produced by the surrounding stroma cells and affecting the tumor progression and resistance, are not cross reactive between human and mice.

Further, the co-culture assay using 6-well, 24-well, 96-well or 384-well plates can easily be expanded to hundreds or thousands of plates. This is important to investigate new extracellular Ras targeting formats alone or in combination with large inhibitor libraries to screen for combination partners in a timely manner.

Moreover, this plate-based assay makes it possible to use a large variety of reagents and equipment using special plates (coated or not, transparent etc.), modified cells (reporter plasmids, gene knock-out or knock-in, resistant genes etc.), selected medium (supplements, pH, osmolality etc.), different oxygen conditions (from hypoxia to hyperoxia) and different analytic instruments (microscopy, luminescence or fluorescence sensor etc.). This is important to test and analyze the extracellular Ras expression, and the effect of modulating factors, in great detail.

Further, such an assay fulfills the "replace" requirements of the 3Rs principle (Re- place, Reduce, Refine) for animal testing. This is important to reduce the number of animals (mice) for this invention.

Co-culture assays, mimicking the complex TME *in vitro,* can for example be used for large-scale automated library screens for small molecule compounds (i.e. modulator compounds) or cellular-target genes modulating the extracellular Ras expression.

In a preferred embodiment, different concentrations of oxygen (O2) are used for cell incubation. From hyperbaric 99% to regular ambient air oxygen concentration of 20,942 %. Or from regular 20,942 % to hypoxemia 0.01%. Low oxygen concentrations are advantageous, because they better reflect the physiological oxygen concentration in the human body ranges from 13-14% in arterial blood to 5-8% in venous blood, lung, liver, kidney, placenta, and bone marrow, to 4% in the brain, 3% in skeletal muscle, and 0.5-4.5% in lymphoid organs.

In another preferred embodiment, cell media with a pH different from the body plasma physiological pH (7.35 to 7.45) are used. In the body the physiological pH is different in different tissues. In the TME the pH can change dramatically with tumor pH values ranging between 5.55 and 7.69. Cell culture media can be used for example with a basic pH in the range between 7.5 and 12.0. Or media with an acidic pH in the range between 7.3 and 1.0.

Different artificial models can for example be used for analyzing Ras protein expression on lipid membranes, binders against Ras proteins or binders against other cell targets modulating the Ras expression on lipid membranes. For example, artificial lipid based membranes or artificial cells may be used. These could include one or more of polymersomes, proteinosomes, colloidosomes, liposomes, micelles, lipid membrane discs as well as membrane-vesicles.

### Modulator compounds

Modulator compounds used in combinations of the present invention are for example small molecule inhibitors (SMI) or fatty acids (lipids, also termed Lipid Mix). SMIs are preferably organic substances, preferably with a low molecular weight ≤ 1000 daltons, that interfere with specific molecules required for cell growth and function. Surprisingly, all combinations with different SMIs from different chemical families with different structures, different targets and different mechanisms of action had a significantly better anti-tumor efficacy than ABPs alone. The tests were done with:

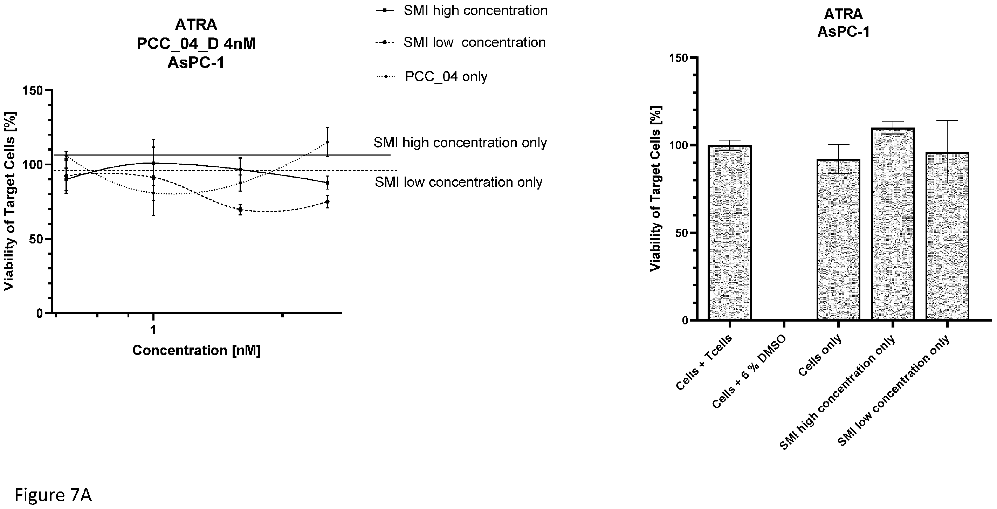

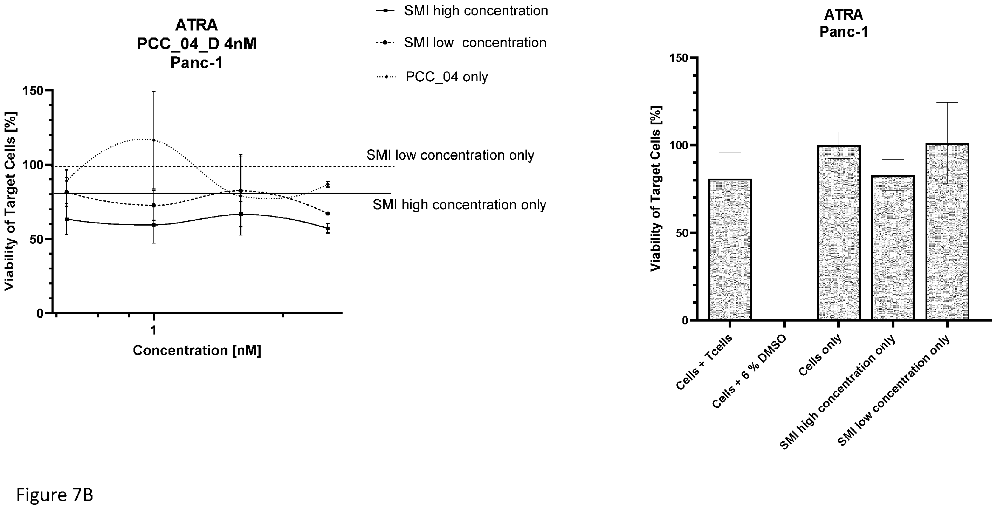

### 1. All-trans-retinoic acid (ATRA) based modulator compounds (Figure 7)

The compound ATRA (DrugBank ID DB00755) used in combination with an extracellular Ras directed ABP, is a metabolite of vitamin A (DrugBank ID DB00162) and mediates the functions of cell growth, differentiation and development mainly through modulating gene expression. ATRA is a natural agonist of RAR nuclear receptors, with IC50s of 14 nM for RARα/β/γ. Retinoic acid bind to PPARβ/δ with Kd of 17 nM. Retinoic acid acts as an inhibitor of transcription factor NFE2L2 through activation of retinoic acid receptor alpha.

Therefore, any compound inhibiting or modulating retinoid X receptors (RXRG Gene ID: 6258 UniProt P48443) (RXRB Gene ID: 6257 UniProt P28702) (RXRA Gene ID: 6256 UniProt P19793) and/or retinoic acid receptors (RARA Gene ID: 5914, UniProt P10276) (RARG Gene ID: 5916, UniProt P13631) (RARB Gene ID: 5915, UniProt 10826) and/or cellular retinoic acid-binding proteins (CRABP2 Gene ID: 1382, UniProt P29373) (CRABP1 Gene ID: 1381, UniProt P29762) and/or NFE2L2 (Gene ID: 4780, UniProt Q16236) can be used for this invention.

Examples for ATRA analogues are shown in Liang, C. et al. (2021) Overview of all-trans-retinoic acid (ATRA) and its analogues: Structures, activities, and mechanisms in acute promyelocytic leukaemia Eur J Med Chem. 220:113451: Metabolites of ATRA, hydrophobic unit-modified analogues, compounds with imidazole modifications in the hydrophobic part, compounds obtained by modifying the hydrophobic part of the molecule with adamantane, compounds which were obtained by replacing the hydrophobic part by a 4-methoxy-2,3,6-trimethylbenzene ring, a compound which was obtained by replacing the trimethyl cyclohexenyl ring of 9-cis-retinoic acid with a tetra-hydronaphthone ring, compounds obtained by connecting 9Z tetraenoic acid with a disubstituted cyclohexene ring, compounds with polar terminus modified analogues, Compounds whose terminal positions are substituted or fused with different groups, compounds with linker unit-modified analogues, compounds with receptor functional analogues, compounds with RARβ selectivity or compounds with RXR selectivity.

As alternative, compounds inducing transcriptional activation and/or inhibition of cell differentiation-related genes, or compounds regulating one or more genes affected by ATRA in acute promyelocytic leukaemia (APL) cells (Table 6), can be used for this invention.

**Table 6: Genes up-regulated by ATRA in U937 cells (acute promyelocytic leukemia, APL)**

| **Gene ID** | **Gene symbol** | | **Gene ID** | **Gene symbol** |
|---|---|---|---|---|
| 2268 | FGR | | 638 | BIK |
| 6688 | SPI1 | | 9516 | LITAF |
| 1050 | CEBPA | | 10397 | NDRG1 |
| 1053 | CEBPE | | 7378 | UPP1 |
| 5585 | PKN1 | | 4046 | LSP1 |
| 7133 | TNFRSF1B | | 3417 | IDH1 |
| 6503 | SLA | | 5660 | PSAP |
| 5294 | PIK3CG | | 8870 | IER3 |
| 3659 | IRF1 | | 5292 | PIM1 |
| 241 | ALOX5AP | | 2000 | ELF4 |
| 7127 | TNFAIP2 | | 6518 | SLC2A5 |
| 3198 | HOXA1 | | 10507 | SEMA4D |
| 5916 | RARG | | 3727 | JUND |
| 3489 | IGFBP6 | | 952 | CD38 |
| 7150 | TOP1 | | | |

### 2. Modulator compounds of the cholesterol and triglyceride synthesis and metabolism (Figures 8-11)

The modulator compounds Simvastatin (DrugBank ID DB00641) (Figure 8), Fluvastatin (DrugBank ID DB01095) (Figure 9), Lapaquistat (DrugBank Accession Number DB16215) (Figure 10) and Bemfivastatin (PubChem CID 11192585, CAS number 805241-79-6) (Figure 11), used in combination with an extracellular Ras directed ABP, are all modulators or inhibitors of the cholesterol and/or fatty acid synthesis. Simvastatin, Fluvastatin and Bemfivastatin are all cholesterol lowering drugs and belong to the group of statins. Simvastatin and Fluvastatin are competitive inhibitors of HMG-CoA reductase (HMGCR) (Gene ID: 3156, UniProt P04035). Bemfivastatin is an HMG-CoA Reductase (HMGCR) inhibitor enhancing the activity of liver extraction. HMG-CoA Reductase (HMGCR) is the rate-limiting enzyme for cholesterol synthesis in the conversion of HMG-CoA to mevalonate. In contrast, Lapaquistat acetate is a squalene synthase FDFT1 (Gene ID: 2222, UniProt P37268) inhibitor for decreasing plasma cholesterol and triglyceride levels, by lowering lipoproteins containing apoB100. Squalene is further downstream in the synthesis of cholesterol compared to HMG-CoA reductase.

Therefore, any compound inhibiting or modulating the synthesis of fatty acids and/or cholesterol, cholesterol oxygenated derivatives and/or cholesterol conjugated derivatives can be used for this invention.

Examples are shown in Table 7.

**Table 7: Different inhibitors or modulators for HMG-CoA reductase (HMGCR) (Gene ID: 3156, UniProt P04035) or squalene (FDFT1 Gene ID: 2222, UniProt P37268) (SQLE Gene ID: 6713, UniProt Q14534).**

| **Name** | **CAS (Chemical Abstracts Service) number** |
|---|---|
| Simvastatin | 79902-63-9 |
| Lovastatin | 75330-75-5 |
| Atorvastatin | 134523-00-5 |
| Rosuvastatin Calcium | 147098-20-2 |
| Mevastatin | 73573-88-3 |
| Pitavastatin | 147511-69-1 |
| Fluvastatin | 93957-54-1 |
| SR12813 | 126411-39-0 |
| Meglutol | 503-49-1 |
| Monacolin J | 79952-42-4 |
| Bemfivastatin | 805241-79-6 |
| Clinofibrate | 30299-08-2 |
| HMG499 | 2416941-68-7 |
| Rosuvastatin | 287714-41-4 |
| β-Amyrin acetate | 1616-93-9 |
| Pravastatin | 81093-37-0 |
| S-2E | 155730-92-0 |
| L-157012 | 114801-28-4 |
| β-Amyrin palmitate | 5973-06-8 |
| Ganomycin I | 1191255-15-8 |
| QH536 | 2754254-07-2 |
| Crilvastatin | 120551-59-9 |
| L-669,262 | 130468-11-0 |
| Cerivastatin | 145599-86-6 |
| Rawsonol | 125111-69-5 |
| Diallyl disulfide | 2179-57-9 |
| Diallyl Trisulfide | 2050-87-5 |
| Allicin | 539-86-6 |
| FIN56 | 1083162-61-1 |
| Procyanidin B-5 3,3'-di-O-gallate | 106533-60-2 |
| Zaragozic acid D | 155179-14-9 |
| Liranaftate | 88678-31-3 |
| YM-53601 | 182959-33-7 |
| SQLE-IN-1 | 1019169-83-5 |
| Terbinafine | 91161-71-6 |
| FR194738 | 204067-52-7 |
| YM-75440 | 780736-74-5 |
| Zaragozic acid A | 142561-96-4 |
| Butenafine | 101828-21-1 |
| Alnusenone | 508-09-8 |
| BPH-1218 | 1426824-36-3 |
| NB-598 Maleate | 155294-62-5 |
| NB-598 | 131060-14-5 |

As alternative, any protein or protein complexes of the human cholesterol and/or lipid metabolism can be targeted with a modulating or inhibiting compound. Examples for these genes are squalene monooxygenase (SQLE) (Gene ID: 6713, UniProt Q14534), or mevalonate kinase (Gene ID: 4598, Q03426), or lanosterol synthase (Gene ID: 4047, UniProt P48449), or ACAT1 (Gene ID: 38, UniProt P24752), or HMGCS1 (Gene ID: 3157, UniProt Q01581), or GNPAT (Gene ID: 8443, UniProt 015228), or GPAM (Gene ID: 57678, UniProt Q9HCL2), or GPAT3 (Gene ID: 84803, UniProt Q53EU6), or AGPS (Gene ID: 8540, UniProt 000116), or PNPLA2 (Gene ID: 57104, UniProt Q96AD5), orACSS2 (Gene ID: 55902, Uniprot Q9NR19), or FASN (Gene ID: 2194, UniProt P49327) or SCD (Gene ID: 6319, UniProt O00767) or APOB (Gene ID: 338, UniProt P04114).

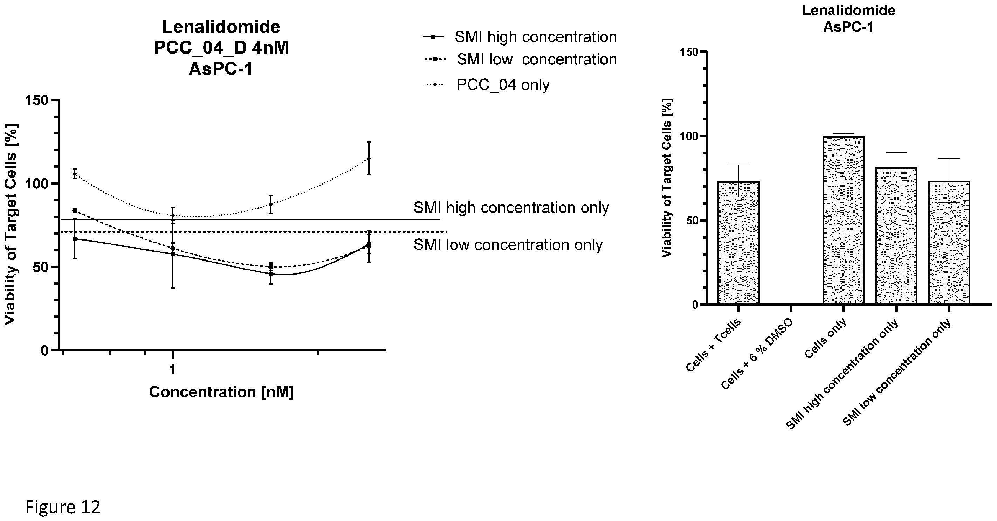

### 3. Immunomodulatory drugs (IMiDs) targeting cereblon (Figures 12-15)

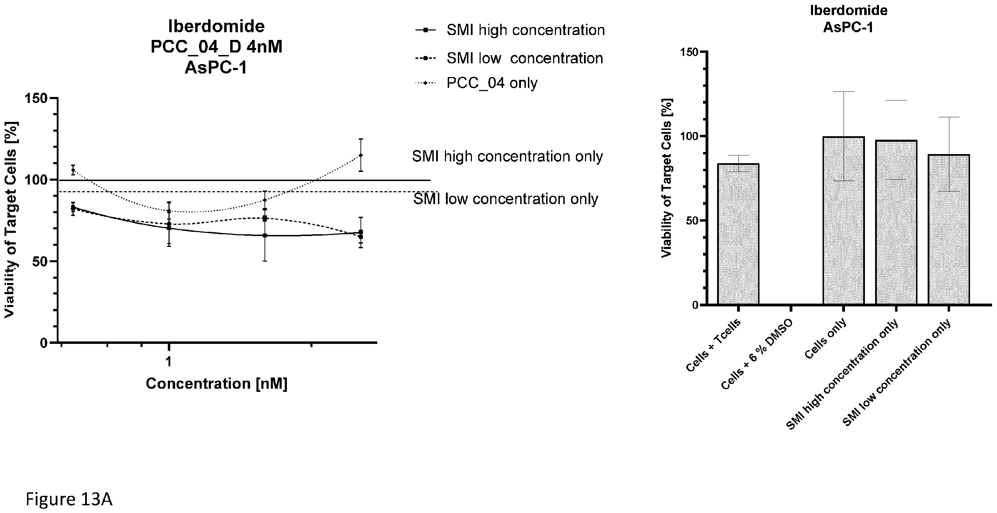

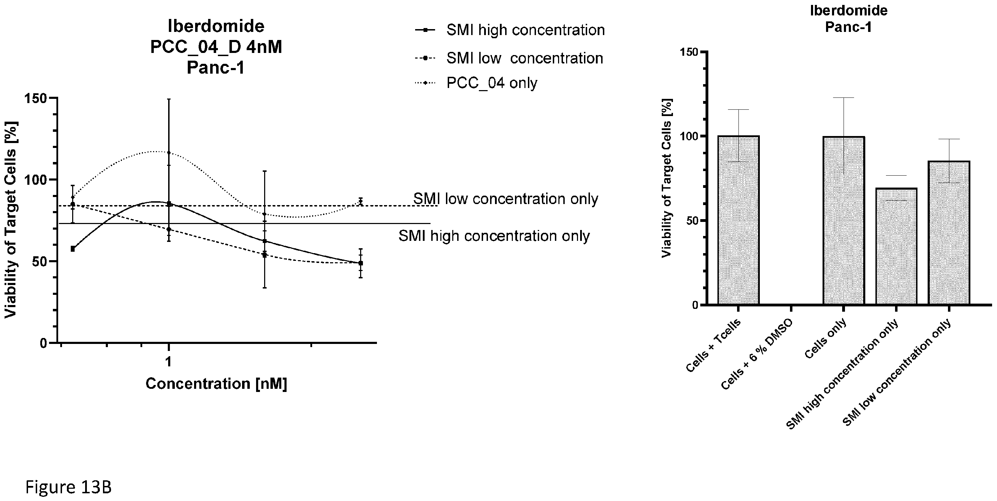

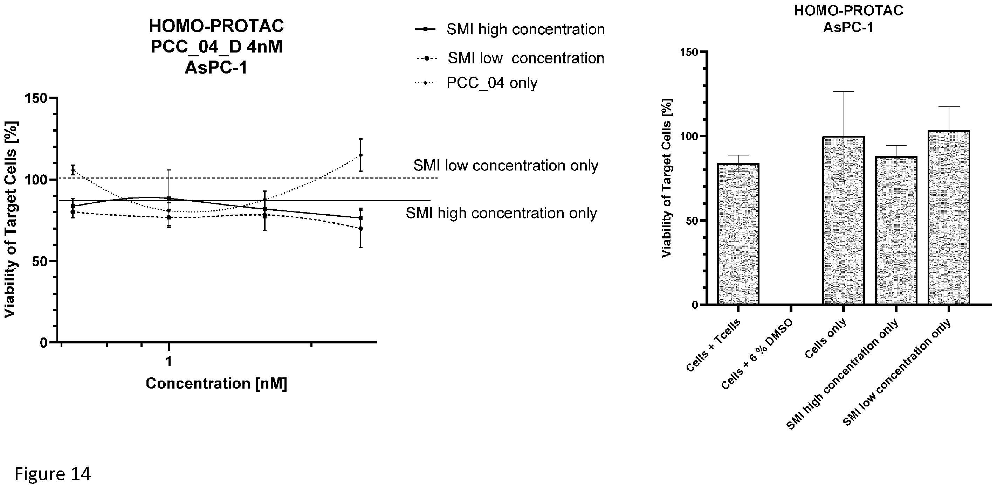

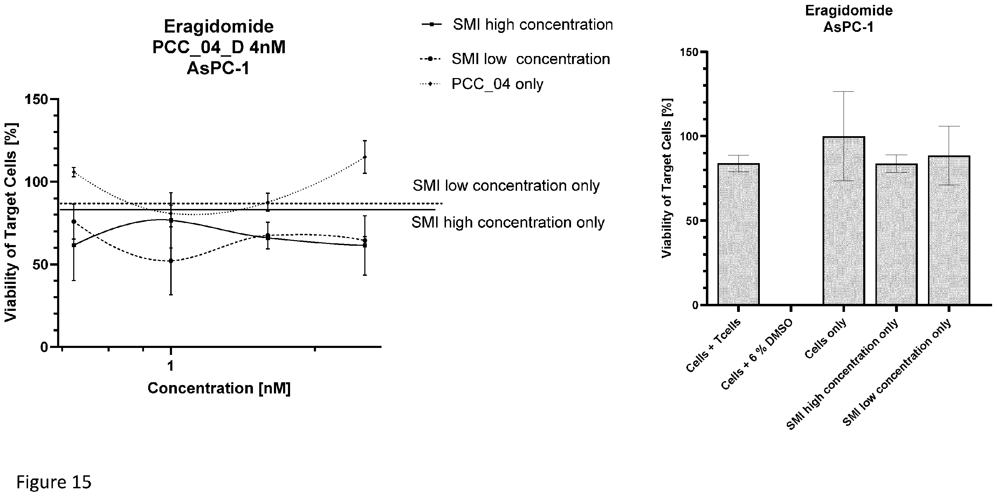

The compounds Lenalidomide (DrugBank ID DB00480) (Figure 12), Iberdomide (DrugBank Accession Number DB12101) (Figure 13), HOMO-Protac cereblon degrader 1 (CAS number 2244520-98-5) (Figure 14) and Eragidomide (DrugBank Accession Number DB19242) (Figure 15), used in combination with an extracellular Ras directed ABP, are all cereblon (CRBN) E3 ligase modulators or inhibitors.

Heterobifunctional degraders belonging to the family of proteolysis targeting chimeras (PROTACs) are a rapidly developing mode of therapeutic activity in which small molecules stabilize the interface between the E3 ubiquitin ligase and its "neosubstrate" protein that normally does not interact with the ligase, thereby promoting their ubiquitination and degradation in a drug-dependent manner. For example the HOMO-Protac cereblon degrader 1 (CAS number 2244520-98-5) was used for combination therapy of this invention (Figure 14). Therefore, any compound with function as PROTACs can be used for this invention.

Also, any compounds inhibiting or modulating the ubiquitin E3 ligase cereblon (Gene ID: 51185, UniProt Q96SW2) can be used in combination with an extracellular Ras directed ABP. Examples are shown in Table 8.

**Table 8: Cereblon (CRBN) inhibitors or modulators and ligands for E3 ligase**

| Name | CAS Number | MedChemExpress (MCE^{®}) numbering / clinicial trials |
|---|---|---|
| CC-885 | 1010100-07-8 | |
| EM12-SO2F | 2803819-61-4 | |
| SMART1 | | HY-W998345 |
| Thalidomide | 50-35-1 | |
| Pomalidomide | 19171-19-8 | |
| E3 ligase Ligand 32 | 2300099-98-1 | |
| BMS-986397 | 2564486-44-6 | |
| PLX-4545 | 2892065-45-9 | |
| PT-179 | 2924858-25-1 | |
| Golcadomide | 2379572-34-4 | |
| ZXH-1-161 | 2407654-51-5 | |
| TD-106 | 2250288-69-6 | |
| CC-17369 | 1547162-46-8 | |
| Cereblon inhibitor 1 | 2672489-14-2 | |
| CRBN modulator-1 | 2407829-65-4 | |
| Cereblon inhibitor 2 | 2639380-62-2 | |
| CRBN ligand-9 | 55003-81-1 | |
| PROTAC CRBN ligand-2 | | HY-158152 |
| CRBN ligand-1 | 3032314-67-0 | |
| BWA-522 intermediate-1 | 2241315-66-0 | |
| Avadomide | 1015474-32-4 | |
| Golcadomide | 2379572-34-4 | |
| Cemsidomide | 2504235-67-8 | |
| BMS-986397 | | NCT04951778 |
| Mezigdomide | 2259648-80-9 | |
| CC-3060 | 444288-86-2 | |

As alternative, any protein or protein complexes of the human ubiquitin E3 ligase family can be targeted with a modulating or inhibiting compound. Examples are:
UBR5 (GenelD 51366, UniProt: 095071), XIAP (GenelD 331, UniProt P98170), ZFP91 (GenelD 80829, UniProt: Q96JP5), TRIM65 (GenelD 201292, UniProt: Q6PJ69), TRIM47 (GenelD 91107, UniProt: Q96LD4), TRIM39 (GenelD 56658, UniProt: Q9HCM9), TRIM3 (GenelD 10612, UniProt: O75382), TRIM25 (GenelD 7706, UniProt: Q14258), SIAH1 (GenelD 6477, UniProt: Q8IUQ4), RNF216 (GenelD 54476, UniProt: Q9NWF9), RNF40 (GenelD 9810, UniProt: 075150), RNF41 (GenelD 10193, UniProt: Q9H4P4), RNF6 (GenelD 6049, UniProt: Q9Y252), PDZRN3 (GenelD 23024, UniProt: Q9UPQ7), NEDD4 (GenelD 4734, UniProt: P46934), Mdm2 (GenelD 4193, UniProt: Q00987), CBL (GenelD 867, UniProt: P22681), BRCA1 (GenelD 672, UniProt: P38398)

Another alternative are modulator compounds inhibiting or modulating the Wnt receptor signalling pathway through beta-catenin or the canonical Wnt receptor signaling pathway, important for cereblon (CRBN) activation and regulation in tumor cells.

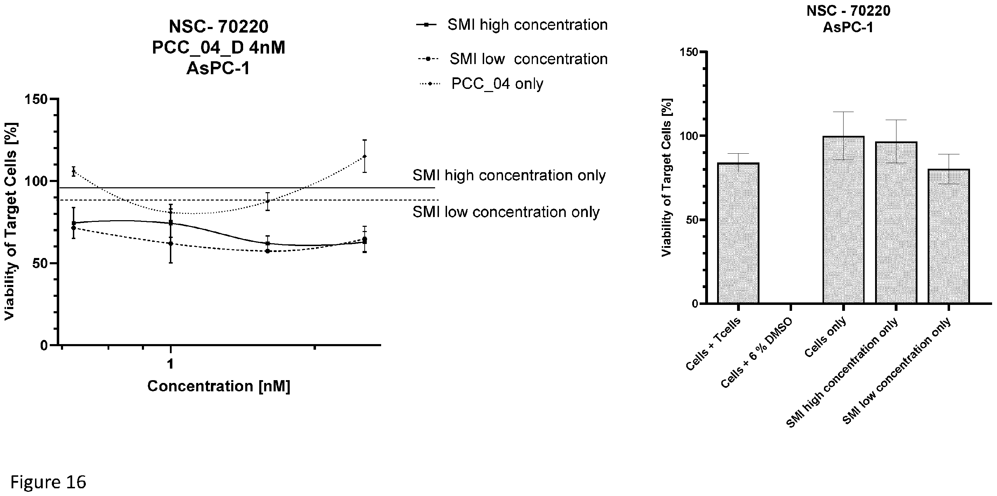

### 4. Modulators of the Ras signaling complex (Figure 16-22)

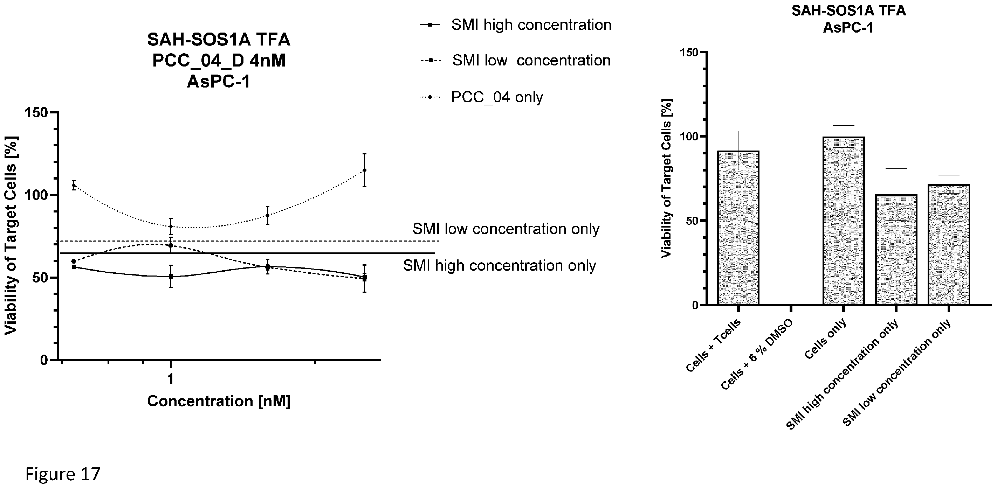

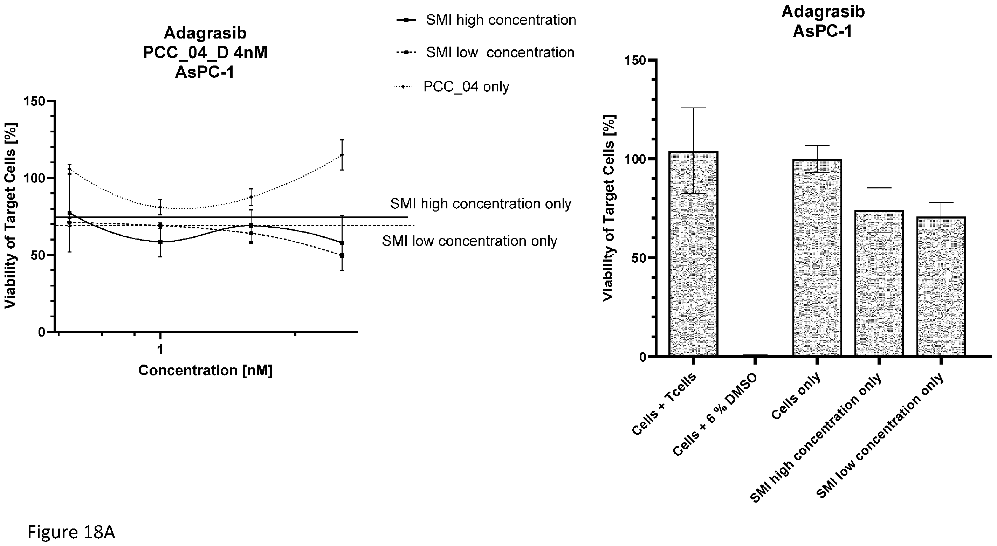

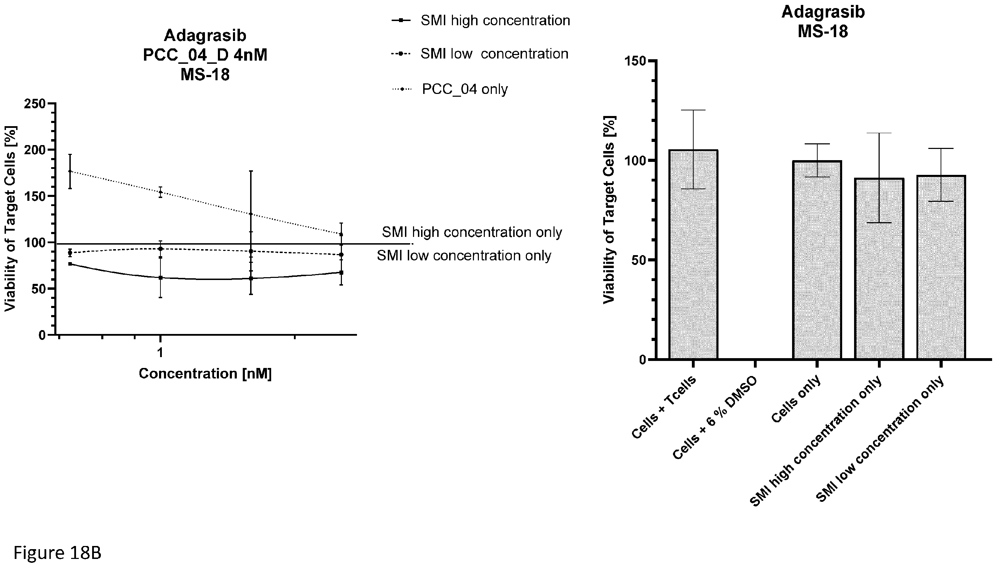

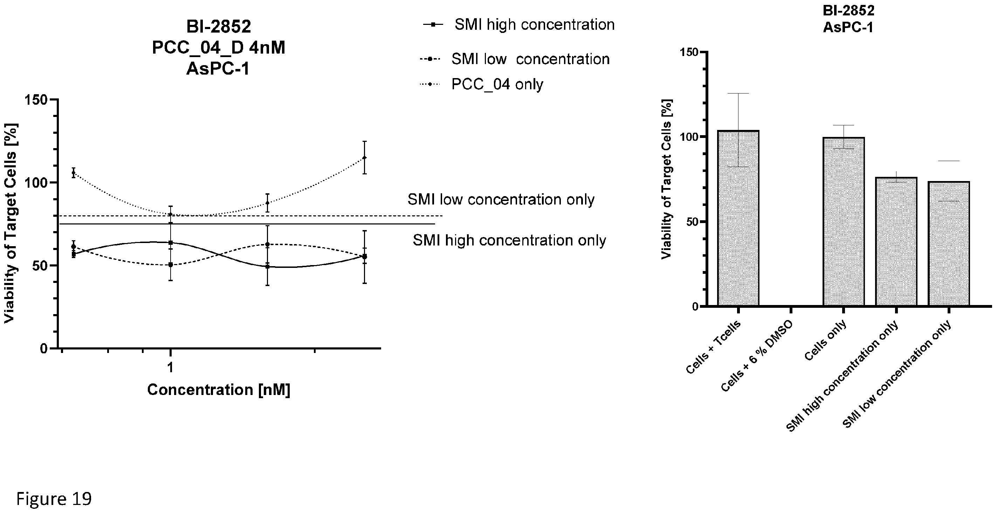

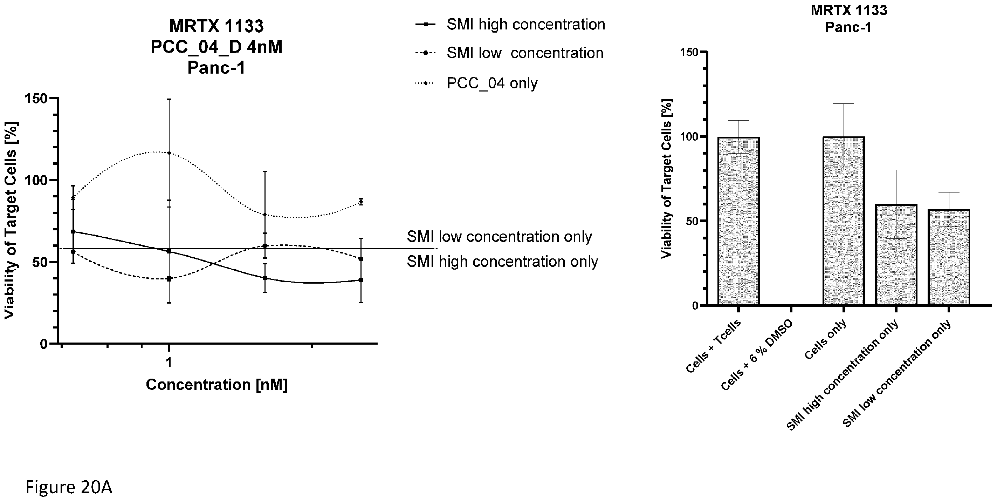

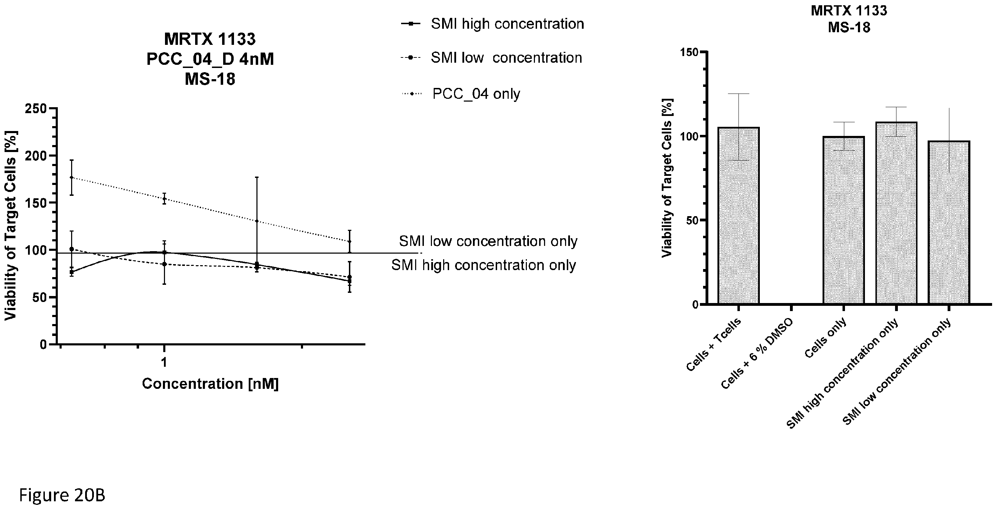

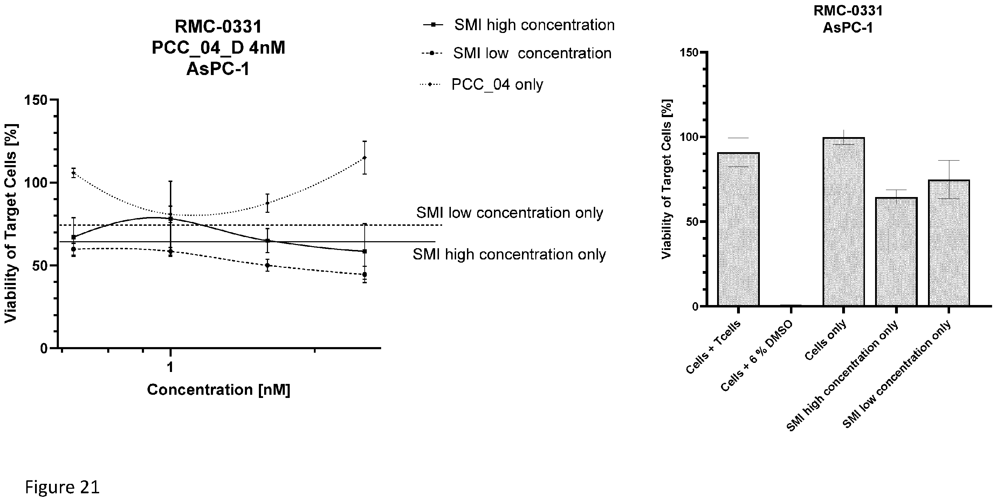

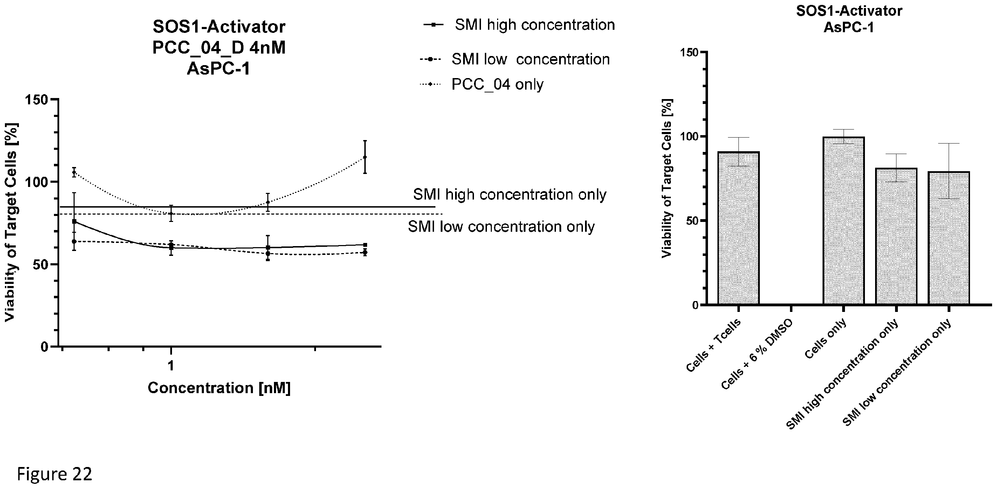

The modulator compounds NSC-70220 (CAS number 4551-00-2) (Figure 16), SAH-SOS1A TFA (2896737-31-6) (Figure 17) Adagrasib (DrugBank ID DB15568) (Figure 18), BI-2852 (CAS number 2375482-51-0) (Figure 19), MRTX-1133 (CAS number 2621928-55-8) (Figure 20), RMC-0331 (CAS number 2488788-52-7) (Figure 21), and SOS1-activator 1 (CAS number 2245237-53-8) (Figure 22), used in combination with an extracellular Ras directed ABP, are all modulators or inhibitors of targets in the Ras signaling complex.

Adagrasib (DrugBank ID DB15568) is a potent, orally-available, and mutation-selective covalent inhibitor of KRAS G12C. BI-2852 (CAS number 2375482-51-0) is a KRAS inhibitor for the switch I/II pocket (SI/II-pocket) by structure-based agent design with nanomolar affinity. BI-2852 is mechanistically distinct from covalent KRASG12C inhibitors (binds to switch II pocket) and binds ten-fold more strongly to active KRASG12D versus KRASwt (740 nM vs 7.5 µM). MRTX-1133 (CAS number 2621928-55-8) is a noncovalent and selective KRAS G12D inhibitor. MRTX1133 optimally fills the switch II pocket and extends three substituents to favorably interact with the protein, resulting in an estimated KD against KRAS G12D of 0.2 pM. MRTX1133 prevents SOS1-catalyzed nucleotide exchange and/or formation of the KRAS G12D/GTP/RAF1 complex, thereby inhibiting mutant KRAS-dependent signal transduction. MRTX1133 selectively inhibits KRAS G12D mutant, but not KRAS wild-type, tumor cells. RMC-0331 (CAS number 2488788-52-7) RMC-0331 (RM-023) is a potent, selective and orally bioavailable SOS1 inhibitor. RMC-0331 is a compound that blocks RAS activation via disruption of the RAS-SOS1 interaction. SOS1-activator 1 (CAS number 2245237-53-8) is a potent activator of SOS1-mediated nucleotide exchange with a Kd of 44 nM. SOS1 (Gene ID: 6654, UniProt Q07889) is a guanine nucleotide exchange factor that catalyzes the exchange of GDP for GTP on RAS. NSC-70220 (CAS number 4551-00-2) is a selective and allosteric SOS1 inhibitor. NSC-70220 inhibits allosteric site activation, and partially inhibited catalytic site activation and SAH-SOS1A TFA (2896737-31-6) is a peptide-based SOS1/KRAS protein interaction inhibitor. SAH-SOS1A TFA binds to wild-type and mutant KRAS (G12D, G12V, G12C, G12S, and Q61H) with nanomolar affinity (EC50=106-175 nM). SAH-SOS1A TFA directly and independently blocks nucleotide association.

Therefore, any compounds inhibiting or modulating the wild type Ras proteins (SEQ ID NOs: 1, 3, 5, 7, 9 or 179 to 182) and/or the mutant Ras proteins (SEQ ID NOs: 11 to 19, 77 to 119, or 183 to 223) can be used in combination with an extracellular Ras directed ABP. Examples for Ras direct or indirect inhibitors or modulators are shown in Table 9.

**Table 9: Different inhibitors or modulators of Ras or farnesyltransferase or the PRMT5-MTA complex or SOS1 or SHP2 or SHP1**

| **Name** | **CAS** | **Clinical study number / Publications / Compound CID** |
|---|---|---|
| A-176120 | 185049-54-1 | |
| RAS/RAS-RAF-IN-1 | 2447039-81-6 | |
| RAS inhibitor Abd-7 | 2351843-48-4 | |
| RMC-6236 | 2765081-21-6 | |
| Rasarfin | 674359-73-0 | |
| Ras modulator-1 | 623935-08-0 | |
| RAS GTPase inhibitor 1 | 2252242-32-1 | |
| Pan-RAS-IN-7 | 2642135-72-4 | |
| K-Ras-IN-1 | 84783-01-7 | |
| Pan-RAS-IN-1 | 1835283-94-7 | |
| K-Ras-IN-4 | 3044773-77-2 | |
| Pan-RAS-IN-4 | 3024060-23-6 | |
| Pan-RAS-IN-3 | 3034588-80-9 | |
| Pan-RAS-IN-2 | 3034673-92-9 | |
| PROTAC K-Ras Degrader-4 | 2938169-99-2 | |
| K-Ras-IN-2 | 905794-70-9 | |
| K-Ras-PDEδ-IN-1 | 1841464-21-8 | |
| PROTAC K-Ras Degrader-1 | 2378258-52-5 | |
| K-Ras-IN-3 | 3024991-82-7 | |
| PROTAC K-Ras Degrader-3 | 3043670-68-1 | |
| K-Ras ligand-Linker Conjugate 1 | 2749492-84-8 | |
| K-Ras ligand-Linker Conjugate 2 | 2741300-61-6 | |
| K-Ras ligand-Linker Conjugate 3 | 2378261-87-9 | |
| K-Ras ligand-Linker Conjugate 4 | 2378261-83-5 | |
| Ftase inhibitor I | 149759-96-6 | |
| UCM-1336 | 1621535-90-7 | |
| ML-099 | 496775-95-2 | |
| MCP110 | 521310-51-0 | |
| SCH-53870 | 188480-50-4 | |
| G DC-6036-N H | 2417918-80-8 | |
| L-731735 | 149756-20-7 | |
| KY1022 | 1029721-36-5 | |
| SOS1 activator 1 | 2245237-53-8 | |
| LUNA18 | 2676177-63-0 | |
| Pan-RAF kinase inhibitor 1 | 2648838-76-8 | |
| RMC-7977 | 2765082-12-8 | |
| Salirasib | 162520-00-5 | |
| FTI-2153 | 344900-92-1 | |
| NSC1011 | 5335-97-7 | |
| LB42908 | 226927-89-5 | |
| L 731734 | 149786-89-0 | |
| NSC-658497 | 909197-38-2 | |
| LB42708 | 226929-39-1 | |
| XMU-MP-9 | 2251130-41-1 | |
| SOS1 agonist-1 | 2245237-61-8 | |
| Lonafarnib | 193275-84-2 | |
| A-176120 | 185049-54-1 | |
| XRP44X | 729605-21-4 | |
| ADT-007 | 1945941-09-2 | |
| Kobe0065 | 436133-68-5 | |
| ML-097 | 743456-83-9 | |
| UC-857993 | 487001-04-7 | |
| Kobe2602 | 454453-49-7 | |
| FTI-277 | 170006-73-2 | |
| RMC-0331 | 2488788-52-7 | |
| G12Si-1 | 2946593-42-4 | |
| POP-3MB | 1144114-27-1 | |
| SCH54292 | 188480-51-5 | |
| BAY-293 | 2244904-70-7 | |
| SML-10-70-1 | 1536470-98-0 | |
| 6-CEPN | 1054549-73-3 | |
| CP-609754 | 1190094-64-4 | |
| ARS-1323 | 1698024-73-5 | |
| KY1220 | 292168-79-7 | |
| ARS-1630 | 1698055-86-5 | |
| ZINC57632462 | 1286482-29-8 | |
| SOS1-IN-16 | 2930763-85-0 | |
| PROTAC SOS1 degrader-7 | 3036155-26-4 | |
| FTI-2148 | 251577-09-0 | |
| RMC-9805 | 2922732-54-3 | |
| L-739749 | 156511-34-1 | |
| KRAS inhibitor-22 | 2042365-57-9 | |
| XR 3054 | 247090-97-7 | |
| Glecirasib | 2657613-87-9 | |
| Vociprotafib | 2172652-48-9 | |
| FGTI-2734 | 1247018-19-4 | |
| SHP099 | 1801747-42-1 | |
| RMC-6291 | 2641998-63-0 | |
| Setidegrasib | 2821793-99-9 | |
| H RS-4642 | - | NCT06620848 |
| | | NCT06587061 |
| MRTX1133 | 2621928-55-8 | |
| BI-3706674 | | JPRN-jRCT2030230343 |
| | | NCT06056024 |
| ERAS-4693 | | DOI: 10.1016/j.taap.2023.116601 |
| ERAS-5024 | | |
| BI-2865 | 2937327-93-8 | |
| BI-2493 | 2937344-16-4 | |
| FMC-376 | | NCT06244771 |
| BBO-8520 | 2893809-51-1 | |
| MRTX9768 | 2629314-68-5 | |
| MRTX-1719 | 2630904-45-7 | |
| M RTX0902 | 2654743-22-1 | |
| MRTX-1257 | 2206736-04-9 | |
| | | |
| MRTX-EX185 | | Compound CID: 163322337 |
| Adagrasib | 2326521-71-3 | |
| BI-2852 | 2375482-51-0 | |
| TH-Z827 | 2847881-81-4 | |
| TH-Z816 | 2847881-42-7 | |
| TH-Z835 | 2766209-50-9 | |
| TH-Z827 | 2847881-81-4 | |
| RMC-4550 | 2172651-73-7 | |
| RMC-3943 | 1801764-60-2 | |
| RMC-6291 | 2641998-63-0 | |
| RMC-4998 | 2642037-07-6 | |
| NSC-70220 | 4551-00-2 | |
| NSC-87877 | 56990-57-9 | |
| sos1-in-15 | | Compound CID: 165437863 |
| sos1-in-14 | | Compound CID: 165413022 |
| SOS1 activator 1 | | Compound CID: 134814234 |
| SAH-SOS1A TFA | 2896737-31-6 | |
| SAH-SOS1A | 1652561-87-9 | |

As alternative, any protein or protein complexes of the Ras signaling pathway can be targeted with a modulating or inhibiting compound. Exemplary RAS pathway gene names are:
BRAF (GenelD: 673, UniProt P15056, H7C560, AOA2U3TZI2, A0A2R8Y8E0), ALK (GenelD: 238, UniProt Q9UM73, A0A087WZL3), AKT1 (GenelD: 207, UniProt P31749-1, P31749-2), AKT2 (GenelD: 208, UniProt P31751-1, P31751-2), BRCA2 (GenelD: 675, UniProt P51587, H0YE37, H0YD86, A0AAQ5BGN2), ERBB2 (GenelD: 2064, UniProt P04626-1, P04626-2, P04626-3, P04626-4, P04626-5, P04626-6), EGFR (GenelD: 1956, UniProt P00533-1, P00533-2, P00533-3, P00533-4), FGFR1 (GenelD: 2260, UniProt P11362-1, P11362-2, P11362-3, P11362-4, P11362-5, P11362-6, P11362-7, P11362-8, P11362-9, P11362-10, P11362-11, P11362-12), FGFR2 (GenelD: 2263, UniProt P21802-1, P21802-2, P21802-3, P21802-4, P21802-5, P21802-6, P21802-7, P21802-8, P21802-14, P21802-15, P21802-16, P21802-17), FGFR3 (GenelD: 2261, UniProt P22607-1, P22607-2, P22607-3, P22607-4), FGFR4 (GenelD: 2264, UniProt, P22455-1, P22455-2), FLT3 (GenelD: 2322, UniProt, P36888-1, P36888-2), GRB10 (GenelD: 2887, UniProt Q13322-1, Q13322-2, Q13322-3, Q13322-4), GRB2 (GenelD: 2885, UniProt P62993-1, P62993-2), KSR1 (GenelD: 8844, UniProt Q8IVT5-1, Q8IVT5-2, Q8IVT5-3, Q8IVT5-4), KSR2 (GenelD: 283455, UniProt Q6VAB6-1, Q6VAB6-2), MET (GenelD: 4233, UniProt P08581-1, P08581-2, P08581-3), MTOR (GenelD: 2475, UniProt P42345), NFKB1 (GenelD: 4790, UniProt P19838-1, P19838-2, P19838-3), PDGFRA (GenelD: 5156, UniProt P16234-1, P16234-2, P16234-3), PDGFRB (GenelD: 5159, UniProt P09619-1, P09619-2), PDPK1 (GenelD: 5170, UniProt 015530-1, 015530-2), RAF1 (GenelD: 5894, UniProt P04049-1, P04049-2), RALA (GenelD: 5898, UniProt P11233), RALB (GenelD: 5899, UniProt P11234-1, P11234-2, P11234-3), RASA1 (GenelD: 5921, UniProt P20936-1, P20936-2, P20936-3, P20936-4), RASA2 (GenelD: 5922, UniProt Q15283-1, Q15283-2), RASA3 (GenelD: 22821, UniProt Q14644-1, Q14644-2), RASGRF1 (GenelD: 5923, UniProt Q13972-1, Q13972-2, Q13972-3), RASGRF2 (GenelD: 5924, UniProt 014827), RHEB (GenelD: 6009, UniProt Q15382), ROCK1 (GenelD: 6093, UniProt Q13464), ROCK2 (GenelD: 9475, UniProt 075116), ROS1 (GenelD: 6098, UniProt P08922), SOS1 (GenelD: 6654, UniProt Q07889-1, Q07889-2), SOS2 (GenelD: 6655, UniProt Q07890-1, Q07890-2), STK11 (GenelD: 6794, UniProt Q15831-1, Q15831-2), STK3 (GenelD: 6788, UniProt Q13188-1, Q13188-2), STK4 (GenelD: 6789, UniProt Q13043-1, Q13043-2).

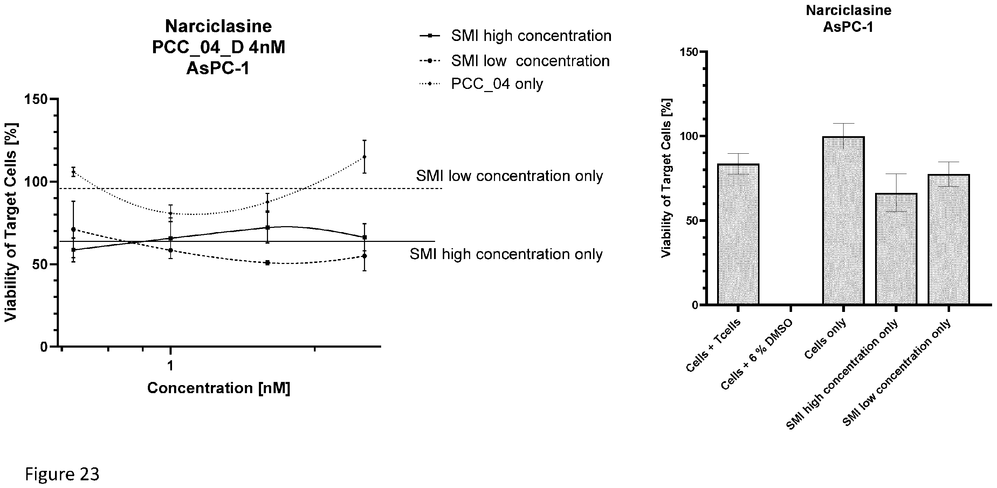

### 5. Modulators of the Rho kinase pathway (Figure 23)

The compounds Narciclasine (CAS number 29477-83-6) (Figure 23) used in combination with an extracellular Ras directed ABP, modulates the ROCK, LIMK and cofilin signaling pathway, greatly increasing GTPase RhoA activity as well as inducing actin stress fiber formation in a RhoA-dependent manner. Narciclasine also broadly altered lipid metabolism via modulation of differential genes in pathways related to phospholipid metabolism.

Therefore, any compounds inhibiting or modulating ROCK1 (Gene ID: 6093, UniProt Q13464) or ROCK2 (Gene ID: 9475, UniProt 075116) or LIMK1 (Gene ID: 3984, UniProt P53667) or LIMK2 (Gene ID: 3985, UniProt P53671) or RHOA (Gene ID: 387, UniProt P61586) can be used in combination with an extracellular Ras directed ABP of this invention.

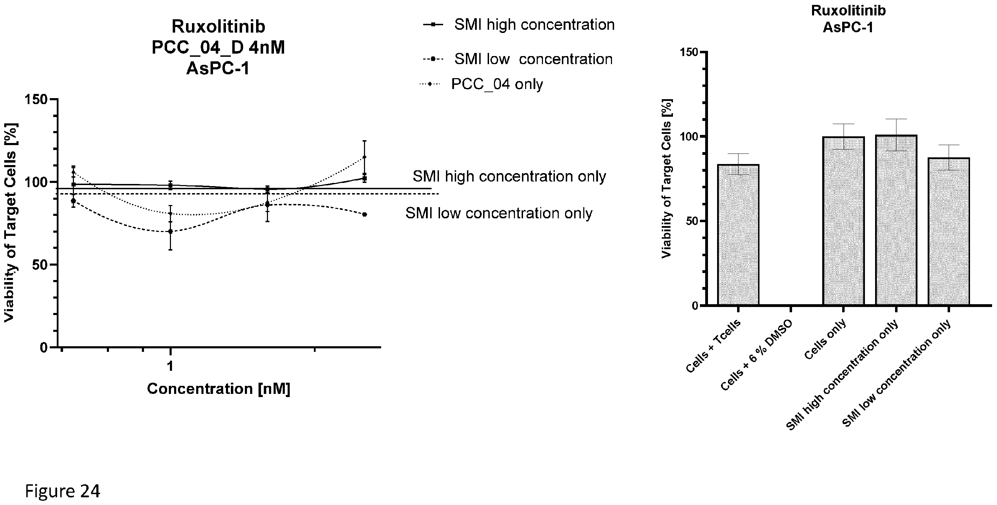

### 6. Modulators of the JAK signaling pathway (Figure 24)

The modulator compound Ruxolitinib (DrugBank ID DB08877) (Figure 24) used in combination with an extracellular Ras directed ABP, is an orally active and selective JAK1/2 inhibitor with IC50s of 3.3 nM and 2.8 nM in cell-free assays, and has 130-fold selectivity for JAK1/2 over JAK3.

Therefore, any compounds inhibiting or modulating the JAK kinase family, JAK1 (Gene ID: 3716, UniProt P23458) or JAK2 (Gene ID: 3717, UniProt O60674) or JAK3 (Gene ID: 3718, UniProt P52333) or TYK2 (Gene ID: 7297, UniProt P29597) can be used for this invention.

Examples for possible JAK inhibitors used in combination for this invention are Abrocitinib (DrugBank ID DB14973), Baricitinib (DrugBank ID DB11817), Delgocitinib (DrugBank Accession Number DB16133), Fedratinib (DrugBank ID DB12500), Filgotinib (DrugBank ID DB14845), Oclacitinib (DrugBank Accession Number DB11441), Peficitinib (DrugBank Accession Number DB11708), Pacritinib (DrugBank ID DB11697), Tofacitinib (DrugBank ID DB08895), Upadacitinib (DrugBank ID DB15091), Brepocitinib (CAS number 1883299-62-4), Cerdulatinib (DrugBank Accession Number DB15499), Momelotinib (DrugBank ID DB11763), Decernotinib (DrugBank Accession Number DB12566), Itacitinib (DrugBank Accession Number DB12154), Gandotinib (DrugBank Accession Number DB13040) and Gusacitinib (DrugBank Accession Number DB15670).

As alternative, any protein or protein complexes of JAK kinase down-stream binding partners can be targeted with a modulating or inhibiting compound. Examples are STAT1 (Gene ID: 6772, UniProt P42224), STAT2 (Gene ID: 6773, UniProt P52630), STAT3 (Gene ID: 6774, P40763), STAT4 (Gene ID: 6775, UniProtQ14765), STAT5A (Gene ID: 6776, UniProt P42229), STAT5B (Gene ID: 6777, UniProt P51692) and STAT6 (Gene ID: 6778, P42226).

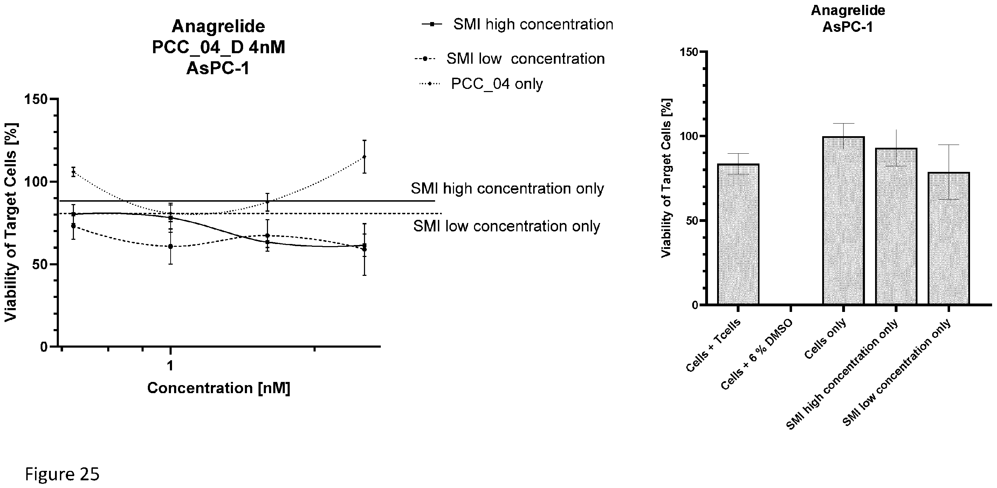

### 7. Modulators of the phosphodiesterase pathway (Figure 25)

The compound Anagrelide (DrugBank ID DB00261) (Figure 25) is an inhibitor of phosphodiesterase type III (PDE3) (IC50=36 nM).

Therefore, any compounds inhibiting or modulating the phosphodiesterase family, PDE1A (Gene ID: 5136, UniProt P54750), PDE1C (Gene ID: 5137, UniProt Q14123), PDE1B (Gene ID: 5153, UniProt Q01064), PDE3A (Gene ID: 5139, UniProt Q14432), PDE3B (Gene ID: 5140, UniProt Q13370), PDE4A (Gene ID: 5141, UniProt P27815), PDE4B (Gene ID: 5142, UniProt Q07343), PDE4C (Gene ID: 5143, UniProt Q08493), PDE4D (Gene ID: 5144, UniProt Q08499), PDE2A (Gene ID: 5138, UniProt O00408), PDE5A (Gene ID: 8654, UniProt O76074), PDE11A (Gene ID: 50940, UniProt Q9HCR9), PDE6A (Gene ID: 5145, UniProt P16499), PDE6B (Gene ID: 5158, UniProt P35913), PDE6C (Gene ID: 5146, UniProt P51160), PDE10A (Gene ID: 10846, UniProt Q9Y233), PDE9A (Gene ID: 5152, O76083), PDE8A (Gene ID: 5151, UniProt O60658), PDE8B (Gene ID: 8622, UniProt O95263), PDE7A (Gene ID: 5150, UniProt Q13946) or PDE7B (Gene ID: 27115, UniProt Q9NP56) can be used for this invention.

Examples for phosphodiesterase inhibitors which can be used for this invention are Milrinone (DrugBank ID DB00235), Amrinone (DrugBank ID DB01427), Enoximone (DrugBank ID DB04880), Tadalafil (DrugBank ID DB00820), Vardenafil (DrugBank ID DB00862), Cilostazol (DrugBank ID DB01166), Piclamilast (DrugBank ID DB01791), Rolipram (DrugBank ID DB01954), OSI-461 (DrugBank ID DB05415), Udenafil (DrugBank ID DB06267), Doxofylline (DrugBank ID DB09273), Trapidil (DrugBank ID DB09283), Pimobendan (DrugBankAccession Number DB11450), Mirodenafil (DrugBank Accession Number DB11792), PF-04447943 (DrugBank Accession Number DB11953), Vesnarinone (DrugBank Accession Number DB12082), Vinpocetine (DrugBank Accession Number DB12131), GSK-256066 (DrugBank Accession Number DB12137), Simendan (DrugBankAccession Number DB12286), MK-0873 (DrugBank Accession Number DB13029), Bucladesine (DrugBank Accession Number DB13242), Roflumilast (DrugBank ID DB01656), E-6005 (DrugBank Accession Number DB12776), Caffeine (DrugBank ID DB00201), Theophylline (DrugBank ID DB00277), Dyphylline (DrugBank ID DB00651), Pentoxifylline (DrugBank ID DB00806), Levosimendan (DrugBank ID DB00922), Dipyridamole (DrugBank ID DB00975), Papaverine (DrugBank ID DB01113), Aminophylline (DrugBank ID DB01223), Cilomilast (DrugBank ID DB03849), Ibudilast (DrugBank ID DB05266), Crisaborole (DrugBank ID DB05219), Zardaverine (DrugBank ID DB02918), Apremilast (DrugBank ID DB05676), Osoresnontrine (DrugBank Accession Number DB16274), Drotaverine (DrugBank ID DB06751), Avanafil (DrugBank ID DB06237), Sildenafil (DrugBank ID DB00203), Dovramilast (DrugBank Accession Number DB16242), Olprinone (DrugBank Accession Number DB16847), Zatolmilast (DrugBank Accession Number DB14790), Etazolate (DrugBank Accession Number DB05881), Ensifentrine (DrugBank ID DB16157).

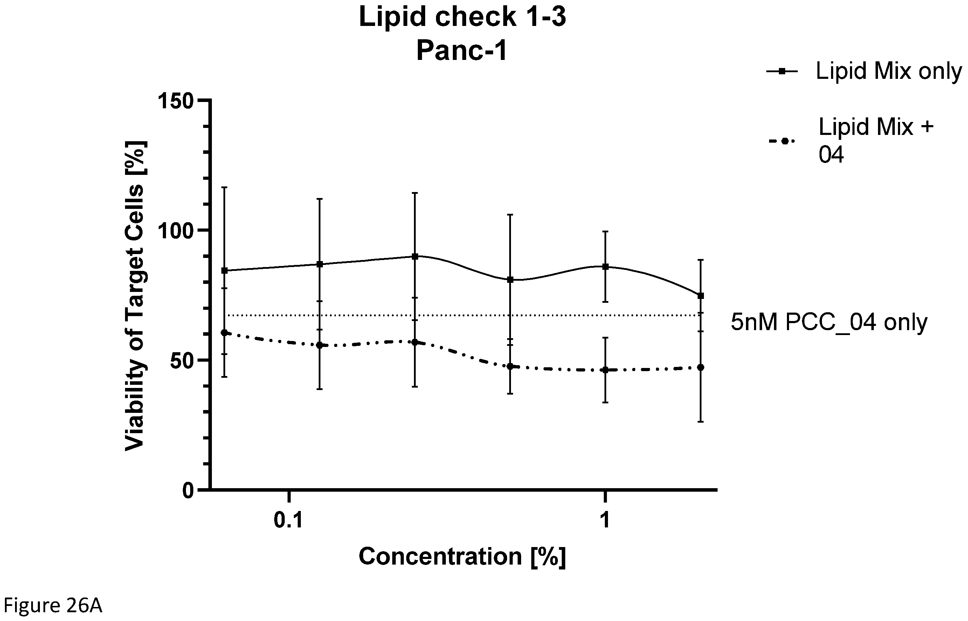

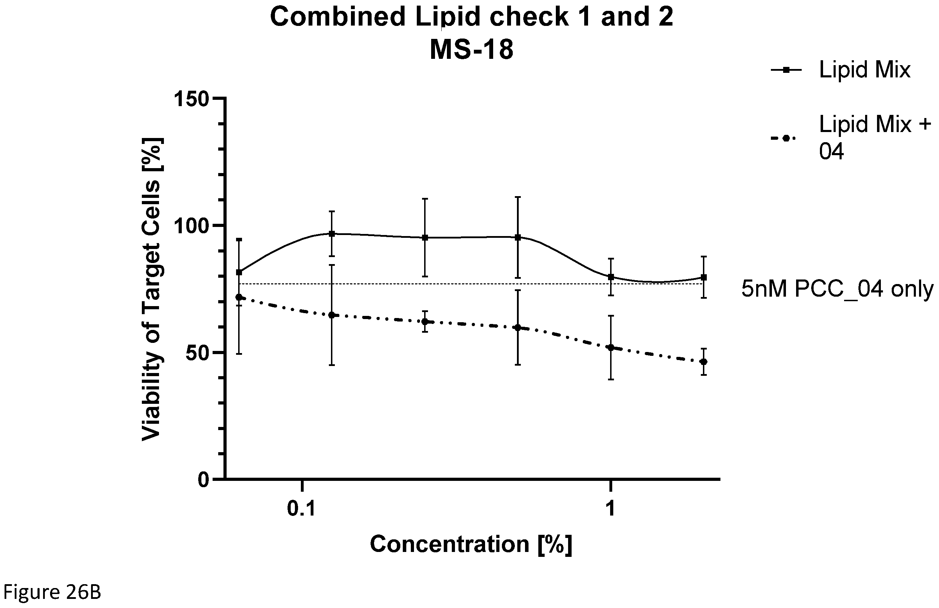

### 8. Compounds of fatty acids and cholesterol (Figure 26)

It was tested if the addition of lipids can increase the anti-tumor efficacy of the PCC04D Diabody (SEQ ID NOs: 23 and 231) in the co-culture assay (Figure 26). A mix containing different non-animal derived fatty acids including arachidonic acid (DrugBank ID DB04557), linoleic acid (DrugBank Accession Number DB14104), linolenic acid (DrugBank ID DB00132), myristic acid (DrugBank ID DB08231), oleic acid (DrugBank ID DB04224), palmitic acid (DrugBank ID DB03796) and stearic acid (DrugBank ID DB03193), as well as animal derived cholesterol (DrugBank ID DB04540) has been used (Lipid Mix). In two different tumor cell lines Panc-1 (Figure 26A) and MS-18 (Figure 26B), the addition of the Lipid mix caused a dose dependent increase in target cell killing by the PCC04D Diabody.

Therefore, different fatty acids can be used in combination therapy for this invention like short-chain fatty acids (SCFAs) with aliphatic tails of five or fewer carbons, or medium-chain fatty acids (MCFAs) with aliphatic tails of 6 to 12 carbons, or long-chain fatty acids (LCFAs) with aliphatic tails of 13 to 21 carbons, or very long chain fatty acids (VLCFAs) with aliphatic tails of 22 or more carbons. These can be saturated fatty acids or unsaturated fatty acids. Examples for different fatty acids are arachidonic acid (DrugBank ID DB04557), linoleic acid (DrugBank Accession Number DB14104), linolenic acid (DrugBank ID DB00132), myristic acid (DrugBank ID DB08231), oleic acid (DrugBank ID DB04224), palmitic acid (DrugBank ID DB03796) or stearic acid (DrugBank ID DB03193).

Further exemplary fatty acids are: Propionic acid, Valeric acid, Lauric acid, Myristic acid, Pentadecylic acid, Palmitic acid, Stearic acid, α-Linolenic acid, Eicosapentaenoic acid, Linoleic acid, Linolelaidic acid, γ-Linolenic acid, Arachidonic acid, Palmitoleic acid, Oleic acid, Gondoic acid, Mead acid, Glycolic acid, Oxalic acid, Propiolic acid, pentanoic acid, pentanedioic acid, hexanoic acid, decanoic acid.

In addition, cholesterol (DrugBank ID DB04540) can be used in combination therapy for this invention.

In addition, different metabolites of cholesterol can be used in combination therapy for this invention (Table 10).

**Table 10: Metabolites of cholesterol and bile acids**

| **Oxysterols** | |
|---|---|
| 4-beta-hydroxycholesterol | (4β-OHC) |
| 5 alpha,6 alpha-epoxycholesterol | (α-epoxyC) |
| 5 beta,6 beta-epoxycholesterol | (β-epoxyC) |
| 5 alpha,6 beta-epoxycholesterol | (5α,6β-diOHC) |
| 7-hydroperoxycholesterol | (7-OOHC) |
| 7-alpha-hydroxycholesterol | (7α-OHC) |
| 7-alpha-hydroxy-4-cholesten-3-one | (7α-OHC4) |
| 7-beta-hydroxycholesterol | (7β-OHC) |
| 7-ketocholesterol | (7-ketoC) |
| 7-dehydrocholesterol | (7-DHC) |
| 24,25-epoxycholesterol | (24,25-epoxyC) |
| 22-hydroxycholesterol | (22-OHC) |
| 24-hydroxycholesterol | (24 OHC) |
| 25-beta-hydroxycholesterol | (25β-OHC) |
| 25-hydroxycholesterol | (25-OHC) |
| 26-hydroxycholesterol | (26-OHC) |
| Cholic acid | 3α,7α,12α-trihydroxy-5β-cholan-24-oic acid |
| Glycocholic acid | N-(3α,7α,12α-Trihydroxy-5β-cholan-24-oyl)glycine |
| Taurocholic acid | 2-(3α,7α,12α-Trihydroxy-5β-cholan-24-amido)ethane-1-sulfonic acid |
| Deoxycholic acid | 3α,12α-Dihydroxy-5β-cholan-24-oic acid |
| Chenodeoxycholic acid | 3α,7α-Dihydroxy-5β-cholan-24-oic acid |
| Glycochenodeoxycholic acid | N-(3α,7α-Dihydroxy-5β-cholan-24-oyl)glycine |
| Taurochenodeoxycholic acid | 2-(3α,7α-Dihydroxy-5β-cholan-24-amido)ethane-1-sulfonic acid |
| Lithocholic acid | 3α-Hydroxy-5β-cholan-24-oic acid |
| Ursodeoxycholic acid | 3α,7β-dihydroxy-5β-cholan-24-oic acid |

These fatty acids and/or cholesterol can be administrated as purified compounds. In addition, these fatty acids and/or cholesterol can be administrated through food and/or special diets.

### 9. Modulation of the gut microbiome

Because fatty acids can improve the extracellular Ras directed therapy, short chain fatty acids (SCFAs) produced as bacterial metabolites of the gut microbiome can be important for this invention. This includes for example acetate (DrugBank Accession Number DB14511), propionate (DrugBank Accession Number DB19384) and butyrate (DrugBank ID DB03568). Therefore modulation of the gut microbiome can be used for this invention.

A method for this modulation is faecal microbiota transplant (FMT). FMT is the administration of stool preparation obtained from a healthy donor to a tumor patient (recipient). Another method for this modulation is faecal filtrate transplant (FFT). FTT aim is to remove the microbial cells but to retain the viral (bacteriophages) and microbial metabolites. Administrations of FMT and FFT are by special covered pills, or lower gastrointestinal endoscopy or with enema. Another method for this modulation is through special diets. Diets with plant foods (fruits, vegetables, wholegrains, legumes, and nuts) rich in naturally occurring fibres. Or fermented food, made through desirable microbial growth and enzymatic conversions of food components. Or prebiotics, defined as a substrate that is selectively utilised by host microorganisms conferring a health benefit. Or probiotics, defined as live microorganisms that, when administered in adequate amounts, confer a health benefit on the host.

Another method for this modulation is phage therapy, using bacteriophages targeting specific bacteria strains in the gut. Another method for this modulation is the use of postbiotics which are functionally bioactive molecules produced by microbes that confers a health benefit on the host. Another method for this modulation is the use of specific antibiotics to reduce individual microbial strains detrimental to this invention. Another method for this modulation is the use of specific antibiotics to reduce or deplete the patients gut microbiome to improve the engraftment of the transplanted new microbiome.

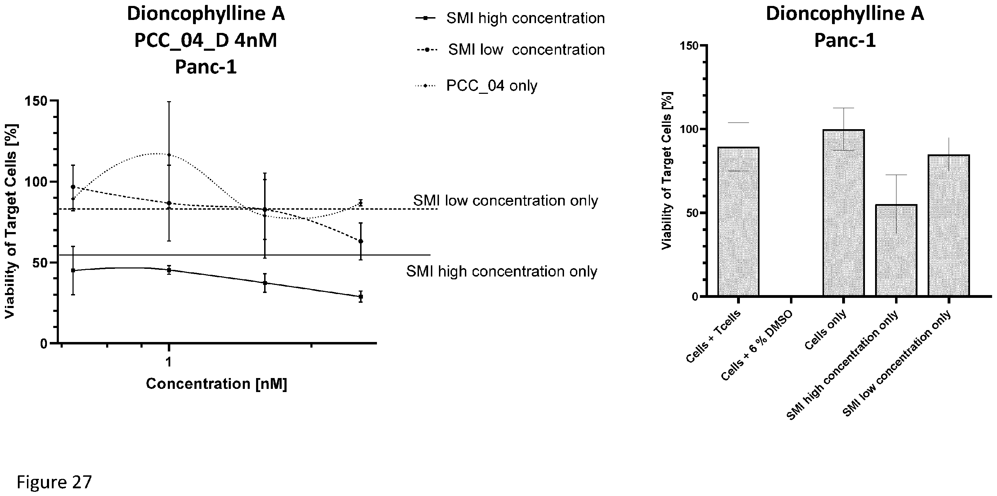

### 10. Inhibitors of the naphthylisoquinoline alkaloid class (Figure 27)

The large and diverse class of naphthylisoquinoline alkaloids are natural biaryl compounds. A unique feature of these compounds is their biosynthetic origin from polyketidic precursors and not, as usual for isoquinoline alkaloids, from aromatic amino acids. Over 280 naphthylisoquinoline alkaloids have so far been isolated, all of them exclusively from tropical plant species belonging to the lianes family Ancistrocladaceae and Dioncophyllaceae.

The compound Dioncophylline A (PubChem ID 3095) (BR-BM 7) (Figure 27), used in combination with an extracellular Ras directed ABP, is a naphthyltetrahydroisoquinoline alkaloid.

Therefore, any compounds of the naphthylisoquinoline alkaloid class can be used in combination with an extracellular Ras directed ABP of this invention.

Based on these data a large variety of different drugs, SMls and other modulator compounds as well as different cellular targets and pathways for inhibition or modulation, can be used in combination therapies with extracellular Ras directed ABPs of this invention. For example, modulator compounds can be (amongst others) any drug, chemical, peptide, protein, ligand and/or small molecule inhibitor. The combinations of the present invention can for example be used for humans or animals in the fields of cancer therapy, therapy of non-malignant disease, treatment of aging, in lifestyle medicine, in diagnostics and/or drug development.

The data testing many different combinations of the present invention demonstrate: (i) The extracellular Ras targeting ABP strategies can be modulated. (ii) This modulation can happen by the combination with a modulator compound. (iii) This combination therapy can increase the anti-tumor effect in a dose dependent manner, (iv) The use of a modulator compound is decreasing IC50 of the Ras targeting ABP.

Combinations comprising more than one modulator compound (i.e. same or different modulator compounds) and/or more than one ABP (same or different ABP) and/or more than one isolated nucleic acid (same or different isolated nucleic acids) comprising a sequence encoding the ABP are also possible. Such combinations can be advantageous in that they show a high anti-tumor activity.

In prior experiments, without extracellular Ras targeting ABPs, the effect of the individual modulator compounds on tumor cells was analyzed to determine a dose range, where the tested compound has reported target activity but only a small or minor negative effect on the tumor cells. If the anti-tumor effect of the compound alone is too high, a possible additional smaller benefit for the combination with extracellular Ras targeting ABP can be missed (data not shown).

ABPs and modulator compounds can be used in a wide variety of concentrations. The person skilled in the art is aware how to test suitable concentrations in that the effect on the tumor cells, with or without T cells, is tested. Also, concentration gradients can be used. Toxicity assays are also standard in the art.

ABPs are preferably in physiologic solutions for administration, like normal 0.9% NaCl solution, 1.3% saline solution, 0.45% half normal saline solution, 5% dextrose in water solution, lactate ringer solution, acetate ringer solution, hartmann solution, PlasmaLyte, Sterofundin ISO, ELO-MEL isoton, lonolyte, Isoplex, Gelaspan, Hextend, Tetraspan, Volulyte. The physiologic solutions have a preferred Osmolarity [mOsm L⁻¹] between 277 and 309.

Possible routes of administration of the ABPs are intravenous, intradermal, subcutaneous, intramuscular, intraperitoneal, intranodal or intrathecal. The administration can happen through fast injection (1 to 60 seconds long), slow injection (1 to 5 minutes), fast infusions (5 to 15 minutes), slow infusions (16 minutes to 8 hours) or continuous infusion (8 hours to 96 hours).

The administration of the modulator compounds can be together with the ABPs administration, or before (1 hour to 48 hours) or afterwards (1 hour to 48 hours). The modulator compounds can also be administrated daily as continuous medication, or every two days, or three times a week, for example.

### In vitro methods for detecting the expression of extracellular Ras proteins on the surface of cells

Development of diagnostic assays is essential for the safe and effective use of new therapeutics (companion diagnostics), for example to analyze before start of therapy, if the tumor cells are positive or negative for extracellular Ras expression. Also, only treating responders by testing for extracellular Ras expression before treatment, reduces costs. Such methods of the present invention are also preferably useful for large-scale and high-throughput drug, compound or targets screens.

In addition, such *in vitro* methods for detecting the expression of extracellular Ras proteins on cells can be used in screening assays in order to identify a compound (e.g. an SMI) or a target modulating extracellular Ras expression.

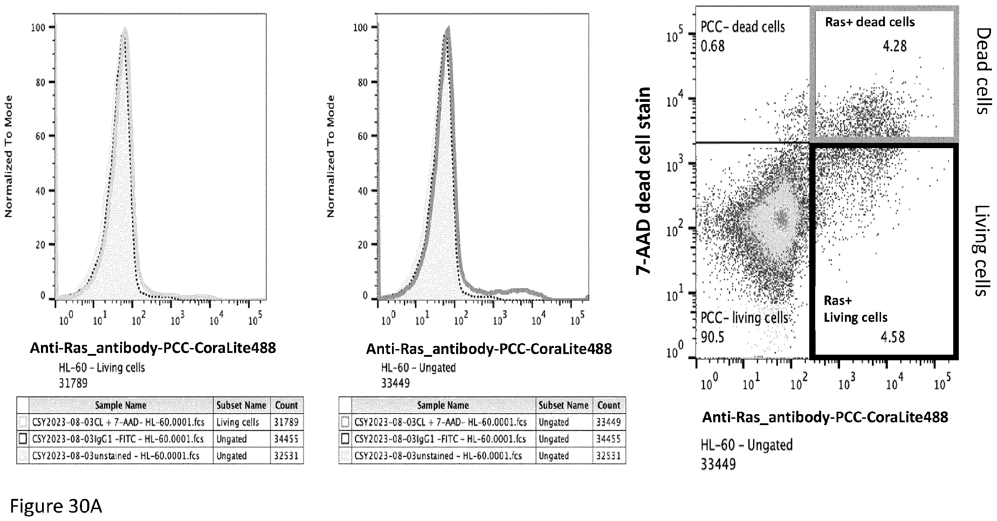

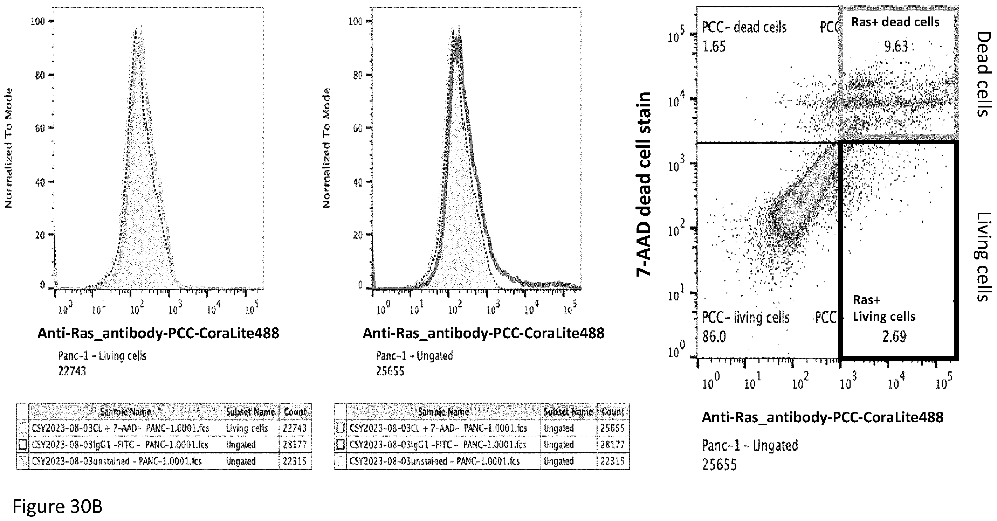

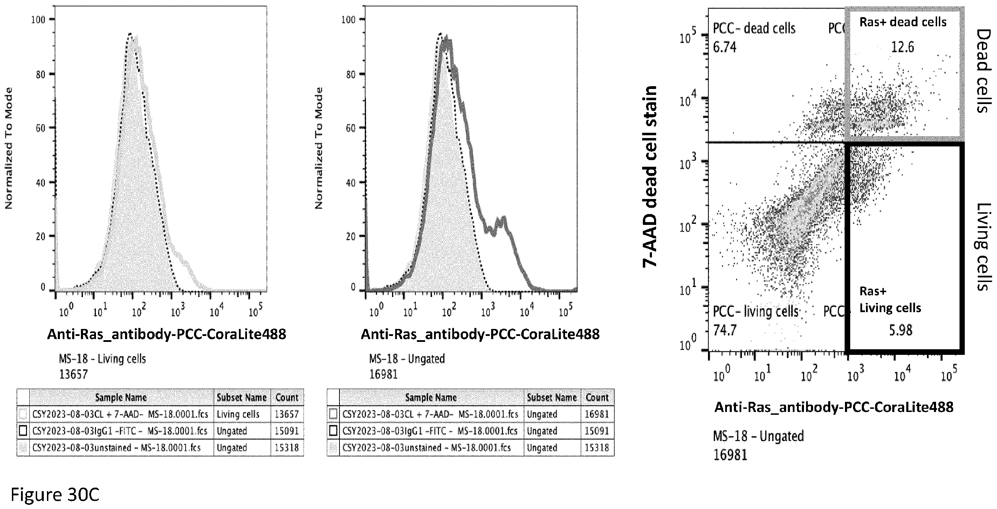

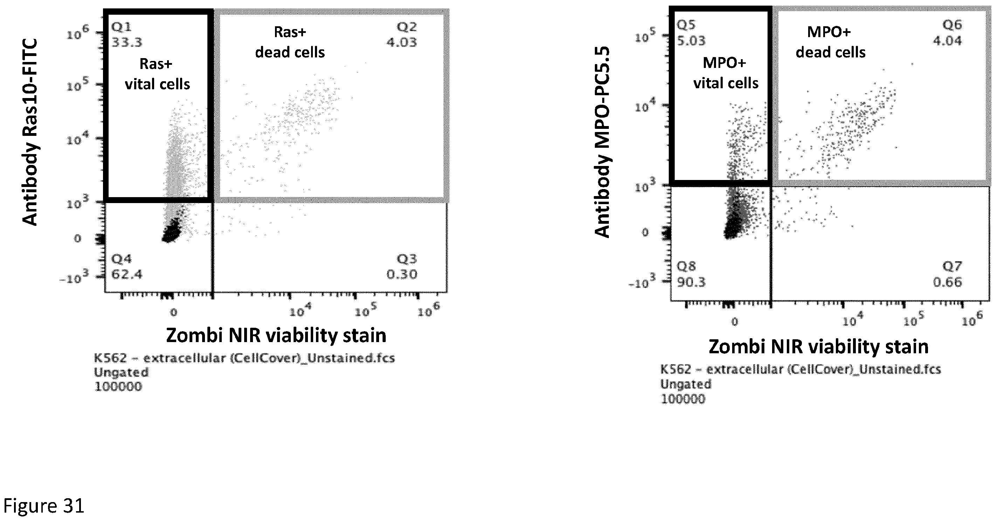

However, there are no prior art methods for detecting and/or measuring the expression of extracellular Ras proteins, in particular on the surface of living cells. There are three major challenges associated with such methods:
(i) Ras proteins are physiologically expressed at high amount in the cytosol of all nucleated cells. Extracellular Ras proteins, however, are expressed at significantly lower levels (Figure 30). This is a major risk for: (1) False positive results, if the intracellular Ras is detected and not separated from the extracellular Ras. (2) False negative results, if the detection of the intracellular Ras gives a background noise, similar or higher as the signal for the low extracellular Ras expression.
(ii) Extracellular Ras protein can also be expressed by dead or dying cells. It was shown herein that different human tumor cell lines express extracellular Ras protein on the surface of life and dead cells (Figure 30). The extracellular Ras expression on the dead cells can even be higher compared to the viable cells. In clinic and diagnostics, patient samples potentially arrive late during the day, or have to be shipped external for analysis. From the time point of extraction from the patient, the cell viability starts to deteriorate, increasing the dead or dying cell population. This is a major risk for: (1) False positive results, if the dead cell population is large and not clearly separated from the life population. (2) False negative, if the tumor cells are more susceptible to the ex vivo or shipping conditions and dye at higher rates as the surrounding healthy cells. (3) Analyzing failure if all or the majority of patient cells are dead.
(iii) Regular compounds used in cell based detection methods can potentially cause false positive results even when used according to protocol (Figure 31). Fixatives used in such methods may bring Ras detection antibody inside the cells without disrupting the cell membrane or killing the cells.

Therefore, in vitro methods for the detection of extracellular Ras proteins on the surface of cells are important tools for the use of the combinations and ABPs of the present invention.

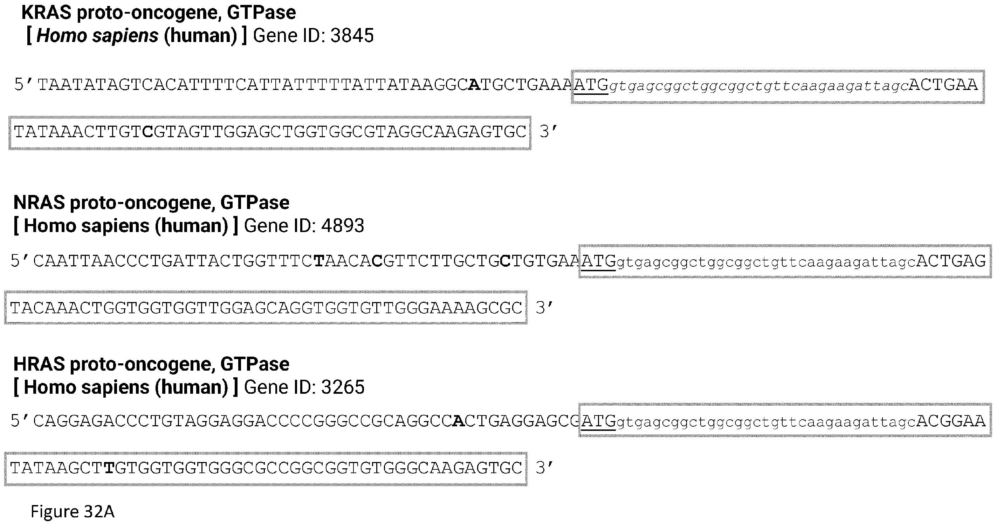

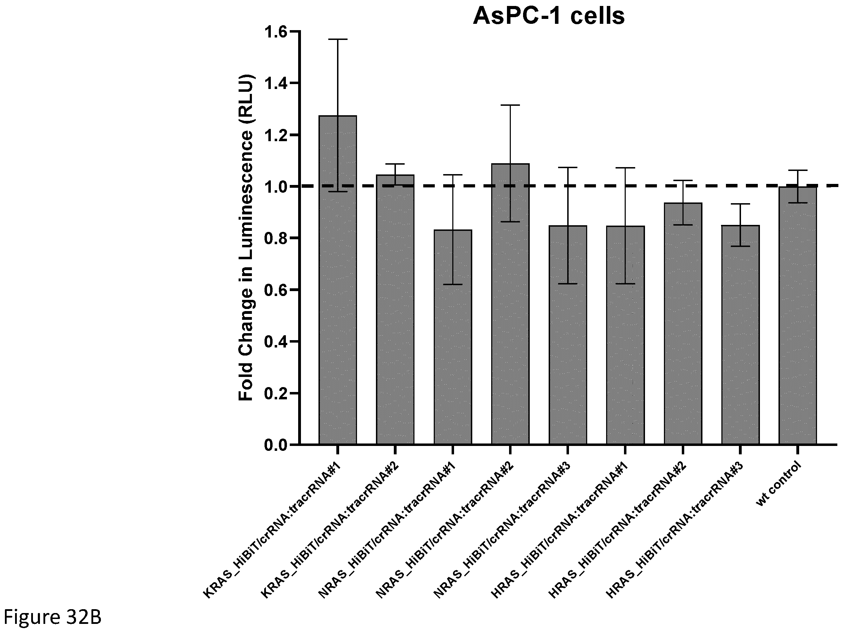

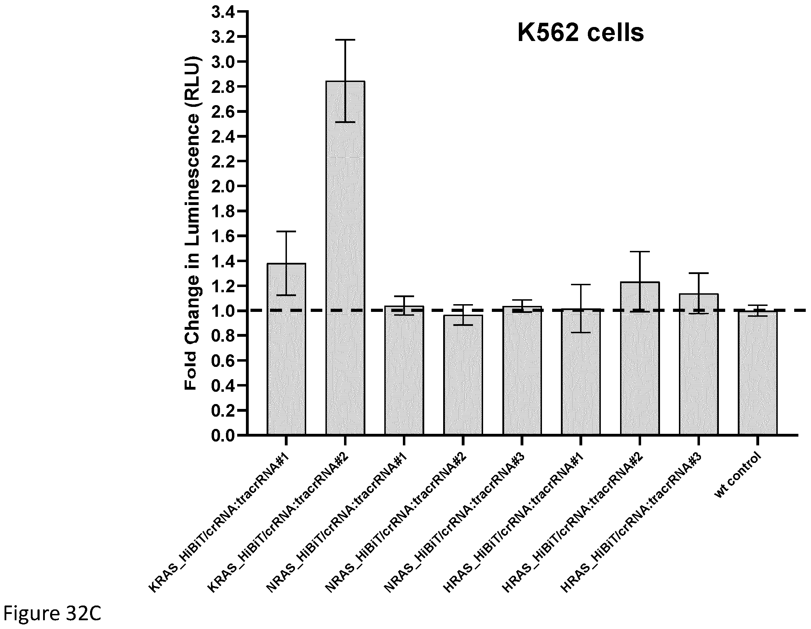

It is presently shown that using genetically modified cells expressing a small HiBiT tag fused to the amino-terminal end of KRAS, NRAS or HRAS, extracellular Ras protein can be detected (Figure 32). This method is using a split-tag for protein-protein interaction. One half of the tag is expressed with Ras, the other half is added into the cell medium not able to penetrate inside the cells. Only extracellular Ras can successfully complement the split-tag halves, reconstituting the luciferase reporter for detection. Another additional important benefit of this used HiBiT tag system is, no additional washing step of the labeled cells is necessary for detection. This is a huge benefit for every large-scale and high-throughput screening assays using hundreds or thousands of plates with cells.

In the present invention, an *in vitro* method is provided for detecting the expression of predominantly extracellular Ras proteins on the surface of predominantly living cells selected from the group consisting of
a) a method using a Ras protein labeling compound with substantially no penetration inside the cell and/or predominantly binding to the extracellular Ras protein
b) a method using an agent blocking binding to intracellular Ras proteins and/or using a washing step to substantially remove binding to intracellular Ras proteins
c) a method using a Ras protein labeling compound as of a) and one or more labeling compounds binding to intracellular proteins not belonging to the Ras protein family
d) a method using a dual-antigen protein-protein interaction (PPI) reporter
e) a method using cells expressing an altered Ras protein, preferably a Ras-tag fusion protein, wherein the tag can be detected on the extracellular side of the cell, optionally wherein the tag requires a second tag to be detectable (split-tag PPI)
f) a method using an ABP as defined herein
g) a method using 2D, 3D cell culture and/or spheroid or organoid cultures and/or stem cell-based embryo-like structures
h) a method combining two or more of a) to g).

The term "predominantly" in the sense of the present invention means "the most part of" or "most of". Hence, "predominantly living cells" means that most cells are living cells. Detecting the expression of predominantly extracellular Ras shall have the meaning that for the most part, the detected Ras protein is extracellular.
An *in vitro* method is also provided for detecting modulation of cell activity induced by binding of ABPs or compounds to extracellular Ras on the surface of predominantly living cells selected from the group consisting of
a) a method using an agent to detect a cytokine level
b) a method using at least one electrode to detect a change in cells' electrical potential
c) a method using genetically modified cells with a reporter plasmid to detect and/or measure activation of a signaling cascade
d) a method combining two or more of a) to c).

Further, an *in silico* method is provided for detecting modulation of extracellular Ras on the surface of artificial cells selected from the group consisting of
a) a method using artificial cell membranes
b) a method using artificial intelligence for calculating the extracellular Ras interface with small molecule compounds, with other proteins, with the lipid cell membrane and/or with the glycocalyx.

### Tag

In another embodiment, labeling compounds against altered Ras protein can be used in an in vitro detection method. Altered in the sense of cellular Ras is preferably man-made genetically modified on the genomic and/or protein level. Altered preferably means a tag fused to a Ras protein to result in a Ras-tag fusion protein. In one embodiment, cells with a specific tag cloned into their genome are used. The tag is for example integrated in one or more of the Ras gene coding sequences. In another embodiment, the tag is integrated outside the Ras gene coding sequences. On the protein level the tag may then interact with Ras protein forming heterodimers.

The tag may be modified to be active only on the extracellular side. For example the tag has a protease cleaving site, specific for intracellular proteases. Or the tag has a binding site for proteasomal degradation in the cytosol. Or the tag is connected via a protease cleaving site to a cap protein which is protecting the tag from binding to a reporter, and the specific protease is added and/or present only in the extracellular solution.

In one embodiment, the tag is a small split-tag for PPI with no own reporter activity. One tag-half may be expressed with the Ras protein, the second tag-half may be expressed with a cell membrane receptor or linked to a reporter compound added into the cell medium and stay extracellular. Only for extracellular Ras protein there may be a tag-tag complementation and a positive signal from the reporter.

For example, this could be the HiBiT tag or SpyTag system (Nano-Glo^{®} HiBiT) (SEQ ID NOs: 245 to 249). HiBiT is a small, 11-amino-acid, epitope tag (SEQ ID NO: 245) capable of complementation with the LgBiT partner (SEQ ID NO: 248). Alternative tags are also possible (SEQ ID NOs: 261 to 289).

The tag can be a split-protein for PPI with own reporter activity like fluorescent, luminescent or esterase. One half may be expressed with the Ras protein, the second half may be added into the cell medium or iexpressed by an extracellular protein. Only for the extracellular Ras there may be a complementation of the split-reporter, giving a signal for positive cells. Examples are split luciferase (1/2luc) complementation, or bimolecular luciferase complementation (BiLC) or split-GFP, split-RFP or split-YFP fluorescent protein complementation. Or a combination of complementation of split luciferase (CSL) and bioluminescence resonance energy transfer (BRET) approaches. Or a split BS2 esterase.

In some embodiments, reconstituted signals are driven by the colocalization of Ras protein with split-fragments on the cell surface. To improve the colocalization, the second fragment not specific for Ras protein, preferably has a specific targeting domain for the extracellular cell membrane, for example a domain against extracellular receptors, a ligand for a receptor or a domain against the cell glycocalyx.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of". The term "consist essentially of", where possible, in particular refers to embodiments wherein the subject-matter comprises 20% or less, in particular 15% or less, 10% or less or especially 5% or less further elements in addition to the specifically listed elements of which the subject-matter consists essentially of.

### DESCRIPTION OF THE FIGURES

Figure 1A shows the Diabody format used in this invention. All Diabodies use the same anti-CD3 binding variable-heavy (VH) and variable light (VL) chain domains (black filled ovals) from the UCHT1 clone (SEQ ID NO: 49) targeting the epsilon chain of the human CD3 receptor complex on T cells (SEQ ID NOs: 49, 53, 54, 55, 59, 60, 61, 64 and 65). The anti-CD3 VL and VH domains are connected via a flexible 20 amino acid long GS linker (grey solid lines) to the Ras ABP (white ovals). Every Diabody has a 6 His tag (SEQ ID NO: 33) connected via a two amino acid GS linker to the C-terminal end (black dotted line). Each Diabody has a different Ras specific ABP (PCC_01D to PCC_012D) (SEQ ID NOs: 20 to 31). The PCC01_D, PCC02_D and PCC03_D are using as Ras ABP the variable-heavy (VH) and variable light (VL) chains from Ras binding antibodies, connected via a 15 amino acid long flexible GS linker (black solid line). The PCC06_D and PCC07_D are using as Ras ABP a FN3 monobody domain. The PCC04_D, PCC05_D and PCC08D to PCC012_D are using as Ras ABP the isolated domains from human proteins with known Ras interaction.
Figure 1B shows an overview of the predicted binding sites to Ras protein for all 12 ABPs (PCC01 to PCC012) used in this invention. On the bottom is the Ras secondary structure with the different domains. The amino acids are numbered. Hypervariable domain (HVR) is important for attachment to the membrane. The black square filled boxes represent the ABP binding sites. Because of the complex tertiary and quaternary structure of Ras, most ABPs have contact with different domains of Ras. The binding site for PCC08 (containing the SOS2-CDC25H domain) was predicted to be similar to the binding site of PCC05 (containing the SOS1-CDC25H domain), due to lack of structural data for the SOS2-KRAS complex, but high similarity between the two CDC25H domains (86% for amino acids 780 - 1019). The binding site for PCC09 (containing the RASGRP3-CDC25 domain), PCC011 (containing the RASGRP1-CDC25 domain) and PCC012 (containing the RASGRP2-CDC25 domain) was predicted to be similar to the binding site of PCC05 (containing the SOS1-CDC25H domain), due to lack of structural data for the RASGRP3-, RASGRP2- and RASGRP1-KRAS complexes. For RASGRP1 the CDC25 domain consists of a compact bundle of 10 helices that forms the core of the structure. Two antiparallel and tightly packed helices that form a prominent hairpin protrude from this core. The structure of nucleotide-free Ras bound to the SOS-CDC25 domain showed that the switch 2 region of Ras docks on the helical bundle of the CDC25 domain. Based on the structural similarity of the CDC25 domains, empty Ras is expected to bind the CDC25 domain of RasGRP1. For RASGRP3 and RASGRP2 the binding to HRAS or NRAS is reported, but it is weaker compared to the binding to other members of the Ras family.

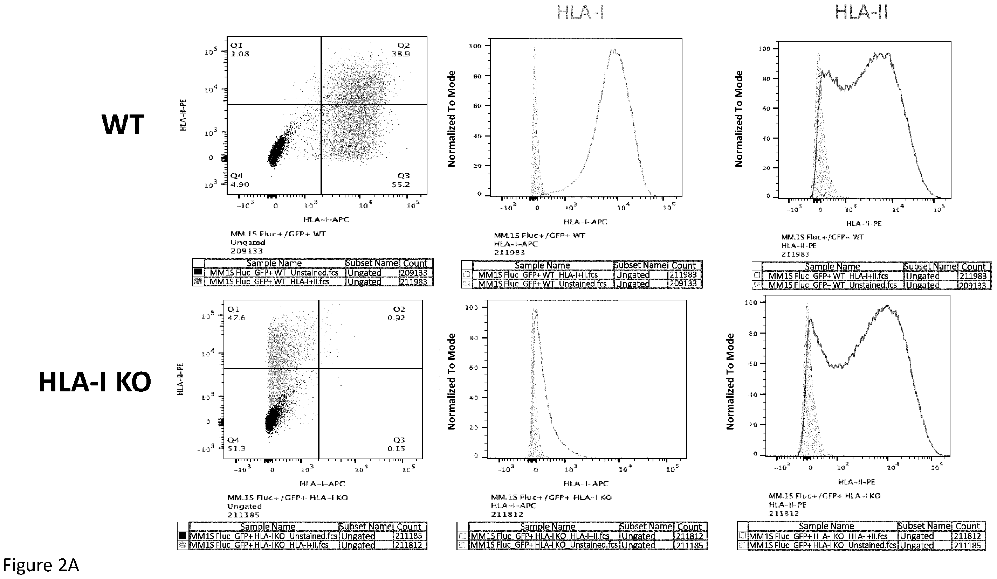

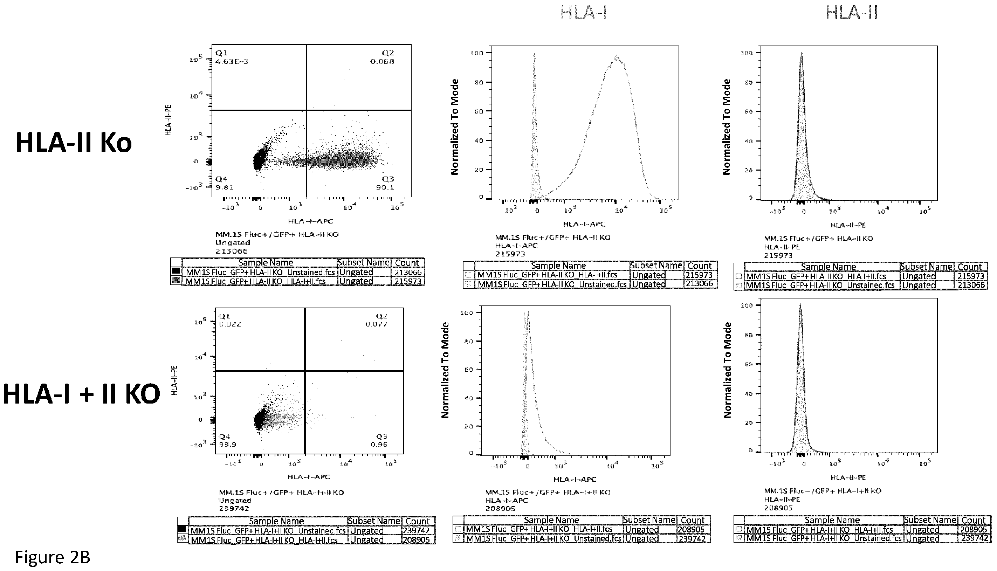

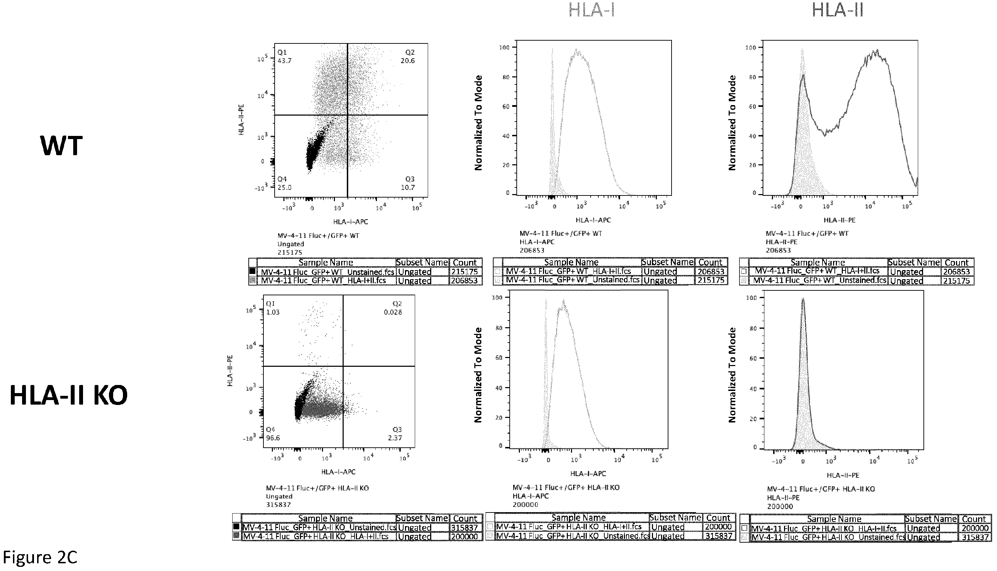

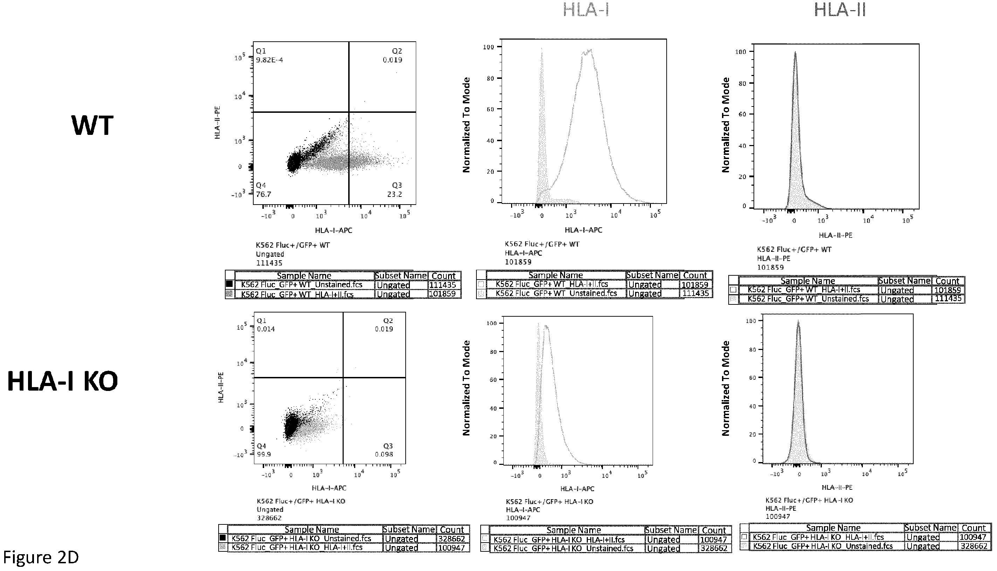

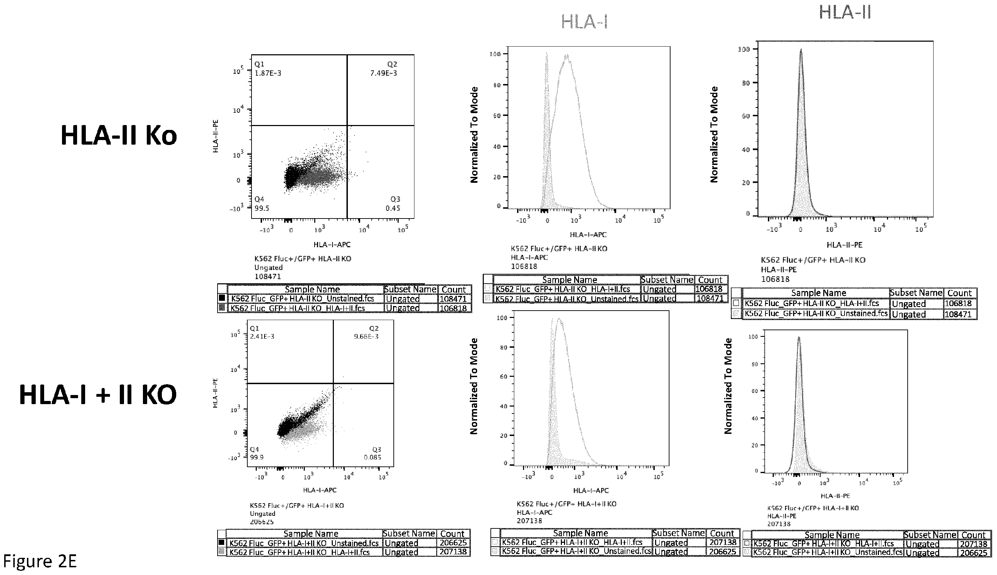

Figure 2 shows expression of HLA-I and HLA-II complex on the surface of different wild type (WT) and HLA knock-down (KO) tumor cells using Flow cytometry. Figure 2A shows MM1.S wild type cells (top row) and MM1.S cells with HLA-I knock-down (bottom row). HLA-I (light blue, middle column) and HLA-II (red, right column) is highly expressed on the wild type cells. In contrast in the HLA-I knock-down cells, the HLA-I expression is significantly reduced, but not the HLA-II expression. Figure 2B vice versa in MM1.S HLA-II knock-down cells (top row), the HLA-II expression (red, right column) is completely lost, but not the HLA-I expression (light blue, middle column). In HLA-I and HLA-II double knock-down cells (bottom row), the HLA-I expression (light blue, middle column) is significantly reduced and the HLA-II expression completely lost (red, right column). Figure 2C shows MV-4-11 wild type cells (top row) and MV-4-11 HLA-II knock-down cells (bottom row). HLA-I (light blue, middle column) and HLA-II (red, right column) is highly expressed on the wild type cells. In contrast in the HLA-II knock-down cells, the HLA-II expression is completely lost, but not the HLA-I expression. Figure 2D shows K562 wild type cells (top row) and K562 HLA-I knock-down cells (bottom row). HLA-I (light blue, middle column) is highly expressed on the wild type cells but not HLA-II (red, right column). In contrast in the HLA-I knock-down cells, the HLA-I expression is significantly reduced and the HLA-II negativity unchanged. Figure 2E shows in the K562 HLA-II knock-down cells (top row), the negative HLA-II expression (red, right column) is not changed, and the HLA-I expression (light blue, middle column) stays the same. In K562 HLA-I and HLA-II double knock-down cells (bottom row), the HLA-I expression (light blue, middle column) is significantly reduced and the negative HLA-II expression unchanged (red, right column).

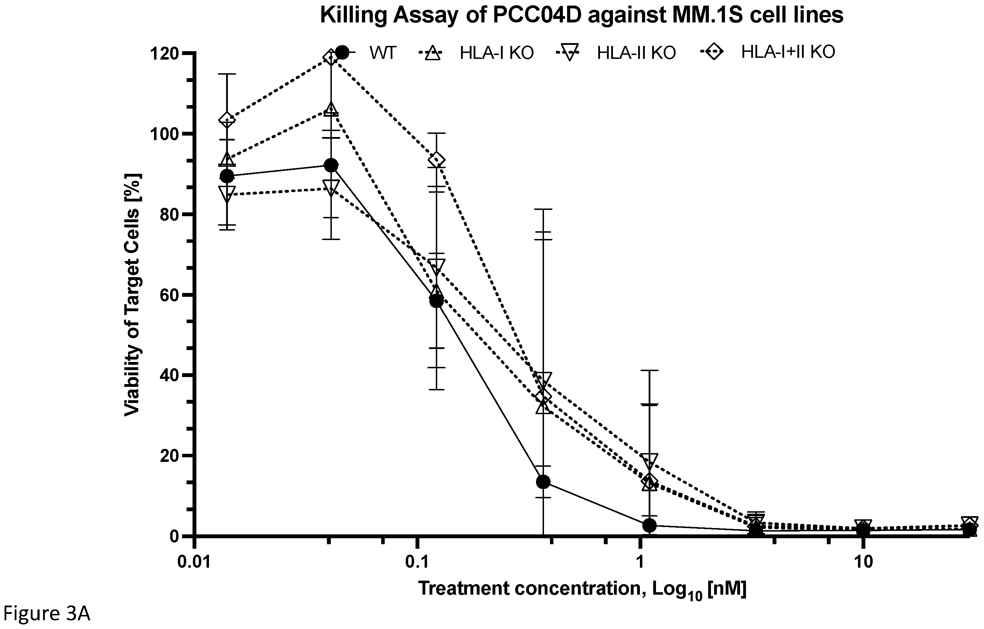

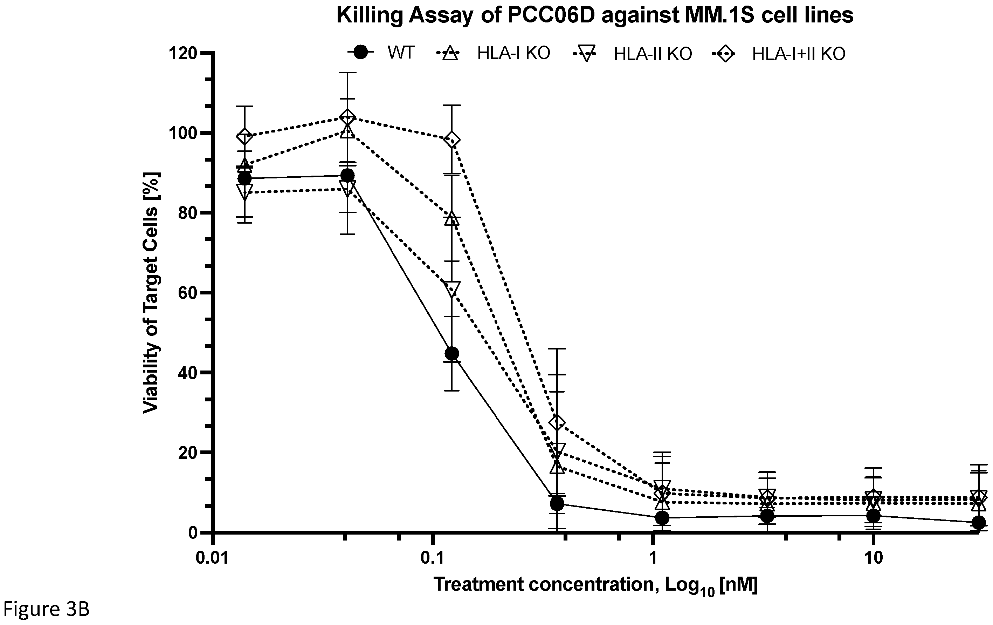

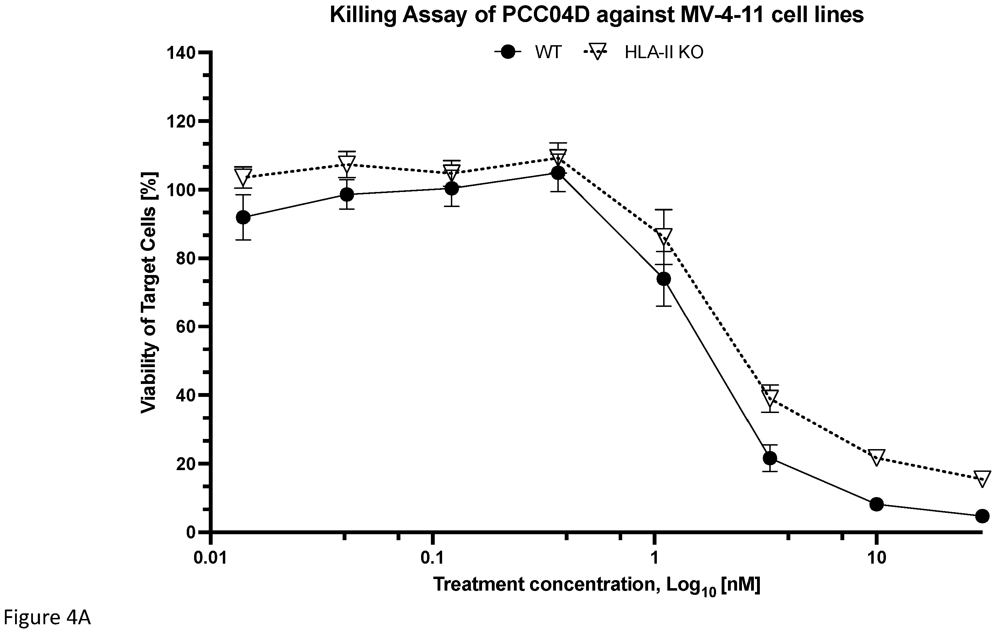

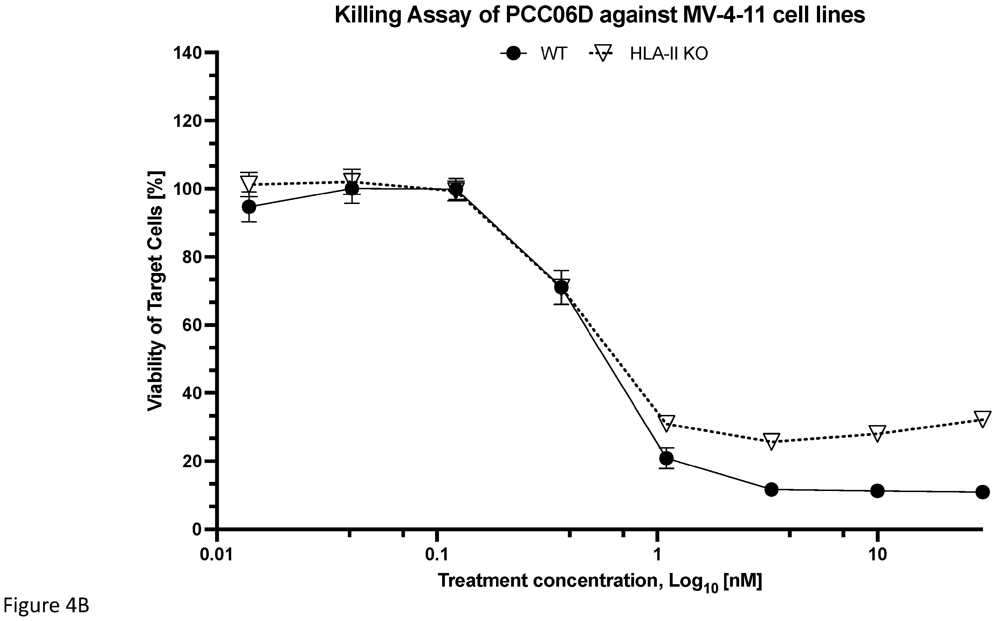

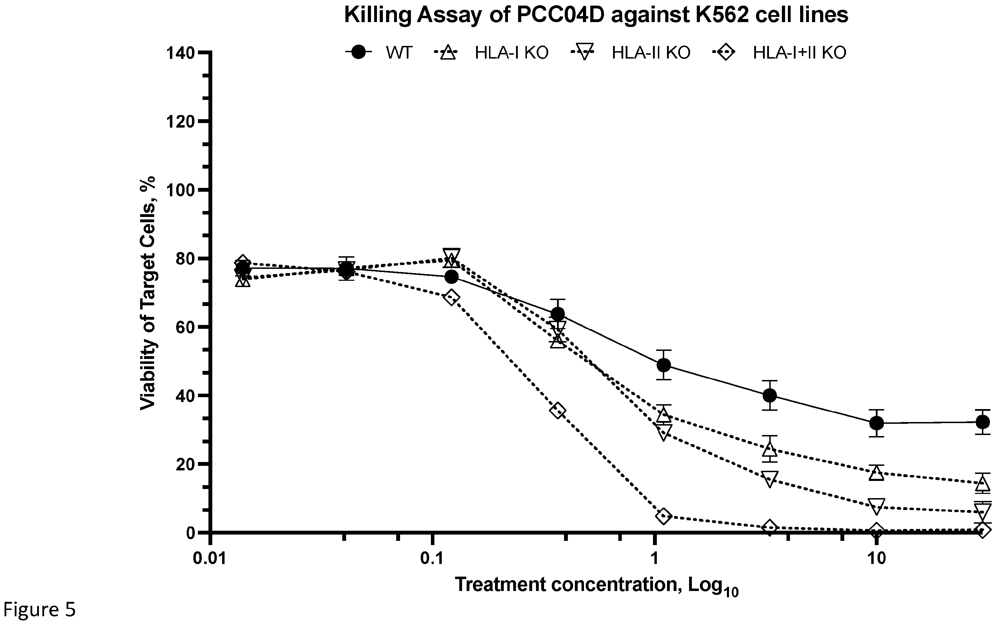

Figures 3, 4, and 5 shows *in vitro* killing in regular 2D cell culture for the different wild type (WT) and HLA knock down (KO) cell lines, analyzed in Figures 2 with Flow cytometry, using two different T cell activating Diabodies PCC04D (SEQ ID NO: 231) and PCC06D (SEQ ID NO: 233) against extracellular Ras. The Diabodies were utilized in a concentration gradient from 30 nM to 0.014 nM, using 1 to 3 dilution steps. The CD8+ T cell to tumor cell (E:T) ratio was 5 to 1. The viability of the luciferase positive tumor cells was measured after 48 hours incubation. Figure 3A shows extraordinary and high killing activity of a construct (PCC04D) against different HLA positive and negative MM1.S cells according to this invention. Against the wild type cells (black circle, solid line), against the HLA-I knock-down cells (white upright triangle, black spotted line), against the HLA-II knock-down cells (white upside-down triangle, black spotted line) and against the HLA-I+II double knock-down cells (white diamond, black spotted line). Importantly, these results show for the anti Ras directed ABPs of this invention, their binding and T cell engaging function is not restricted to HLA alleles presented on the tumor cell surface by the MHC. These results clearly indicate that said extracellular Ras antigen is not part of the HLA-peptide complex. Figure 3B shows analogous experiment to Figure 3A with extraordinary and high killing activity of a construct (PCC06D) against different HLA positive and negative MM1.S cells in low nanomolar range. Against the wild type cells (black spot, solid line), against the HLA-I knock-down cells (white upright triangle, black spotted line), against the HLA-II knock-down cells (white upside-down triangle, black spotted line) and against the HLA-I+II double knock-down cells (white diamond, black spotted line). Again, these results show for the anti Ras directed ABPs of this invention, their binding and T cell engaging function is not restricted to HLA alleles presented on the tumor cell surface by the MHC. Figure 4A shows extraordinary and high killing activity of a construct (PCC04D) against HLA positive and negative MV-4-11 cells according to this invention. Against the wild type cells (black circle, solid line) and against the HLA-II knock-down cells (white upside-down triangle, black spotted line). Figure 4B shows analogous experiments to Figure 4A with extraordinary and high killing activity of a construct (PCC06D) against HLA positive and negative MV-4-11 cells according to this invention. Against the wild type cells (black circle, solid line) and against the HLA-II knock-down cells (white upside-down triangle, black spotted line). Again, these results show for the anti Ras directed ABPs of this invention, their binding and T cell engaging function is not restricted to HLA allel presented on the tumor cell surface by the MHC. These results clearly indicate that said extracellular Ras antigen is not part of the HLA-peptide complex. Figure 5 shows extraordinary and high killing activity of a construct (PCC04D) against different HLA positive and negative K562 cells according to this invention. Against the wild type cells (black circle, solid line), against the HLA-I knock-down cells (white upright triangle, black spotted line), against the HLA-II knock-down cells (white upside-down triangle, black spotted line) and against the HLA-I+II double knock-down cells (white diamond, black spotted line). Interestingly, the tumor cell killing was even higher in the HLA knock-down cells compared to the wild type cells in higher nM range. Importantly, these results again show for the anti Ras directed ABPs of this invention, their binding and T cell engaging function is not restricted to HLA alleles presented on the tumor cell surface by the MHC. These results clearly indicate that said extracellular Ras antigen is not part of the HLA-peptide complex.
Figure 6A shows an illustration of the regular 2D cell culture (top) used in the experiments depicted in Figures 3 to 5, 29 and 30, and the 3D co-culture assay (bottom) used in the experiments depicted in Figures 7 to 28. In regular 2D cell culture tumor cells (white cells with black spots) are mixed with healthy T cells (round grey cells). In 3D co-culture different non-malign cells (grey cells) are mixed with the tumor cells. Also in the co-culture assay is methylcellulose (grey mesh) to form a semi-solid medium, and Corning Matrigel^{®} a solubilized basement membrane preparation extracted from the Engelbreth-Holm-Swarm mouse sarcoma, a tumor rich in extracellular matrix proteins, including laminin, collagen IV, heparan sulfate proteoglycans and entactin/ nidogen. Figure 6B shows human pancreatic cancer cell line Panc-1 in regular 2D cell culture medium (left), growing adherent mostly in monolayer with a spindle shape form. The Panc-1 cells growing in 3D co-culture medium (right), together with healthy human HUAEC and NHDF cells, forming cell clusters.
Figures 7 to 25 and 27 to 28 show the extraordinary benefit for a combination therapy of different small molecule inhibitors (SMI) with anti Ras directed construct of this invention. For this, the 3D co-culture assay was used with human CD8 positive T cells, the Diabody PCC04D (SEQ ID NO: 231) and different human tumor cells (AsPc-1, Panc-1 and MS18). The Diabody was used in a low nM concentration gradient. Different controls were used to distinguish the anti-tumor effects of the respective single components concerning the Diabody, the SMI and the T cells, from the anti-tumor effect of the respective Diabody and SMI combination therapy. For this, the SMI effect on the tumor cells was analyzed at two different concentrations (horizontal dotted line, SMI low concentration only) (horizontal solid line, SMI high concentration only). The effect of a Diabody PCC04D (SEQ ID NO: 231) monotherapy against the tumor cells was also analyzed (grey dotted line, PCC_04 only). Tumor cells with CD8+ T cells (cells + T cells) and tumor cells only (cells only) were used as negative control to exclude possible T-cell response against cell line neoantigens. 6% DMSO in cell culture medium was used as positive control for tumor cell killing (cells + 6% DMSO). For the combination therapy, the Diabody was used in the same concentration gradient as in the PCC04 monotherapy together with the respective SMI at the same concentration as in the SMI only groups (black solid line with filled black rectangle, combination Diabody and SMI high concentration) (black dotted line with filled black circle, for combination Diabody and SMI low concentration).
Figure 7A shows in AsPc-1 co-culture, for ATRA with PCC04D a significant increase in anti-tumor activity for the combination therapy, using ATRA in low concentration (black dotted line with filled black circle). ATRA low concentration (400 nM) and high concentration (2 µM). Importantly, these results show for the anti Ras directed ABPs of this invention, the increase in therapeutic efficacy in combination with an ATRA based compound. Figure 7B shows analogous experiments to Figure 7A in Panc-1 co-culture, for ATRA with PCC04D a significant increase in anti-tumor activity for the combination therapy, using ATRA in high concentration (black solid line with filled black rectangle). ATRA low concentration (400 nM) and high concentration (2 µM). Importantly, these results again show for the anti Ras directed ABPs of this invention, the increase in therapeutic efficacy in combination with an ATRA based compound.

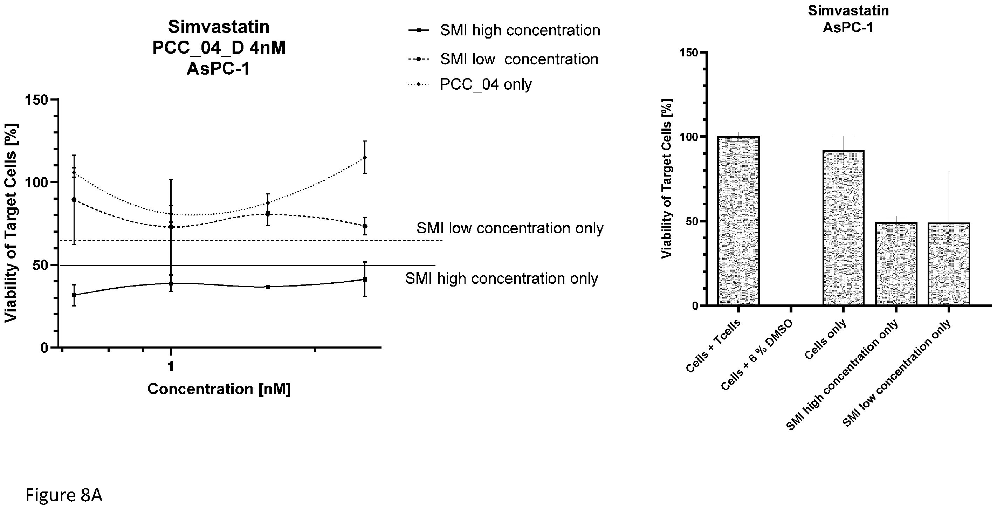

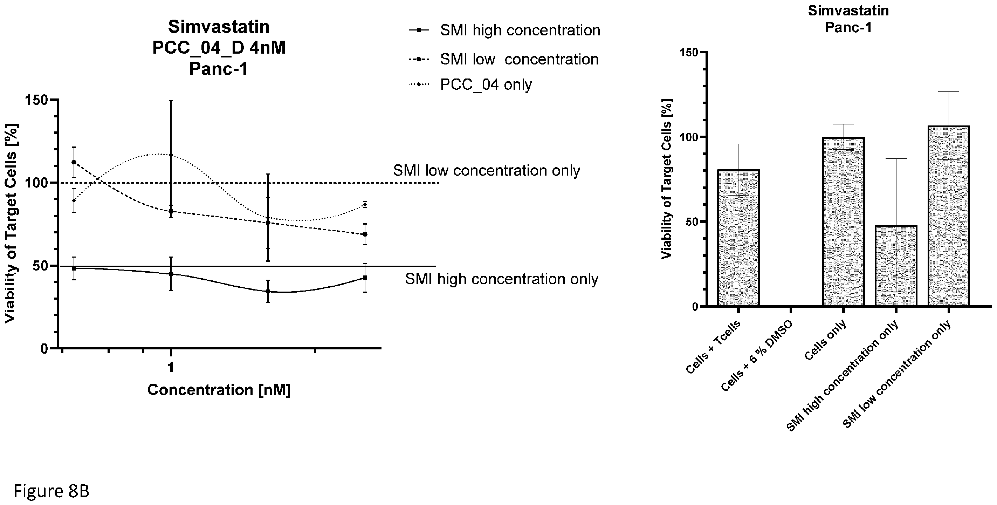

Figure 8A shows in AsPc-1 co-culture, for Simvastatin with PCC04D a significant increase in anti-tumor activity for the combination therapy, using Simvastatin in high concentration (black solid line with filled black rectangle). Simvastatin low concentration (2 µM) and high concentration (10 µM). Importantly, these results show for the anti Ras directed ABPs of this invention, the increase in therapeutic efficacy in combination with a Statin based compound. Figure 8B shows analogous experiments to Figure 8A in Panc-1 co-culture, for Simvastatin with PCC04D a significant increase in anti-tumor activity for the combination therapy, using Simvastatin in high concentration (black solid line with filled black rectangle). Simvastatin low concentration (2 µM) and high concentration (10 µM). Importantly, these results again show for the anti Ras directed ABPs of this invention, the increase in therapeutic efficacy in combination with a Statin based compound.

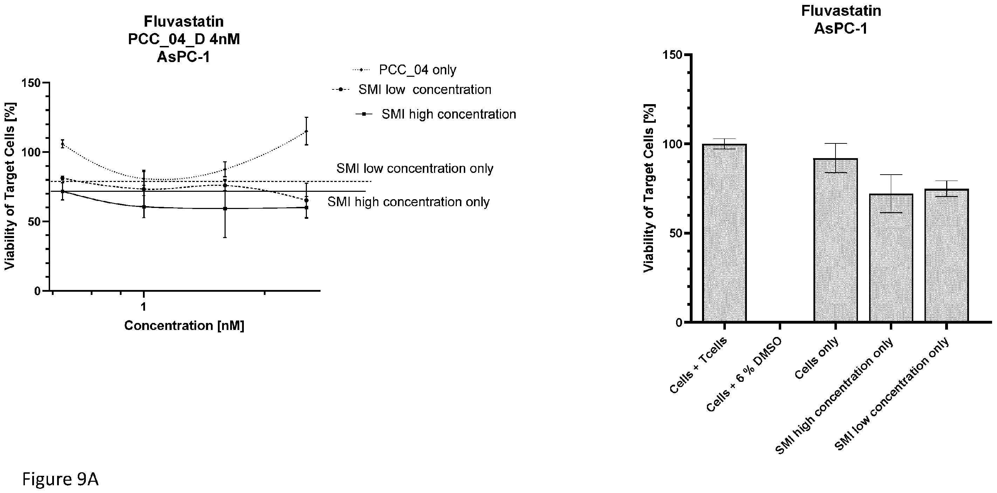

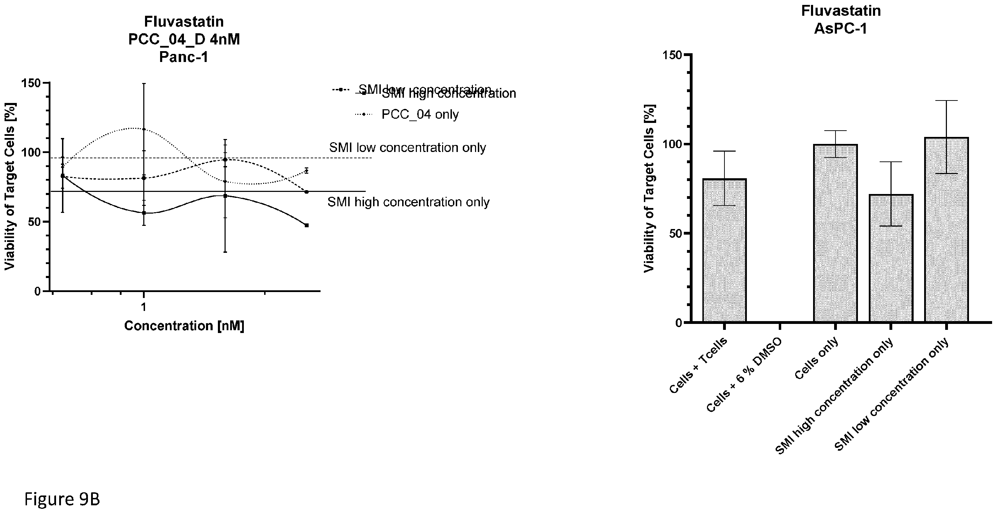

Figure 9A shows in AsPc-1 co-culture, for Fluvastatin with PCC04D a significant increase in anti-tumor activity for the combination therapy, using Fluvastatin in high concentration (black solid line with filled black rectangle). Fluvastatin low concentration (200 nM) and high concentration (1 µM). Importantly, these results show for the anti Ras directed ABPs of this invention, the increase in therapeutic efficacy in combination with a Statin based compound. Figure 9B shows analogous experiments to Figure 9A in Panc-1 co-culture, for Fluvastatin with PCC04D a significant increase in anti-tumor activity for the combination therapy, using Fluvastatin in high concentration (black solid line with filled black rectangle). Fluvastatin low concentration (200 nM) and high concentration (1 µM). Importantly, these results again show for the anti Ras directed ABPs of this invention, the increase in therapeutic efficacy in combination with a Statin based compound.

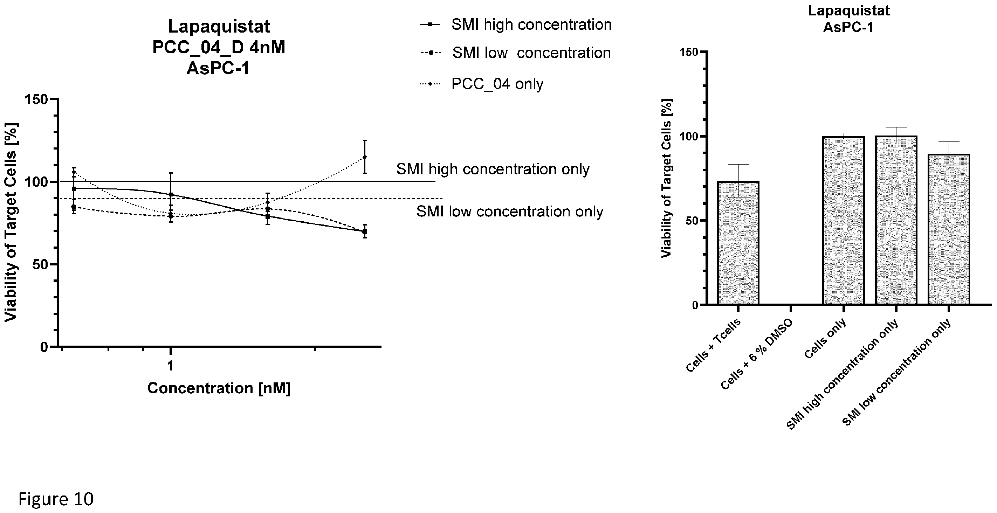

Figure 10 shows in AsPc-1 co-culture, for Lapaquistat with PCC04D at high concentration (4nM) a significant increase in anti-tumor activity for the combination therapy, using Lapaquistat in high concentration (black solid line with filled black rectangle) and low concentration (black dotted line with filled black circle). Lapaquistat low concentration (2 µM) and high concentration (10 µM). Importantly, these results show for the anti Ras directed ABPs of this invention, the increase in therapeutic efficacy in combination with a Statin based compound.

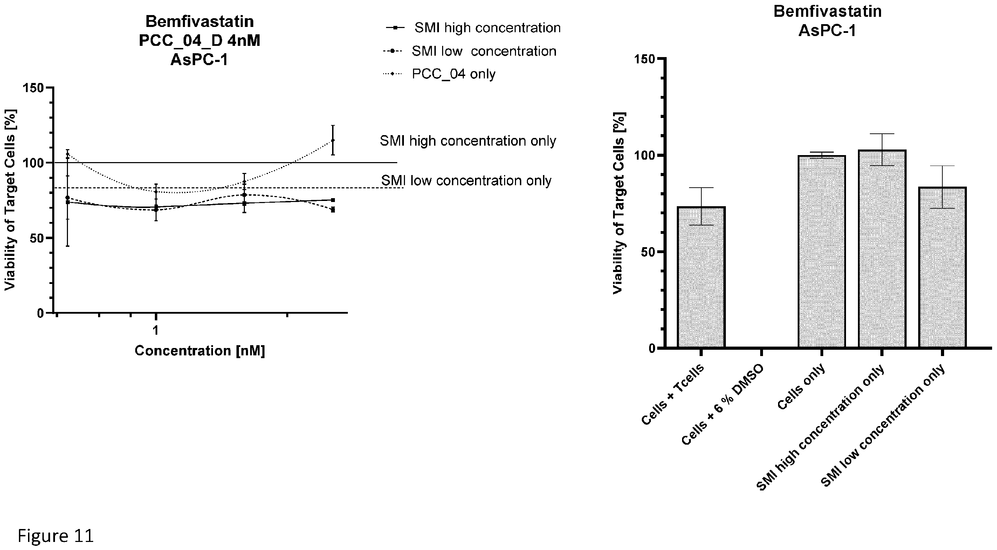

Figure 11 shows in AsPc-1 co-culture, for Bemfivastatin with PCC04D a significant increase in anti-tumor activity for the combination therapy, using Bemfivastatin in high concentration (black solid line with filled black rectangle) and low concentration (black dotted line with filled black circle). Bemfivastatin low concentration (2 µM) and high concentration (10 µM). Importantly, these results show for the anti Ras directed ABPs of this invention, the increase in therapeutic efficacy in combination with a Statin based compound.
Figure 12 shows in AsPc-1 co-culture, for Lenalidomide with PCC04D a significant increase in anti-tumor activity for the combination therapy, using Lenalidomide in high concentration (black solid line with filled black rectangle) and low concentration (black dotted line with filled black circle). Lenalidomide low concentration (2 µM) and high concentration (10 µM). Importantly, these results show for the anti Ras directed ABPs of this invention, the increase in therapeutic efficacy in combination with a cereblon (CRBN) or E3 ligase based compound or inhibitor.
Figure 13A shows in AsPc-1 co-culture, for Iberdomide with PCC04D a significant increase in anti-tumor activity for the combination therapy, using Iberdomide in high concentration (black solid line with filled black rectangle) and low concentration (black dotted line with filled black circle). Iberdomide low concentration (4 µM) and high concentration (20 µM). Importantly, these results show for the anti Ras directed ABPs of this invention, the increase in therapeutic efficacy in combination with a cereblon (CRBN) or E3 ligase based compound or inhibitor. Figure 13B shows analogous experiments to Figure 13A in Panc-1 co-culture, for Iberdomide with PCC04D at high concentration (4nM) a significant increase in anti-tumor activity for the combination therapy, using Iberdomide in high concentration (black solid line with filled black rectangle) and low concentration (black dotted line with filled black circle). Iberdomide low concentration (4 µM) and high concentration (20 µM). Importantly, these results again show for the anti Ras directed ABPs of this invention, the increase in therapeutic efficacy in combination with a cereblon (CRBN) or E3 ligase based compound or inhibitor.
Figure 14 shows in AsPc-1 co-culture, for HOMO-Protac cereblon degrader 1 with PCC04D at high concentration (4nM) a significant increase in anti-tumor activity for the combination therapy, using HOMO-Protac cereblon degrader 1 in high concentration (black solid line with filled black rectangle) and low concentration (black dotted line with filled black circle). HOMO-Protac cereblon degrader 1 low concentration (2 µM) and high concentration (10 µM). Importantly, these results show for the anti Ras directed ABPs of this invention, the increase in therapeutic efficacy in combination with a cereblon (CRBN) or E3 ligase based compound or inhibitor.
Figure 15 shows in AsPc-1 co-culture, for Eragidomide with PCC04D a significant increase in anti-tumor activity for the combination therapy, using Eragidomide in high concentration (black solid line with filled black rectangle) and low concentration (black dotted line with filled black circle). Eragidomide low concentration (20 nM) and high concentration (100 nM). Importantly, these results show for the anti Ras directed ABPs of this invention, the increase in therapeutic efficacy in combination with a cereblon (CRBN) or E3 ligase based compound or inhibitor.
Figure 16 shows in AsPc-1 co-culture, for NSC-70220 with PCC04D a significant increase in anti-tumor activity for the combination therapy, using NSC-70220 in high concentration (black solid line with filled black rectangle) and low concentration (black dotted line with filled black circle). NSC-70220 low concentration (2 µM) and high concentration (10 µM). Importantly, these results show for the anti Ras directed ABPs of this invention, the increase in therapeutic efficacy in combination with a Ras inhibiting or modulating compound.
Figure 17 shows in AsPc-1 co-culture, for SAH-SOS1A TFA with PCC04D a significant increase in anti-tumor activity for the combination therapy, using SAH-SOS1A TFA in high concentration (black solid line with filled black rectangle). SAH-SOS1A TFA low concentration (4 µM) and high concentration (20 µM). Importantly, these results show for the anti Ras directed ABPs of this invention, the increase in therapeutic efficacy in combination with a Ras inhibiting or modulating compound.
Figure 18A shows in AsPc-1 co-culture, for Adagrasib with PCC04D at high concentration (4nM) a significant increase in anti-tumor activity for the combination therapy, using Adagrasib in high concentration (black solid line with filled black rectangle) and low concentration (black dotted line with filled black circle). Adagrasib low concentration (600 nM) and high concentration (3 µM). Importantly, these results show for the anti Ras directed ABPs of this invention, the increase in therapeutic efficacy in combination with a Ras inhibiting or modulating compound. Figure 18B shows analogous experiments to Figure 18A in MS-18 co-culture, for Adagrasib with PCC04D a significant increase in anti-tumor activity for the combination therapy, using Adagrasib in high concentration (black solid line with filled black rectangle). Adagrasib low concentration (600 nM) and high concentration (3 µM). Importantly, these results again show for the anti Ras directed ABPs of this invention, the increase in therapeutic efficacy in combination with a Ras inhibiting or modulating compound.
Figure 19 shows in AsPc-1 co-culture, for BI-2852 with PCC04D a significant increase in anti-tumor activity for the combination therapy, using BI-2852 in high concentration (black solid line with filled black rectangle) and low concentration (black dotted line with filled black circle). Bl-2852 low concentration (4 µM) and high concentration (20 µM). Importantly, these results show for the anti Ras directed ABPs of this invention, the increase in therapeutic efficacy in combination with a Ras inhibiting or modulating compound.
Figure 20A shows in Panc-1 co-culture, for MRTX-1133 with PCC04D at high concentration (2nM and 4nM) a significant increase in anti-tumor activity for the combination therapy, using MRTX-1133 in high concentration (black solid line with filled black rectangle). MRTX-1133 low concentration (600 nM) and high concentration (3 µM). Importantly, these results show for the anti Ras directed ABPs of this invention, the increase in therapeutic efficacy in combination with a Ras inhibiting or modulating compound. Figure 20B shows analogous experiments to Figure 20A in MS-18 co-culture, for MRTX-1133 with PCC04D at high concentration (2nM and 4nM) a significant increase in anti-tumor activity for the combination therapy, using MRTX-1133 in high concentration (black solid line with filled black rectangle) and low concentration (black dotted line with filled black circle). MRTX-1133 low concentration (600 nM) and high concentration (3 µM). Importantly, these results again show for the anti Ras directed ABPs of this invention, the increase in therapeutic efficacy in combination with a Ras inhibiting or modulating compound.
Figure 21 shows in AsPc-1 co-culture, for RMC-0331 with PCC04D at high concentration (2nM and 4nM) a significant increase in anti-tumor activity for the combination therapy, using RMC-0331 in low concentration (black dotted line with filled black circle). RMC-0331 low concentration (2 µM) and high concentration (10 µM). Importantly, these results show for the anti Ras directed ABPs of this invention, the increase in therapeutic efficacy in combination with a Ras inhibiting or modulating compound.
Figure 22 shows in AsPc-1 co-culture, for SOS1-activator 1 with PCC04D a significant increase in anti-tumor activity for the combination therapy, using SOS1-activator 1 in high concentration (black solid line with filled black rectangle) and low concentration (black dotted line with filled black circle). SOS1-activator 1 low concentration (400 nM) and high concentration (2 µM). Importantly, these results show for the anti Ras directed ABPs of this invention, the increase in therapeutic efficacy in combination with a Ras inhibiting or modulating compound.
Figure 23 shows in AsPc-1 co-culture, for Narciclasine with PCC04D at high concentration (2nM and 4nM) a significant increase in anti-tumor activity for the combination therapy, using Narciclasine in low concentration (black dotted line with filled black circle). Narciclasine low concentration (4 nM) and high concentration (20 nM). Importantly, these results show for the anti Ras directed ABPs of this invention, the increase in therapeutic efficacy in combination with a Rho kinase pathway inhibiting or modulating compound.
Figure 24 shows in AsPc-1 co-culture, for Ruxolitinib with PCC04D a significant increase in anti-tumor activity for the combination therapy, using Ruxolitinib in low concentration (black dotted line with filled black circle). Ruxolitinib low concentration (4 µM) and high concentration (20 µM). Importantly, these results show for the anti Ras directed ABPs of this invention, the increase in therapeutic efficacy in combination with a Jak signaling pathway inhibiting or modulating compound.
Figure 25 shows in AsPc-1 co-culture, for Anagrelide with PCC04D a significant increase in anti-tumor activity for the combination therapy, using Anagrelide in high concentration (black solid line with filled black rectangle) and low concentration (black dotted line with filled black circle). Anagrelide low concentration (4 µM) and high concentration (20 µM). Importantly, these results show for the anti Ras directed ABPs of this invention, the increase in therapeutic efficacy in combination with a phosphodiesterase inhibiting or modulating compound.
Figure 26A shows in Panc-1 co-culture, for a mixture of different lipids with PCC04D a significant increase in anti-tumor activity for the combination therapy. The lipid mix contained the fatty acids arachidonic, linoleic, linolenic, myristic, oleic, palmitic and stearic acid and cholesterol (Lipid Mixture 1, Chemically Defined, L0288, Sigma-Aldrich^{®}), and was used in a concentration gradient from 2.0% to 0.06% (Concentration %). For this experiment two similar Panc-1 co-cultures were used, one with medium only containing the lipid mix gradient (solid line with filled black rectangles, Lipid Mix only) and one with medium containing the lipid mix gradient and a fix concentration of 5nM PCC04D (dotted line with filled black circles). The anti-tumor efficacy of the PCC04D as monotherapy was measured at 5nM in medium without lipid mix (small grey dotted horizontal line, 5nM PCC_04 only). Importantly, these results show for the anti Ras directed ABPs of this invention, the increase in therapeutic efficacy in combination with different fatty acids and cholesterol. Figure 26B shows analogous experiments to Figure 26A in MS-18 co-culture, for a mixture of different lipids with PCC04D a significant increase in anti-tumor activity for the combination therapy. The same methods and experimental setup was used as described in Figure 26A. Importantly, these results again show for the anti Ras directed ABPs of this invention, the increase in therapeutic efficacy in combination with different fatty acids and cholesterol.
Figure 27 shows in Panc-1 co-culture, for Dioncophylline A with PCC04D a significant increase in anti-tumor activity for the combination therapy, using Dioncophylline A in high concentration (black solid line with filled black rectangles). Dioncophylline A low concentration (50 nM) and high concentration (250 nM). Importantly, these results show for the anti Ras directed ABPs of this invention, the increase in therapeutic efficacy in combination with naphthylisoquinoline alkaloid compounds.

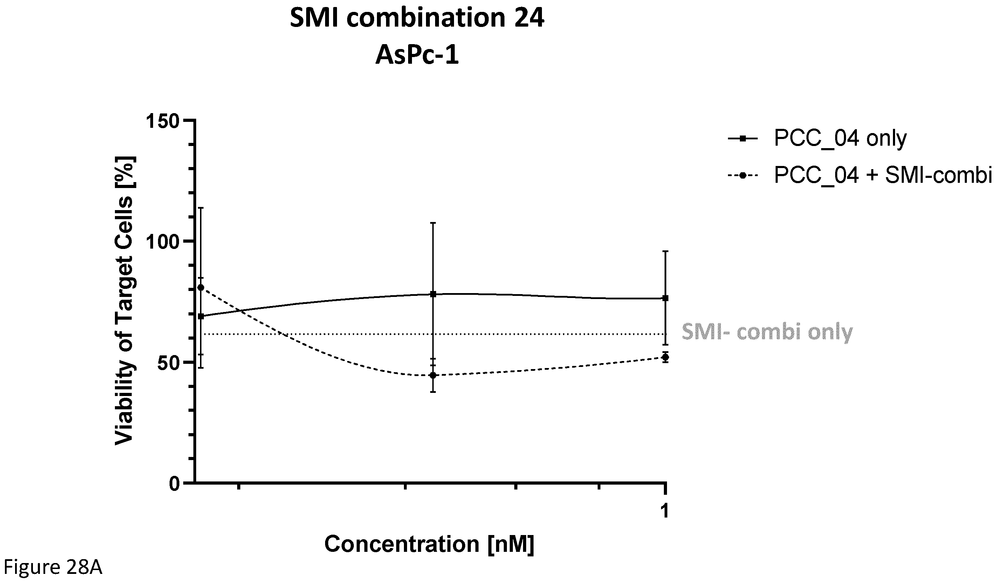

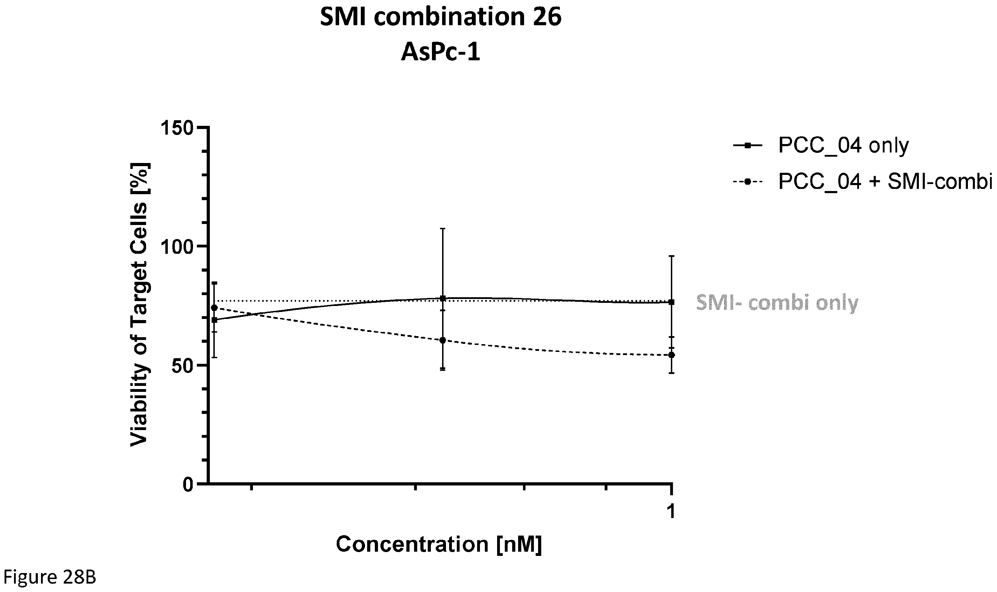

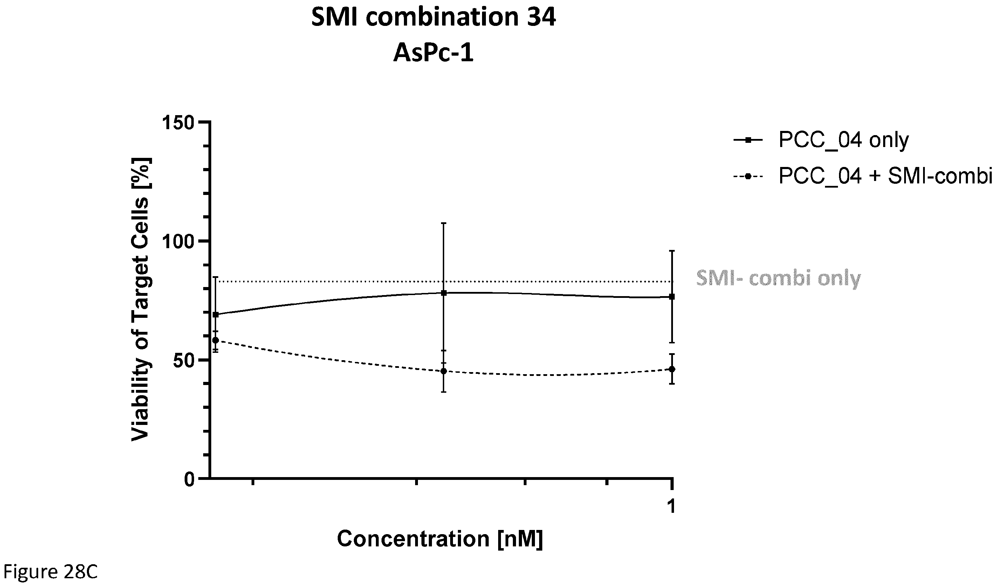

Figure 28A shows in AsPc-1 co-culture, for the combination of three inhibitors (Fluvastatin, CC-90009 and MRTX-1133) with PCC04D at high concentration (1nM and 0.5nM) a significant increase in anti-tumor activity for the combination therapy (dotted line with filled black circles) compared to PCC04D monotherapy (solid line with filled black rectangle). The anti-tumor effect of the three inhibitor combination without Diabody was also analyzed (small dotted horizontal line, SMI-combi only). Fluvastatin was used with 500nM, CC-90009 with 500nM and MRTX-1133 also with 500nM. Importantly, these results show for the anti Ras directed ABPs of this invention, the increase in therapeutic efficacy in combination with more than two compounds targeting each different pathways. Figure 28B shows analogous experiments to Figure 28A in AsPc-1 co-culture, for the combination of three inhibitors (Fluvastatin, Lenalidomide, and BI-2865) with PCC04D at high concentration (1nM and 0.5nM) a significant increase in anti-tumor activity for the combination therapy (dotted line with filled black circles) compared to PCC04D monotherapy (solid line with filled black rectangles). The anti-tumor effect of the three inhibitor combination without Diabody was also analyzed (small dotted horizontal line, SMI-combi only). Fluvastatin was used with 500nM, Lenalidomide with 500nM and BI-2865 also with 500nM. Importantly, these results show again for the anti Ras directed ABPs of this invention, the increase in therapeutic efficacy in combination with more than two compounds targeting each different pathways. Figure 28C shows analogous experiments to Figure 28A and 28B in AsPc-1 co-culture, for the combination of three inhibitors (Fluvastatin, Lenalidomide, and SOS1-activator 1) with PCC04D at high concentration (1nM and 0.5nM) a significant increase in anti-tumor activity for the combination therapy (dotted line with filled black circles) compared to PCC04D monotherapy (solid line with filled black rectangles). The anti-tumor effect of the three inhibitor combination without Diabody was also analyzed (small dotted horizontal line, SMI-combi only). Fluvastatin was used with 500nM, Lenalidomide with 500nM and SOS1-activator 1 also with 500nM. Importantly, these results show again for the anti Ras directed ABPs of this invention, the increase in therapeutic efficacy in combination with more than two compounds targeting each different pathways.

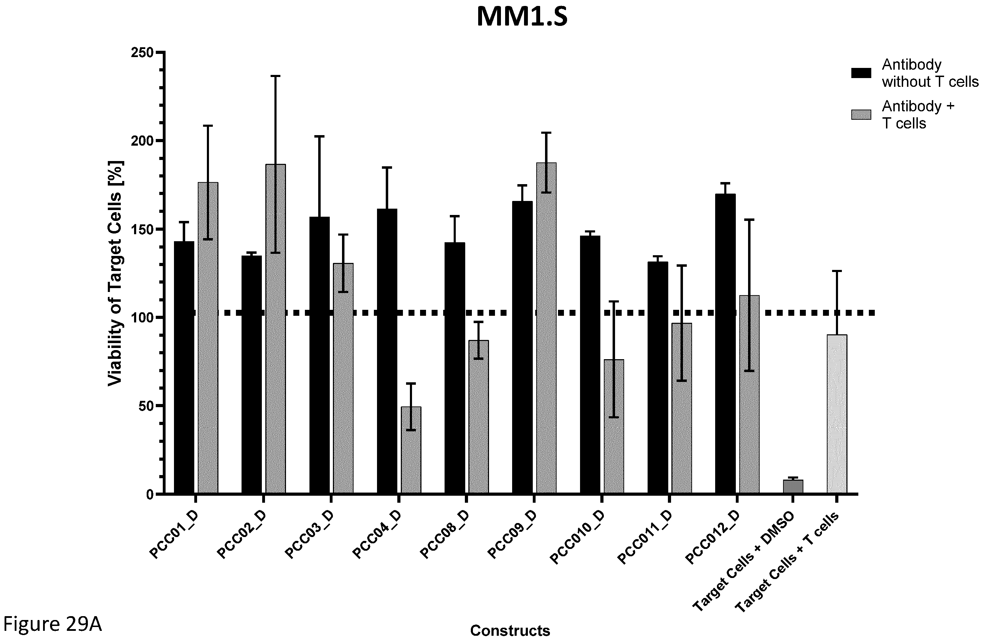

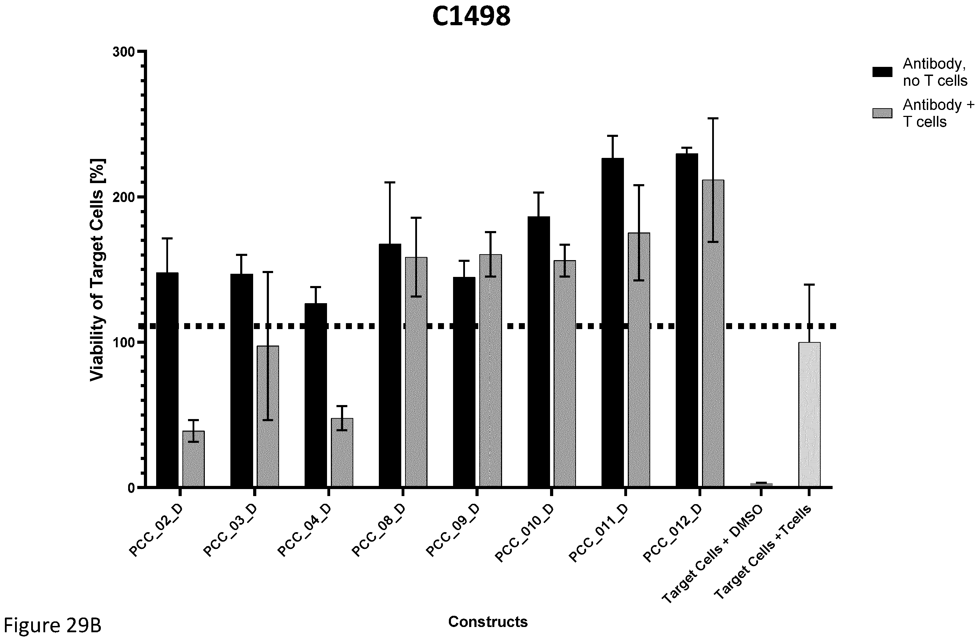

Figure 29A shows 9 different anti Ras Diabodies (SEQ ID NOs: 228 to 239) against human MM1.S cells in the absence of human T cells (black bars = Antibody without T cells) and in the presence of human CD8+ T cells (grey bars = Antibody + T-cells). The Diabody concentration in the medium was 30 nM. No Diabody showed strong or moderate killing activity in the absence of T cells. But without T cells there was a strong stimulation of cells compared to untreated cells. The addition of T cells was able to abrogate this proliferative effect via tumor cell killing, for example for PCC04D, but not for all constructs. Importantly, these results indicate for the anti Ras directed ABPs of this invention, there is possible direct stimulatory effect on the target cells metabolism. Figure 29B shows analogous experiments to Figure 29A for 8 different anti Ras Diabodies (SEQ ID NOs: 228 to 239) against the murine C1498 cells in the absence of human T cells (black bars = Antibody without T cells) and in the presence of human CD8+ T cells (grey bars = Antibody + T-cells). The Diabody concentration in the medium was 30 nM. Again, no Diabody showed strong or moderate killing activity in the absence of T cells. But without T cells there was again a strong stimulation of cells compared to untreated cells. The addition of T cells was able to abrogate this proliferative effect via tumor cell killing, for example for PCC02D and PCC04D, but not for all constructs. Importantly, these results again indicate for the anti Ras directed ABPs of this invention, there is possible direct stimulatory effect on the target cells metabolism, also for different species.
Figure 30A shows the extracellular Ras expression on HL-60 cells using Flow cytometry. To measure the expression on living and dead cells, a cell viability stain was used. The Ras expression on the dead cells (grey boxed quadrant) was higher compared to the living cells (black boxed quadrant). Importantly, these results indicate for any diagnostic test used to detect Ras expression on the cell surface, to gain on specificity it is beneficial to exclude or minimize the influence of the expression on the dead cell population. Notably, the correct binding of a Ras detection construct to its target, on a cell population not the primary focus for prognostic evaluation (dead cells), has the risk to cause false positive results for every standard and/or current diagnostic test used in clinic today. Figure 30B shows in analogous experiments to Figure 30A the extracellular Ras expression on Panc-1 cells using Flow cytometry. To measure the expression on living and dead cells, a cell viability stain was used. The Ras expression on the dead cells (grey boxed quadrant) was higher compared to the living cells (black boxed quadrant). Importantly, these results again indicate it is beneficial for any diagnostic test to exclude the Ras expression on the dead cells. Figure 30C shows in analogous experiments to Figure 30A and 30B the extracellular Ras expression on MS-18 cells using Flow cytometry. To measure the expression on living and dead cells, a cell viability stain was used. The Ras expression on the dead cells (grey boxed quadrant) was again higher compared to the living cells (black boxed quadrant). Importantly, these results again indicate it is beneficial for any diagnostic test to exclude the Ras expression on the dead cells.
Figure 31 shows the false positive detection of extracellular Ras on K562 cells using a standard fixation method used for Flow cytometry. For this, cells were treated before antibody staining with CellCover fixative according to manufacturer's protocol. For extracellular Ras detection (left side), the same antibody clone (Ras10) was used as in Figures 30A-C. To control for intra cellular proteins, an antibody against the cytoplasmic protein MPO was used (right side). Both antibodies were used on the freshly fixed cells with an extracellular staining protocol. To measure the expression on living cells, a cell viability stain was used. The fixation caused a significant high unspecific binding of anti Ras detection antibody to the vital cells. This is most likely caused by fixative effects against the cell membrane, not high enough to kill the cells, but high enough for binding of the Ras detection antibody to the much larger intracellular Ras population. The binding of the MPO antibody to the cells supports the detection of intracellular proteins. Importantly, these results indicate for any diagnostic test used to detect Ras expression on the cell surface, to gain on specificity it is beneficial to exclude or minimize the effect of the large intracellular Ras expression for analysis. Notably, the correct binding of a Ras detection construct to its target, on a cell population which is the primary focus for prognostic evaluation (living cells), has the risk to cause false positive results for standard and/or current diagnostic test, if steps or methods are used which are not specifically optimized for the difference in the extracellular versus intracellular Ras expression ratios. Therefore, these data again clearly indicate the need for new and specific methods to detect extracellular Ras expression by this invention.
Figure 32A shows an illustration for the novel HiBiT-tag expression method used to screen for extracellular Ras protein expression on the cell surface. Depicted is the nucleotide insertion of a HiBiT tag into the DNA of three human RAS genes, KRAS (top), NRAS (middle) and HRAS (bottom). The DNA is shown in 5' to 3' orientation, with the ATG start codon (underlined), followed by the coding sequence for the amino acids (grey boxed), the point mutations used in the single-stranded oligodeoxynucleotides (ssODNs) (SEQ ID NOs: 252, 256, and 260) ordered as Ultramer^{®} DNA Oligo (fat black) and the inserted HiBiT sequence (small cursive letters). Figure 32B shows low expression of the KRAS-HiBiT-fusion (#1) on the cell surface of AsPc-1 cells. For this, the HiBiT tag was cloned separately into the three Ras genes, KRAS, NRAS and HRAS, using different CRISPR RNA (crRNA) (SEQ ID NOs: 250, 251, 253, 254, 255, 257, 258, and 259). Figure 32C shows in analogous experiments to Figure 32B extraordinarily high expression of the KRAS-HiBiT-fusion (#2) on the cell surface of K562 cells. The same CRISPR RNAs (crRNA) were used as for Figure 32B. Importantly, these results show novel detection methods using genetically modified cells, can be used to screen for extracellular Ras expression. Therefore, these data again clearly indicate the need for new and specific methods to detect extracellular Ras expression by this invention.

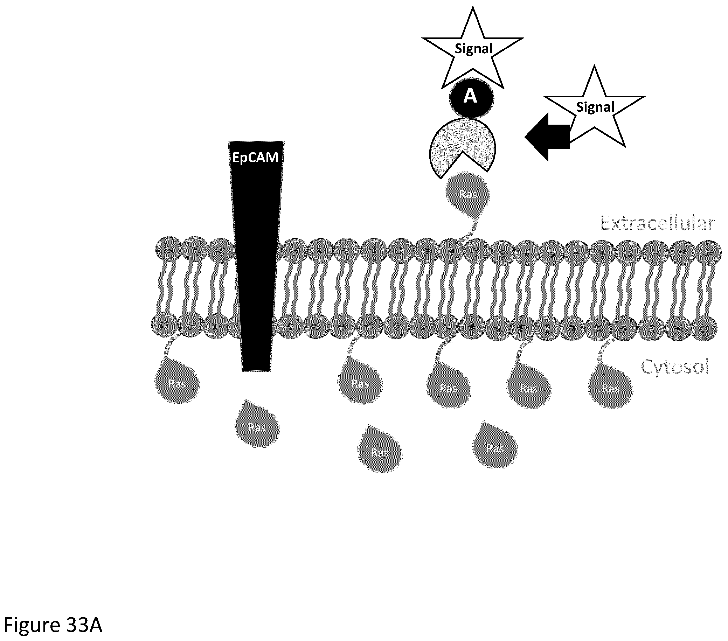

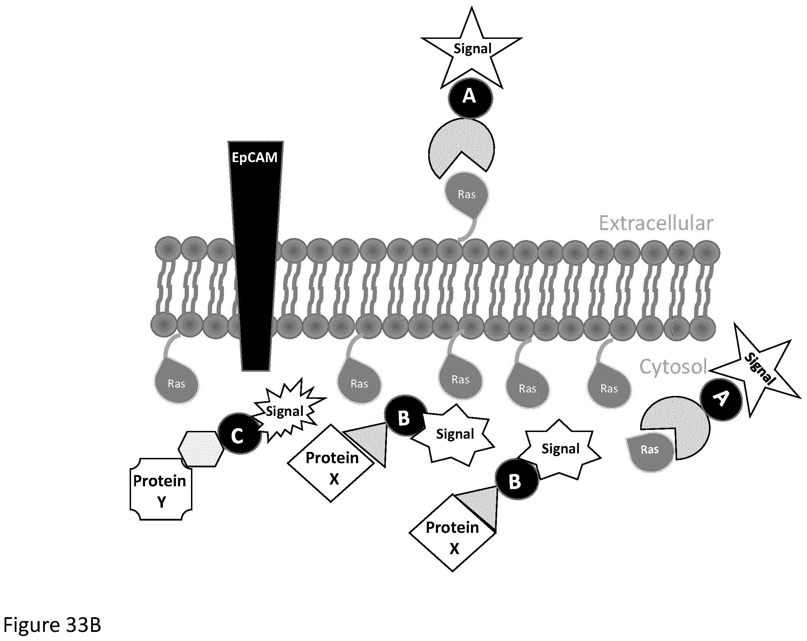

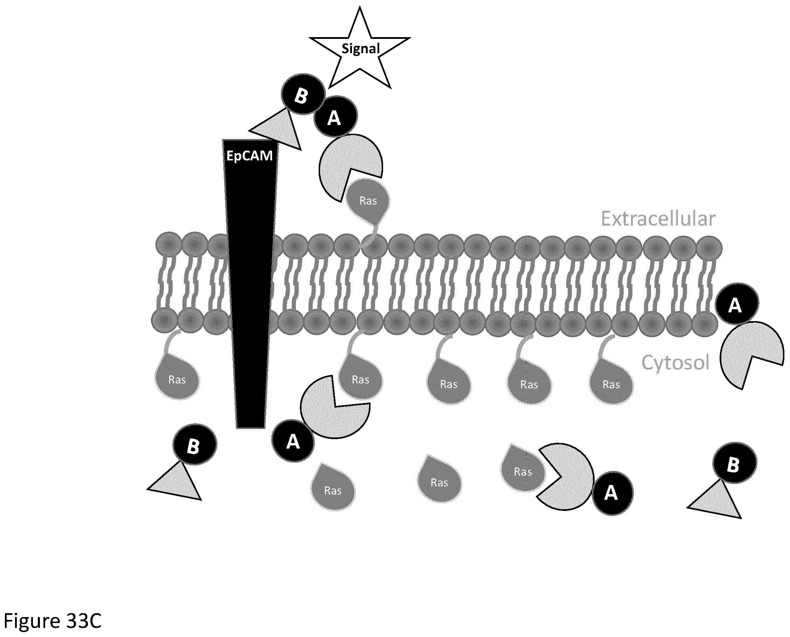

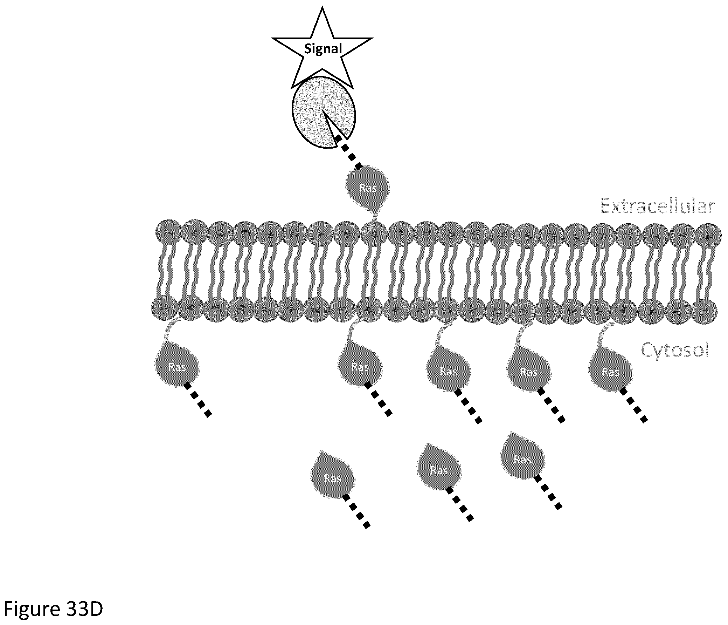

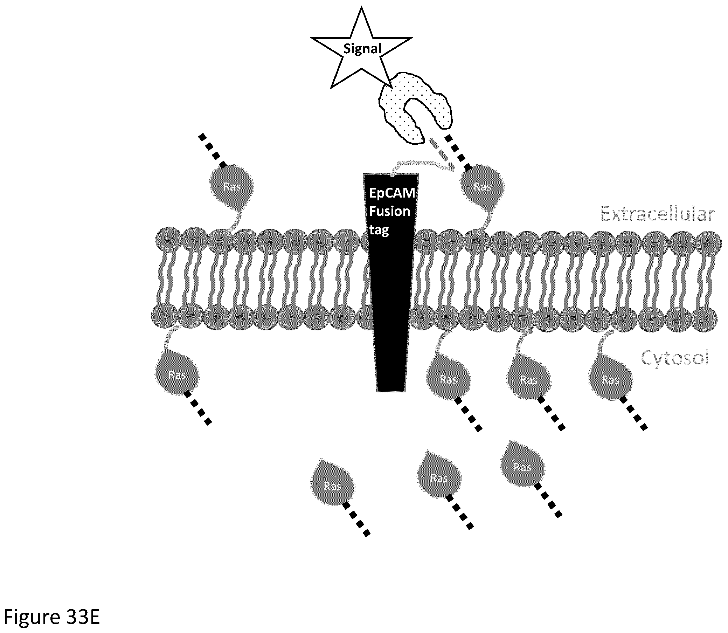

Figure 33A shows a possible *in vitro* method for detecting the expression of predominantly extracellular Ras proteins on the surface of predominantly living cells using a Ras-protein-labeling-compound (Ras-PLC) (grey pac-man circle) against extracellular Ras with substantially no penetration inside the cell. EpCAM represents a known transmembrane receptor (black square frustum), Ras proteins (grey tear-shaped). A reporter signal (white star) is directly labeled (A) to the Ras-PLC, or the reporter signal is indirectly labeled to the Ras-PLC (black arrow). Figure 33B shows another illustration of an *in vitro* method for detecting the expression of predominantly extracellular Ras proteins on the surface of predominantly living cells. Using in addition to the Ras-PLC (grey pac-man circle), one or more labeling-compounds against intracellular proteins not belonging to the Ras targets (grey triangles and grey hexagon), for detection of cells with damaged membranes and/or to preferably exclude them from analysis. Ras proteins (grey tear-shaped). A reporter signal (white 5 arm star) is directly labeled (A) to the Ras-PLC, and two other reporter signals (white 7 arm star and white 14 arm star) are directly labeled to the labeling-compounds against intracellular proteins not belonging to the Ras targets (B and C). Figure 33C shows another illustration of an *in vitro* method for detecting the expression of predominantly extracellular Ras proteins on the surface of predominantly living cells using a dual-antigen protein-protein interaction (PPI) reporter. For this, the first labeled protein is targeting Ras (grey pac-man circle) and the second labeled protein (grey triangle) is targeting the extracellular domain of a known membrane receptor, in this depicted case EpCAM (black square frustum), Ras proteins (grey tear-shaped). For detection, the reporter (white star) only gives a signal if both proteins bind to their respective targets in close proximity on the cell surface and reconstitute the reporter binding site (B-A). Figure 33D shows another illustration of an *in vitro* method for detecting the expression of predominantly extracellular Ras proteins on the surface of predominantly living cells using man-made genetically modified cells, expressing a Ras-tag fusion protein. Ras proteins (Ras, grey tear-shaped) fused with a tag (black dotted line). A detection compound, with preferably substantially no penetration inside the cell, will only detect extracellular Ras expression. Because there are modified tag-detection compounds with extremely high specificity and sensitivity, higher than the kd (dissociation constant) of regular antibodies, this method will be beneficial for large-scale screening assays detecting even small differences in extracellular Ras expression levels. Figure 33E shows another illustration of an *in vitro* method for detecting the expression of predominantly extracellular Ras proteins on the surface of predominantly living cells using man-made genetically modified cells, expressing two different tags each on a different antigen, for a split-tag PPI. One tag (black dotted line) is fused to the Ras proteins (Ras) and the second tag (grey dotted line) is fused with the extracellular part of a known cell membrane receptor, in this depicted case EpCAM (black square frustum). Only for extracellular Ras there is a tag-tag PPI complementation for the detection reporter (white star).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples. Standard methods of molecular biology were used (see, e.g. Sambrook et al., Molecular Cloning: A Laboratory Manual, Cold Spring Harbor Laboratory Press, New York (2001)).

### The examples show:

### 1. Isolation of human CD8+ T cells from blood healthy donors

CD8+ T cells, from healthy human donors, were used as effector cells to test the *in vitro* activity of the anti Ras antibodies. First, the mononuclear cells were separated from the peripheral blood through Ficoll density gradient centrifugation (Ficoll-Paque^{™} Plus, Cytivia) at 3000 rpm with acceleration at 5 and deceleration at 1 for 18 minutes. Afterward the separated peripheral blood mononuclear cells (PBMCs) were collected and washed with 40ml PBS. The PBMCs were then subjected to red blood cell lysis with 10 ml of Gibco's ACK lysing buffer for 15 minutes at room temperature. Then, the PBMCs were washed once with 40 ml PBS and proceed for magnetic selection according to the user manual of the magnetic beads provided by the company (CD8+ T Cell Isolation Kit, human, Miltenyi). A negative selection was used to isolate the CD8+ T cell population from the CD8 negative PBMC cells. Briefly, the number of PBMNCs was first determined and 1x 10⁸ PBMNCs were resuspended in 400 µl of MACS buffer (PBS at pH 7.2 supplemented with 0.5% BSA and 2 mM EDTA). Then, 200 µl of MicroBeads (to deplete CD8 negative cells) was added to the PBMNCs suspension, mixed well and incubated for 15 minutes and then washed with 50 ml MACS buffer and resuspended in 500 µl of MACS buffer. The labelled PBMNC were then loaded onto their respective columns according to the cell number. All magnetic labelled CD8-negative cells were captured on to the magnetic column while the CD8-positive cells were eluted by washing the column with MACS buffer. The CD8-positive cells were then collected and cultivated in RPMI1640 supplemented with 10% (v/v) FBS, 1% (v/v) GlutaMAX^{™}, 1% (v/v) penicillin-streptomycin-neomycin antibiotic mixture, 0.05 mM 2-mercaptoethanol and 50 IU/ml human IL-2 (# AF-200-02-100UG, ThermoFisher Scientific). In order to expand the cells, Gibco^{™} Dynabeads^{™} Human T-Activator CD3/CD28 for T Cell Expansion and Activation was added to the CD8+ T cells with one bead to five cells ratio and the expansion was carried out for five days. After the 5 days, the CD3/CD28 Dynabeads^{™} were captured and removed from the proliferating CD8 positive T cells, using a magnet. The CD8+ T cells were used directly for *in vitro* killing assays or cryopreserved in 50% (v/v) complete RPMI1640 supplemented with 45% (v/v) FBS and 10% (v/v) DMSO for future use.

### 2. PCC antibody generation

Custom gene synthesis services including chemical synthesis was used (GeneArt^{®}, Thermo Fisher Scientific Inc.). Each DNA plasmid had their specific restriction enzyme cloning sites at the end for subcloning into the expression plasmids. For the cloning of the Diabody constructs, a pMA-RQ plasmid was ordered containing the nucleotide sequence for the Diabody back bone with the split anti CD3 VL and VH domains (from the UCHT1 clone) already included (SEQ ID NO: 224). The 12 different Ras ABPs (PCC01 to PCC012, SEQ ID NOs: 20 to 31) were in the pCEP4 plasmid (SEQ ID NOs: 122 to 133). For sub-cloning their DNA sequences, amplification via PCR was used with specific primers for each construct (SEQ ID NOs: 251 to 274). For each PCR reaction was used: 36.25 µl water, 10 µl 5x Q5^{®} reaction buffer, 1 µl dNTP mix (10 µM), 1.25 µl Primer-mix forward and reverse (10 µM), 1 µl ABP-DNA-plasmid, 0.5 µl Q5^{®} High-Fidelity DNA polymerase (New England Biolabs GmbH) and with PCR cycling conditions (step-1 98°C for 1minute, step-2 98°C for 15 seconds, step-3 60°C for 30 seconds, step-4 72°C for 3 minutes, step-5 is go-to step-2 for 5 more times, step-6 98°C for 15 seconds, step-7 66°C for 30 seconds, step-8 72°C for 3 minutes, step-9 is go-to step-6 for 38 more times, final step-10 72°C for 3 minutes, then hold reaction at 12°C). The PCR product was loaded onto a 1% agarose gel and run for 1 hour at 80 volt. After the run, the individual bands were extracted using the MiniElute^{®} Gel extraction kit (^{©} QIAGEN 2013-24). Following restriction enzyme digestion of the Diabody backbone pMA-RQ plasmid and the gel extracted 12 ABPs (PCC01 to PCC012) with the FastDigest Restriction enzyme system from Thermo Scientific^{™}. The DNA was cut with the FastDigest Ehel (Thermo Scientific^{™}, #FD0444) and FastDigest BamHI (Thermo Scientific^{™}, #FD0054). Then, the cut Diabody backbone was dephosphorylated using the FastAP Thermosensitive Alkaline Phosphatase (Thermo Scientific^{™}, #EF0654). Subsequently, the ABPs were ligated into the Diabody backbone using the T4 DNA Ligase (New England BioLabs, #M0202S). For the ligation 30ng of linearized pMA-RQ vector DNA, 5 to 10ng DNA of PCC insert, 10x T4-ligase buffer and 5 U/µl T4 DNA Ligase, incubated at 16°C over night. The 5 µl ligation mixture was used for each construct for transformation of MachT1 cells for plasmid preparation as descript in section (3) Production of plasmids in bacteria.

The 12 different Diabodies (according to any one of SEQ ID NOs: 228 to 239) were sub-cloned into the inducible prokaryote expression plasmid pColdlV (Takara Bio Group, #3364). The pCOLD system is using the cold-shock Protein A (cspA) promoter for expression of high-purity, high-yield recombinant protein in E. coli. The vector selectively induces target protein synthesis at a low temperature (12 to 16°C), a condition which suppresses the expression of host proteins and decreases protease activity. This results in high yields of target proteins. For higher yields and correctly folded recombinant ABP production, a bacterial expression and folding leader was used at the N-terminal domain (starting at the Vector multiple cloning site mRNA transcription ATG start codon), the trigger factor from bacterium Pseudoalteromonas distincta (UniProt F3BFD4). Only the amino acid 112 to 434 domain from the original protein (F3BFD4), without the ribosom binding site, was used in this invention (SEQ ID NO: 243). For higher purity of recombinant ABP production, the CL7 tag protein (SEQ ID NO: 45), connected via a 15 amino acid long flexible GS linker (SEQ ID NO: 40) to a 3C protease cutting site (SEQ ID NO: 47), was connected to the individual folding leaders (SEQ ID NOs: 240, 226 and 227).

To ensure the correct cloning, 20 µl (300 - 800 ng DNA) of each plasmid was send for DNA sequencing by LGC Genomics (Berlin, Germany), using the sequencing primers pCold Fwd (5'-ACGCCATATCGCCGAAAGG-3'; SEQ ID NO: 275) and pCold Rev (5'-GGCAGGGATCTTAGATTCTG-3'; ; SEQ ID NO: 276).

### 3. Production of plasmids in bacteria

The different pColdlV vectors carrying the respective individual final Diabody sequences (PCC01_D, PCC02_D, PCC03_D, PCC04_D, PCC05_D, PCC06_D, PCC07_D, PCC08_D, PCC09_D, PCC010_D, PCC011_D and PCC012_D, SEQ ID NOs: 228 to 239), were transformed separately into *E. coli* Mach1 cells (Invitrogen, C862003). After short thawing E. *coli* cells on ice, 10 µL of plasmid Vector (10-100 ng DNA) was mixed with 90µL of cells by gentle pipetting and incubated for 20 minutes on ice. Afterwards, a heat-shock was performed at 42°C for 90 seconds followed by incubation on ice for 5 minutes. The cells were then transferred into 600 µL of 2xYT medium (Carl Roth, X966.3) and incubated at 37°C and 230 rpm for one hour. After a subsequent centrifugation at 13000 rpm for two minutes, 500 µL of the supernatant was discarded. The cells were resuspended in the remaining 200 µL of medium and plated out on LB (Carl Roth, 6673.1) agar plates containing a plasmid specific selection antibiotic (100 µg/mL Carbenicillin). The plates were then incubated upside down at 37°C overnight.

On the next day, a single cell colony was picked and inoculated in 5 mL of 2xYT medium (100 µg/mL Carbenicillin) and incubated at 37°C over-night. An aliquot of each over-night culture was stored at 4°C for short-term preservation. The over-night cultures were centrifuged at room temperature and 4500 g for 10 minutes after which the plasmids were isolated according to the QlAprep Spin Miniprep Kit (QIAGEN, 27115). For the elution step, 20 µl pre-warmed UltraPure Water (Invitrogen, 11538646) was added to the middle of the column membrane and incubated at room temperature for two minutes before centrifugation at 13000 rpm for three minutes. The elution step was repeated one more time to maximize the final plasmid DNA yield. To determine the concentration and purity of the plasmids, the products were analyzed photometrically at 260nm and 280 nm using the NanoDrop^{®} 2000 (Thermo Scientific, ND-2000). The plasmids were stored at -20°C until further use. For large-scale plasmid amplifications, short-term preserved *E. coli* Mach1 cultures, positive for the respective plasmid, were inoculated in 500 mL of 2xYT medium (100 µg/mL Carbenicillin) and incubate at 37°C and 230 rpm overnight. An aliquot (1 ml) of each overnight culture was mixed with the same volume of LB-medium (+ 30% glycerol) and stored at -80°C for long-term preservation. The overnight cultures (499 ml) were centrifuged at 5000 g and 4°C for 30 minutes after which the plasmids were isolated according to the NucleoBond Xtra Maxi Kit (Maschery Nagel, 740414.50) while considering low copy numbers. The final purified plasmids were reconstituted in UltraPure Water (Invitrogen, 11538646). To determine the concentration and purity of the plasmids, the products were analyzed photometrically at 260nm and 280 nm using the NanoDrop^{®} 2000 (Thermo Scientific, ND-2000). The plasmids were stored at -20°C until further use.

### 4. Recombinant Diabody production in bacteria

For each Diabody the pColdlV plasmid was transfected into SHuffle^{®} T7 Competent E. coli cells (New England Biolabs Inc.). Single bacteria clones were used for fermentation and production in a larger volume, using 2x YT medium supplemented with 100 µg/ml carbenicilin and 0.2% (w/v) glucose. Diabody protein production was induced adding 0.1 mM IPTG and incubated at 14°C for 18 - 20 hours. Afterward from the E. coli cell pellet the Diabodies were extracted using first a His-tag purification followed by a CL7/IM7-purification (Trialtus Bioscience). For bacteria lysis the Emulsiflex C3 (Avestin^{®}) was used. For this the bacteria suspension is loaded into the sample reservoir, with set air pressure to 60-80 psi, and run a total of three times thru the machine. The lysate was then centrifuged 3 times for 30-45 min at 5000 g and 4°C. The clarified supernatant was then used for His-tag purification. First the supernatant was transferred into dialysis tubes (Membra-Cel^{™} 14000 dalton, Carl Roth^{®}) and dialyzed against 1× PBS (pH 7.4) at 4 °C overnight. The dialysate was then transferred into falcon tubes and incubated with Co-IMAC beads (400 µL of pure beads per 50 mL of supernatant, Agarose Bead Technology, 6BCL-QHNi-X) at 4°C overnight under constant rotation. The bead-protein suspension was then centrifuged at 800 g and 4°C for 30 minutes. The bead-protein pellet is resuspended in CO-IMAC loading buffer (50mM Na phosphate buffer (pH 7.5), 300mM NaCl, 10mM Imidazol (pH 8.0)) and centrifuged at 800 g and 4°C for 30 minutes. This was performed for two more times. Then the pellet was resuspended in 1ml Co-IMAC loading buffer and transferred onto a flow column (Themo Scientific, 29924). Afterwards, the column with the loaded beads was washed three times with 10 mL of cooled Co-IMAC loading buffer each. For the elution, 1 ml of cooled Cobalt-IMAC elution buffer (50mM Na phosphate buffer (pH 7.5), 300mM NaCl, 150mM Imidazol (pH 8.0)) was loaded while the column was closed with a stopper. After an incubation of 5 minutes, the elution fraction with the antibodies was collected. Afterward, the elution fraction was transferred into dialysis tubes (Membra-Cel^{™} 14000 dalton, Carl Roth^{®}) and dialyzed against 1× PBS (pH 7.4) at 4 °C overnight. The dialysate was then transferred into a falcon tube with 200 µL of IM7-Beads added (Trialtus Bioscience^{®}). The mix was incubated at 4°C overnight in rotation. The next day, the mixture was centrifuged at 800 g and 4°C for 20 minutes. The CL7-pellet was transferred onto a 10 mL column. The column was washed stepwise with 10 mL of different washing Buffers (Table 11).

**Table 11: List of buffers**

| Steps | Buffers | Components |
|---|---|---|
| 1 | PBS | 1x phosphate buffer solution pH 7.4 |
| 2 | A1 | pH 8.0, 2M NaCl, 300 mM Tris, 5% Glycerol, 0.02% (w/v) sodium azide |
| 3 | Buffer Acide | pH 5.6, 40 mM citrate-phosphate, 0.02% (w/v) sodium azide |
| 4 | PBS | 1x phosphate buffer solution pH 7.4 |
| 5 | Buffer Alkaline | pH 10.0, 100 mM sodium carbonate, 0.02% (w/v) sodium azide |
| 6 | PBS | 1x phosphate buffer solution pH 7.4 |
| 7 | Buffer Imidazole | pH 8.0, 1 M NaCl, 0.02 M Tris, 5% Glycerol, 0.3 M Imidazole, 0.02% (w/v) sodium azide |
| 8 | A2 | pH 9.0, 25 mM Tris, 10% isopropanol, 2M urea, 0.02% (w/v) sodium azide |
| 9 | PBS | 1x phosphate buffer solution pH 7.4 |
| 10 | A3 | pH 8.0, 600 mM NaCl, 300 mM Tris ph 8, 0, 5% Glycerol |
| 11 | Elution 3C | pH 7.4, 3C-Protease, PBS + 300 mM NaCl |

To cleave off the bacterial-folding-tag and the CL7-tag from the N-terminal end of the Diabodies, 800 µL of Elution 3C Buffer, containing 1,25 µg/mL PreScission Protease (Trialtus Bioscience, 30-2030), was added on the column. The column was closed with a stopper, and rotated at 4°C for three hours. Afterward the column was eluted using gravity and the elution fraction with the Diabodies was saved (E1). Then, another 800 µL of Elution 3C Buffer without PreScission Protease was added to the column, incubated for three minutes, and the elution fractions were saved (E2-E7) for six more times. From the different washing and elution fractions, 21 µL was mixed with 7 µL of 4x protein loading buffer and incubated at 95°C for 5 minutes. Afterwards, the protein samples were loaded onto a 12% SDS-PAGE gel and run first at 90 volt for 20 minutes and then at 130 volt for the final 90 minutes. After the run, the gels were stained with Coomassie Blue solution for 10 minutes and afterwards washed in de-staining solution for 10 minutes and then transferred into VE-water for imaging.

For each CL7-tag purified Diabody, the elution fractions with the highest purity (often excluding E1 fraction) were pooled, concentrated using Aquacide II ((Sigma Aldrich, 17851-M) tubes, and dialyzed against 1x PBS (pH 7,4) at 4°C over night. D-Trehalose was added to the Diabodies at a final concentration of 60 mM as a solvent and preservative. The Diabody solution (PBS pH 7,4 + 60 mM D-Trehalose) was sterilized through 0,2 µm filtration and kept at 4°C until further use.

After the CL7 tag purification with 3C protease digestion, the final purified Diabodies (according to any one of SEQ ID NOs: 228 to 239) have the amino acid sequence GPGT (SEQ ID NO: 277) attached at the N-terminal end. GP amino acids are left from the 3C protease cutting side (SEQ ID NO: 47) and GT amino acids are from the Kpnl restriction enzyme cloning side.

### 5. Measuring PCC Diabody concentration in solution

To determine the final Diabody concentration, the Pierce^{™} BCA Protein Assay Kit (Thermo Scientific^{™}, #23225) was used after the manufactures protocol. In short, protein-standard and Diabody samples were pipette into separate test tubes. Then working reagent was added to each tube, mixed and incubate at room temperature for 30-60 minutes in the dark. With a spectrophotometer set to 562 nm the absorbance of all samples was measured within 10 minutes. The average 562 nm absorbance measurement of the Blank standard was subtracted from the 562nm absorbance measurement of all other individual standards and Diabody samples, to generate a standard curve to determine the protein concentration of each Diabody sample.

### 6. Testing in vitro activity of Diabodies against tumor cells in regular 2D cell culture

Recombinant produced Diabodies (SEQ ID NOs: 228 to 239) were tested in cell culture against different human and murine tumor cell lines (Table 12). The tumor cells are positive for the luziferase fire fly protein, expressed by viable cells only. The tumor cells were seeded into 96-well cell culture plates (7500 cells in 50 µL per well for 150 cells per µL) in their respective media and incubated at 37°C and 5% CO2 for 24 hours. The next day, fresh human CD3+/CD8+ T-cells were added to each well, with 50 IU/ml human IL-2 (#AF-200-02-100UG, ThermoFisher Scientific), for a final tumor cell to T cells ratio of 1 to 3 or 1 to 5. The Diabodies were added at their respective concentration in 20 µL per well. As positive control for target cell killing a DMSO solution (#472301, MERCK) with a final concentration of 5-10% (v/v) in the medium was used. As reference control, to control for unspecific donor T cell activation against the target cells, target cells only and target cells mixed with T cells was used. The plates were incubated at 37°C and 5% CO2 for 24 or 48 hours. For measuring cell viability 20 µL of 3.5 mM luciferin solution was added to each well (final concentration 0.5 mM) and the plates were incubate at 37°C for 30 minutes. Afterwards, the bioluminescence of the firefly-luciferase was measured, using a Tecan Spark plate reader (with an absorbance scan from 350 nm to 700 nm with an integration time interval of 1000 ms).

**Table 12: Tumor cell lines**

| Cell Line | ATCC / DSMZ Number | Cell type | Medium |
|---|---|---|---|
| AsPC-1 | CRL-1682 | Pancreas Adenocarcinoma | RPMI1640 supplemented with 10% (v/v) FBS, 1% (v/v) GlutaMAX^{™}, 1% (v/v) penicillin-streptomycin-neomycin antibiotic mixture |
| MV4-11 | CRL-9591 | Macrophage Biphenotypic B Myelomonocytic Leukemia | RPMI1640 supplemented with 10% (v/v) FBS, 1% (v/v) GlutaMAX^{™}, 1% (v/v) penicillin-streptomycin-neomycin antibiotic mixture |
| MM.1S | CRL-2974 | B lymphoblast Multiple Myeloma | RPMI1640 supplemented with 10% (v/v) FBS, 1% (v/v) GlutaMAX^{™}, 1% (v/v) penicillin-streptomycin-neomycin antibiotic mixture |
| MS18 | - | Neuroendocrine carcinoma NET G3 of the rectum | DMEM/F12 supplemented with 10% (v/v) FBS, 1% (v/v) GlutaMAX^{™}, 1% (v/v) penicillin-streptomycin-neomycin antibiotic mixture, 1% (v/v) Gibco^{™} insulin-transferrin-selenium (ITS-G) |
| Panc-1 | CRL-1469 | Pancreas Epithelioid Carcinoma | DMEM (4.5 g/L) supplemented with 10% (v/v) FBS, 1% (v/v) GlutaMAX^{™}, 1% (v/v) penicillin-streptomycin-neomycin antibiotic mixture |
| HL-60 | CCL-240 | Promyelocytic leukemia | RPMI1640 supplemented with 10% (v/v) FBS, 1% (v/v) GlutaMAX^{™}, 1% (v/v) penicillin-streptomycin-neomycin antibiotic mixture, + 0.05 mM 2-mercaptoethanol |
| K562 | CCL-243 | Chronic myeloid leukemia cells | RPMI1640 supplemented with 10% (v/v) FBS, 1% (v/v) GlutaMAX^{™}, 1% (v/v) penicillin-streptomycin-neomycin antibiotic mixture |
| C1498 | ATCC: TIB-49^{™} | Cells from a female mouse with acute myeloid leukemia | DMEM (Sigma-Aldrich # D6429) with 10% v/v FBS; 1% v/v PSN; 1% v/v GlutaMAX |

### 7. 3D co-culture assay

A mix of tumor cells and healthy human cells (Table 13) is seeded into 96-well cell culture plates. Per well a mix of 600 tumor cells, 600 HUEC, 600 HDLEC, 600 NHDF and 1800 T cells in 100 µL medium. The medium consist of RPMI1640 supplemented with 10% (v/v) FBS, 1% (v/v) GlutaMAX^{™}, 1% (v/v) penicillin-streptomycin-neomycin antibiotic mixture, 50 IU/ml human IL-2 (# AF-200-02-100UG, ThermoFisher Scientific), 10% (v/v) cultrex basement membrane extract (BME) containing laminin, collagen IV, entactin and heparin sulfate proteoglycan (#3434-050-RTU, R&D Systems^{®}), 10% (v/v) methocult (#04434, StemCell^{™}) and 1% lipid-mix (v/v) (Lipid Mixture 1, Chemically Defined, L0288, Sigma-Aldrich^{®}). The co-culture plates are incubated at 37°C and 5% CO2.

**Table 13: Healthy human tissue cells**

| Cell Line | PromoCell Number | Cell type | Medium |
|---|---|---|---|
| HUAEC | C-12202 | Umbilical artery Endothelial cells | Endothelial Cell GrowthMedium MV 2 (PromoCell # C-22022), Growth Medium MV 2 SupplementMix; 1% v/v PSN |
| HaCaT | C-12006 | Keratinocytes | Keratinocyte Growth Medium 2 (PromoCell # C-20011), Growth Medium SupplementMix; 1% v/v PSN |
| NHDF | C-12300 | Fibroblast | Fibroblast Growth Medium (PromoCell # C-23020), Growth Medium SupplementMix; 1% v/v PSN |
| HDLEC | C-12217 | Lymphatic Endothelial Cells | Endothelial Cell GrowthMedium MV 2 (PromoCell # C-22022), Growth Medium MV 2 SupplementMix; 1% v/v PSN |

### 8. Testing the in vitro activity of Diabodies against tumor cells in 3D co-culture

At day 1 the luziferase positive tumor cells are seeded in the co-culture assay and incubated for 5 days at 37°C and 5% CO2. At day 5 Diabody PCC04D (SEQ ID NO: 231) was added (10 µL per well) at the respective concentration. Also 1800 fresh human T cells (in 10 µL pure RPMI medium per well) were added. As positive control for target cell killing a DMSO solution (#472301, MERCK) with a final concentration of 5-10% (v/v) in the medium was used. As reference control, to control for unspecific donor T cell activation against the target cells, target cells only and target cells mixed with T cells was used. The plates were incubated at 37°C and 5% CO2 for 3 more days. At day 8 the tumor cell viability was measured adding 20 µL of 3.5 mM luciferin solution to each well (final concentration 0.5 mM) and the plates were incubate at 37°C for 30 minutes. Afterwards, the bioluminescence of the firefly-luciferase was measured, using a Tecan Spark plate reader (with an absorbance scan from 350 nm to 700 nm with an integration time interval of 1000 ms).

### 9. Testing in vitro the combination of PCC04D with different small molecule inhibitors

At day 1 the luziferase positive tumor cells are seeded in the co-culture assay and incubated for 5 days at 37°C and 5% CO2. At day 5 the Diabody PCC04D (SEQ ID NO: 231) was added (10 µL per well) at the respective concentration. Also 1800 fresh human T cells (in 10 µL per well) were added. For inhibitor treatment, the respective SMI were resuspended in pure RPMI medium and 20 µL was added per well. For combination of two or more SMI, the respective inhibitors were combined before in medium and added in 20 µL per well. Most SMI were ordered from commercial companies, and most SMI were resuspended in DMSO for stock concentrations and stored at -80°C. SMIs ordered from MedChemExpress^{®} are ATRA (HY-14649), Simvastatin (HY-17502), Fluvastatin (HY-14664), Lapaquistat (HY-14925), Bemfivastatin (HY-106281), Lenalidomide (HY-A0003), Iberdomide (HY-101291), HOMO-PROTAC cereblon degrader 1 (HY-111594), Eragidomide (HY-130800), Adagrasib (HY-130149), BI-2852 (HY-126247), MRTX-1133 (HY-134813), RMC-0331 (HY-134885), SOS1 activator 1(HY-111671), Narciclasine (HY-16563), Ruxolitinib (HY-50856), Anagrelide hydrochloride (HY-B0523A), NSC-70220 (HY-101796), SAH-SOS1A TFA (HY-P2265A). The SMI Dioncophyllin A (BR-BM 7) is a gift from Dr. h.c. mult. Gerhard Bringmann Institut für Organische Chemie, Am Hubland D-97074 Wuerzburg, Germany. The SMI SAH-SOS1A TFA was dissolved in pure H₂O. As positive control for target cell killing, DMSO (#472301, MERCK) at a final concentration of 5-10% (v/v) in the medium was used. As reference control, to control for unspecific donor T cell activation against the target cells, target cells only and target cells mixed with T cells was used. The plates were incubated at 37°C and 5% CO2 for 3 more days. At day 8 the tumor cell viability was measured adding 20 µL of 3.5 mM luciferin solution to each well (final concentration 0.5 mM) and the plates were incubated at 37°C for 30 minutes. Afterwards, the bioluminescence of the firefly-luciferase was measured, using a Tecan Spark plate reader (with an absorbance scan from 350 nm to 700 nm with an integration time interval of 1000 ms).

### 10. Testing the expression of Ras protein on the cell surface with flow cytometry

The day before, the tumor cells in regular 2D-culture were washed 1x with PBS and resuspended in their respective medium without FBS (serum starving). The next day, 10-14 hours before the flow cytometry analysis (FACS) the cells are returned in regular medium with FBS. Two hundred thousand cells were used for each flow cytometry sample. For adherent cell lines, cell culture medium was first removed and the cells were washed one time with PBS.

After washing, 1 ml of 0.5% Trypsin-EDTA (Gibco, #25300054) was added to trypsinise the cells for 3 min at 37°C. Then, 9 ml of complete medium was added to stop trypsinisation process. Suspension cells were directly taken from the cell culture medium. Cell number of each sample was determined via Trypan Blue staining and a total of 2 X 10⁶ cells were harvested and washed one time with ice-cold PBS. The cells were then resuspended into 1 ml of ice-cold FACS buffer (PBS supplemented with 2% FBS) and blocked with 10 µl of human FcR Blocking Reagent (MACS Miltenyi #130-059-901) for 10 min at 4°C. The cells were then aliquoted into a 96-well round bottom plate with 100 µl per well. For antibody staining, 1 µl of primary antibody (1 to 100 dilution) was added per sample. For detecting Ras protein on the cell surface the anti-Ras antibodies (Ras10 clone, Invitrogen #MA1-012) was used, or primary labeled PCC04D Diabody (10). For detecting human HLA-I and HLA-II antigens on the cell surface the HLA-I-APC (MACS Miltenyi #130-120-569) and the HLA-II-PE (MACS Miltenyi #130-120-784) antibodies. For detecting intra cellular myeloperoxidase (MPO), a direct labeled antibody was used (Beckman Coulter^{®}, #B23132). To detect the dead cell population, 1 µl of cell viability stain was added per sample. Two viability stains were used, 7-AAD (Biolegend^{®}, #420404) or Zombie NIR^{™} (BioLegend^{®}, #423105). Cells were incubated for 30 minutes at 4°C in the dark. Then, cells were pelleted through centrifugation at 1500 rpm for 5 min at 4°c and washed with 5 ml of FACS buffer for 3 times. After last wash, cells were resuspended into 100 µl FACS buffer. For detecting the Ras10 antibody clone, 1 µl of the secondary antibodies (FITC anti-mouse IgG (Biolegend #406001) was added into their respective wells and incubated for 30 min at 4°C in dark. The cells were washed 3 times with FACS buffer after secondary antibody incubation and finally resuspended into 100 µl FACS buffer. Flow cytometry analysis was performed with BD FACSCanto II Flow Cytometry System. Single staining controls were performed to correct spectral spillover.

### 11. Labeling PCC04D Diabody for flow cytometry

First the Diabody PCC04D (SEQ ID NO: 231) was conjugated with streptavidin (abcam #ab102921) after manual procedure. Then PCC04D-straptavidin was conjugated with biotin-FITC (Invitrogen #22030) at 4°C overnight. The next day the samples was dialyzed with Slide-A-Lyzer^{™} 3,5 K MWCO (ThermoFisher #69550) into PBS for 3 hours at 4°C to remove unconjugated Biotin-FITC. The final PCC04D-straptavidin-biotin-FITC Diabody was resuspended at 0.35 mg/ml in FACS buffer.

### 12. Fixation of tumor cells with CellCover for flow cytometry

For adherent cell lines, cell culture medium was first removed and the cells were washed one time with PBS. After washing, 1 ml of 0.5% Trypsin-EDTA (Gibco, #25300054) was added to trypsinise the cells for 3 min at 37°C. Then, 9 ml of complete medium was added to stop trypsinisation process. Suspension cells were directly taken from the cell culture medium. Cells were washed one time with ice-cold PBS and resuspended into ice-cold (4°C) Cell Cover (Biotrend^{®}, #800-125). For FACS, the CellCover fixation was performed first, followed by FcR blocking and antibody staining according to manufacturer's protocol.

### 13. Generating HLA-I and HLA-II knock down cells

To knock down the HLA-I complex on cells, the gene B2M (Gene ID: 567) was targeted, and for HLA-II complex knock down the gene CIITA (Gene ID: 4261) was targeted. For this base editing was performed using the luziferase positive cell lines K562, MM.1S and MV-4-11. Cells were cultivated at 2 X 10⁶ cells/ml in their respective medium. Adenine based editor (Addgene #136300) was cloned into an *in vitro transcription* backbone (Addgene #178113) with restriction enzymes Kpnl (New England Biolabs #R3142) and Pacl (New England Biolabs #R0547S), digested at 37°C for 1 hour. Gel electrophoresis was performed with 0.7% TAE agarose gel (ThermoFisher #16500100). DNA band of 3 kb was extracted with NucleoSpin Gel and PCR Clean-up kit (Machery-Nagel #740609.50) according to manufacturer's protocol. ABE 8.20m was amplified with Phusion high fidelity PCR kit (ThermoFisher #F553L) with specific primers (SEQ ID NOs: 391 and 392). In parallel, bridge oligos (SEQ ID NOs: 393 and 394) required for the cloning were annealed by adding 1 µL of each at 100 µM to 23 µL of r2.1 buffer (New England Biolabs #B7202S) and heated at 95°C for 3 minutes, then gradually cooled at 0.1°C/second to 22 °C. ABE8.20m, bridge oligos and digested backbones were mixed using a Gibson assembly mix (New England Biolabs #E2611S) and incubated for 1 hour at 50°C. The mix was then diluted at 1/4 and transformed into One Shot^{™} TOP10 Chemically Competent E. coli (Invitrogen #C404010). Plasmid with correct sequences was transcribed in vitro according to protocol from Doman et al., 2022. In short, 2 µg of mRNA coding for Adenine base editor was mixed with 100 pmol of the appropriate gRNA (for HLA-I SEQ ID NO: 395, and for HLA-II SEQ ID NO: 396) and then electroporated into the cells using Lonza 4D-Nucleofector System (MM.1S: SF kit, CA-137 pulse code / K562: SF kit, FF-120 pulse code / MV-4-11: SF kit, DJ-100 pulse code). Post nucleofection the cells were incubated in 80 µL of pure medium at 37°C with 5% CO2 for 15 minutes. The nucleofected cells were then transferred into a 48-well plate containing fresh complete medium. The cells were then expanded and sorted for population single negative for HLA-I and HLA-II as well as the double negative population with MACSQuant^{®} Tyto Cell Sorter (Miltenyi Biotec) according to manufacturer's protocol.

### 14. Sorting of HLA knock-down cells

For sorting the cells with a successful HLA knock-down, the MACSQuant^{®} Tyto^{®} Cell Sorter according to manufacturer's protocol was used. In short, 1 × 10⁷ cells were washed one time with FACS buffer (2% FBS in PBS). The cells were then resuspended in 500 µl of FACS buffer and stained with 5 µl of HLA-I-APC (MACS Miltenyi #130-120-569) and the HLA-II-PE (MACS Miltenyi #130-120-784) antibodies for 30 min at 4°C. Afterward the cells were two times centrifuged at 1200 rpm for 5 min and washed with FACS buffer. The cells were then resuspended in 10 ml of MACSQuant^{®} Tyto^{®} Running Buffer (#130-107-207). In the meantime, MACSQuant^{®} Tyto^{®} Cartridge (#130-104-791) was primed with MACSQuant^{®} Tyto^{®} running Buffer according to manufacturer's protocol. The cells were then passed through a 20 µm pre-separation filter (MACS Miltenyi #130-101-812) into the sorting cartridge using a 10 ml syringe (Dispomed^{®} #21010). The loaded cartridge was installed in the MACSQuant^{®} Tyto^{®} Cell Sorter and sorted for the respective HLA-I and HLA-II single and double negative populations. The sorted cells were recovered in 2 ml of complete medium in a 6-well plate (Sarstedt #83.3920) for standard cell culture.

### 15. Generating HiBiT positive tumor cells

For this specific cRNA and ssODN for the three human Ras genes KRAS, NRAS and HRAS was used (SEQ ID Nos: 250 to 260) (Integrated DNA Technologies GmbH), and the Cas9 protein (MERCK, #CAS9PROT). The lyophilized guide RNA was reconstituted in nuclease-free water, annealed at 95 °C for 5 minutes and cooled to room temperature. The rinonucleoprotein (RNP) complex was prepared after manufacturer's protocol. In short, the Cas9 protein (100 pmol) was slowly added and mixed to tracrRNA:crRNA (120 pmol) to avoid precipitation. The RNP complex was allowed to form for 20 minutes at room temperature. For transfection the cells were resuspended in nucleofector buffer at final concentration of 200,000 cells in 20 µl. For K562 cells the SF Cell Line kit was used, and for AsPc-1 cells the SE Cell Line kit (FisherScientific, #13171463 and #10377959). For electroporation the Lonza 4D-Nucleofector System was used. After electroporation the cells were immediately added back into pure RPMI medium and incubated at 37 °C, 5% Co2 for 10 minutes. Afterward the cells were transferred into regular complete medium for standard cell culture.

### 16. Detecting HiBiT expression on the cell surface

First the Nano-Glo^{®} HiBiT Extracellular Detection mix was prepared (Promega, #N2420). For this, 4ml of extracellular buffer, 40µl of LgBiT Protein and 80µl of substrate were mixed together. The HiBiT transfected cells in their respective complete medium, and the respective wild type cells as negative controls, were taken out of the incubator and equilibrated to room temperature for 5 minutes. Then extracellular reagent mix was added in equal volume direct into the culture medium, mixed with the cells gently by pipetting and incubated for 10 minutes. Afterwards, the bioluminescence of the HiBiT-tag complex was measured, using a Tecan Spark plate reader (with an absorbance scan from 350 nm to 700 nm with an integration time interval of 1000 ms).

### EMBODIMENTS

1. Combination of
   - an antigen binding protein (ABP) or an isolated nucleic acid comprising a sequence encoding the ABP and
   - a modulator compound
   for use as medicament, wherein the ABP comprises at least a first antigen binding domain capable of binding to an extracellular Ras antigen and wherein the modulator compound is selected from the group consisting of
   a) an All-trans-retinoic acid (ATRA) based modulator,
   b) a modulator of the cholesterol and triglyceride synthesis or metabolism, preferably Simvastatin, Fluvastatin, Lapaquistat, or Bemfivastatin
   c) an immunomodulatory drug targeting cereblon, preferably Lenalidomide, Iberdomide, HOMO-Protac cereblon degrader 1, or Eragidomide,
   d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
   e) a kinase modulator, preferably of the Rho kinase pathway, preferably Narciclasine,
   f) a tyrosine kinase modulator, preferably of the JAK signaling pathway, preferably Ruxolitinib,
   g) a modulator of esterases, preferably of the phosphodiesterase pathway, preferably Anagrelide,
   h) a lipid, preferably a lipid part of a cell membrane
   i) a fatty acid, preferably a saturated fatty acid or unsaturated fatty acid
   j) a modulator of the gut microbiome, and
   k) an inhibitor of the naphthylisoquinoline alkaloid class, preferably Dioncophylline A.
   l) a combination of two or more of a) to k).
2. The combination for use according to embodiment 1, wherein said extracellular Ras antigen is not part of, or is not restricted to, a human leukocyte antigen (HLA)-peptide complex, or specific HLA alleles, presented on the cell surface by the major histocompatibility complex (MHC).
3. The combination for use according to embodiment 1 or 2, wherein said ABP comprises one or more additional antigen binding domain(s) that is capable of binding to antigen(s) present on a mammalian T-cell, preferably a human cluster of differentiation 3 (CD3) antigen or a human T cell receptor (TCR).
4. The combination for use according to any one of embodiments 1 to 3, wherein the first antigen binding domain comprises an amino acid sequence having a sequence identity of at least 70%, more preferably at least 80%, even more preferably at least 90%, and most preferably at least 95% sequence identity, to an amino acid sequence selected from SEQ ID NO: 20 to 31, 66 to 71, 134 to 178 and 290 to 396, or, in each case independently, optionally with no more than ten, nine, eight, seven, six, five, four, preferably no more than three, two or one, amino acid substitution(s), insertion(s) or deletion(s) compared to these sequences.
5. The combination for use according to any one of embodiments 1 to 4, wherein the ABP is a Chimeric Antigen Receptor (CAR) comprising from N-terminus to C-terminus (i) an extracellular domain comprising the at least first antigen binding domain, (ii) an extracellular hinge domain, (iii) a transmembrane domain, and (iv) a cytoplasmic domain, optionally wherein the ABP is a CAR which is expressed by a T cell (CAR T cell).
6. The combination for use according to any one of embodiments 1 to 5, wherein the isolated nucleic acid is comprised in an expression construct, preferably further comprising promoter and/or terminator sequences.
7. The combination for use according to embodiment 6, wherein the isolated nucleic acid and/or the expression construct is comprised in a recombinant host cell.
8. The combination for use according to embodiment 7, wherein the recombinant host cell is preferably selected from effector cells of the immune system, such as lymphocytes, for example CD8 positive cytotoxic lymphocytes, CD4 positive T cells, T helper cells, or Th17 T cells, natural killer (NK) cells, natural killer T (NKT) cells, dendritic cells, killer dendritic cells, B cells, γδ T cells, and a lymphocyte preparation containing NK cells and NKT cells mast cells.
9. The combination for use according to any one of embodiments 1 to 8, wherein the modulator compound is not an immunomodulatory drug targeting cereblon.
10. The combination for use according to any one of embodiments 1 to 9, wherein the ABP is PCC04D diabody (SEQ ID NOs 23 and 231).
11. The combination for use according to any one of embodiments 1 to 10, wherein the ABP enhances a cell-mediated immune response, such as the immune response mediated by an activated cytotoxic T-cell (CTL) to a mammalian cell expressing said extracellular Ras antigen.
12. The combination for use according to any one of embodiments 1 to 11, wherein the extracellular Ras antigen comprises, preferably consists of, the sequence according to any one of SEQ ID NOs: 1, 3, 5, 7, 9, or 179 to 182, or comprises, preferably consists of, a sequence having at least 75% sequence identity, preferably at least 80%, more preferably at least 90%, and most preferably at least 95% sequence identity with any one of SEQ ID NOs: 1, 3, 5, 7, 9, or 179 to 182, optionally wherein the extracellular Ras antigen is a mutant Ras antigen comprising, preferably consisting of, a sequence having at least 75% sequence identity, preferably at least 80%, more preferably at least 90%, and most preferably at least 95% sequence identity with a sequence according to any one of SEQ ID NOs: 11 to 19, 77 to 119, or 183 to 223.
13. The combination for use according to any one of embodiments 1 to 12, wherein the ABP is selected from the group consisting of an immunoglobulin molecule, such as an IgG, IgE, IgD, IgA, or IgM immunoglobulin, preferably an IgG immunoglobulin, a monoclonal antibody, a chimeric antibody, a bispecifc ABP, such as a bispecific antibody, a CDR-grafted antibody, a humanized antibody, a single domain antibody, such as a VHH single domain antibody, a hemibody antibody, a single-chain KeyLock-antibody, a diabody, a single chain diabody, a variable domain of the antibody heavy chain or antibody light chain, a multispecific antibody, a Chimeric Antigen Receptor (CAR), alternative protein binders including monobodies (derived from fibronectin type III), anticalins (derived from lipocalins), affibodies (derived from immunoglobulin-binding protein A), DARPins (Designed Ankyrin Repeat Proteins), proteins with repeating motifs like leucine-rich repeats (LRRs), ankyrin repeats (ARs), Armadillo repeats (Arms), tetratricopeptide repeats (TPRs), and/or a fragment of an antibody, such as a fragment of a monoclonal antibody, for example a single chain Fv (scFv), (scFv)2, a Fv, a disulfide linked Fv, Fab, Fab', F(ab')2 or a scFv-Fc, preferably wherein said ABP is a bispecific antibody or a diabody.
14. The combination for use according to any one of embodiments 1 to 13, wherein the ABP is a bispecific antibody, and comprises at least one additional antigen binding domain that binds to a human cluster of differentiation 3 (CD3) antigen, preferably wherein the antigen binding domain that binds to a human cluster of differentiation 3 (CD3) antigen comprises, preferably consists of, a sequence having at least 75% sequence identity, preferably at least 80%, more preferably at least 90%, and most preferably at least 95% sequence identity with an amino acid sequence according to SEQ ID No: 48 or 49.
15. The combination for use according to any one of embodiments 1 to 14, wherein the ABP comprises
   (i) a heavy chain variable domain comprising the CDRH1 region set forth in any one of SEQ ID NO: 50, 53, 135, 149, 162, 292, 300, or 338, the CDRH2 region set forth in any one of SEQ ID NO: 51, 54, 137, 151, 164, 293, 301, or 339, and the CDRH3 region set forth in any one of SEQ ID NO: 52, 55, 139, 153, 166, 294, 302, or 340, or wherein in each case independently the CDRH1, CDRH2 and/or CDRH3 comprise a sequence having no more than three or two, preferably no more than one amino acid substitution(s), deletion(s) or insertion(s) compared to any one of SEQ ID NO: 50, 53, 135, 149, 162, 292, 300, or 338, SEQ ID NO: 51, 54, 137, 151, 164, 293, 301, or 339, or SEQ ID NO: 52, 55, 139, 153, 166, 294, 302, or 340, respectively; or comprising a CDRH1, CDRH2 or CDRH3 sequence having at least 75% sequence identity, preferably at least 80% sequence identity, more preferably at least 90% sequence identity, and most preferably at last 95% sequence identity with any one of SEQ ID NO: 50, 53, 135, 149, 162, 292, 300, or 338, SEQ ID NO: 51, 54, 137, 151, 164, 293, 301, or 339, or SEQ ID NO: 52, 55, 139, 153, 166, 294, 302, or 340, respectively; and
   (ii) a light chain variable domain comprising the CDRL1 region set forth in any one of SEQ ID NO:56, 59, 142, 156, 169, 295, 303, or 341, the CDRL2 region set forth in any one of SEQ ID NO: 57, 60, 144, 158, 171, 296, 304, or 342 and the CDRL3 region set forth in any one of SEQ ID NO: 58, 61, 146, 160, 173, 297, 305, or 343 or wherein in each case independently CDRL1, CDRL2 and/or CDRL3 comprise a sequence having no more than three or two, preferably no more than one amino acid substitution(s), deletion(s) or insertion(s) compared to any one of SEQ ID NO: 56, 59, 142, 156, 169, 295, 303, or 341, SEQ ID NO: 57, 60, 144, 158, 171, 296, 304, or 342, or SEQ ID NO: 58, 61, 146, 160, 173, 297, 305, or 343, respectively; or comprising a CDRL1, CDRL2 or CDRL3 sequence having at least 75% sequence identity, preferably at least 80% sequence identity, more preferably at least 90% sequence identity, and most preferably at last 95% sequence identity with any one of SEQ ID NO: 56, 59, 142, 156, 169, 295, 303, or 341, SEQ ID NO: 57, 60, 144, 158, 171, 296, 304, or 342, or SEQ ID NO: 58, 61, 146, 160, 173, 297, 305, or 343, respectively.
16. The combination for use according to any one of embodiments 1 to 15, wherein the ABP comprises a heavy chain variable region which comprises an amino acid sequence having a sequence identity of at least 70%, preferably at least 80%, more preferably at least 90%, and most preferably at last 95% sequence identity, to an amino acid sequence selected from SEQ ID NO: 62 or 64, or, in each case independently, optionally with no more than ten, nine, eight, seven, six, five, four, preferably no more than three, two or one, amino acid substitution(s), insertion(s) or deletion(s) compared to these sequences; and/or wherein the ABP comprises a light chain variable region which comprises the amino acid sequence having a sequence identity of at least 70%, preferably at least 80%, more preferably at least 90%, and most preferably at last 95% sequence identity, to the amino acid sequence selected from SEQ ID NO: 63 or 65, or, in each case independently, optionally with no more than ten, nine, eight, seven, six, five, four, preferably no more than three, two or one, amino acid substitution(s), insertion(s) or deletion(s) compared to these sequences.
17. The combination for use according to any one of embodiments 1 to 16, wherein the ABP comprises a heavy chain variable region which comprises an amino acid sequence having a sequence identity of at least 70%, preferably at least 80%, more preferably at least 90%, and most preferably at last 95% sequence identity, to an amino acid sequence selected from SEQ ID NO: 66, 68, 70, 391, 393, or 395, or, in each case independently, optionally with no more than ten, nine, eight, seven, six, five, four, preferably no more than three, two or one, amino acid substitution(s), insertion(s) or deletion(s) compared to these sequences; and/or wherein the ABP comprises a light chain variable region which comprises the amino acid sequence having a sequence identity of at least 70%, preferably at least 80%, more preferably at least 90%, and most preferably at last 95% sequence identity, to the amino acid sequence selected from SEQ ID NO: 67, 69, 71, 392, 394, or 396, or, in each case independently, optionally with no more than ten, nine, eight, seven, six, five, four, preferably no more than three, two or one, amino acid substitution(s), insertion(s) or deletion(s) compared to these sequences.
18. The combination for use according to any one of embodiments 1 to 17, wherein the ABP is a heterodimer comprising two different heavy chain immunoglobulin constant domains, wherein the first heavy chain comprises a first mutation (such as a knob mutation), and the pairing second heavy chain comprises a corresponding second mutation (such as a hole mutation), wherein the first mutation and the second mutation allow to form a heterodimer, optionally wherein the first heavy chain comprises an amino acid sequence according to any one of SEQ ID No: 42, 120 and 121, and the second heavy chain comprises an amino acid sequence according to any one of SEQ ID No: 43, and 122 to 133.
19. The combination for use according to any one of embodiments 1 to 18, wherein the ABP comprises a fibronectin type III domain (FN3) binder with four different antigen binding loops (BC, DE, FG and CD), comprising the BC loop region set forth in SEQ ID NO: 175, 307, 312, 349, 354, or 359, the DE loop region set forth in SEQ ID NO: 176, 309, 314, 351, 356, or 361, the FG loop region set forth in SEQ ID NO: 177, 310, 315, 352, 357, or 362, and the CD loop region set forth in SEQ ID NO: 178, 308, 313, 350, 355, or 360, or wherein in each case independently the BC loop region, the DE loop region, the FG loop region, and the CD loop region, comprise a sequence having no more than three or two, preferably no more than one, amino acid substitution(s), deletion(s) or insertion(s) compared to any one of SEQ ID NO: 175, 176, 177, 178, 307 to 310, 312 to 315, 349 to 352, 354 to 357, and/or 359 to 362, respectively; or comprising a BC, DE, FG and/or CD loop region sequence having at least 75% sequence identity, preferably at least 80% sequence identity, more preferably at least 90% sequence identity, and most preferably at last 95% sequence identity with any one of SEQ ID NO: 175, 176, 177, 178, 307 to 310, 312 to 315, 349 to 352, 354 to 357, and/or 359 to 362.
20. The combination for use according to any one of embodiments 1 to 19, wherein the ABP comprises at least one monobody having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from SEQ ID NOs: 25, 26, 306, 311, 348, 353, or 358.
21. The combination for use according to any one of embodiments 1 to 20, wherein the ABP comprises at least one antigen binding domain capable of binding with one, two, three, four, or preferable more than five amino acids to a Ras antigen, wherein the at least one antigen binding domain is alone or in a bipartite complex with another Ras binding protein, or in a tripartite complex with another Ras binding protein and the membrane, or in a multipartite complex with one or more Ras binding proteins and/or the membrane, and wherein said amino acids in the ABP optionally comprise:
   (i) a domain comprising the RBD-CRD region (amino acids 52 to 188) of the human RAF1 protein as set forth in SEQ ID NO: 23,
   (ii) optionally wherein one or multiple amino acids as depicted in Tables A and B are in contact with KRAS residues, or wherein one or multiple amino acids as depicted in Table C are in contact with the membrane, or wherein one or multiple amino acids as depicted in Table D are in contact with KRAS residues in a tripartite complex comprised of RBD-CRD, KRAS and the membrane, or
   (iii) a domain comprising the CDC25H region (amino acids 780 to 1019) of the human SOS1 protein as set forth in SEQ ID NO: 24, optionally wherein one or multiple amino acids as depicted in Table E are in contact with KRAS residues, or
   (iv) a domain comprising the CDC25 region (amino acids 1038 to 1270) of the human RASGRF1 protein as set forth in SEQ ID NO: 29, optionally wherein one or multiple amino acids as depicted in Table F are in contact with Ras residues in a tripartite complex with Ras, Sos1 and RasGRF1, or
   (v) a domain comprising the RAS binding region (amino acids 274 to 364) of the human RASSF5 protein (UniProt Q8WWW0-1) as set forth in SEQ ID NO: 377, optionally wherein one or multiple amino acids are in contact with RAS residues, or
   (vi) a domain comprising the RAS binding region (amino acids 201 to 363) of a splice variant of the human RASSF5 protein (UniProt Q8WWW0-2) as set forth in SEQ ID NO: 378, optionally wherein one or multiple amino acids are in contact with RAS residues, or
   (vii)a domain comprising the RAS binding region (amino acids 201 to 363) of the human RASSF1 protein (UniProt Q9NS23-1) as set forth in SEQ ID NO: 379, optionally wherein one or multiple amino acids are in contact with RAS residues, or
   (viii) a domain comprising the RAS binding region (amino acids 176 to 264) of the human RASSF2 protein (UniProt P50749-1) as set forth in SEQ ID NO: 380, optionally wherein one or multiple amino acids are in contact with RAS residues, or
   (ix) a domain comprising the RAS binding region (amino acids 6 to 89) of the human RASSF7 protein (UniProt Q02833-1) as set forth in SEQ ID NO: 381, optionally wherein one or multiple amino acids are in contact with RAS residues, or
   (x) a domain comprising the RAS binding region (amino acids 19 to 91) of the human ARAF protein (UniProt P10398-1) as set forth in SEQ ID NO: 382, optionally wherein one or multiple amino acids are in contact with RAS residues, or
   (xi) a domain comprising the RAS binding region (amino acids 19 to 148) of the human ARAF protein (UniProt P10398-1) as set forth in SEQ ID NO: 383, optionally wherein one or multiple amino acids are in contact with RAS residues, or
   (xii)a domain comprising the RAS binding region (amino acids 151 to 232) of the human BRAF protein (UniProt P15056) as set forth in SEQ ID NO: 384, optionally wherein one or multiple amino acids are in contact with RAS residues, or
   (xiii) a domain comprising the RAS binding region (amino acids 151 to 320) of the human BRAF protein (UniProt P15056) as set forth in SEQ ID NO: 385, optionally wherein one or multiple amino acids are in contact with RAS residues, or
   (xiv) a domain comprising the RAS binding region (amino acids 56 to 131) of the human RAF1 protein (UniProt P04049-1) as set forth in SEQ ID NO: 386, optionally wherein one or multiple amino acids are in contact with RAS residues, or
   (xv) a domain comprising the RAS binding region (amino acids 1235 to 1451) of the human NF1 protein (UniProt P21359-1) as set forth in SEQ ID NO: 387, optionally wherein one or multiple amino acids are in contact with RAS residues, or
   (xvi) a domain comprising the RAS binding region (amino acids 748 to 942) of the human RASA1 protein (UniProt P20936-1) as set forth in SEQ ID NO: 388, optionally wherein one or multiple amino acids are in contact with RAS residues, or
   (xvii) a domain comprising the RAS binding region (amino acids 302 to 512) of the human RASA4 protein (UniProt O43374-1) as set forth in SEQ ID NO: 389, optionally wherein one or multiple amino acids are in contact with RAS residues, or
   (xviii) a domain comprising the RAS binding region (amino acids 938 to 1130) of the human RGS12 protein (UniProt O14924-1) as set forth in SEQ ID NO: 390, optionally wherein one or multiple amino acids are in contact with RAS residues.
22. The combination for use according to any one of embodiments 1 to 21, wherein the ABP comprises at least one antigen binding domain capable of binding to an extracellular Ras antigen, preferably provided that said extracellular Ras antigen is not part of, or is not restricted to, a HLA-peptide complex, or specific HLA alleles presented on the cell surface, and wherein said Ras binding ABP comprises a diabody format as set forth in SEQ ID NO: 228 to 239, wherein in each case independently comprise a sequence having no more than three or two, preferably no more than one amino acid substitution(s), deletion(s) or insertion(s) compared to SEQ ID NO: 224, and 228 to 239, respectively; or comprising a sequence having at least 75% sequence identity, preferably at least 80% sequence identity, more preferably at least 90% sequence identity, and most preferably at last 95% sequence identity with SEQ ID NO: 224, and 228 to 239.
23. The combination for use according to any one of embodiments 1 to 22 for use in a method of diagnosis, prevention and/or treatment of a proliferative disorder, such as cancer, and/or for use in adoptive, target-cell specific immunotherapy and/or for use in drug development and/or for use in a method of diagnosis, prevention and/or treatment of a non-malignant disease.
24. The combination for use according to embodiment 23, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancers, skin cancer, leukaemia, lymphoma, myeloma, myeloid malignancies, lymphoid malignancies, breast cancer, ovarian cancer, neuroendocrine carcinoma, endometrial cancer, vaginal cancers, blood-related cancers, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3), optionally wherein the cancer is metastatic, stage III cancer, or stage IV cancer, optionally wherein the cancer is a Ras dependent cancer.
25. Pharmaceutical composition for use as medicament comprising the combination according to any one of embodiments 1 to 24 and a pharmaceutically acceptable carrier, stabilizer and/or excipient.
26. Composition comprising
   - an antigen binding protein (ABP) or an isolated nucleic acid comprising a sequence encoding the ABP and
   - a modulator compound,
   wherein the ABP comprises at least a first antigen binding domain capable of binding to an extracellular Ras antigen and wherein the modulator compound is selected from the group consisting of
   a) an All-trans-retinoic acid (ATRA) based modulator,
   b) a modulator of the cholesterol and triglyceride synthesis or metabolism, preferably Simvastatin, Fluvastatin, Lapaquistat, or Bemfivastatin
   c) an immunomodulatory drug targeting cereblon, preferably Lenalidomide, Iberdomide, HOMO-Protac cereblon degrader 1, or Eragidomide,
   d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
   e) a kinase modulator, preferably of the Rho kinase pathway, preferably Narciclasine,
   f) a tyrosine kinase modulator, preferably of the JAK signaling pathway, preferably Ruxolitinib,
   g) a modulator of esterases, preferably of the phosphodiesterase pathway, preferably Anagrelide,
   h) a lipid, preferably a lipid part of a cell membrane
   i) a fatty acid, preferably a saturated fatty acid or unsaturated fatty acid
   j) a modulator of the gut microbiome, and
   k) an inhibitor of the naphthylisoquinoline alkaloid class, preferably Dioncophylline A.
   l) a combination of two or more of a) to k).
27. The composition according to embodiment 26, wherein the composition is a pharmaceutical composition, preferably additionally comprising a pharmaceutically acceptable carrier, stabilizer and/or excipient.
28. A kit comprising
   - an antigen binding protein (ABP) or an isolated nucleic acid comprising a sequence encoding the ABP and
   - a modulator compound,
   wherein the ABP comprises at least a first antigen binding domain capable of binding to an extracellular Ras antigen and wherein the modulator compound is selected from the group consisting of
   a) an All-trans-retinoic acid (ATRA) based modulator,
   b) a modulator of the cholesterol and triglyceride synthesis or metabolism, preferably Simvastatin, Fluvastatin, Lapaquistat, or Bemfivastatin
   c) an immunomodulatory drug targeting cereblon, preferably Lenalidomide, Iberdomide, HOMO-Protac cereblon degrader 1, or Eragidomide,
   d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
   e) a kinase modulator, preferably of the Rho kinase pathway, preferably Narciclasine,
   f) a tyrosine kinase modulator, preferably of the JAK signaling pathway, preferably Ruxolitinib,
   g) a modulator of esterases, preferably of the phosphodiesterase pathway, preferably Anagrelide,
   h) a lipid, preferably a lipid part of a cell membrane
   i) a fatty acid, preferably a saturated fatty acid or unsaturated fatty acid
   j) a modulator of the gut microbiome, and
   k) an inhibitor of the naphthylisoquinoline alkaloid class, preferably Dioncophylline A.
   l) a combination of two or more of a) to k).
29. An *in vitro* 3D cell culture model comprising tumor cells and at least one non-malignant cell selected from the group comprising or consisting of a fibroblast, an endothelial cell of an arterial blood vessel, an endothelial cell of a venous blood vessel, an endothelial cell of a lymphatic vessel, a tissue macrophage, a fat cell, an osteoblast, a chondrocyte, a smooth muscle cell, a preadipocyte, a pericyte, a mesenchymal stem cell, a melanocyte, a keratinocyte, hematopoietic progenitors, a dendritic cell, a skeletal muscle cell, a T cell and a B cell, preferably wherein the at least non-malignant cell is a T cell, a fibroblast and an endothelial cell.
30. The *in vitro* 3D cell culture model according to embodiment 29, further comprising the combination according to any one of embodiments 1 to 24 or the ABP as defined in any one of embodiments 1 to 24 for testing the influence of the combination on the viability and extracellular Ras expression of the at least one tumor cell.
31. The *in vitro* 3D cell culture model according to embodiment 29 or 30, further comprising a cellulose, preferably methylcellulose, and/or Matrigel^{®}.
32. The *in vitro* 3D cell culture model according to any one of embodiments 29 to 31 comprising at least one tumor cell, at least one T cell, at least one fibroblast, at least one aortic endothelial cell, at least one lymphoid endothelial cell and a cellulose, preferably methylcellulose, and further comprising the combination according to any one of embodiments 1 to 24 for testing the influence of the combination on the viability and extracellular Ras expression of the at least one tumor cell.
33. The *in vitro* 3D cell culture model according to any one of embodiments 29 to 32, wherein the cells are human cells.
34. An ABP as defined in any one of embodiments 1 to 24 for use in a method of diagnosis, prevention and/or treatment of a non-malignant disease.
35. An *in vitro* method for detecting the expression of predominantly extracellular Ras proteins on the surface of predominantly living cells selected from the group consisting of
   a) a method using a Ras protein labeling compound with substantially no penetration inside the cell and/or predominantly binding to the extracellular Ras protein
   b) a method using an agent blocking binding to intracellular Ras proteins and/or using a washing step to substantially remove binding to intracellular Ras proteins
   c) a method using a Ras protein labeling compound as of a) and one or more labeling compounds binding to intracellular proteins not belonging to the Ras protein family
   d) a method using a dual-antigen protein-protein interaction (PPI) reporter
   e) a method using cells expressing an altered Ras protein, preferably a Ras-tag fusion protein, wherein the tag can be detected on the extracellular side of the cell, optionally wherein the tag requires a second tag to be detectable (split-tag PPI)
   f) a method using an ABP as defined in any one of embodiments 1 to 24
   g) a method using 2D, 3D cell culture and/or spheroid or organoid cultures and/or stem cell-based embryo-like structures
   h) a method combining two or more of a) to g).
36. An *in vitro* method for detecting modulation of cell activity induced by binding of ABPs or compounds to extracellular Ras on the surface of predominantly living cells selected from the group consisting of
   a) a method using an agent to detect a cytokine level
   b) a method using at least one electrode to detect a change in cells' electrical potential
   c) a method using genetically modified cells with a reporter plasmid to detect and/or measure activation of a signaling cascade
   d) a method combining two or more of a) to c).
37. An *in silico* method for detecting modulation of extracellular Ras on the surface of artificial cells selected from the group consisting of
   a) a method using artificial cell membranes
   b) a method using artificial intelligence for calculating the extracellular Ras interface with small molecule compounds, with other proteins, with the lipid cell membrane and/or with the glycocalyx.

### REFERENCES

[1] Zhou Y, Hancock JF. "RAS nanoclusters are cell surface transducers that convert extracellular stimuli to intracellular signalling." FEBS Lett. 2023 Mar;597(6):892-908. doi: 10.1002/1873-3468.14569. Epub 2023 Jan 18. PMID: 36595205; PMCID: PMC10919257.
[2] Yang X, et al. "RAS signaling in carcinogenesis, cancer therapy and resistance mechanisms." J Hematol Oncol. 2024 Nov 9;17(1):108. doi: 10.1186/s13045-024-01631-9. PMID: 39522047; PMCID: PMC11550559.
[3]Wang, et al. "Identification of T-cell Receptors Targeting KRAS-Mutated Human Tumors." Cancer immunology research vol. 4,3 (2016): 204-14. doi:10.1158/2326-6066.CIR-15-0188.
[4]WO 2018/231759 A1
[5]WO 2019/241315 A1
[6]US 2015/166661 A1
[7]WO 2021/127184 A1
[8]Gulotta MR et al. "A Rational Design of α-Helix-Shaped Peptides Employing the Hydrogen-Bond Surrogate Approach: A Modulation Strategy for Ras-RasGRF1 Interaction in Neuropsychiatric Disorders." Pharmaceuticals (Basel). 2021 Oct 28;14(11):1099. doi: 10.3390/ph14111099. PMID: 34832880; PMCID: PMC8623491.
[9]Waters AM, et al. "Evaluation of the selectivity and sensitivity of isoform- and mutation-specific RAS antibodies." Sci Signal. 2017 Sep 26;10(498):eaao3332. doi: 10.1126/scisignal.aao3332. PMID: 28951536; PMCID: PMC5812265
[10]Blair HA. "Sotorasib: First Approval. Drugs". 2021 Sep;81(13):1573-1579. doi: 10.1007/s40265-021-01574-2. Erratum in: Drugs. 2021 Nov;81(16):1947. PMID: 34357500; PMCID: PMC8531079.
[11]Mulcahy LS, et al. "Requirement for ras proto-oncogene function during serum-stimulated growth of NIH 3T3 cells." Nature. 1985 Jan 17-23;313(5999):241-3. doi: 10.1038/313241a0. PMID: 3918269.
[12]Tomazini A, Shifman JM. "Targeting Ras with protein engineering." Oncotarget. 2023 Jul 1;14:672-687. doi: 10.18632/oncotarget.28469. PMID: 37395750; PMCID: PMC10317039.
[13]Ueda O, et al. "Corrigendum: Entire CD3ε, δ, and γ humanized mouse to evaluate human CD3-mediated therapeutics." Sci Rep. 2018 Mar 19;8:46960. doi: 10.1038/srep46960. Erratum for: Sci Rep. 2017 Apr 03;7:45839. PMID: 29553142; PMCID: PMC5857968.
[14]Yang JL et al. "A novel anti-p21Ras scFv antibody reacting specifically with human tumour cell lines and primary tumour tissues." BMC Cancer. 2016 Feb 20;16:131. doi: 10.1186/s12885-016-2168-6. PMID: 26897358; PMCID: PMC4761205.
[15]Wang P et al. "The immunoreactivity of the anti-p21Ras single-chain fragment variant KGH-R1 and its predicted binding sites to p21Ras." Immunotherapy. 2020 Aug;12(12):879-890. doi: 10.2217/imt-2019-0222. Epub 2020 Jul 14. PMID: 32664770.

SEQUENCE LISTING

SEQ ID NO 1: KRAS4A (amino acid sequence)







SEQ ID NO 2: KRAS4A (nucleotide sequence)

atgactgaat ataaacttgt ggtagttgga gctggtggcg taggcaagag tgccttgacg

atacagctaa ttcagaatca ttttgtggac gaatatgatc caacaataga ggattcctac

aggaagcaag tagtaattga tggagaaacc tgtctcttgg atattctcga cacagcaggt

caagaggagt acagtgcaat gagggaccag tacatgagga ctggggaggg ctttctttgt

gtatttgcca taaataatac taaatcattt gaagatattc accattatag agaacaaatt

aaaagagtta aggactctga agatgtacct atggtcctag taggaaataa atgtgatttg

ccttctagaa cagtagacac aaaacaggct caggacttag caagaagtta tggaattcct

tttattgaaa catcagcaaa gacaagacag agagtggagg atgcttttta tacattggtg
agagagatcc gacaatacag attgaaaaaa atcagcaaag aagaaaagac tcctggctgt
gtgaaaatta aaaaatgcat tataatgtaa

SEQ ID NO 3: KRAS4B (amino acid sequence)




SEQ ID NO 4: KRAS4B (nucleotide sequence)
atgactgaat ataaacttgt ggtagttgga gctggtggcg taggcaagag tgccttgacg
atacagctaa ttcagaatca ttttgtggac gaatatgatc caacaataga ggattcctac
aggaagcaag tagtaattga tggagaaacc tgtctcttgg atattctcga cacagcaggt
caagaggagt acagtgcaat gagggaccag tacatgagga ctggggaggg ctttctttgt
gtatttgcca taaataatac taaatcattt gaagatattc accattatag agaacaaatt
aaaagagtta aggactctga agatgtacct atggtcctag taggaaataa atgtgatttg
ccttctagaa cagtagacac aaaacaggct caggacttag caagaagtta tggaattcct
tttattgaaa catcagcaaa gacaagacag ggtgttgatg atgccttcta tacattagtt
cgagaaattc gaaaacataa agaaaagatg agcaaagatg gtaaaaagaa gaaaaagaag
tcaaagacaa agtgtgtaat tatgtaa

SEQ ID NO 5: NRAS (amino acid sequence)
MTEYKLVVVGAGGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 6: NRAS (nucleotide sequence)
atgactgag tacaaactgg tggtggttgg agcaggtggt gttgggaaaa gcgcactgac
aatccagcta atccagaacc actttgtaga tgaatatgat cccaccatag aggattctta
cagaaaacaa gtggttatag atggtgaaac ctgtttgttg gacatactgg atacagctgg
acaagaagag tacagtgcca tgagagacca atacatgagg acaggcgaag gcttcctctg
tgtatttgcc atcaataata gcaagtcatt tgcggatatt aacctctaca gggagcagat
taagcgagta aaagactcgg atgatgtacc tatggtgcta gtgggaaaca agtgtgattt
gccaacaagg acagttgata caaaacaagc ccacgaactg gccaagagtt acgggattcc
attcattgaa acctcagcca agaccagaca gggtgttgaa gatgcttttt acacactggt

aagagaaata cgccagtacc gaatgaaaaa actcaacagc agtgatgatg ggactcaggg
ttgtatggga ttgccatgtg tggtgatgtaa

SEQ ID NO 7: HRAS isoform 1 (amino acid sequence)




SEQ ID NO 8: HRAS isoform 1 (nucleotide sequence)
atgacg gaatataagc tggtggtggt gggcgccggc ggtgtgggca agagtgcgct
gaccatccag ctgatccaga accattttgt ggacgaatac gaccccacta tagaggattc
ctaccggaag caggtggtca ttgatgggga gacgtgcctg ttggacatcc tggataccgc
cggccaggag gagtacagcg ccatgcggga ccagtacatg cgcaccgggg agggcttcct
gtgtgtgttt gccatcaaca acaccaagtc ttttgaggac atccaccagt acagggagca
gatcaaacgg gtgaaggact cggatgacgt gcccatggtg ctggtgggga acaagtgtga
cctggctgca cgcactgtgg aatctcggca ggctcaggac ctcgcccgaa gctacggcat
cccctacatc gagacctcgg ccaagacccg gcagggagtg gaggatgcct tctacacgtt
ggtgcgtgag atccggcagc acaagctgcg gaagctgaac cctcctgatg agagtggccc
cggctgcatg agctgcaagt gtgtgctctc ctga

SEQ ID NO 9: HRAS isoform 2 (amino acid sequence)
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGSRSGSSSSSGTLWDPPGPM

SEQ ID NO 10: HRAS isoform 2 (nucleotide sequence)
atgacg gaatataagc tggtggtggt gggcgccggc ggtgtgggca agagtgcgct
gaccatccag ctgatccaga accattttgt ggacgaatac gaccccacta tagaggattc
ctaccggaag caggtggtca ttgatgggga gacgtgcctg ttggacatcc tggataccgc
cggccaggag gagtacagcg ccatgcggga ccagtacatg cgcaccgggg agggcttcct
gtgtgtgttt gccatcaaca acaccaagtc ttttgaggac atccaccagt acagggagca
gatcaaacgg gtgaaggact cggatgacgt gcccatggtg ctggtgggga acaagtgtga
cctggctgca cgcactgtgg aatctcggca ggctcaggac ctcgcccgaa gctacggcat
cccctacatc gagacctcgg ccaagacccg gcagggcagc cgctctggct ctagctccag
ctccgggacc ctctgggacc ccccgggacc catgtga

SEQ ID NO 11: HRAS G12A mutant
MTEYKLVVVGAAGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 12: HRAS G12C mutant
MTEYKLVVVGACGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 13: HRAS G12D mutant
MTEYKLVVVGADGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 14: HRAS G12R mutant
MTEYKLVVVGARGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 15: HRAS G12S mutant
MTEYKLVVVGASGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 16: HRAS G12V mutant




SEQ ID NO 17: HRAS G13C mutant
MTEYKLVVVGAGCVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 18: HRAS G13D mutant




SEQ ID NO 19: HRAS G13R mutant
MTEYKLVVVGAGRVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 20: RAS binder PCC01 (scFv = G12V-34)
QVQLQQPGAE LVRPGASVKL SCKTS GYSFTSYW MNWVKQRP GQGLEWIGY INPSTGYT
EYNQKFKDKA TLTADKSSSTAY MQLSSLTSEDSAVYYC ARRDYRYFDV
WGAGTTVTVSSGGGGSGGGGSGGGGSDIVMTQSPALM SAALGERVTM TCSAS SSVSY MHWYQQKS
GTSPKRWIY DTS KLASGVPARFSGS GSGTSYSLTISSMEA EDAATYYC QQWSSNPFT
FGSGTKLEIK

SEQ ID NO 21: RAS binder PCC02 (scFv = G13D-18)
GSELVRPGASV KLSCKAS GYTFTNYW IHWVKQRP GQGLEWIGN VYPGTGTT YYDEKFKSKA
SLTVDTSSSTAFM QLSSLTSEDSAVYYC TRIDYESLYFGV
WGAGTTLTVSSGGGGSGGGGSGGGGSDIQLTQSPASL SASVGETVTIT CRAS ENIYSY
LAWYQQKQG KSPQLLVY AAT NLADGVPSRFSGSGSGT QYSLKINSLQSEDFGSYYC
QHFWGTPFT FGSGTKLEIK

SEQ ID NO 22: RAS binder PCC03 (scFv = KGH_R1)
MAQVKLQESGPELVRPGTSVKVSCKASGYAFTNYLIEWIKQRPGQGLEWIGVIHPGNGGTNYNENFKG
KATLTADKSSSTAYMQLSSLTSDDSAVYFCASGNDGSYWGQGTTVTVSSGGGGSGGGGSGGGGSDIEL
TQSPSSLTVTAGEKVTMSCKSSQSLLNSGDQKIYLTWYQQKPGQPPKLLIYWASTRESGVPDRFTGSV
SGTDFTLTISSVQAEDLAVYYCQNDYNYPYTFGGGTKLEIKR

SEQ ID NO 23: RAS binder PCC04 (RAF1_RBD_CRD (AA 52 - 188))
KTSNTIRV FLPNKQRTVV NVRNGMSLHD CLMKALKVRG LQPECCAVFRLLHEHKGKKA
RLDWNTDAAS LIGEELQVDF LDHVPLTTHN FARKTFLKLAFCDICQKFLL NGFRCQTCGY
KFHEHCSTKV PTMCVDWS

SEQ ID NO 24: RAS binder PCC05 (SOS1_CDC25H (AA 780 - 1019))
HPIEIARQLTLLESDLYRAVQPSELVGSVWTKEDKEINSPNLLKMIRHTTNLTLWFEKCIVETENLEE
RVAVVSRIIEILQVFQELNNFNGVLEVVSAMNSSPVYRLDHTFEQIPSRQKKILEEAHELSEDHYKKY
LAKLRSINPPCVPFFGIYLTNILKTEEGNPEVLKRHGKELINFSKRRKVAEITGEIQQYQNQPYCLRV
ESDIKRFFENLNPMGNSMEKEFTDYLFNKSLEIEPR

SEQ ID NO 25: RAS binder PCC06 (VHH, NS1_Crystal)
gsvssvptkl evvaatptsl liswdapavt vdyyvityge tggnspvqkf evpgskstat
isglkpgvdy titvyawgwh gqvyyymgsp isinyrt

SEQ ID NO 26: RAS binder PCC07 (VHH, NS1_Patent)
SSVPTKLEVVAATSLLISWDAPAVTVDYYVITYGETGGNSPVQKFEVPGSKSTATISGLKPGVDYTIT
VYA WGWHGQVYYYMGSPISINYRT

SEQ ID NO 27: RAS binder PCC08 (SOS2_CDC25H (AA 778 - 1017))
HPI EIARQLTLLE SDLYRKVQPSELVGSVWTKE DKEINSPNLL KMIRHTTNLT LWFEKCIVEA
ENFEERVAVLSRIIEILQVFQDLNNFNGVLEIVSAVNSVSVYRLDHTFEALQERKRKILDEAVELSQD
HF KKYLVKLKSI NPPCVPFFGI YLTNILKTEE GNNDFLKKKGKDLINFSKRR KVAEITGEIQ
QYQNQPYCLR IEPDMRRFFE NLNPMGSASEKEFTDYLFNK SLEIEPR

SEQ ID NO 28: RAS binder PCC09 (RASGRP3_GEF (AA 152 - 383))
EPIELAEHLTFLEHKSFRRISFTDYQSYVIHGCLENNPTLERSIALFNGISKWVQLMVLS
KPTPQQRAEVITKFINVAKKLLQLKNFNTLMAVVGGLSHSSISRLKETHSHLSSEVTKNW
NEMTELVSSNGNYCNYRKAFADCDGFKIPILGVHLKDLIAVHVIFPDWTEENKVNIVKMH
QLSVTLSELVSLQNASHHLEPNMDLINLLTLSLDLYHTEDDIYKLSLVLEPR

SEQ ID NO 29: RAS binder PCC010 (RASGRF1_GEF (AA 1038-1270))
SALEIAEQLTLLDHLVFKKIPYEEFFGQGWMKLEKNERTPYIMKTTKHFNDISNLIASEI
IRNEDINARVSAIEKWVAVADICRCLHNYNAVLEITSSMNRSAIFRLKKTWLKVSKQTKA
LIDKLQKLVSSEGRFKNLREALKNCDPPCVPYLGMYLTDLAFIEEGTPNYTEDGLVNFSK
MRMISHIIREIRQFQQTAYKIEHQAKVTQYLLDQSFVMDEESLYESSLRIEPK

SEQ ID NO 30: RAS binder PCC011 (RASGRP1_GEF (AA 205 - 436))

EPEELS EHLTYLEFKS FRRISFSDYQ NYLVNSCVKE NPTMERSIALCNGISQWVQL
MVLSRPTPQL RAEVFIKFIQ VAQKLHQLQN FNTLMAVIGGLCHSSISRLK ETSSHVPHEI
NKVLGEMTEL LSSSRNYDNY RRAYGECTDFKIPILGVHLK DLISLYEAMP DYLEDGKVNV
HKLLALYNHI SELVQLQEVAPPLEANKDLV HLLTLSLDLY YTEDEIYELS YAREPR

SEQ ID NO 31: RAS binder PCC012 (RASGRP2_GEF (AA 154 - 387))
EPMELAE HLTYLEYRSF CKILFQDYHS FVTHGCTVDN PVLERFISLFNSVSQWVQLM
ILSKPTAPQR ALVITHFVHV AEKLLQLQNF NTLMAVVGGLSHSSISRLKE THSHVSPETI
KLWEGLTELV TATGNYGNYR RRLAACVGFRFPILGVHLKD LVALQLALPD WLDPARTRLN
GAKMKQLFSI LEELAMVTSL RPPVQANPDL LSLLTVSLDQ YQTEDELYQL SLQREPR

SEQ ID NO 32: long His tag (for antibody purification and detection)
HHHHHHHH

SEQ ID NO 33: short His tag (for antibody purification and detection)
HHHHHH

SEQ ID NO 34: Protein linker (between Ig constant domain and Ras
binders)
GSGGGGSGGGGSGGGGS

SEQ ID NO 35: Protein linker (between Ig constant domain and Ras
binders)
GSGGS

SEQ ID NO 36: Protein linker (between Ig constant domain and Ras
binders)
GA

SEQ ID NO 37: Protein linker (between Ig constant domain and Ras
binders)
AS

SEQ ID NO 38: Protein linker (between Ig constant domain and Ras
binders, and/or His tag and Ras binders)

GS

SEQ ID NO 39: Protein linker (between Ig constant domain and Ras
binders)
GG

SEQ ID NO 40: Protein linker (between antibody variable domains)
GGGGSGGGGSGGGGS

SEQ ID NO 41: Protein linker (between CL7 tag and 3c protease site)
SGGGGSGGGGSGGGGSGGGGSGGGG

SEQ ID NO 42: FC_Knop_WDN (Ig constant domain 1)
GSGGGGGSDKTHTCPPCPAPEAAGGPSVFLFPPKPKDTLMISRTPEVTCVVVDVSHEDPEVKFNWYVD
GVEVHNAKTKPREEQYASTYRVVSVLTVLHQDWLNGKEYKCKVSNKALAAPIEKTISKAKGQPREPQV
YTLPPCRDELTKNQVSLWCLVKGFYPSDIAVEWESNGQPENNYKTTPPVLDSDGSFFLYSKLTVDKSR
WQQGNVFSCSVMHEALHNHYTQKSLSLSPGK

SEQ ID NO 43: FC_hole_WDN (Ig constant domain 2)
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGKGS

SEQ ID NO 44: Protein eukaryote secretion leader
METDTLLVFVLLVWVPAGNG

SEQ ID NO 45: CL7 tag (for antibody purification and detection)
EPGEATGEGEPVNNKWLDNAGKDLGSPVPDRIADKLRDQEFESFDDFRETFWEEVSKDPELSKQFSRN
NNDRMKVGKAPKTRTQDVSGKRTSFELNHEKPIEENGGVYDMDNISVVTPKRNIDIE

SEQ ID NO 46: alternative CL7 tag (for antibody purification and
detection)
SKSNEPGKATGEGKPVNNKWLNNAGKDLGSPVPDRIANKLRDKEFESFDDFRETFWEEVSKDPELSKQ
FSRNNNDRMKVGKAPKTRTQDVSGKRTSFELNHQKPIEQNGGVYDMDNISVVTPKRNIDIEG

SEQ ID NO 47: 3C protease site
LEVLFQGP

SEQ ID NO 48: anti CD3 (scFv = diL2K)
DVQLVQSGAEVKKPGASVKVSCKASGYTFTRYTMHWVRQAPGQGLEWIGYINPSRGYTNYADSVKGRF
TITTDKSTSTAYMELSSLRSEDTATYYCARYYDDHYCLDYWGQGTTVTVSSGGGGSGGGGSGGGGSDI
VLTQSPATLSLSPGERATLSCRASQSVSYMNWYQQKPGKAPKRWIYDTSKVASGVPARFSGSGSGTDY
SLTINSLEAEDAATYYCQQWSSNPLTFGGGTKVEIK

SEQ ID NO 49: anti CD3 (scFv = UCHT1)
EVQLVESGGGLVQPGGSLRLSCAASGYSFTGYTMNWVRQAPGKGLEWVALINPYKGVS
TYNQKFKDRFTISVDKSKNTAYLQMNSLRAEDTAVYYCARSGYYGDSDWYFDVWGQGTLV
TVSSGGGGSGGGGSGGGGSDIQMTQSPSSLSASVGDRVTITCRASQDIRNYLNWYQQKPG
KAPKLLIYYTSRLESGVPSRFSGSGSGTDYTLTISSLQPEDFATYYCQQGNTLPWTFGQG
TKVEIK

SEQ ID NO 50: diL2K CDRH1
GYTFTRY

SEQ ID NO 51: diL2K CDRH2
NPSRG

SEQ ID NO 52: diL2K CDRH3
YDDHYC

SEQ ID NO 53: UCHT1 CDRH1
GYSFTGY

SEQ ID NO 54: UCHT1 CDRH2
NPYKG

SEQ ID NO 55: UCHT1 CDRH3
SGYYGDSDWY

SEQ ID NO 56: diL2K CDRL1
ASQSVSYM

SEQ ID NO 57: diL2K CDRL2
YDTSKV

SEQ ID NO 58: diL2K CDRL3
QQWSSNPLT

SEQ ID NO 59: UCHT1 CDRL1
ASQDIRNYL

SEQ ID NO 60: UCHT1 CDRL2
YTSRLES

SEQ ID NO 61: UCHT1 CDRL3
QQGNTLPWT

SEQ ID NO 62: diL2K VH
DVQLVQSGAEVKKPGASVKVSCKASGYTFTRYTMHWVRQAPGQGLEWIGYINPSRGYT
NYADSVKGRFTITTDKSTSTAYMELSSLRSEDTATYYCARYYDDHYCLDYWGQGTTVTVS
S

SEQ ID NO 63: diL2K VL
DIVLTQSPATLSLSPGERATLSCRASQSVSYMNWYQQKPGKAPK
RWIYDTSKVASGVPARFSGSGSGTDYSLTINSLEAEDAATYYCQQWSSNPLTFGGGTKVE
IK

SEQ ID NO 64: UCHT1 VH




SEQ ID NO 65: UCHT1 VL





SEQ ID NO 66: RAS binder PCC01 (G12V-34) VH
QVQLQQPGAE LVRPGASVKL SCKTS GYSFTSYW MNWVKQRP GQGLEWIGY INPSTGYT
EYNQKFKDKA TLTADKSSSTAY MQLSSLTSEDSAVYYC ARRDYRYFDV WGAGTTVTVSS

SEQ ID NO 67: RAS binder PCC01 (G12V-34) VL
DIVMTQSPALM SAALGERVTM TCSAS SSVSY MHWYQQKS GTSPKRWIY DTS
KLASGVPARFSGS GSGTSYSLTISSMEA EDAATYYC QQWSSNPFT FGSGTKLEIK

SEQ ID NO 68: RAS binder PCC02 (G13D-18) VH
GSELVRPGASV KLSCKAS GYTFTNYW IHWVKQRP GQGLEWIGN VYPGTGTT YYDEKFKSKA
SLTVDTSSSTAFM QLSSLTSEDSAVYYC TRIDYESLYFGV WGAGTTLTVSS

SEQ ID NO 69: RAS binder PCC02 (G13D-18) VL
DIQLTQSPASL SASVGETVTIT CRAS ENIYSY LAWYQQKQG KSPQLLVY AAT
NLADGVPSRFSGSGSGT QYSLKINSLQSEDFGSYYC QHFWGTPFT FGSGTKLEIK

SEQ ID NO 70: RAS binder PCC03 (KGH_R1) VH
MAQVKLQESGPELVRPGTSVKVSCKASGYAFTNYLIEWIKQRPGQGLEWIGVIHPGNGGTNYNENFKG
KATLTADKSSSTAYMQLSSLTSDDSAVYFCASGNDGSYWGQGTTVTVSS

SEQ ID NO 71: RAS binder PCC03 (KGH_R1) VL
DIELTQSPSSLTVTAGEKVTMSCKSSQSLLNSGDQKIYLTWYQQKPGQPPKLLIYWASTRESGVPDRF
TGSVSGTDFTLTISSVQAEDLAVYYCQNDYNYPYTFGGGTKLEIKR

SEQ ID NO 72: CAR T cell constant domain (-CD8 hinge_CD8 TM_ 4-
1BB_CD3zeta)
AAATTTPAPRPPTPAPTIASQPLSLRPEACRPAAGGAVHTRGLDFACDIYIWAPLAGTCGVLLLSLVI
TLYCKRGRKKLLYIFKQPFMRPVQTTQEEDGCSCRFPEEEEGGCELRVKFSRSADAPAYQQGQNQLYN
ELNLGRREEYDVLDKRRGRDPEMGGKPRRKNPQEGLYNELQKDKMAEAYSEIGMKGERRRGKGHDGLY
QGLSTATKDTYDALHMQALPPR

SEQ ID NO 73: SFV01_Fc knop

GPGADVQLVQSGAEVKKPGASVKVSCKASGYTFTRYTMHWVRQAPGQGLEWIGYINPSRGYTNYADSV
KGRFTITTDKSTSTAYMELSSLRSEDTATYYCARYYDDHYCLDYWGQGTTVTVSSGGGGSGGGGSGGG
GSDIVLTQSPATLSLSPGERATLSCRASQSVSYMNWYQQKPGKAPKRWIYDTSKVASGVPARFSGSGS
GTDYSLTINSLEAEDAATYYCQQWSSNPLTFGGGTKVEIKGSGGGGSDKTHTCPPCPAPEAAGGPSV
FLFPPKPKDTLMISRTPEVTCVVVDVSHEDPEVKFNWYVDGVEVHNAKTKPREEQYASTYRVVSVLTV
LHQDWLNGKEYKCKVSNKALAAPIEKTISKAKGQPREPQVYTLPPCRDELTKNQVSLWCLVKGFYPSD
IAVEWESNGQPENNYKTTPPVLDSDGSFFLYSKLTVDKSRWQQGNVFSCSVMHEALHNHYTQKSLSLS
PGK

SEQ ID NO 74: PCC01_Fc hole

DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGKGSGGS QVQLQQPGAE LVRPGASVKL SCKTS GYSFTSYW
MNWVKQRP GQGLEWIGY INPSTGYT EYNQKFKDKA TLTADKSSSTAY MQLSSLTSEDSAVYYC
ARRDYRYFDV WGAGTTVTVSSGGGGSGGGGSGGGGSDIVMTQSPALM SAALGERVTM TCSAS
SSVSY MHWYQQKS GTSPKRWIY DTS KLASGVPARFSGS GSGTSYSLTISSMEA EDAATYYC
QQWSSNPFT FGSGTKLEIK
GSHHHHHHHH

SEQ ID NO 75: SFV02_Fc knop
GPGAEVQLVESGGGLVQPGGSLRLSCAASGYSFTGYTMNWVRQAPGKGLEWVALINPYKGVSTYNQKF
KDRFTISVDKSKNTAYLQMNSLRAEDTAVYYCARSGYYGDSDWYFDVWGQGTLV
TVSSGGGGSGGGGSGGGGSDIQMTQSPSSLSASVGDRVTITCRASQDIRNYLNWYQQKPG
KAPKLLIYYTSRLESGVPSRFSGSGSGTDYTLTISSLQPEDFATYYCQQGNTLPWTFGQG
TKVEIKGSGGGGSDKTHTCPPCPAPEAAGGPSVFLFPPKPKDTLMISRTPEVTCVVVDVSHEDPEVK
FNWYVDGVEVHNAKTKPREEQYASTYRVVSVLTVLHQDWLNGKEYKCKVSNKALAAPIEKTISKAKGQ
PREPQVYTLPPCRDELTKNQVSLWCLVKGFYPSDIAVEWESNGQPENNYKTTPPVLDSDGSFFLYSKL
TVDKSRWQQGNVFSCSVMHEALHNHYTQKSLSLSPGK

SEQ ID NO 76: PCC06_Fc hole
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS

CSVMHEALHNHYTQKSLSLSPGKGS GGS GSVSSVPTKL EVVAATPTSL LISWDAPAVT
VDYYVITYGE TGGNSPVQKF EVPGSKSTAT ISGLKPGVDY TITVYAWGWH GQVYYYMGSP
ISINYRT GSHHHHHHHH

SEQ ID NO 77: HRAS G13S mutant
MTEYKLVVVGAGSVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 78: HRAS G13V mutant
MTEYKLVVVGAGVVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 79: HRAS Q61H mutant
MTEYKLVVVGAGGVGKSALTIQLIQNHFVDEYDPTIEDSYRKQVVIDGETCLLDILDTAGHEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 80: HRAS Q61K mutant
MTEYKLVVVGAGGVGKSALTIQLIQNHFVDEYDPTIEDSYRKQVVIDGETCLLDILDTAGKEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 81: HRAS Q61L mutant
MTEYKLVVVGAGGVGKSALTIQLIQNHFVDEYDPTIEDSYRKQVVIDGETCLLDILDTAGLEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 82: HRAS Q61P mutant
MTEYKLVVVGAGGVGKSALTIQLIQNHFVDEYDPTIEDSYRKQVVIDGETCLLDILDTAGPEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 83: HRAS Q61R mutant

MTEYKLVVVGAGGVGKSALTIQLIQNHFVDEYDPTIEDSYRKQVVIDGETCLLDILDTAGREEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 84: KRAS4A G12A mutant
MTEYKLVVVGAAGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 85: KRAS4A G12C mutant
MTEYKLVVVGACGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 86: KRAS4A G12D mutant
MTEYKLVVVGADGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 87: KRAS4A G12R mutant
MTEYKLVVVGARGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 88: KRAS4A G12S mutant
MTEYKLVVVGASGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 89: KRAS4A G12V mutant
MTEYKLVVVGAVGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 90: KRAS4A G13A mutant

MTEYKLVVVGAGAVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 91: KRAS4A G13C mutant
MTEYKLVVVGAGCVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 92: KRAS4A G13D mutant
MTEYKLVVVGAGDVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 93: KRAS4A G13R mutant
MTEYKLVVVGAGRVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 94: KRAS4A G13S mutant
MTEYKLVVVGAGSVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM
SEQ ID NO 95: KRAS4A G13V mutant
MTEYKLVVVGAGVVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 96: KRAS4A Q61E mutant
MTEYKLVVVGAGGVGKSALTIQLIQNHFVDEYDPTIEDSYRKQVVIDGETCLLDILDTAGEEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 97: KRAS4A Q61H mutant

MTEYKLVVVGAGGVGKSALTIQLIQNHFVDEYDPTIEDSYRKQVVIDGETCLLDILDTAGHEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 98: KRAS4A Q61K mutant
MTEYKLVVVGAGGVGKSALTIQLIQNHFVDEYDPTIEDSYRKQVVIDGETCLLDILDTAGKEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 99: KRAS4A Q61L mutant
MTEYKLVVVGAGGVGKSALTIQLIQNHFVDEYDPTIEDSYRKQVVIDGETCLLDILDTAGLEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 100: KRAS4A Q61P mutant
MTEYKLVVVGAGGVGKSALTIQLIQNHFVDEYDPTIEDSYRKQVVIDGETCLLDILDTAGPEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 101: KRAS4A Q61R mutant
MTEYKLVVVGAGGVGKSALTIQLIQNHFVDEYDPTIEDSYRKQVVIDGETCLLDILDTAGREEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 102: NRAS G12A mutant
MTEYKLVVVGAAGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 103: NRAS G12C mutant
MTEYKLVVVGACGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 104: NRAS G12D mutant





SEQ ID NO 105: NRAS G12R mutant




SEQ ID NO 106: NRAS G12S mutant
MTEYKLVVVGASGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 107: NRAS G12V mutant
MTEYKLVVVGAVGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 108: NRAS G13A mutant
MTEYKLVVVGAGAVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 109: NRAS G13C mutant
MTEYKLVVVGAGCVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 110: NRAS G13D mutant
MTEYKLVVVGAGDVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 111: NRAS G13R mutant

MTEYKLVVVGAGRVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 112: NRAS G13S mutant
MTEYKLVVVGAGSVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 113: NRAS G13V mutant
MTEYKLVVVGAGVVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 114: NRAS Q61E mutant
MTEYKLVVVGAGGVGKSALTIQLIQNHFVDEYDPTIEDSYRKQVVIDGETCLLDILDTAGEEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 115: NRAS Q61H mutant




SEQ ID NO 116: NRAS Q61K mutant
MTEYKLVVVGAGGVGKSALTIQLIQNHFVDEYDPTIEDSYRKQVVIDGETCLLDILDTAGKEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 117: NRAS Q61L mutant




SEQ ID NO 118: NRAS Q61P mutant

MTEYKLVVVGAGGVGKSALTIQLIQNHFVDEYDPTIEDSYRKQVVIDGETCLLDILDTAGPEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 119: NRAS Q61R mutant
MTEYKLVVVGAGGVGKSALTIQLIQNHFVDEYDPTIEDSYRKQVVIDGETCLLDILDTAGREEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 120: PCC01 - PCC012 Fc_Knop_WDN_UCHT1
GP
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSSGGGGSGGGGSGGGG
SDIQMTQSPSSLSASVGDRVTITCRASQDIRNYLNWYQQKPGKAPKLLIYYTSRLESGVPSRFSGSGS
GTDYTLTISSLQPEDFATYYCQQGNTLPWTFGQGTKVEIK GS
GGGGGSDKTHTCPPCPAPEAAGGPSVFLFPPKPKDTLMISRTPEVTCVVVDVSHEDPEVKFNWYVDGV
EVHNAKTKPREEQYASTYRVVSVLTVLHQDWLNGKEYKCKVSNKALAAPIEKTISKAKGQPREPQVYT
LPPCRDELTKNQVSLWCLVKGFYPSDIAVEWESNGQPENNYKTTPPVLDSDGSFFLYSKLTVDKSRWQ
QGNVFSCSVMHEALHNHYTQKSLSLSPGK

SEQ ID NO 121: PCC01 - PCC012 Fc_Knop_WDN_diL2K
GP
DVQLVQSGAEVKKPGASVKVSCKASGYTFTRYTMHWVRQAPGQGLEWIGYINPSRGYTNYADSVKGRF
TITTDKSTSTAYMELSSLRSEDTATYYCARYYDDHYCLDYWGQGTTVTVSSGGGGSGGGGSGGGGSDI
VLTQSPATLSLSPGERATLSCRASQSVSYMNWYQQKPGKAPKRWIYDTSKVASGVPARFSGSGSGTDY
SLTINSLEAEDAATYYCQQWSSNPLTFGGGTKVEIK GS
GGGGGSDKTHTCPPCPAPEAAGGPSVFLFPPKPKDTLMISRTPEVTCVVVDVSHEDPEVKFNWYVDGV
EVHNAKTKPREEQYASTYRVVSVLTVLHQDWLNGKEYKCKVSNKALAAPIEKTISKAKGQPREPQVYT
LPPCRDELTKNQVSLWCLVKGFYPSDIAVEWESNGQPENNYKTTPPVLDSDGSFFLYSKLTVDKSRWQ
QGNVFSCSVMHEALHNHYTQKSLSLSPGK

SEQ ID NO 122: PCC01 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS

CSVMHEALHNHYTQKSLSLSPGK GS GGS QVQLQQPGAE LVRPGASVKL SCKTS GYSFTSYW
MNWVKQRP GQGLEWIGY INPSTGYT EYNQKFKDKA
TLTADKSSSTAYMQLSSLTSEDSAVYYCARRDYRYFDVWGAGTTVTVSSGGGGSGGGGSGGGGSDIVM
TQSPALM SAALGERVTM TCSAS SSVSY MHWYQQKS GTSPKRWIY DTS KLASGVPARFSGS
GSGTSYSLTISSMEA EDAATYYC QQWSSNPFT FGSGTKLEIKGSHHHHHHHH

SEQ ID NO 123: PCC02 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGS GSELVRPGASV KLSCKAS GYTFTNYW IHWVKQRP
GQGLEWIGN VYPGTGTT YYDEKFKSKA SLTVDTSSSTAFM QLSSLTSEDSAVYYC
TRIDYESLYFGV WGAGTTLTVSSGGGGSGGGGSGGGGSDIQLTQSPASL SASVGETVTIT CRAS
ENIYSY LAWYQQKQG KSPQLLVY AAT NLADGVPSRFSGSGSGT QYSLKINSLQSEDFGSYYC
QHFWGTPFT FGSGTKLEIKGSHHHHHHHH

SEQ ID NO 124: PCC03 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS
GGSMAQVKLQESGPELVRPGTSVKVSCKASGYAFTNYLIEWIKQRPGQGLEWIGVIHPGNGGTNYNEN
FKGKATLTADKSSSTAYMQLSSLTSDDSAVYFCASGNDGSYWGQGTTVTVSSGGGGSGGGGSGGGGSD
IELTQSPSSLTVTAGEKVTMSCKSSQSLLNSGDQKIYLTWYQQKPGQPPKLLIYWASTRESGVPDRFT
GSVSGTDFTLTISSVQAEDLAVYYCQNDYNYPYTFGGGTKLEIKRGSHHHHHHHH

SEQ ID NO 125: PCC04 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGGGSGGGGSGGGGSKTSNTIRV FLPNKQRTVV
NVRNGMSLHD CLMKALKVRG LQPECCAVFRLLHEHKGKKA RLDWNTDAAS LIGEELQVDF
LDHVPLTTHN FARKTFLKLAFCDICQKFLL NGFRCQTCGY KFHEHCSTKV
PTMCVDWSGSHHHHHHHH

SEQ ID NO 126: PCC05 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS
GGGGSGGGGSGGGGSHPIEIARQLTLLESDLYRAVQPSELVGSVWTKEDKEINSPNLLKMIRHTTNLT
LWFEKCIVETENLEERVAVVSRIIEILQVFQELNNFNGVLEVVSAMNSSPVYRLDHTFEQIPSRQKKI
LEEAHELSEDHYKKYLAKLRSINPPCVPFFGIYLTNILKTEEGNPEVLKRHGKELINFSKRRKVAEIT
GEIQQYQNQPYCLRVESDIKRFFENLNPMGNSMEKEFTDYLFNKSLEIEPRGSHHHHHHHH

SEQ ID NO 127: PCC06 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGS gsvssvptkl evvaatptsl liswdapavt
vdyyvityge tggnspvqkf evpgskstat isglkpgvdy titvyawgwh gqvyyymgsp
isinyrt GSHHHHHHHH

SEQ ID NO 128: PCC07 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGS
SSVPTKLEVVAATSLLISWDAPAVTVDYYVITYGETGGNSPVQKFEVPGSKSTATISGLKPGVDYTIT
VYA WGWHGQVYYYMGSPISINYRTGSHHHHHHHH

SEQ ID NO 129: PCC08 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGGGSGGGGSGGGGSHPI EIARQLTLLE
SDLYRKVQPSELVGSVWTKE DKEINSPNLL KMIRHTTNLT LWFEKCIVEA
ENFEERVAVLSRIIEILQVFQDLNNFNGVLEIVSAVNSVSVYRLDHTFEALQERKRKILDEAVELSQD
HF KKYLVKLKSI NPPCVPFFGI YLTNILKTEE GNNDFLKKKGKDLINFSKRR KVAEITGEIQ
QYQNQPYCLR IEPDMRRFFE NLNPMGSASEKEFTDYLFNK SLEIEPRGSHHHHHHHH

SEQ ID NO 130: PCC09 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS
GGGGSGGGGSGGGGSEPIELAEHLTFLEHKSFRRISFTDYQSYVIHGCLENNPTLERSIALFNGISKW
VQLMVLS
KPTPQQRAEVITKFINVAKKLLQLKNFNTLMAVVGGLSHSSISRLKETHSHLSSEVTKNW
NEMTELVSSNGNYCNYRKAFADCDGFKIPILGVHLKDLIAVHVIFPDWTEENKVNIVKMH
QLSVTLSELVSLQNASHHLEPNMDLINLLTLSLDLYHTEDDIYKLSLVLEPRGSHHHHHHHH

SEQ ID NO 131: PCC010 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS
GGGGSGGGGSGGGGSSALEIAEQLTLLDHLVFKKIPYEEFFGQGWMKLEKNERTPYIMKTTKHFNDIS
NLIASEI
IRNEDINARVSAIEKWVAVADICRCLHNYNAVLEITSSMNRSAIFRLKKTWLKVSKQTKA
LIDKLQKLVSSEGRFKNLREALKNCDPPCVPYLGMYLTDLAFIEEGTPNYTEDGLVNFSK
MRMISHIIREIRQFQQTAYKIEHQAKVTQYLLDQSFVMDEESLYESSLRIEPK GSHHHHHHHH

SEQ ID NO 132: PCC011 Fc hole_WDN
DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGGGSGGGGSGGGGSEPEELS EHLTYLEFKS
FRRISFSDYQ NYLVNSCVKE NPTMERSIALCNGISQWVQL MVLSRPTPQL RAEVFIKFIQ
VAQKLHQLQN FNTLMAVIGGLCHSSISRLK ETSSHVPHEI NKVLGEMTEL LSSSRNYDNY
RRAYGECTDFKIPILGVHLK DLISLYEAMP DYLEDGKVNV HKLLALYNHI
SELVQLQEVAPPLEANKDLV HLLTLSLDLY YTEDEIYELS YAREPRGSHHHHHHHH

SEQ ID NO 133: PCC012 Fc hole_WDN

DKTHTCPPCPAPEAAGGPSVFLFPPKPKDTLMISRTPEVTCVVVDVSHEDPEVKFNWYVDGVEVHNAK
TKPREEQYASTYRVVSVLTVLHQDWLNGKEYKCKVSNKALAAPIEKTISKAKGQPREPQVCTLPPSRD
ELTKNQVSLSCAVKGFYPSDIAVEWESNGQPENNYKTTPPVLDSDGSFFLVSKLTVDKSRWQQGNVFS
CSVMHEALHNHYTQKSLSLSPGK GS GGGGSGGGGSGGGGSEPMELAE HLTYLEYRSF
CKILFQDYHS FVTHGCTVDN PVLERFISLFNSVSQWVQLM ILSKPTAPQR ALVITHFVHV
AEKLLQLQNF NTLMAVVGGLSHSSISRLKE THSHVSPETI KLWEGLTELV TATGNYGNYR
RRLAACVGFRFPILGVHLKD LVALQLALPD WLDPARTRLN GAKMKQLFSI LEELAMVTSL
RPPVQANPDL LSLLTVSLDQ YQTEDELYQL SLQREPRGSHHHHHHHH

SEQ ID NO 134: VH from PCC01 (scFv = G12V-34) FR1
QVQLQQPGAELVRPGASVKLSCKTS

SEQ ID NO 135: VH from PCC01 (scFv = G12V-34) CDRH1
GYSFTSYW

SEQ ID NO 136: VH from PCC01 (scFv = G12V-34) FR2
MNWVKQRPGQGLEWIGY

SEQ ID NO 137: VH from PCC01 (scFv = G12V-34) CDRH2
INPSTGYT

SEQ ID NO 138: VH from PCC01 (scFv = G12V-34) FR3
EYNQKFKDKATLTADKSSSTAYMQLSSLTSEDSAVYYC

SEQ ID NO 139: VH from PCC01 (scFv = G12V-34) CDRH3
ARRDYRYFDV

SEQ ID NO 140: VH from PCC01 (scFv = G12V-34) FR4
WGAGTTVTVSS

SEQ ID NO 141: VL from PCC01 (scFv = G12V-34) FR1
DIVMTQSPALMSAALGERVTMTCSAS

SEQ ID NO 142: VL from PCC01 (scFv = G12V-34) CDRL1
SSVSY

SEQ ID NO 143: VL from PCC01 (scFv = G12V-34) FR2
MHWYQQKSGTSPKRWIY

SEQ ID NO 144: VL from PCC01 (scFv = G12V-34) CDRL2
DTS

SEQ ID NO 145: VL from PCC01 (scFv = G12V-34) FR3
KLASGVPARFSGSGSGTSYSLTISSMEAEDAATYYC

SEQ ID NO 146: VL from PCC01 (scFv = G12V-34) CDRL3
QQWSSNPFT

SEQ ID NO 147: VL from PCC01 (scFv = G12V-34) FR4
FGSGTKLEIK

SEQ ID NO 148: VH from PCC02 (scFv = G13D-18) FR1
GSELVRPGASVKLSCKAS

SEQ ID NO 149: VH from PCC02 (scFv = G13D-18) CDRH1
GYTFTNYW

SEQ ID NO 150: VH from PCC02 (scFv = G13D-18) FR2
IHWVKQRPGQGLEWIGN

SEQ ID NO 151: VH from PCC02 (scFv = G13D-18) CDRH2
VYPGTGTT

SEQ ID NO 152: VH from PCC02 (scFv = G13D-18) FR3
YYDEKFKSKASLTVDTSSSTAFMQLSSLTSEDSAVYYC

SEQ ID NO 153: VH from PCC02 (scFv = G13D-18) CDRH3
TRIDYESLYFGV

SEQ ID NO 154: VH from PCC02 (scFv = G13D-18) FR4

WGAGTTLTVSS

SEQ ID NO 155: VL from PCC02 (scFv = G13D-18) FR1
DIQLTQSPASLSASVGETVTITCRAS

SEQ ID NO 156: VL from PCC02 (scFv = G13D-18) CDRL1
ENIYSY

SEQ ID NO 157: VL from PCC02 (scFv = G13D-18) FR2
LAWYQQKQGKSPQLLVY

SEQ ID NO 158: VL from PCC02 (scFv = G13D-18) CDRL2
AAT

SEQ ID NO 159: VL from PCC02 (scFv = G13D-18) FR3
NLADGVPSRFSGSGSGTQYSLKINSLQSEDFGSYYC

SEQ ID NO 160: VL from PCC02 (scFv = G13D-18) CDRL3
QHFWGTPFT

SEQ ID NO 160: VL from PCC02 (scFv = G13D-18) FR4
FGSGTKLEIK

SEQ ID NO 161: VH from PCC03 (scFv = KGH_R1) FR1
MAQVKLQESGPELVRPGTSVKVSCKAS

SEQ ID NO 162: VH from PCC03 (scFv = KGH_R1) CDRH1
GYAFTNYL

SEQ ID NO 163: VH from PCC03 (scFv = KGH_R1) FR2
IEWIKQRPGQGLEWIGV

SEQ ID NO 164: VH from PCC03 (scFv = KGH_R1) CDRH2
IHPGNGGT

SEQ ID NO 165: VH from PCC03 (scFv = KGH_R1) FR3
NYNENFKGKATLTADKSSSTAYMQLSSLTSDDSAVYFC

SEQ ID NO 166: VH from PCC03 (scFv = KGH_R1) CDRH3
ASGNDGSY

SEQ ID NO 167: VH from PCC03 (scFv = KGH_R1) FR4
WGQGTTVTVSS

SEQ ID NO 168: VL from PCC03 (scFv = KGH_R1) FR1
DIELTQSPSSLTVTAGEKVTMSCKSS

SEQ ID NO 169: VL from PCC03 (scFv = KGH_R1) CDRL1
QSLLNSGDQKIY

SEQ ID NO 170: VL from PCC03 (scFv = KGH_R1) FR2
LTWYQQKPGQPPKLLIY

SEQ ID NO 171: VL from PCC03 (scFv = KGH_R1) CDRL2
WASTRES

SEQ ID NO 172: VL from PCC03 (scFv = KGH_R1) FR3
GVPDRFTGSVSGTDFTLTISSVQAEDLAVYYC

SEQ ID NO 173: VL from PCC03 (scFv = KGH_R1) CDRL3
QNDYNYPYT

SEQ ID NO 174: VL from PCC03 (scFv = KGH_R1) FR4
FGGGTKLEIKR

SEQ ID NO 175: FN3 loop BC
DYYV

SEQ ID NO 176: FN3 loop DE
KFE

SEQ ID NO 177: FN3 loop FG
WGWHGQVYYYMG

SEQ ID NO 178: FN3 loop CD
GNSPV

SEQ ID NO 179: protease amino acids removed from KRAS4A
TEYKLVVVGAGGVGKSALTIQLIQNHFVDEYDPTIEDSYRKQVVIDGETCLLDILDTAGQEEYSAMRD
QYMRTGEGFLCVFAINNTKSFEDIHHYREQIKRVKDSEDVPMVLVGNKCDLPSRTVDTKQAQDLARSY
GIPFIETSAKTRQRVEDAFYTLVREIRQYRLKKISKEEKTPGCVKIKKC

SEQ ID NO 180: protease amino acids removed from KRAS4B
TEYKLVVVGAGGVGKSALTIQLIQNHFVDEYDPTIEDSYRKQVVIDGETCLLDILDTAGQEEYSAMRD
QYMRTGEGFLCVFAINNTKSFEDIHHYREQIKRVKDSEDVPMVLVGNKCDLPSRTVDTKQAQDLARSY
GIPFIETSAKTRQGVDDAFYTLVREIRKHKEKMSKDGKKKKKKSKTKC

SEQ ID NO 181: protease amino acids removed from NRAS
TEYKLVVVGAGGVGKSALTIQLIQNHFVDEYDPTIEDSYRKQVVIDGETCLLDILDTAGQEEYSAMRD
QYMRTGEGFLCVFAINNSKSFADINLYREQIKRVKDSDDVPMVLVGNKCDLPTRTVDTKQAHELAKSY
GIPFIETSAKTRQGVEDAFYTLVREIRQYRMKKLNSSDDGTQGCMGLPC

SEQ ID NO 182: protease amino acids removed from HRAS
TEYKLVVVGAGGVGKSALTIQLIQNHFVDEYDPTIEDSYRKQVVIDGETCLLDILDTAGQEEYSAMRD
QYMRTGEGFLCVFAINNTKSFEDIHQYREQIKRVKDSDDVPMVLVGNKCDLAARTVESRQAQDLARSY
GIPYIETSAKTRQGVEDAFYTLVREIRQHKLRKLNPPDESGPGCMSCKC

SEQ ID NO 183: KRAS4B G12A mutant




SEQ ID NO 184: KRAS4B G12C mutant

MTEYKLVVVGACGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 185: KRAS4B G12D mutant




SEQ ID NO 186: KRAS4B G12R mutant
MTEYKLVVVGARGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 187: KRAS4B G12S mutant




SEQ ID NO 188: KRAS4B G12V mutant
MTEYKLVVVGAVGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 189: KRAS4B G13A mutant
MTEYKLVVVGAGAVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 190: KRAS4B G13C mutant
MTEYKLVVVGAGCVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 191: KRAS4B G13D mutant





SEQ ID NO 192: KRAS4B G13R mutant
MTEYKLVVVGAGRVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 193: KRAS4B G13S mutant
MTEYKLVVVGAGSVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 194: KRAS4B G13V mutant
MTEYKLVVVGAGVVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 195: KRAS4B Q61H mutant




SEQ ID NO 196: KRAS4B Q61K mutant
MTEYKLVVVGAGGVGKSALTIQLIQNHFVDEYDPTIEDSYRKQVVIDGETCLLDILDTAGKEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 197: KRAS4B Q61L mutant
MTEYKLVVVGAGGVGKSALTIQLIQNHFVDEYDPTIEDSYRKQVVIDGETCLLDILDTAGLEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 198: KRAS4B Q61P mutant

MTEYKLVVVGAGGVGKSALTIQLIQNHFVDEYDPTIEDSYRKQVVIDGETCLLDILDTAGPEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 199: KRAS4B Q61R mutant
MTEYKLVVVGAGGVGKSALTIQLIQNHFVDEYDPTIEDSYRKQVVIDGETCLLDILDTAGREEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 200: KRAS4B A146T mutant




SEQ ID NO 201: KRAS4B A146P mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSPKTRQGVDDAFYTLVREIRKHKEKMSKDGKKKKKKSKTKCVIM

SEQ ID NO 202: KRAS4B A146V mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSVKTRQGVDDAFYTLVREIRKHKEKMSKDGKKKKKKSKTKCVIM

SEQ ID NO 203: KRAS4B A59E mutant
MTEYKLVVVGAGGVGKSALTIQLIQNHFVDEYDPTIEDSYRKQVVIDGETCLLDILDTEGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 204: KRAS4B A59G mutant
MTEYKLVVVGAGGVGKSALTIQLIQNHFVDEYDPTIEDSYRKQVVIDGETCLLDILDTGGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 205: KRAS4B A59T mutant

MTEYKLVVVGAGGVGKSALTIQLIQNHFVDEYDPTIEDSYRKQVVIDGETCLLDILDTTGQEEYSAMR
DQYMRTGEGFLCVFAINNTKSFEDIHHYREQIKRVKDSEDVPMVLVGNKCDLPSRTVDTKQAQDLARS
YGIPFIETSAKTRQGVDDAFYTLVREIRKHKEKMSKDGKKKKKKSKTKCVIM

SEQ ID NO 206: KRAS4A A146T mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSTKTRQRVEDAFYTLVREIRQYRLKKISKEEKTPGCVKIKKCIIM

SEQ ID NO 207: KRAS4A A146P mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSPKTRQRVEDAFYTLVREIRQYRLKKISKEEKTPGCVKIKKCIIM

SEQ ID NO 208: KRAS4A A146V mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHHYREQIKRVKDSEDVPMVLVGNKCDLPSRTVDTKQAQDLARS
YGIPFIETSVKTRQRVEDAFYTLVREIRQYRLKKISKEEKTPGCVKIKKCIIM

SEQ ID NO 209: KRAS4A A59E mutant
MTEYKLVVVGAGGVGKSALTIQLIQNHFVDEYDPTIEDSYRKQVVIDGETCLLDILDTEGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 210: KRAS4A A59G mutant
MTEYKLVVVGAGGVGKSALTIQLIQNHFVDEYDPTIEDSYRKQVVIDGETCLLDILDTGGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 211: KRAS4A A59T mutant
MTEYKLVVVGAGGVGKSALTIQLIQNHFVDEYDPTIEDSYRKQVVIDGETCLLDILDTTGQEEYSAMR
DQYMRTGEGFLCVFAINNTKSFEDIHHYREQIKRVKDSEDVPMVLVGNKCDLPSRTVDTKQAQDLARS
YGIPFIETSAKTRQRVEDAFYTLVREIRQYRLKKISKEEKTPGCVKIKKCIIM

SEQ ID NO 212: NRAS  A146T mutant

MTEYKLVVVGAGGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSTKTRQGVEDAFYTLVREIRQYRMKKLNSSDDGTQGCMGLPCVVM

SEQ ID NO 213: NRAS A146P mutant
MTEYKLVVVGAGGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSPKTRQGVEDAFYTLVREIRQYRMKKLNSSDDGTQGCMGLPCVVM

SEQ ID NO 214: NRAS A146V mutant
MTEYKLVVVGAGGVGKSALTIQLIQNHFVDEYDPTIEDSYRKQVVIDGETCLLDILDTAGQEEYSAMR
DQYMRTGEGFLCVFAINNSKSFADINLYREQIKRVKDSDDVPMVLVGNKCDLPTRTVDTKQAHELAKS
YGIPFIETSVKTRQGVEDAFYTLVREIRQYRMKKLNSSDDGTQGCMGLPCVVM

SEQ ID NO 215: NRAS A59E mutant
MTEYKLVVVGAGGVGKSALTIQLIQNHFVDEYDPTIEDSYRKQVVIDGETCLLDILDTEGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 216: NRAS A59G mutant
MTEYKLVVVGAGGVGKSALTIQLIQNHFVDEYDPTIEDSYRKQVVIDGETCLLDILDTGGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 217: NRAS A59T mutant
MTEYKLVVVGAGGVGKSALTIQLIQNHFVDEYDPTIEDSYRKQVVIDGETCLLDILDTTGQEEYSAMR
DQYMRTGEGFLCVFAINNSKSFADINLYREQIKRVKDSDDVPMVLVGNKCDLPTRTVDTKQAHELAKS
YGIPFIETSAKTRQGVEDAFYTLVREIRQYRMKKLNSSDDGTQGCMGLPCVVM

SEQ ID NO 218: HRAS A146T mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSTKTRQGVEDAFYTLVREIRQHKLRKLNPPDESGPGCMSCKCVLS

SEQ ID NO 219: HRAS A146P mutant

MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSPKTRQGVEDAFYTLVREIRQHKLRKLNPPDESGPGCMSCKCVLS

SEQ ID NO 220: HRAS A146V mutant
MTEYKLVVVGAGGVGKSALTIQLIQNHFVDEYDPTIEDSYRKQVVIDGETCLLDILDTAGQEEYSAMR
DQYMRTGEGFLCVFAINNTKSFEDIHQYREQIKRVKDSDDVPMVLVGNKCDLAARTVESRQAQDLARS
YGIPYIETSVKTRQGVEDAFYTLVREIRQHKLRKLNPPDESGPGCMSCKCVLS

SEQ ID NO 221: HRAS A59E mutant
MTEYKLVVVGAGGVGKSALTIQLIQNHFVDEYDPTIEDSYRKQVVIDGETCLLDILDTEGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 222: HRAS A59G mutant
MTEYKLVVVGAGGVGKSALTIQLIQNHFVDEYDPTIEDSYRKQVVIDGETCLLDILDTGGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 223: HRAS A59T mutant
MTEYKLVVVGAGGVGKSALTIQLIQNHFVDEYDPTIEDSYRKQVVIDGETCLLDILDTTGQEEYSAMR
DQYMRTGEGFLCVFAINNTKSFEDIHQYREQIKRVKDSDDVPMVLVGNKCDLAARTVESRQAQDLARS
YGIPYIETSAKTRQGVEDAFYTLVREIRQHKLRKLNPPDESGPGCMSCKCVLS

SEQ ID NO 224: Diabody (aCD3VL - ABP insert - aCD3VH-6xHis-tag)
DIQMTQSPSSLSASVGDRVTITCRASQDIRNYLNWYQQKPGKAPKLLIYYTSRLESGVPSRFSGSGSG
TDYTLTISSLQPEDFATYYCQQGNTLPWTFGQGTKVEIK
GGGGSGGGGSGGGGSGGGGAASGASGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 225: Thioredox_Ecoli
M S D K I I H L T D D S F D T D V L K A D G A I L V D F W A E W C G P
C K M I A P I L D E I A D E Y Q G K L T V A K L N I D Q N P G T A P K
Y G I R G I P T L L L F K N G E V A A T K V G A L S K G Q L K E F L D

A                                    N                                    L                                    A
LEEPGEATGEGEPVNNKWLDNAGKDLGSPVPDRIADKLRDQEFESFDDFRETFWEEVSKDPELSKQFS
RNNNDRMKVGKAPKTRTQDVSGKRTSFELNHEKPIEENGGVYDMDNISVVTPKRNIDIESGGGGSGGS
GGSGGSLEVLFQGP

SEQ ID NO 226: TF_PCs_Ecoli
MYPEVELQGLEAIEVEKPIVEVTDADVDGMLDTLRKQQATWKEKDGAVEAEDRVTIDFTGSVDGEEFE
GGKASDFVLAMGQGRMIPGFEDGIKGHKAGEEFTIDVTFPEEYHAENLKGKAAKFAINLKKVEERELP
ELTAEFIKRFGVEDGSVEGLRAEVRKNMERELKSAIRNRVKSQAIEGLVKANDIDVPAALIDSEIDVL
RRQAAQRFGGNEKQALELPRELFEEQAKRRVVVGLLLGEVIRTNELKADEERVKGLIEEMASAYEDPK
EVIEFYSKNKELMDNMRNVALEEQAVEAVLAKAKVTEKETTFNELMNQQASGGGGSGGSGGSGGSLEE
PGEATGEGEPVNNKWLDNAGKDLGSPVPDRIADKLRDQEFESFDDFRETFWEEVSKDPELSKQFSRNN
NDRMKVGKAPKTRTQDVSGKRTSFELNHEKPIEENGGVYDMDNISVVTPKRNIDIESGGGGSGGSGGS
GGSLEVLFQGP

SEQ ID NO 227: TF_PCs_ Pseudo
MYPEVEVQGLDKITVEKPAVTVTDEDLANMLETLRKQHAAWADVDAAAGENDRVTVDFVGTIDGEVFE
GGKAEDFPLELGQGRMIPGFEDNIVGKKAGEEVVADVTFPDDYHAENLKGKAAQFTITVKKVEAQELP
ELSEEFATKFGVTEGGVDALKEEVKKNMTRELDQAVKASVKDQAIKGLLANNEIEVPKALVDQEVDAL
RQQAAQRFGGDAQNMPELPAELFHEQAVTRVKTGLLLGEVIKANDIKVDDAKVEALIATVASAYEDPT
EVVEYYKGNDQLMQQMRNVAMEEQAVEAILAKAVVTDVEKAFDDIMNPQQGASGGGGSGGSGGSGGSL
EEPGEATGEGEPVNNKWLDNAGKDLGSPVPDRIADKLRDQEFESFDDFRETFWEEVSKDPELSKQFSR
NNNDRMKVGKAPKTRTQDVSGKRTSFELNHEKPIEENGGVYDMDNISVVTPKRNIDIESGGGGSGGSG
GSGGSLEVLFQGP

SEQ ID NO 228: PCC01_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK      GGGGSGGGGSGGGGSGGGGA
QVQLQQPGAE  LVRPGASVKL  SCKTS  GYSFTSYW  MNWVKQRP  GQGLEWIGY  INPSTGYT
EYNQKFKDKA       TLTADKSSSTAY       MQLSSLTSEDSAVYYC       ARRDYRYFDV
WGAGTTVTVSSGGGGSGGGGSGGGGSDIVMTQSPALM SAALGERVTM TCSAS SSVSY MHWYQQKS
GTSPKRWIY  DTS  KLASGVPARFSGS  GSGTSYSLTISSMEA  EDAATYYC  QQWSSNPFT
FGSGTKLEIKGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 229: PCC02_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK GGGGSGGGGSGGGGSGGGGA
EVQLVRPGASV KLSCKAS GYTFTNYW IHWVKQRP GQGLEWIGN VYPGTGTT YYDEKFKSKA
SLTVDTSSSTAFM QLSSLTSEDSAVYYC TRIDYESLYFGV
WGAGTTLTVSSGGGGSGGGGSGGGGSDIQLTQSPASL SASVGETVTIT CRAS ENIYSY
LAWYQQKQG KSPQLLVY AAT NLADGVPSRFSGSGSGT QYSLKINSLQSEDFGSYYC
QHFWGTPFT FGSGTKLEIKGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 230: PCC03_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK GGGGSGGGGSGGGGSGGGGA
QVKLQESGPELVRPGTSVKVSCKASGYAFTNYLIEWIKQRPGQGLEWIGVIHPGNGGTNYNENFKGKA
TLTADKSSSTAYMQLSSLTSDDSAVYFCASGNDGSYWGQGTTVTVSSGGGGSGGGGSGGGGSDIELTQ
SPSSLTVTAGEKVTMSCKSSQSLLNSGDQKIYLTWYQQKPGQPPKLLIYWASTRESGVPDRFTGSVSG
TDFTLTISSVQAEDLAVYYCQNDYNYPYTFGGGTKLEIKRGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 231: PCC04_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGAKTSNTIRV FLPNKQRTVV NVRNGMSLHD CLMKALKVRG
LQPECCAVFRLLHEHKGKKA RLDWNTDAAS LIGEELQVDF LDHVPLTTHN
FARKTFLKLAFCDICQKFLL NGFRCQTCGY KFHEHCSTKV
PTMCVDWSGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 232: PCC05_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK

GGGGSGGGGSGGGGSGGGGAHPIEIARQLTLLESDLYRAVQPSELVGSVWTKEDKEINSPNLLKMIRH
TTNLTLWFEKCIVETENLEERVAVVSRIIEILQVFQELNNFNGVLEVVSAMNSSPVYRLDHTFEQIPS
RQKKILEEAHELSEDHYKKYLAKLRSINPPCVPFFGIYLTNILKTEEGNPEVLKRHGKELINFSKRRK
VAEITGEIQQYQNQPYCLRVESDIKRFFENLNPMGNSMEKEFTDYLFNKSLEIEPRGSGGSGGGGSGG
GGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 233: PCC06_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGAgsvssvptkl evvaatptsl liswdapavt vdyyvityge
tggnspvqkf evpgskstat isglkpgvdy titvyawgwh gqvyyymgsp
isinyrtGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 234: PCC07_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGASSVPTKLEVVAATSLLISWDAPAVTVDYYVITYGETGGNSPVQKFEVP
GSKSTATISGLKPGVDYTITVYA WGWHGQVYYYMGSPISINYRTGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 235: PCC08_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK GGGGSGGGGSGGGGSGGGGAHPI
EIARQLTLLE SDLYRKVQPSELVGSVWTKE DKEINSPNLL KMIRHTTNLT LWFEKCIVEA
ENFEERVAVLSRIIEILQVFQDLNNFNGVLEIVSAVNSVSVYRLDHTFEALQERKRKILDEAVELSQD
HF KKYLVKLKSI NPPCVPFFGI YLTNILKTEE GNNDFLKKKGKDLINFSKRR KVAEITGEIQ
QYQNQPYCLR IEPDMRRFFE NLNPMGSASEKEFTDYLFNK
SLEIEPRGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 236: PCC09_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGAEPIELAEHLTFLEHKSFRRISFTDYQSYVIHGCLENNPTLERSIALFN
GISKWVQLMVLSKPTPQQRAEVITKFINVAKKLLQLKNFNTLMAVVGGLSHSSISRLKETHSHLSSEV
TKNWNEMTELVSSNGNYCNYRKAFADCDGFKIPILGVHLKDLIAVHVIFPDWTEENKVNIVKMHQLSV
TLSELVSLQNASHHLEPNMDLINLLTLSLDLYHTEDDIYKLSLVLEPRGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 237: PCC010_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGASALEIAEQLTLLDHLVFKKIPYEEFFGQGWMKLEKNERTPYIMKTTKH
FNDISNLIASEIIRNEDINARVSAIEKWVAVADICRCLHNYNAVLEITSSMNRSAIFRLKKTWLKVSK
QTKALIDKLQKLVSSEGRFKNLREALKNCDPPCVPYLGMYLTDLAFIEEGTPNYTEDGLVNFSKMRMI
SHIIREIRQFQQTAYKIEHQAKVTQYLLDQSFVMDEESLYESSLRIEPKGSGGSGGGGSGGGGSGGGG
S
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 238: PCC011_D
GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGAEPEELS EHLTYLEFKS FRRISFSDYQ NYLVNSCVKE
NPTMERSIALCNGISQWVQL MVLSRPTPQL RAEVFIKFIQ VAQKLHQLQN
FNTLMAVIGGLCHSSISRLK ETSSHVPHEI NKVLGEMTEL LSSSRNYDNY
RRAYGECTDFKIPILGVHLK DLISLYEAMP DYLEDGKVNV HKLLALYNHI
SELVQLQEVAPPLEANKDLV HLLTLSLDLY YTEDEIYELS YAREPRGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 239: PCC012_D

GPGTDIQMTQSPSSLSASVGDRVTITCRASQDIRNYLNWYQQKPGKAPKLLIYYTSRLESGVPSRFSG
SGSGTDYTLTISSLQPEDFATYYCQQGNTLPWTFGQGTKVEIK
GGGGSGGGGSGGGGSGGGGAEPMELAE HLTYLEYRSF CKILFQDYHS FVTHGCTVDN
PVLERFISLFNSVSQWVQLM ILSKPTAPQR ALVITHFVHV AEKLLQLQNF
NTLMAVVGGLSHSSISRLKE THSHVSPETI KLWEGLTELV TATGNYGNYR
RRLAACVGFRFPILGVHLKD LVALQLALPD WLDPARTRLN GAKMKQLFSI LEELAMVTSL
RPPVQANPDL LSLLTVSLDQ YQTEDELYQL SLQREPRGSGGSGGGGSGGGGSGGGGS
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSS GS HHHHHH

SEQ ID NO 240: Flexible GS linker (15 amino acids)
SGGGGSGGSGGSGGSLE

SEQ ID NO 241: Thioredox folding leader for bacteria Ras ABP production
M S D K I I H L T D D S F D T D V L K A D G A I L V D F W A E W C G P
C K M I A P I L D E I A D E Y Q G K L T V A K L N I D Q N P G T A P K
Y G I R G I P T L L L F K N G E V A A T K V G A L S K G Q L K E F L D
A N L A

SEQ ID NO 242: Trigger Factor folding leader from E. coli for bacteria
Ras ABP production (AA 112 - 432)
YPEVELQGLEAIEVEKPIVEVTDADVDGMLDTLRKQQATWKEKDGAVEAEDRVTIDFTGSVDGEEFEG
GKASDFVLAMGQGRMIPGFEDGIKGHKAGEEFTIDVTFPEEYHAENLKGKAAKFAINLKKVEERELPE
LTAEFIKRFGVEDGSVEGLRAEVRKNMERELKSAIRNRVKSQAIEGLVKANDIDVPAALIDSEIDVLR
RQAAQRFGGNEKQALELPRELFEEQAKRRVVVGLLLGEVIRTNELKADEERVKGLIEEMASAYEDPKE
VIEFYSKNKELMDNMRNVALEEQAVEAVLAKAKVTEKETTFNELMNQQA

SEQ ID NO 243: Trigger Factor folding leader from Pseudoalteromonas
distincta for Ras ABP production in bacteria (AA 112 - 434)
YPEVEVQGLDKITVEKPAVTVTDEDLANMLETLRKQHAAWADVDAAAGENDRVTVDFVGTIDGEVFEG
GKAEDFPLELGQGRMIPGFEDNIVGKKAGEEVVADVTFPDDYHAENLKGKAAQFTITVKKVEAQELPE
LSEEFATKFGVTEGGVDALKEEVKKNMTRELDQAVKASVKDQAIKGLLANNEIEVPKALVDQEVDALR
QQAAQRFGGDAQNMPELPAELFHEQAVTRVKTGLLLGEVIKANDIKVDDAKVEALIATVASAYEDPTE
VVEYYKGNDQLMQQMRNVAMEEQAVEAILAKAVVTDVEKAFDDIMNPQQGA

SEQ ID NO 244: Anti EpCAM BiTE antibody (antiCD3 scFv - antiEpCAM scFv
with a His-tag at the c-terminal end)
EVQLVESGGGLVQPGGSLRLSCAASGYSFTGYTMNWVRQAPGKGLEWVALINPYKGVSTYNQKFKDRF
TISVDKSKNTAYLQMNSLRAEDTAVYYCARSGYYGDSDWYFDVWGQGTLVTVSSGGGGSGGGGSGGGG
SDIQMTQSPSSLSASVGDRVTITCRASQDIRNYLNWYQQKPGKAPKLLIYYTSRLESGVPSRFSGSGS
GTDYTLTISSLQPEDFATYYCQQGNTLPWTFGQGTKVEIKSGGGGSGGGGSGGGGSGGGGEVQLLEQS
GAELVRPGTSVKISCKASGYAFTNYWLGWVKQRPGHGLEWIGDIFPGSGNIHYNEKFKGKATLTADKS
SSTAYMQLSSLTFEDSAVYFCARLRNWDEPMDYWGQGTTVTVSSGGGGSGGGGSGGGGSGGGGSELVM
TQSPSSLTVTAGEKVTMSCKSSQSLLNSGNQKNYLTWYQQKPGQPPKLLIYWASTRESGVPDRFTGSG
SGTDFTLTISSVQAEDLAVYYCQNDYSYPLTFGAGTKLEIKGSHHHHHHHH

SEQ ID NO 245: HiBiT
VSGWRLFKKIS

SEQ ID NO 246: SpyTag13
AHIVMVDAYKPTK

SEQ ID NO 247: SpyTag14
VPTIVMVDAYKRYK

SEQ ID NO 248: LgBiT
MVFTLEDFVGDWEQTAAYNLDQVLEQGGVSSLLQNLAVSVTPIQRIVRSGENALKIDIHVIIPY
EGLSADQMAQIEEVFKVVYPVDDHHFKVILPYGTLVIDGVTPNMLNYFGRPYEGIAVFDGKKI
TVTGTLWNGNKIIDERLITPDGSMLFRVTINS

SEQ ID NO 249: SpyCatcher = 4MLI_1
VDTLSGLSSEQGQSGDMTIEEDSATHIKFSKRDEDGKELAGATMELRDSSGKTISTWISDGQ
VKDFYLYPGKYTFVETAAPDGYEVATAITFTVNEQGQVTVNGKATKGDAHI

SEQ ID NO 250: KRAS crRNA#1
TATATTCAGTCATTTTCAGC

SEQ ID NO 251: KRAS crRNA#2
AATGACTGAATATAAACTTG

SEQ ID NO 252: KRAS HiBiT ssODN HiBiT sequence underlined
CTAATATAGTCACATTTTCATTATTTTTATTATAAGGCATGCTGAAAATGgtgagcggctggcggctgt
tcaagaagattagcACTGAATATAAACTTGTCGTAGTTGGAGCTGGTGGCGTAGGCAAGAGTG
C

SEQ ID NO 253: NRAS crRNA#1
GGTTTCCAACAGGTTCTTGC

SEQ ID NO 254: NRAS crRNA#2
TCACACCAGCAAGAACCTGT
SEQ ID NO 255: NRAS crRNA#3
CCTGATTACTGGTTTCCAAC

SEQ ID NO 256: NRAS HiBiT ssODN HiBiT sequence underlined
CAATTAACCCTGATTACTGGTTTCTAACACGTTCTTGCTGCTGTGAAATGgtgagcggctggcgg
ctgttcaagaagattagcACTGAGTACAAACTGGTGGTGGTTGGAGCAGGTGGTGTTGGGAAAA
GCGC

SEQ ID NO 257: HRAS crRNA#1
TATTCCGTCATCGCTCCTCA
SEQ ID NO 258: HRAS crRNA#2
ATATTCCGTCATCGCTCCTC
SEQ ID NO 259: HRAS crRNA#3
AGCGATGACGGAATATAAGC
SEQ ID NO 260: HRAS HiBiT ssODN HiBiT sequence underlined
CAGGAGACCCTGTAGGAGGACCCCGGGCCGCAGGCCACTGAGGAGCGATGgtgagcggct
ggcggctgttcaagaagattagcACGGAATATAAGCTTGTGGTGGTGGGCGCCGGCGGTGTGGGC
AAGAGTGC

SEQ ID NO 261: ALFA-atg
SRLEEELRRRLTE

SEQ ID NO 262: AviTag

GLNDIFEAQKIEWHE

SEQ ID NO 263: C-tag
EPEA

SEQ ID NO 264: Calmodulin-tag
KRRWKKNFIAVSAANRFKKISSSGAL

SEQ ID NO 265: iCapTag™
MIKIATRKYLGKQNVYGIGVERDHNFALKNGFIAHN

SEQ ID NO 266: Mono-Q
EEEEEE

SEQ ID NO 267: E-tag
GAPVPYPDPLEPR

SEQ ID NO 268: 6His-tag
HHHHHHAS

SEQ ID NO 269: 8His-tag
HHHHHHHHAS

SEQ ID NO 270: 10His-tag
HHHHHHHHHHAS

SEQ ID NO 271: AS5His-tag
HHHHHAS

SEQ ID NO 272: ASA5His-tag
AHHHHHAS

SEQ ID NO 273: Myc-tag
EQKLISEEDL

SEQ ID NO 274: NE-tag
TKENPRSNQEESYDDNES

SEQ ID NO 275: Rho1D4-tag
TETSQVAPA

SEQ ID NO 276: S-tag
KETAAAKFERQHMDS

SEQ ID NO 277: SBP-tag
MDEKTTGWRGGHVVEGLAGELEQLRARLEHHPQGQREP

SEQ ID NO 278: Softag 1
SLAELLNAGLGGS

SEQ ID NO 279: Softag 3
TQDPSRVG

SEQ ID NO 280: Spot-tag
PDRVRAVSHWSS

SEQ ID NO 281: Strep-tag II
WSHPQFEK

SEQ ID NO 282: T7-tag
MASMTGGQQMG

SEQ ID NO 283: TC tag
CCPGCC

SEQ ID NO 284: Ty tag
EVHTNQDPLD

SEQ ID NO 285: V5 tag
GKPIPNPLLGLDST

SEQ ID NO 286: VSV-tag
YTDIEMNRLGK

SEQ ID NO 287: Xpress tag
DLYDDDDK

SEQ ID NO 288: peptide-PAINT tag
RGSIDTWV

SEQ ID NO 289: Erbin PDZ domain tag
DTWV

SEQ ID NO 290: PCC_013VHVL = Y13-259_scFv_VHVL
QVQLQQSGGGLVQPGRSLKLSCVVSGFTFSNYGMNWIRQTPGKGLEWVAYISSGSSYLYY
AETVKGRFTISRDNAKNTLYLQMTSLRSEDTALYYCARHEGTGTDFFDYWGQGTTVTVSS
GGGGSGGGGSGGGGSGGGGSDIQLTQSPHSLSASLGETVSIECLASEGISNYLAWYQQKP
GKSPQLLIYYASSLQDGVPSRFSGSGSGTQFSLKISNMQPEDEGVYYCQQAYKYPSTFGAG
TKLEIK
SEQ ID NO 291: PCC_013VLVH = Y13-259_scFv_VLVH
DIQLTQSPHSLSASLGETVSIECLASEGISNYLAWYQQKPGKSPQLLIYYASSLQDGVPSRFS
GSGSGTQFSLKISNMQPEDEGVYYCQQAYKYPSTFGAGTKLEIKGGGGSGGGGSGGGGS
GGGGSQVQLQQSGGGLVQPGRSLKLSCVVSGFTFSNYGMNWIRQTPGKGLEWVAYISSG
SSYLYYAETVKGRFTISRDNAKNTLYLQMTSLRSEDTALYYCARHEGTGTDFFDYWGQGTT
VTVSS
SEQ ID NO 292: PCC_013 = Y13-259_CDRH1
NYGMN
SEQ ID NO 293: PCC_013 = Y13-259_ CDRH2
YISSGSSYLYYAETVKG
SEQ ID NO 294: PCC_013 = Y13-259_CDRH3
HEGTGTDFFDY
SEQ ID NO 295: PCC_013 = Y13-259_CDRL1

LASEGISNYLA
SEQ ID NO 296: PCC_013 = Y13-259_CDRL2
YASSLQD
SEQ ID NO 297: PCC_013 = Y13-259_CDRL3
QAYKYPST

SEQ ID NO 298: PCC_014VHVL = Y13-238_scFv_VHVL
QVQLQESGGGLVQPGRSLKLSCEASGFTVSNYGMAWIRQAPTKGLEWVASISYDGSSTYY
RDSVKGRFTISRDNGKSTLYLQMDSLRSEDTATYYCARPKVPGYNLNWFAYWGQGNTVTV
SSGGGGSGGGGSGGGGSGGGGSQFVLTQPTSVSLSLGSTVKLSCTLSSGNIENSYVHWY
QQYEGRSPTTMIYNDDKRPDGVPDRFSGSIDSSSNSAFLTINNVEIEDEAIYFCHSYVSSIHIF
GGGTKLTVL
SEQ ID NO 299: PCC_014VLVH = Y13-238_scFv_VLVH
QFVLTQPTSVSLSLGSTVKLSCTLSSGNIENSYVHWYQQYEGRSPTTMIYNDDKRPDGVPD
RFSGSIDSSSNSAFLTINNVEIEDEAIYFCHSYVSSIHIFGGGTKLTVLGGGGSGGGGSGGGG
SGGGGSQVQLQESGGGLVQPGRSLKLSCEASGFTVSNYGMAWIRQAPTKGLEWVASISYD
GSSTYYRDSVKGRFTISRDNGKSTLYLQMDSLRSEDTATYYCARPKVPGYNLNWFAYWGQ
GNTVTVSS
SEQ ID NO 300: PCC_014 = Y13-238_CDRH1
NYGMA
SEQ ID NO 301: PCC_014 = Y13-238_CDRH2
SISYDGSSTYYRDSVK
SEQ ID NO 302: PCC_014 = Y13-238_CDRH3
PKVPGYNLN
SEQ ID NO 303: PCC_014 = Y13-238_CDRL1
TLSSGNIENSYVH
SEQ ID NO 304: PCC_014 = Y13-238_CDRL2
NDDKRPD
SEQ ID NO 305: PCC_014 = Y13-238_CDRL3
HSYVSSIHI

SEQ ID NO 306: PCC_015 = 12VC1.1_Monobody / FN3 binding loops = BC, CD, DE, FG
VSSVPTELEVVAATPTSLLISWDAPAVTVFFYVITYGETGHGVGAFQAFKVPGSRSTATISGLE
PGVDYTITVYARGYSKQGPYKPSPISINYRT

SEQ ID NO 307: PCC_015 = 12VC1.1_BC
FFYV
SEQ ID NO 308: PCC_015 = 12VC1.1_CD
HGVGAF
SEQ ID NO 309: PCC_015 = 12VC1.1_DE
AFKVP
SEQ ID NO 310: PCC_015 = 12VC1.1_FG
RGYSKQGPYKP

SEQ ID NO 311: PCC_016 = 12VC1.2_Monobody / FN3 binding loops = BC, CD, DE, FG
VSSVPTELEVVAATPTSLLISWDAPAVTVFFYVITYGETGHGVGAFQAFAVPGSRSTATISGLE
PGVDYTITVYARGYSKQGPYKPSPISINYRT
SEQ ID NO 312: PCC_016 = 12VC1.2_BC
FFYV
SEQ ID NO 313: PCC_016 = 12VC1.2_CD
HGVGAF
SEQ ID NO 314: PCC_016 = 12VC1.2_DE
AFAVP
SEQ ID NO 315: PCC_016 = 12VC1.2_FG
RGYSKQGPYKP

SEQ ID NO 316: PCC_017 = RT4_VHH
EVQLVESGGGLVQPGGSLRLSCAASGFTFSSYAMSWVRQAPGKGLEWVSTISRSGHSTYY
ADSVKGRFTISRDNSKNTLYLQMNSLRAEDTAVYYCAKRFGSIVFDYWGQGTLVTVSS
SEQ ID NO 317: PCC_017 = RT4_CDR1
SYAMS
SEQ ID NO 318: PCC_017 = RT4_CDR2
TISRSGHSTYYADSVKG
SEQ ID NO 319: PCC_017 = RT4_CDR3
RFGSIVFDY

SEQ ID NO 320: PCC_018 = RT11_VHH
EVQLVESGGGLVQPGGSLRLSCAASGFTFSSYSMSWVRQAPGKGLEWVSYISRTSHTTYY
ADSVKGRFTISRDNSKNTLYLQMNSLRAEDTAVYYCARGFFSIVMDYWGQGTLVTVSS

SEQ ID NO 321: PCC_018 = RT11_CDR1
SYSMS
SEQ ID NO 322: PCC_018 = RT11_CDR2
YISRTSHTTYYADSVKG
SEQ ID NO 323: PCC_018 = RT11_CDR3
GFFSIVMDY

SEQ ID NO 324: PCC_019 = epRT22_VHH
EVQLVESGGGLVQPGGSLRLSCAASGFTFSDFSMSWVRQAPGKGLEWVSYISRTSHTTYY
ADSVKGRFTISRDNSKNTLYLQMNSLRAEDTAVYYCARGFKMDYWGQGTLVTVSS
SEQ ID NO 325: PCC_019 = epRT22_CDR1
DFSMS
SEQ ID NO 326: PCC_019 = epRT22_CDR2
YISRTSHTTYYADSVKG
SEQ ID NO 327: PCC_019 = epRT22_CDR3
GFKMDY

SEQ ID NO 328: PCC_020 = iDab6_VHH
EVQLLESGGGLVQPGGSLRLSCAASGFSFSTFSMNWVRQAPGKGLEWVSYISRTSKTIYYA
DSVKGRFTISRDNSKNTLYLQMNSLRAEDTAVYYCARGRFFDYWGQGTLVTVSS
SEQ ID NO 329: PCC_020 = iDab6_CDR1
SFSTFSMN
SEQ ID NO 330: PCC_020 = iDab6_CDR2
YISRTSKTIYYADSVKG
SEQ ID NO 331: PCC_020 = iDab6_CDR3
GRFFDY

SEQ ID NO 332: PCC_021 = iDab10_VHH
EVQLLESGGGLVQPGGSLRLSCAASGFSFSTFSMNWVRQAPGKGLEWVSYISTSGRTIYYA
DSVKGRFTISRDNSKNTLYLQMNSLRAEDTAVYYCARGQKFDYWGQGTLVTVSS
SEQ ID NO 333: PCC_021 = iDab10_CDR1
SFSTFSMN
SEQ ID NO 334: PCC_021 = iDab10_CDR2
YISTSGRTIYYADSVKG

SEQ ID NO 335: PCC_021 = iDab10_CDR3
GQKFDY

SEQ ID NO 336: PCC_022VHVL = I21R33_scFv_VHVL
EVQLLESGGGLVQPGGSLRLSSAASGFSFSTFSMNWVRQAPGKGLEWVSYISSSGRTIYYA
DSVKGRFTISRDNSKNTLYLQMNSLRAEDTAVYYSARGRFFDYWGQGTLVTVSS
GGGGSGGGGSGGGGSGGGGSTDIQMTQSPSSLSASVGDRVTITCRASQSISRYLNWYQQ
KPGKAPKLLIYAASSLQSGVPSRFSGSGSGTDFTLTISSLQPEDFATYYCQQSYSTLLTFGQG
TKVEIKR
SEQ ID NO 337: PCC_022VLVH = I21R33_scFv_VLVH
TDIQMTQSPSSLSASVGDRVTITCRASQSISRYLNWYQQKPGKAPKLLIYAASSLQSGVPSR
FSGSGSGTDFTLTISSLQPEDFATYYCQQSYSTLLTFGQGTKVEIKRGGGGSGGGGSGGGG
SGGGGSEVQLLESGGGLVQPGGSLRLSSAASGFSFSTFSMNWVRQAPGKGLEWVSYISSS
GRTIYYADSVKGRFTISRDNSKNTLYLQMNSLRAEDTAVYYSARGRFFDYWGQGTLVTVSS
SEQ ID NO 338: PCC_022 = I21R33_CDRH1
GFSFSTFS
SEQ ID NO 339: PCC_022 = I21R33_CDRH2
ISSSGRTI
SEQ ID NO 340: PCC_022 = I21R33_CDRH3
ARGRFFDY
SEQ ID NO 341: PCC_022 = I21R33_CDRL1
QSISRY
SEQ ID NO 342: PCC_022 = I21R33_CDRL2
AAS
SEQ ID NO 343: PCC_022 = I21R33_CDRL3
QQSYSTLLT

SEQ ID NO 344: PCC_041 = KRAS_VHH
DVQLQESGGGLVQAGGSLRLSCVASGRTFSTYPTGWFRQAPGKEREFVARINLSGGITNYA
DSVKGRFTISRDNAKNTVYLQMNSLKPEDTAVYYCGGGSTTWAGGIPTNFDYWGQGTQVT
VSS
FR and CDR regions based on the nanobody nb.F3 sequence = PDB: 8E0E_B
SEQ ID NO 345: PCC_041 = KRAS_nanobody_CDR1
SGRTFSTY

SEQ ID NO 346: PCC_041 = KRAS_nanobody_CDR2
REFVARINLSGGITNYA
SEQ ID NO 347: PCC_041 = KRAS_nanobody_CDR3
GSTTWAGGIPTNFD

SEQ ID NO 348: PCC_042 = Monobody_12D2 / FN3 binding loops = BC, CD, DE, FG
VSSVPTKLEVVAATPTSLLVSWDAPAVTVVFYDITYGETGGNSPVQEFTVSGSKSTATISGLK
PGVDYTITVYAKYLWYSGYSSPISINYRT
SEQ ID NO 349: PCC_042 = Monobody_12D2_BC
VFYD
SEQ ID NO 350: PCC_042 = Monobody_12D2_CD
GNSPV
SEQ ID NO 351: PCC_042 = Monobody_12D2_DE
EFTVS
SEQ ID NO 352: PCC_042 = Monobody_12D2_FG
KYLWYSGYS

SEQ ID NO 353: PCC_043 = Monobody_12D3 / FN3 binding loops = BC, CD, DE, FG
VSSVPTKLEVVAATPTSLLVSWDAPAVTVVFYDITYGETGGNSPVQEFTVSGSKSTATISGLK
PGVDYTITVYAKYLFWKGYSSPISINYRT
SEQ ID NO 354: PCC_043 = Monobody_12D3_BC
VFYD
SEQ ID NO 355: PCC_043 = Monobody_12D3_CD
GNSPV
SEQ ID NO 356: PCC_043 = Monobody_12D3_DE
EFTVS
SEQ ID NO 357: PCC_043 = Monobody_12D3_FG
KYLFWKGYS

SEQ ID NO 358: PCC_044 = Monobody_12D4 / FN3 binding loops = BC, CD, DE, FG
VSSVPTKLEVVAATPTSLLVSWDAPAVTVVFYDITYGETGGNSPVQEFTVSGSKSTATISGLK
PGVDYTITVYAKYLFWSGYSSPISINYRT
SEQ ID NO 359: PCC_044 = Monobody_12D4_BC
VFYD

SEQ ID NO 360: PCC_044 = Monobody_12D4_CD
GNSPV
SEQ ID NO 361: PCC_044 = Monobody_12D4_DE
EFTVS
SEQ ID NO 362: PCC_044 = Monobody_12D4_FG
KYLFWSGYS

SEQ ID NO 363: PCC_045 = Affimer K3 / Variable Region 1 and 2
MASNSLEIEELARFAVDEHNKKENALLEFVRVVKAKEQHSIDIWYDFTMYYLTLEAKDGGKK
KLYEAKVWVKKLNNSHTYKNFKELQEFKPVG

SEQ ID NO 364: PCC_045 = Affimer K3_VR1
HSIDIWYDF
SEQ ID NO 365: PCC_045 = Affimer K3_VR2
KLNNSHTYK

SEQ ID NO 366: PCC_046 = Affimer K6 / Variable Region 1 and 2
MASNSLEIEELARFAVDEHNKKENALLEFVRVVKAKEQHFTPWFQRNTMYYLTLEAKDGGK
KKLYEAKVWVKRIMVTDKMRNFKELQEFKPVG

SEQ ID NO 367: PCC_046 = Affimer K6_VR1
HFTPWFQRN
SEQ ID NO 368: PCC_046 = Affimer K6_VR2
RIMVTDKMR

SEQ ID NO 369: PCC_047= Affimer K69 / Variable Region 1 and 2
MASNSLEIEELARFAVDEHNKKENALLEFVRVVKAKEQWHFDYQQYNTMYYLTLEAKDGGK
KKLYEAKVWVKRQLRMGSMNNFKELQEFKPVG

SEQ ID NO 370: PCC_047 = Affimer K69_VR1
WHFDYQQYN
SEQ ID NO 371: PCC_047 = Affimer K69_VR2
RQLRMGSMN

SEQ ID NO 372: PCC_023 = Darpin 55 / Residues underlined form the antigen-binding
interface
DLGKKLLEAARAGQDDEVRILMANGADVNANDSAGHTPLHLAAKRGHLEIVEVLLKHGADV
NAMDNTGFTPLHLAALRGHLEIVEVLLKNGADVNAQDRTGRTPLHLAAKLGHLEIVEVLLKN
GADVNAQDKFGKTAFDISIDNGNEDLAEILQKL

SEQ ID NO 373: PCC_024 = Darpin 27 / Residues underlined form the antigen-binding
interface
DLGKKLLEAARAGQDDEVRILMANGADVNAHDTFGFTPLHLAALYGHLEIVEVLLKNGADVN
ADDSYGRTPLHLAAMRGHLEIVEVLLKYGADVNAADEEGRTPLHLAAKRGHLEIVEVLLKNG
ADVNAQDKFGKTAFDISIDNGNEDLAEILQKL

SEQ ID NO 374: PCC_025 = R11.1.6 / Residues of the peptide binding surface are
underlined
ATVKFTHQGEEKQVDISKIKWVIRWGQYIWFKYDEDGGAKGWGYVSEKDAPKELLQMLKK
R

SEQ ID NO 375: PCC_026 = R11.1.6_Mut2 / Residues of the peptide binding surface are
underlined
ATVKFTHQGEEKQVDISKIKWSRRWGQYIWFKYDEDGGAKGWGYVSEKDAPKELLQMLKK
R

SEQ ID NO 376: PCC_027 = R11.1.6_Mut6 / Residues of the peptide binding surface are
underlined
ATVKFTHQGEEKQVDISKIKWQRRWGQYRWFKYDEDGGAKGWGYVSEKDAPKELLQMLK
KR

SEQ ID NO 377: PCC_028 = RASSF5_01 (AA 274 – 364)
TDKRTSFYLPLDAIKQLHISSTTTVSEVIQGLLKKFMVVDNPQKFALFKRIHKDGQVLFQKLSI
ADRPLYLRLLAGPDTEVLSFVLKENET

SEQ ID NO 378: PCC_029 = RASSF5_02 (AA 201 – 363)

ETQRPPTLQEIKQKIDSYNTREKNCLGMKLSEDGTYTGFIKVHLKLRRPVTVPAGIRPQSIYD
AIKEVNLAATTDKRTSFYLPLDAIKQLHISSTTTVSEVIQGLLKKFMVVDNPQKFALFKRIHKDG
QVLFQKLSIADRPLYLRLLAGPDTEVLSFVLKENE

SEQ ID NO 379: PCC_030 = RASSF1 (AA 198 – 292)
RGTSVRRRTSFYLPKDAVKHLHVLSRTRAREVIEALLRKFLVVDDPRKFALFERAERHGQVY
LRKLLDDEQPLRLRLLAGPSDKALSFVLKENDS

SEQ ID NO 380: PCC_031 = RASSF2 (AA 176 – 264)
YNHKTSVFTPAYGSVTNVRINSTMTTPQVLKLLLNKFKIENSAEEFALYVVHTSGEKQKLKAT
DYPLIARILQGPCEQISKVFLMEKDQ

SEQ ID NO 381: PCC_032 = RASSF7 (AA 6 – 89)
AAMELKVWVDGIQRVVCGVSEQTTCQEVVIALAQAIGQTGRFVLVQRLREKERQLLPQECP
VGAQATCGQFASDVQFVLRRTGP

SEQ ID NO 382: PCC_033 = ARAF_RBD (AA 19 – 91)
GTVKVYLPNKQRTVVTVRDGMSVYDSLDKALKVRGLNQDCCVVYRLIKGRKTVTAWDTAIA
PLDGEELIVEVL

SEQ ID NO 383: PCC_034 = ARAF_RBD_CRD (AA 19 – 148)
GTVKVYLPNKQRTVVTVRDGMSVYDSLDKALKVRGLNQDCCVVYRLIKGRKTVTAWDTAIA
PLDGEELIVEVLEDVPLTMHNFVRKTFFSLAFCDFCLKFLFHGFRCQTCGYKFHQHCSSKVP
TVCVDMS

SEQ ID NO 384: PCC_035 = BRAF_RBD (P15056, RBD Domain, AA 151 – 232)
SPQKPIVRVFLPNKQRTVVPARCGVTVRDSLKKALMMRGLIPECCAVYRIQDGEKKPIGWDT
DISWLTGEELHVEVLENVPL

SEQ ID NO 385: PCC_036 = BRAF_RBD_CRD (AA 151 – 320)
SPQKPIVRVFLPNKQRTVVPARCGVTVRDSLKKALMMRGLIPECCAVYRIQDGEKKPIGWDT
DISWLTGEELHVEVLENVPLTTHNFVRKTFFTLAFCDFCRKLLFQGFRCQTCGYKFHQRCST
EVPLMCVNYDQLDLLFVSKFFEHHPIPQEEASLAETALTSGSSPSA

SEQ ID NO 386: PCC_037 = RAF1_RBD (AA 56 – 131)
NTIRVFLPNKQRTVVNVRNGMSLHDCLMKALKVRGLQPECCAVFRLLHEHKGKKARLDWNT
DAASLIGEELQVDFL

SEQ ID NO 387: PCC_038 = NF1_Ras_GAP (AA 1235 – 1451)
QWDELARVLVTLFDSRHLLYQLLWNMFSKEVELADSMQTLFRGNSLASKIMTFCFKVYGATY
LQKLLDPLLRIVITSSDWQHVSFEVDPTRLEPSESLEENQRNLLQMTEKFFHAIISSSSEFPP
QLRSVCHCLYQATCHSLLNKATVKEKKENKKSVVSQRFPQNSIGAVGSAMFLRFINPAIVSPY
EAGILDKKPPPRIERGLKLMSKILQSIAN

SEQ ID NO 388: PCC_039 = RASA1_Ras_GAP (AA 748 – 942)
DRTLLASILLRIFLHEKLESLLLCTLNDREISMEDEATTLFRATTLASTLMEQYMKATATQFVHH
ALKDSILKIMESKQSCELSPSKLEKNEDVNTNLTHLLNILSELVEKIFMASEILPPTLRYIYGCLQ
KSVQHKWPTNTTMRTRVVSGFVFLRLICPAILNPRMFNIISDSPSPIAARTLILVAKSVQNLAN

SEQ ID NO 389: PCC_040 = RASA4_Ras_GAP (AA 302 – 512)
CRQDVATNLLKLFLGQGLAKDFLDLLFQLELSRTSETNTLFRSNSLASKSMESFLKVAGMQY
LHGVLGPIINKVFEEKKYVELDPSKVEVKDVGCSGLHRPQTEAEVLEQSAQTLRAHLGALLS
ALSRSVRACPAVVRATFRQLFRRVRERFPGAQHENVPFIAVTSFLCLRFFSPAIMSPKLFHLR
ERHADARTSRTLLLLAKAVQNVGN

SEQ ID NO 390: PCC_048 = RGS12_RBD1_RBD2 (AA 938 – 1130)
VSSAGSLDLSEACRTLAPEKDKATKHCCIHLPDGTSCVVAVKAGFSIKDILSGLCERHGINGA
AADLFLVGGDKPLVLHQDSSILESRDLRLEKRTLFRLDLVPINRSVGLKAKPTKPVTEVLRPVV
ARYGLDLSGLLVRLSGEKEPLDLGAPISSLDGQRVVLEEKDPSRGKASADKQKGVP
VKQNTAVNSS

SEQ ID NO 391: PCC_013VH = Y13-259_VH
QVQLQQSGGGLVQPGRSLKLSCVVSGFTFSNYGMNWIRQTPGKGLEWVAYISSGSSYLYY
AETVKGRFTISRDNAKNTLYLQMTSLRSEDTALYYCARHEGTGTDFFDYWGQGTTVTVSS

SEQ ID NO 392: PCC_013VL = Y13-259_VL
DIQLTQSPHSLSASLGETVSIECLASEGISNYLAWYQQKPGKSPQLLIYYASSLQDGVPSRFS
GSGSGTQFSLKISNMQPEDEGVYYCQQAYKYPSTFGAGTKLEIK

SEQ ID NO 393: PCC_014VH = Y13-238_VH
QVQLQESGGGLVQPGRSLKLSCEASGFTVSNYGMAWIRQAPTKGLEWVASISYDGSSTYY
RDSVKGRFTISRDNGKSTLYLQMDSLRSEDTATYYCARPKVPGYNLNWFAYWGQGNTVTV
SS

SEQ ID NO 394: PCC_014VL = Y13-238_VL
QFVLTQPTSVSLSLGSTVKLSCTLSSGNIENSYVHWYQQYEGRSPTTMIYNDDKRPDGVPD
RFSGSIDSSSNSAFLTINNVEIEDEAIYFCHSYVSSIHIFGGGTKLTVL

SEQ ID NO 395: PCC_022VH = I21R33_VH
EVQLLESGGGLVQPGGSLRLSSAASGFSFSTFSMNWVRQAPGKGLEWVSYISSSGRTIYYA
DSVKGRFTISRDNSKNTLYLQMNSLRAEDTAVYYSARGRFFDYWGQGTLVTVSS

SEQ ID NO 396: PCC_022VL = I21R33_VL
TDIQMTQSPSSLSASVGDRVTITCRASQSISRYLNWYQQKPGKAPKLLIYAASSLQSGVPSR
FSGSGSGTDFTLTISSLQPEDFATYYCQQSYSTLLTFGQGTKVEIKR

SEQ ID NO 391: Primer amplification ABE 8.20m Forward primer
AAATAAGAGAGAAAAGAAGAGTAAGAAGAAATATAAGAGCCACCATGAAACGGACAGCC
GACGGAAGCG

SEQ ID NO 392: Primer amplification ABE 8.20m Reverse primer
AGCCCCGCAGAAGGCAGCTTAATTAATTAGACTTTCCTCTTCTTCTTGGGCTCGAATTCG
CT

SEQ ID NO 393: Bridge oligos Forward primer
ACCGTCAGATCTCGAGCTCGGTACCTAATACGACACACTATAAGGAAATAAGAGAGAAAA
GAAGAGTAAGAAGAAATATA

SEQ ID NO 394: Bridge oligos Reverse primer
TATATTTCTTCTTACTCTTCTTTTCTCTCTTATTTCCTTATAGTGTGTCGTATTAGGTACCGA
GCTCGAGATCTGACGGT

SEQ ID NO 395: HLA-I gRNA sequences
CTTACCCCACTTAACTATCT

SEQ ID NO 396: HLA-II gRNA sequences
CACTCACCTTAGCCTGAGCA

## Claims

1. Combination of
- an antigen binding protein (ABP) or an isolated nucleic acid comprising a sequence encoding the ABP and
- a modulator compound
for use as medicament, wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

2. The combination for use according to claim 1, wherein said extracellular Ras antigen is not part of, or is not restricted to, a human leukocyte antigen (HLA)-peptide complex, or specific HLA alleles, presented on the cell surface by the major histocompatibility complex (MHC).

3. The combination for use according to claim 1 or 2, wherein said ABP comprises one or more additional antigen binding domain(s) that is capable of binding to antigen(s) present on a mammalian T-cell, preferably a human cluster of differentiation 3 (CD3) antigen or a human T cell receptor (TCR).

4. The combination for use according to any one of claims 1 to 3, wherein the first antigen binding domain comprises an amino acid sequence having a sequence identity of at least 70%, more preferably at least 80%, even more preferably at least 90%, and most preferably at least 95% sequence identity, to an amino acid sequence selected from SEQ ID NO: 20 to 31, 66 to 71, 134 to 178 and 290 to 396, or, in each case independently, optionally with no more than ten, nine, eight, seven, six, five, four, preferably no more than three, two or one, amino acid substitution(s), insertion(s) or deletion(s) compared to these sequences.

5. The combination for use according to any one of claims 1 to 4, wherein the isolated nucleic acid is comprised in an expression construct, preferably further comprising promoter and/or terminator sequences.

6. The combination for use according to claim 5, wherein the isolated nucleic acid and/or the expression construct is comprised in a recombinant host cell.

7. The combination for use according to any one of claims 1 to 6, wherein the ABP is PCC04D diabody (SEQ ID NOs 23 and 231).

8. The combination for use according to any one of claims 1 to 7, wherein the ABP enhances a cell-mediated immune response, such as the immune response mediated by an activated cytotoxic T-cell (CTL) to a mammalian cell expressing said extracellular Ras antigen.

9. The combination for use according to any one of claims 1 to 8, wherein the ABP is selected from the group consisting of an immunoglobulin molecule, such as an IgG, IgE, IgD, IgA, or IgM immunoglobulin, preferably an IgG immunoglobulin, a monoclonal antibody, a chimeric antibody, a bispecifc ABP, such as a bispecific antibody, a CDR-grafted antibody, a humanized antibody, a single domain antibody, such as a VHH single domain antibody, a hemibody antibody, a single-chain KeyLock-antibody, a diabody, a single chain diabody, a variable domain of the antibody heavy chain or antibody light chain, a multispecific antibody, a Chimeric Antigen Receptor (CAR), alternative protein binders including monobodies (derived from fibronectin type III), anticalins (derived from lipocalins), affibodies (derived from immunoglobulin-binding protein A), DARPins (Designed Ankyrin Repeat Proteins), proteins with repeating motifs like leucine-rich repeats (LRRs), ankyrin repeats (ARs), Armadillo repeats (Arms), tetratricopeptide repeats (TPRs), and/or a fragment of an antibody, such as a fragment of a monoclonal antibody, for example a single chain Fv (scFv), (scFv)2, a Fv, a disulfide linked Fv, Fab, Fab', F(ab')2 or a scFv-Fc, preferably wherein said ABP is a bispecific antibody or a diabody.

10. The combination for use according to any one of claims 1 to 9, wherein the ABP comprises at least one antigen binding domain capable of binding with one, two, three, four, or preferable more than five amino acids to a Ras antigen, wherein the at least one antigen binding domain is alone or in a bipartite complex with another Ras binding protein, or in a tripartite complex with another Ras binding protein and the membrane, or in a multipartite complex with one or more Ras binding proteins and/or the membrane, and wherein said amino acids in the ABP optionally comprise:
(i) a domain comprising the RBD-CRD region (amino acids 52 to 188) of the human RAF1 protein as set forth in SEQ ID NO: 23,
(ii) optionally wherein one or multiple amino acids as depicted in Tables A and B are in contact with KRAS residues, or wherein one or multiple amino acids as depicted in Table C are in contact with the membrane, or wherein one or multiple amino acids as depicted in Table D are in contact with KRAS residues in a tripartite complex comprised of RBD-CRD, KRAS and the membrane, or
(iii) a domain comprising the CDC25H region (amino acids 780 to 1019) of the human SOS1 protein as set forth in SEQ ID NO: 24, optionally wherein one or multiple amino acids as depicted in Table E are in contact with KRAS residues, or
(iv) a domain comprising the CDC25 region (amino acids 1038 to 1270) of the human RASGRF1 protein as set forth in SEQ ID NO: 29, optionally wherein one or multiple amino acids as depicted in Table F are in contact with Ras residues in a tripartite complex with Ras, Sos1 and RasGRF1, or
(v) a domain comprising the RAS binding region (amino acids 274 to 364) of the human RASSF5 protein (UniProt Q8WWW0-1) as set forth in SEQ ID NO: 377, optionally wherein one or multiple amino acids are in contact with RAS residues, or
(vi) a domain comprising the RAS binding region (amino acids 201 to 363) of a splice variant of the human RASSF5 protein (UniProt Q8WWW0-2) as set forth in SEQ ID NO: 378, optionally wherein one or multiple amino acids are in contact with RAS residues, or
(vii)a domain comprising the RAS binding region (amino acids 201 to 363) of the human RASSF1 protein (UniProt Q9NS23-1) as set forth in SEQ ID NO: 379, optionally wherein one or multiple amino acids are in contact with RAS residues, or
(viii) a domain comprising the RAS binding region (amino acids 176 to 264) of the human RASSF2 protein (UniProt P50749-1) as set forth in SEQ ID NO: 380, optionally wherein one or multiple amino acids are in contact with RAS residues, or
(ix) a domain comprising the RAS binding region (amino acids 6 to 89) of the human RASSF7 protein (UniProt Q02833-1) as set forth in SEQ ID NO: 381, optionally wherein one or multiple amino acids are in contact with RAS residues, or
(x) a domain comprising the RAS binding region (amino acids 19 to 91) of the human ARAF protein (UniProt P10398-1) as set forth in SEQ ID NO: 382, optionally wherein one or multiple amino acids are in contact with RAS residues, or
(xi) a domain comprising the RAS binding region (amino acids 19 to 148) of the human ARAF protein (UniProt P10398-1) as set forth in SEQ ID NO: 383, optionally wherein one or multiple amino acids are in contact with RAS residues, or
(xii)a domain comprising the RAS binding region (amino acids 151 to 232) of the human BRAF protein (UniProt P15056) as set forth in SEQ ID NO: 384, optionally wherein one or multiple amino acids are in contact with RAS residues, or
(xiii) a domain comprising the RAS binding region (amino acids 151 to 320) of the human BRAF protein (UniProt P15056) as set forth in SEQ ID NO: 385, optionally wherein one or multiple amino acids are in contact with RAS residues, or
(xiv) a domain comprising the RAS binding region (amino acids 56 to 131) of the human RAF1 protein (UniProt P04049-1) as set forth in SEQ ID NO: 386, optionally wherein one or multiple amino acids are in contact with RAS residues, or
(xv) a domain comprising the RAS binding region (amino acids 1235 to 1451) of the human NF1 protein (UniProt P21359-1) as set forth in SEQ ID NO: 387, optionally wherein one or multiple amino acids are in contact with RAS residues, or
(xvi) a domain comprising the RAS binding region (amino acids 748 to 942) of the human RASA1 protein (UniProt P20936-1) as set forth in SEQ ID NO: 388, optionally wherein one or multiple amino acids are in contact with RAS residues, or
(xvii) a domain comprising the RAS binding region (amino acids 302 to 512) of the human RASA4 protein (UniProt O43374-1) as set forth in SEQ ID NO: 389, optionally wherein one or multiple amino acids are in contact with RAS residues, or
(xviii) a domain comprising the RAS binding region (amino acids 938 to 1130) of the human RGS12 protein (UniProt O14924-1) as set forth in SEQ ID NO: 390, optionally wherein one or multiple amino acids are in contact with RAS residues.

11. The combination for use according to any one of claims 1 to 10, wherein the ABP comprises at least one antigen binding domain capable of binding to an extracellular Ras antigen, preferably provided that said extracellular Ras antigen is not part of, or is not restricted to, a HLA-peptide complex, or specific HLA alleles presented on the cell surface, and wherein said Ras binding ABP comprises a diabody format as set forth in SEQ ID NO: 228 to 239, wherein in each case independently comprise a sequence having no more than three or two, preferably no more than one amino acid substitution(s), deletion(s) or insertion(s) compared to SEQ ID NO: 224, and 228 to 239, respectively; or comprising a sequence having at least 75% sequence identity, preferably at least 80% sequence identity, more preferably at least 90% sequence identity, and most preferably at last 95% sequence identity with SEQ ID NO: 224, and 228 to 239.

12. The combination for use according to any one of claims 1 to 11 for use in a method of diagnosis, prevention and/or treatment of a proliferative disorder, such as cancer, and/or for use in adoptive, target-cell specific immunotherapy and/or for use in drug development and/or for use in a method of diagnosis, prevention and/or treatment of a non-malignant disease.

13. Pharmaceutical composition for use as medicament comprising the combination according to any one of claims 1 to 12 and a pharmaceutically acceptable carrier, stabilizer and/or excipient.

14. Composition comprising
- an antigen binding protein (ABP) or an isolated nucleic acid comprising a sequence encoding the ABP and
- a modulator compound,
wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

15. A kit comprising
- an antigen binding protein (ABP) or an isolated nucleic acid comprising a sequence encoding the ABP and
- a modulator compound,
wherein the ABP comprises at least a first antigen binding domain capable of binding to an extracellular Ras antigen and wherein the modulator compound is selected from the group consisting of
a) an All-trans-retinoic acid (ATRA) based modulator,
b) a modulator of the cholesterol and triglyceride synthesis or metabolism, preferably Simvastatin, Fluvastatin, Lapaquistat, or Bemfivastatin
c) an immunomodulatory drug targeting cereblon, preferably Lenalidomide, Iberdomide, HOMO-Protac cereblon degrader 1, or Eragidomide,
d) a modulator of monomeric GTP-binding proteins, preferably the Ras family, preferably the Ras signaling complex, preferably NSC-70220, SAH-SOS1A TFA, Adagrasib, BI-2852, MRTX-1133, RMC-0331 or SOS1-activator 1,
e) a kinase modulator, preferably of the Rho kinase pathway, preferably Narciclasine,
f) a tyrosine kinase modulator, preferably of the JAK signaling pathway, preferably Ruxolitinib,
g) a modulator of esterases, preferably of the phosphodiesterase pathway, preferably Anagrelide,
h) a lipid, preferably a lipid part of a cell membrane
i) a fatty acid, preferably a saturated fatty acid or unsaturated fatty acid
j) a modulator of the gut microbiome, and
k) an inhibitor of the naphthylisoquinoline alkaloid class, preferably Dioncophylline A.
l) a combination of two or more of a) to k).

16. An *in vitro* 3D cell culture model comprising tumor cells and at least one non-malignant cell selected from the group comprising or consisting of a fibroblast, an endothelial cell of an arterial blood vessel, an endothelial cell of a venous blood vessel, an endothelial cell of a lymphatic vessel, a tissue macrophage, a fat cell, an osteoblast, a chondrocyte, a smooth muscle cell, a preadipocyte, a pericyte, a mesenchymal stem cell, a melanocyte, a keratinocyte, hematopoietic progenitors, a dendritic cell, a skeletal muscle cell, a T cell and a B cell, preferably wherein the at least non-malignant cell is a T cell, a fibroblast and an endothelial cell.

17. The *in vitro* 3D cell culture model according to claim 16, further comprising the combination according to any one of claims 1 to 12 or the ABP as defined in any one of claims 1 to 12 for testing the influence of the combination on the viability and extracellular Ras expression of the at least one tumor cell.

18. An *in vitro* method for detecting the expression of predominantly extracellular Ras proteins on the surface of predominantly living cells selected from the group consisting of
a) a method using a Ras protein labeling compound with substantially no penetration inside the cell and/or predominantly binding to the extracellular Ras protein
b) a method using an agent blocking binding to intracellular Ras proteins and/or using a washing step to substantially remove binding to intracellular Ras proteins
c) a method using a Ras protein labeling compound as of a) and one or more labeling compounds binding to intracellular proteins not belonging to the Ras protein family
d) a method using a dual-antigen protein-protein interaction (PPI) reporter
e) a method using cells expressing an altered Ras protein, preferably a Ras-tag fusion protein, wherein the tag can be detected on the extracellular side of the cell, optionally wherein the tag requires a second tag to be detectable (split-tag PPI)
f) a method using an ABP as defined in any one of claims 1 to 12
g) a method using 2D, 3D cell culture and/or spheroid or organoid cultures and/or stem cell-based embryo-like structures
h) a method combining two or more of a) to g).

19. An *in vitro* method for detecting modulation of cell activity induced by binding of ABPs or compounds to extracellular Ras on the surface of predominantly living cells selected from the group consisting of
a) a method using an agent to detect a cytokine level
b) a method using at least one electrode to detect a change in cells' electrical potential
c) a method using genetically modified cells with a reporter plasmid to detect and/or measure activation of a signaling cascade
d) a method combining two or more of a) to c).

20. An *in silico* method for detecting modulation of extracellular Ras on the surface of artificial cells selected from the group consisting of
a) a method using artificial cell membranes
b) a method using artificial intelligence for calculating the extracellular Ras interface with small molecule compounds, with other proteins, with the lipid cell membrane and/or with the glycocalyx.
